# EUROPEAN PATENT APPLICATION

(11) **EP 1 184 382 A1**
(43) Date of publication of application: **06.03.2002**
(21) Application number: 00935605.6
(22) Date of filing: 09.06.2000
(51) Int. Cl.: C07D 413/12

(54) **OXIME DERIVATIVES, PROCESS FOR THE PREPARATION THEREOF AND PESTICIDES**

(30) Priority: 09.06.1999 JP 16247299; 09.06.1999 JP 16247399
(71) Applicant: DAINIPPON INK AND CHEMICALS, INC., Itabashi-ku, Tokyo 174-8520 (JP)
(72) Inventor: KOBORI, Takeo, Inba-gun Chiba 270-1600 (JP); KONDO, Hitoshi, Chiba-shi Chiba-ken (JP); TSUBOI, Hiroyuki, Yachiyo-shi Chiba 276-0034 (JP); IIYAMA, Mika, Koutou-ki Tokyo, (JP); ASADA, Toru, Inba-gun Chiba 285-0922 (JP); GOTO, Takashi, Sakura-shi Chiba-ken, (JP); SATO, Kenichi, Sagamihara-shi Kanagawa 229-1123 (JP); HAGIWARA, Emiko, Sagamihara-shi Kanagawa 229-0034 (JP); GOTO, Tomoko, Sakura-shi Chiba-ken, (JP); MATSUTANI, Sanae Hitachikasei Matsushiro-hausu, Tsukuba-shi Ibaraki 305-0035 (JP)
(74) Representative: Albrecht, Thomas, Dr.
(86) International application number: JP0003753
(87) International publication number: WO0075138

(57) **Abstract**

An object of the present invention is to provide a novel oxime derivative which is less likely to cause chemical injury to useful plants and also has sufficient plant disease controlling activity, an agricultural chemical containing the same as an active ingredient, especially a plant disease controlling agent, a method of preparing the same, and a novel hydroxime derivative suited for use as an intermediate in the preparation method. The object is achieved by an oxime derivative represented by the following general formula (1): 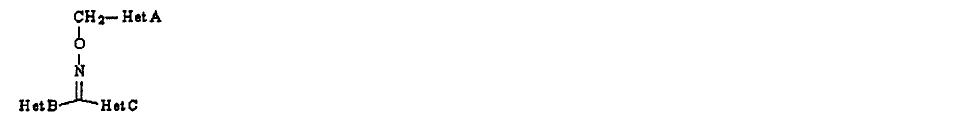 wherein Het A is a group represented by any one of: 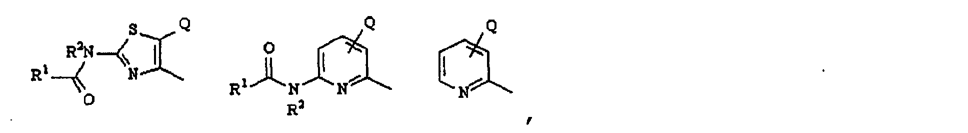 Het B is a group represented by any one of: 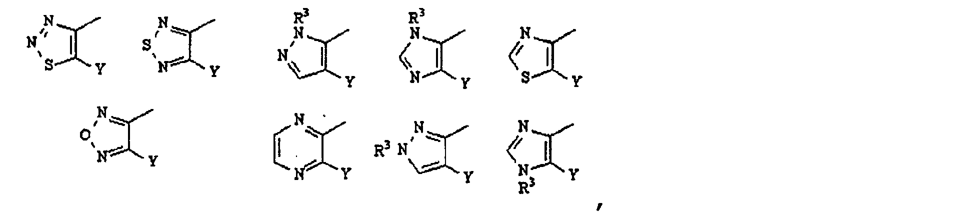 and Het C is a group represented by any one of:

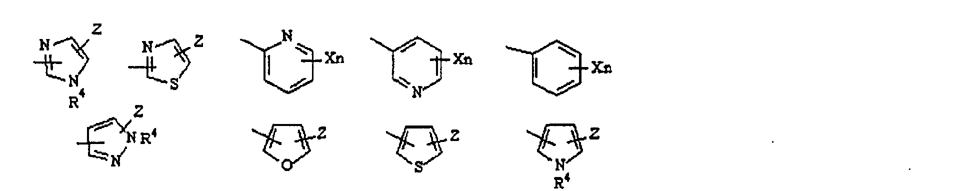

## Description

### TECHNICAL FIELD

The present invention relates to: a novel oxime derivative; an agricultural chemical containing the same as an active ingredient, particularly a plant disease controlling agent; a method of preparing the same, and a novel hydroxime derivative suitable for use as an intermediate in the preparation method.

### BACKGROUND ART

With respect to an oxime derivative having functions and effects as agricultural chemicals, for example, Japanese Unexamined Patent Application, First Publication No. Hei 7-252242, according to an invention of the present inventors, discloses that 4,5-substituted 1,2,3-thiadiazole derivatives act as plant disease controlling agents. Although these compounds exhibit considerable chemical efficacy, it is particularly necessary to develop chemicals which exert superior effects using small amounts thereof.

### DISCLOSURE OF INVENTION

An object of the present invention is to provide novel oxime derivatives which are less likely to chemically injure useful plants and also has sufficient plant disease controlling activity, an agricultural chemical containing the same as an active ingredient, especially a plant disease controlling agent, a method of preparing the same, and a novel hydroxime derivative suited for use as an intermediate of the preparation method.

To achieve such an object, the present inventors synthesized various oxime derivatives, tested the bioactivity thereof, and performed intensive research. As a result, they have found that an oxime derivative represented by the general formula (1) is less likely to cause chemical injury to useful plants and also exhibits particularly superior plant disease controlling activity as an agricultural chemical, and they have also discovered a method of preparing the same and a hydroxime derivative represented by the general formula (2) as an intermediate in the preparation method, and thus the present invention has been completed. The present invention provides the following aspects.

### Aspect 1

An oxime derivative represented by the following general formula (1): 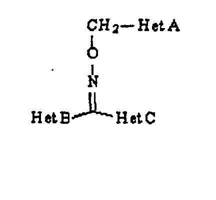 wherein Het A represents a group represented by any one of the following three formulas: 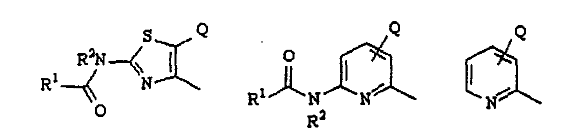 (wherein Q represents a hydrogen atom, a halogen atom, or a lower alkyl group; R¹ represents a hydrogen atom, a lower alkyl group, a lower alkyl group substituted with a halogen atom, a cycloalkyl group, a lower alkenyl group, a lower alkynyl group, an aralkyl group, an aryl group, or an aryl group substituted with one or more substituents selected from the group consisting of halogen atom, lower alkyl group, and lower alkoxy group; and R² represents a hydrogen atom or a lower alkyl group); Het B represents a group represented by any one of the following nine formulas: 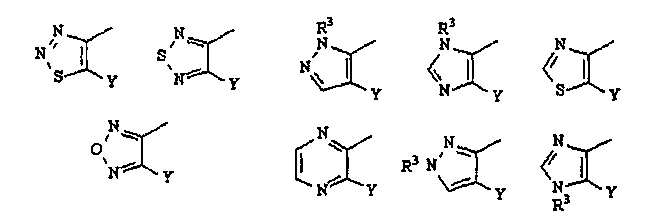 (wherein Y represents a hydrogen atom or a lower alkyl group; and R³ represents a hydrogen atom, a lower alkyl group, or a lower alkyl group substituted with a lower alkoxy group which may have a silyl group); and Het C represents a group represented by any one of the following nine formulas: 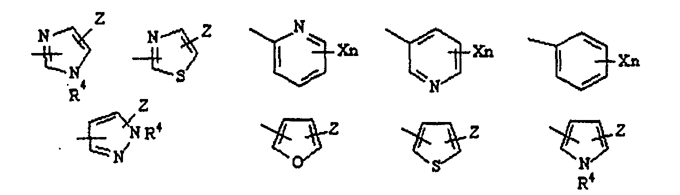 (wherein R⁴ represents a hydrogen atom, a lower alkyl group, or a lower alkyl group substituted with a lower alkoxy group which may have a silyl group; X represents a hydrogen atom, a halogen atom, a lower alkyl group, a lower alkyl group substituted with a halogen atom, a lower alkoxy group, a lower alkoxy group substituted with a halogen atom, or a cyano group; Z represents a hydrogen atom, a halogen atom, or a lower alkyl group; and n is from 0 to 3); with the exception of the case where Het A is a group represented by any one of the following two formulas: 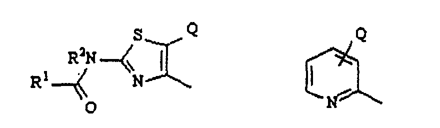 (wherein Q represents a hydrogen atom, a halogen atom, or a lower alkyl group; R¹ represents a hydrogen atom, a lower alkyl group, a lower alkyl group substituted with a halogen atom, a cycloalkyl group, a lower alkenyl group, a lower alkynyl group, an aralkyl group, an aryl group, or an aryl group substituted with one or more substituents selected from the group consisting of halogen atom, lower alkyl group, and lower alkoxy group; and R² represents a hydrogen atom or a lower alkyl group); Het B is a group represented by any one of the ring structures of the following three formulas: 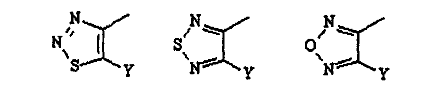 (wherein Y represents a hydrogen atom or a lower alkyl group); and Het C is a group represented by the following formula: 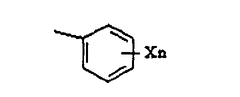 (wherein X represents a hydrogen atom, a halogen atom, a lower alkyl group, a lower alkyl group substituted with a halogen atom, a lower alkoxy group, a lower alkoxy group substituted with a halogen atom, or a cyano group; and n is from 0 to 3).

### Aspect 2

An oxime derivative represented by the following general formula (1): 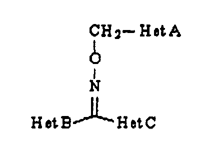 wherein Het A represents a group represented by any one of the following three formulas: 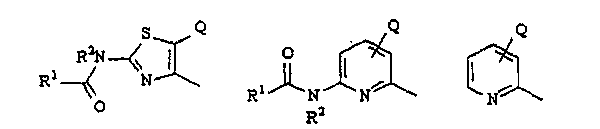 (wherein Q represents a hydrogen atom, a halogen atom, or a lower alkyl group; R¹ represents a hydrogen atom, a lower alkyl group, a lower alkyl group substituted with a halogen atom, a cycloalkyl group, a lower alkenyl group, a lower alkynyl group, an aralkyl group, an aryl group, or an aryl group substituted with one or more substituents selected from the group consisting of halogen atom, lower alkyl group, and lower alkoxy group; and R² represents a hydrogen atom or a lower alkyl group); Het B represents a group represented by any one of the ring structures of the following three formulas: 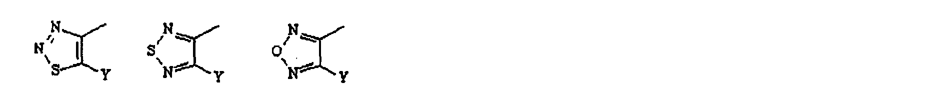 (wherein Y represents a hydrogen atom or a lower alkyl group); and Het C represents a group represented by any one of the ring structures of the following eight formulas: 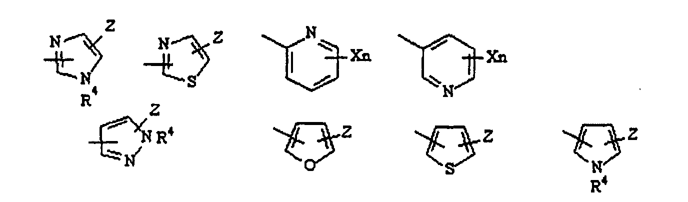 (wherein Z represents a hydrogen atom, a halogen atom, or a lower alkyl group; R⁴ represents a hydrogen atom, a lower alkyl group, or a lower alkyl group substituted with a lower alkoxy group which may have a silyl group; X represents a hydrogen atom, a halogen atom, a lower alkyl group, or a lower alkoxy group; and n is from 0 to 3).

### Aspect 3

An oxime derivative represented by the following general formula (1): 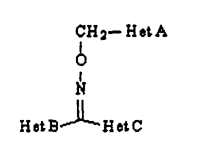 wherein Het A represents a group represented by the following formula: 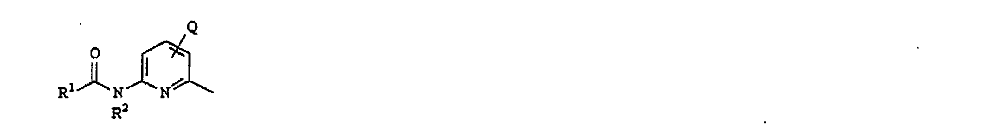 (wherein Q represents a hydrogen atom, a halogen atom, or a lower alkyl group; R¹ represents a hydrogen atom, a lower alkyl group, a lower alkyl group substituted with a halogen atom, a cycloalkyl group, a lower alkenyl group, a lower alkynyl group, an aralkyl group, an aryl group, or an aryl group substituted with one or more substituents selected from the group consisting of halogen atom, lower alkyl group, and lower alkoxy group; and R² represents a hydrogen atom or a lower alkyl group); Het B represents a group represented by any one of the ring structures of the following three formulas: 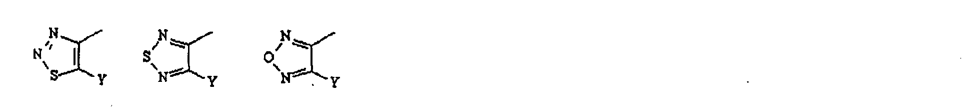 (wherein Y represents a hydrogen atom or a lower alkyl group); and Het C represents a group represented by the following formula: 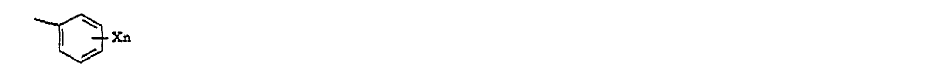 (wherein X represents a hydrogen atom, a halogen atom, a lower alkyl group, a lower alkyl group substituted with a halogen atom, a lower alkoxy group, a lower alkoxy group substituted with a halogen atom, or a cyano group; and n is from 0 to 3).

### Aspect 4

An oxime derivative represented by the following general formula (1): 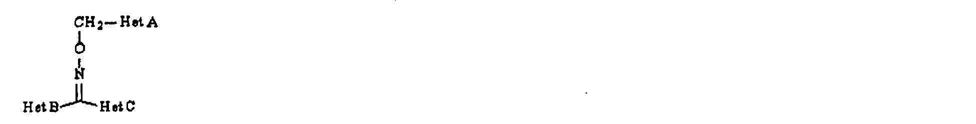 wherein Het A represents a group represented by any one of the following three formulas: 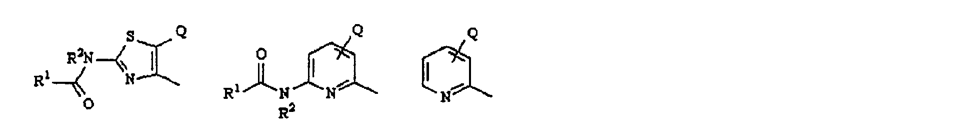 (wherein Q represents a hydrogen atom, a halogen atom, or a lower alkyl group; R¹ represents a hydrogen atom, a lower alkyl group, a lower alkyl group substituted with a halogen atom, a cycloalkyl group, a lower alkenyl group, a lower alkynyl group, an aralkyl group, an aryl group, or an aryl group substituted with one or more substituents selected from the group consisting of halogen atom, lower alkyl group, and lower alkoxy group; and R² represents a hydrogen atom or a lower alkyl group); Het B represents a group represented by any one of the following six formulas: 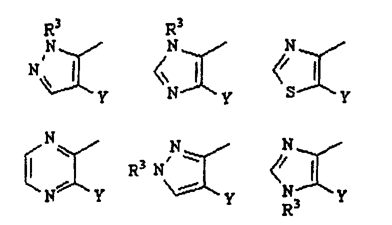 (wherein Y represents a hydrogen atom or a lower alkyl group; and R³ represents a hydrogen atom, a lower alkyl group, or a lower alkyl group substituted with a lower alkoxy group which may have a silyl group); and Het C represents a group represented by any one of the following six formulas: 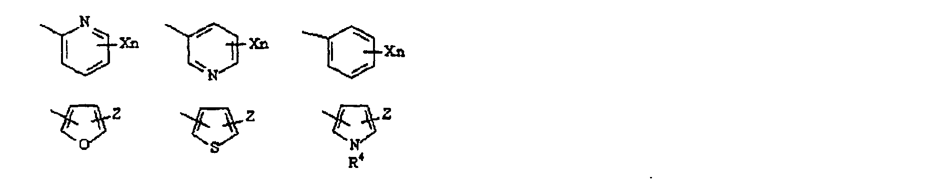 (wherein R⁴ represents a hydrogen atom or a lower alkyl group; X represents a hydrogen atom, a halogen atom, a lower alkyl group, a lower alkyl group substituted with a halogen atom, a lower alkoxy group, or a lower alkoxy group substituted with a halogen atom; Z represents a hydrogen atom, a halogen atom, or a lower alkyl group; and n is from 0 to 3).

### Aspect 5

An oxime derivative described in aspect 2, wherein Het C is a group represented by any one of the ring structures of the following three formulas: 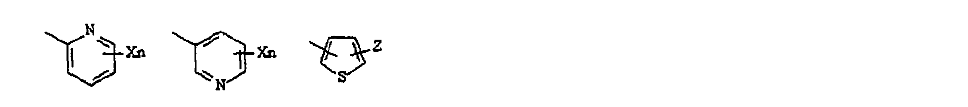 (wherein Z represents a hydrogen atom, a halogen atom, or a lower alkyl group; X represents a hydrogen atom, a halogen atom, a lower alkyl group, or a lower alkoxy group; and n is 0 or 1) in the general formula (1).

### Aspect 6

An oxime derivative described in aspect 2, wherein Het A is a group represented by the following formula: 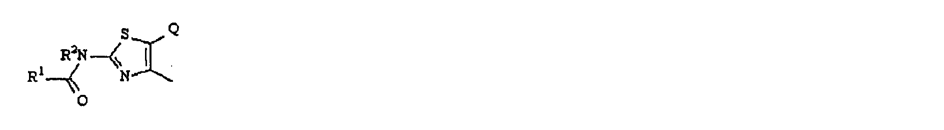 (wherein Q represents a hydrogen atom, a halogen atom, or a lower alkyl group; R¹ represents a lower alkyl group or a cycloalkyl group; and R² represents a hydrogen atom); and Het C is a group represented by the following formula: 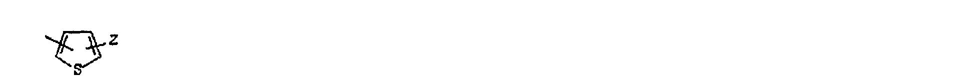 (wherein Z represents a hydrogen atom) in the general formula (1).

### Aspect 7

An oxime derivative described in aspect 2, wherein Het A is a group represented by the following formula: 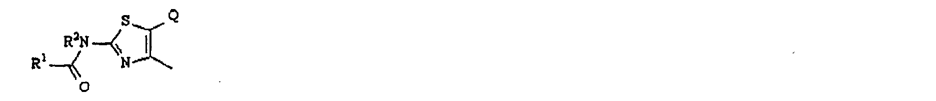 (wherein Q represents a hydrogen atom, a halogen atom, or a lower alkyl group; R¹ represents a lower alkyl group or a cycloalkyl group; and R² represents a hydrogen atom); and Het C is a group represented by the following formula: 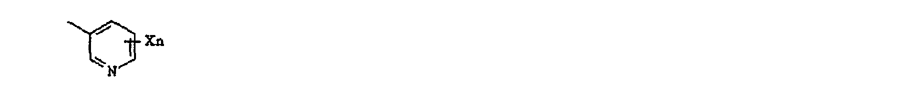 (wherein X represents a hydrogen atom or a halogen atom; and n is 0 or 1) in the general formula (1).

### Aspect 8

An oxime derivative described in aspect 2, wherein Het A is a group represented by the following formula: 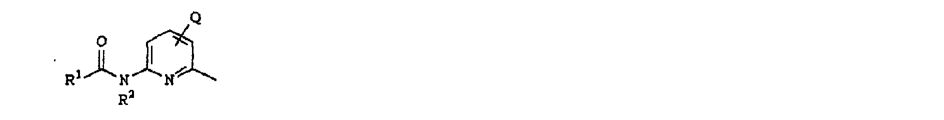 (wherein Q represents a hydrogen atom or a lower alkyl group; R¹ represents a lower alkyl group or a cycloalkyl group; and R² represents a hydrogen atom); and Het C is a group represented by any one of the following three formulas: 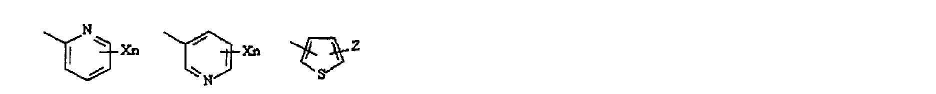 (wherein X represents a hydrogen atom or a halogen atom; z represents a hydrogen atom; and n is 0 or 1) in the general formula (1).

### Aspect 9

An oxime derivative described in aspect 3, wherein Het A is a group represented by the following formula: 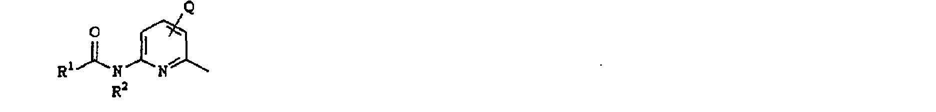 (wherein Q represents a hydrogen atom or a lower alkyl group; R¹ represents a lower alkyl group or a cycloalkyl group; and R² represents a hydrogen atom); and Het C is a group represented by the following formula: 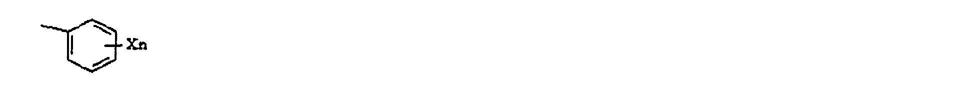 (wherein X represents a hydrogen atom, a halogen atom, a lower alkyl group, a lower alkyl group substituted with a halogen atom, or a cyano group; and n is from 0 to 3) in the general formula (1).

### Aspect 10

An oxime derivative described in aspect 4, wherein Het C is a group represented by the following formula: 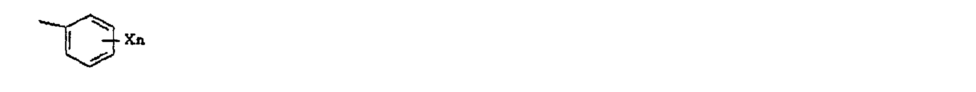 (wherein X represents a hydrogen atom, a halogen atom, a lower alkyl group, a lower alkyl group substituted with a halogen atom, a lower alkoxy group, or a lower alkoxy group substituted with a halogen atom; and n is from 0 to 3) in the general formula (1).

### Aspect 11

An oxime derivative described in aspect 4, wherein Het A is a group represented by the following formula: 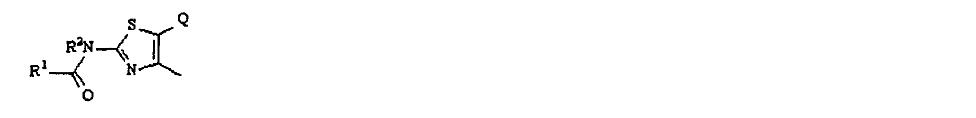 (wherein Q represents a hydrogen atom, a halogen atom, or a lower alkyl group; R¹ represents a lower alkyl group or a cycloalkyl group; and R² represents a hydrogen atom); Het B is a group represented by any one of the following three formulas: 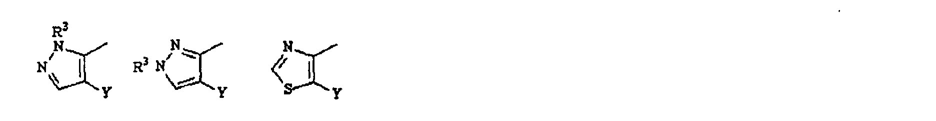 (wherein Y represents a hydrogen atom or a lower alkyl group; and R³ represents a hydrogen atom, a lower alkyl group, or a lower alkyl group substituted with a lower alkoxy group which may have a silyl group); and Het C is a group represented by the following formula: 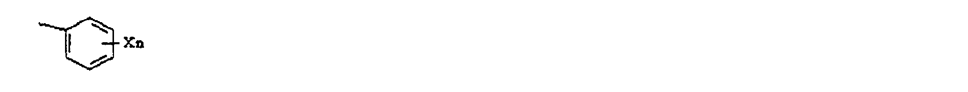 (wherein X represents a hydrogen atom, a halogen atom, a lower alkyl group, or a lower alkyl group substituted with a halogen atom; and n is 0 or 1) in the general formula (1).

### Aspect 12

A hydroxime derivative represented by the following general formula (2): 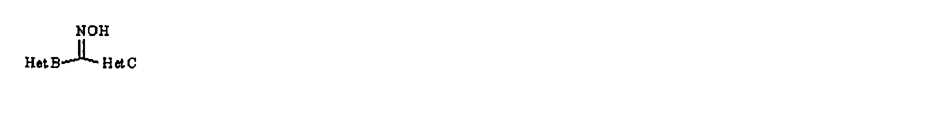 wherein Het B represents a group represented by any one of the following nine formulas: 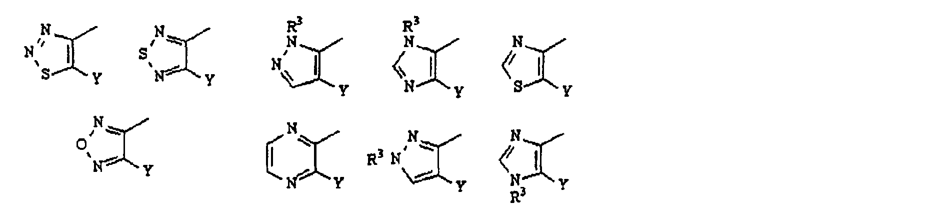 (wherein Y represents a hydrogen atom or a lower alkyl group; and R³ represents a hydrogen atom, a lower alkyl group, or a lower alkyl group substituted with a lower alkoxy group which may have a silyl group); and Het C represents a group represented by any one of the following nine formulas: 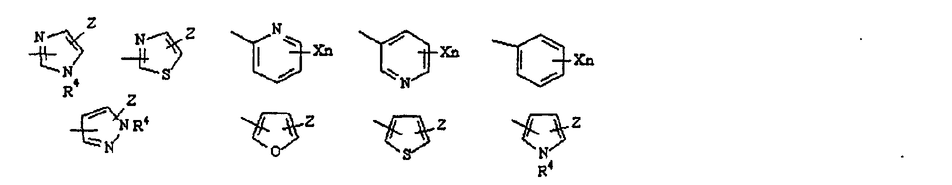 (wherein R⁴ represents a hydrogen atom, a lower alkyl group, or a lower alkyl group substituted with a lower alkoxy group which may have a silyl group; X represents a hydrogen atom, a halogen atom, a lower alkyl group, a lower alkyl group substituted with a halogen atom, a lower alkoxy group, or a lower alkoxy group substituted with a halogen atom; Z represents a hydrogen atom, a halogen atom, or a lower alkyl group; and n is from 0 to 3); with the exception of the case of the following compounds: 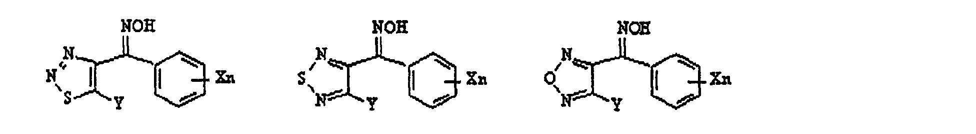 (wherein X represents a hydrogen atom, a halogen atom, a lower alkyl group, a lower alkyl group substituted with a halogen atom, a lower alkoxy group, a lower alkoxy group substituted with a halogen atom, or a cyano group; and n is 0 or 1), the following compound: 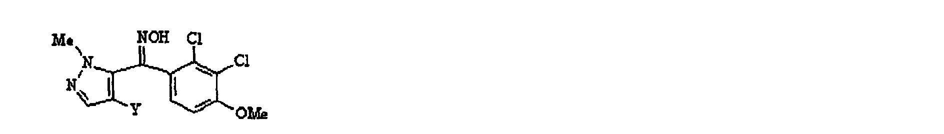 and the following compounds:

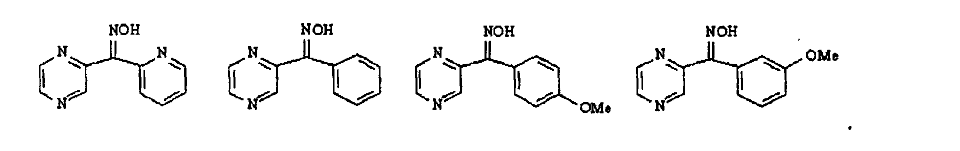

### Aspect 13

A hydroxime derivative described in aspect 12, wherein Het B is a group represented by any one of the ring structures of the following three formulas: 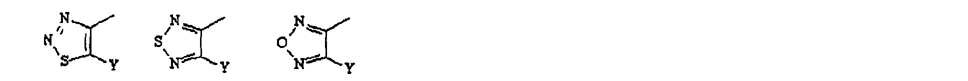 (wherein Y represents a hydrogen atom or a lower alkyl group); and Het C is a group represented by any one of the ring structures of the following eight formulas: 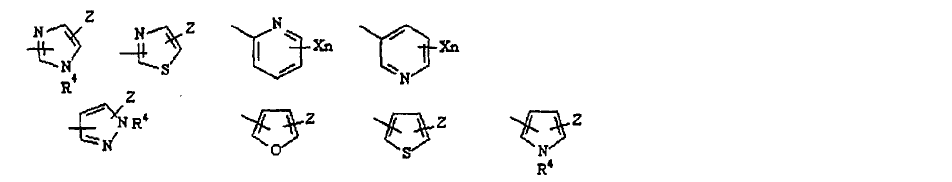 (wherein Z represents a hydrogen atom, a halogen atom, or a lower alkyl group; R⁴ represents a hydrogen atom, a lower alkyl group, or a lower alkyl group substituted with a lower alkoxy group which may have a silyl group; X represents a hydrogen atom, a halogen atom, a lower alkyl group, or a lower alkoxy group; and n is from 0 to 3) in the general formula (2).

### Aspect 14

A hydroxime derivative described in aspect 12, wherein Het B is a group represented by any one of the following six formulas: 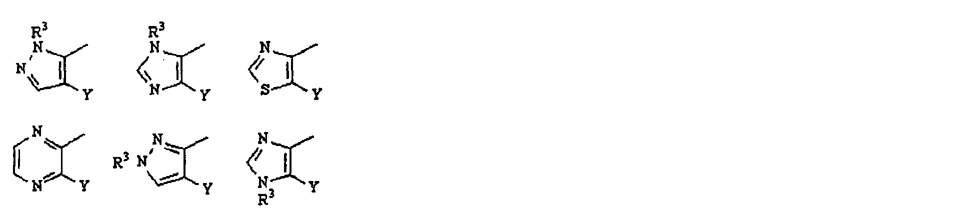 (wherein Y represents a hydrogen atom or a lower alkyl group; and R³ represents a hydrogen atom, a lower alkyl group, or a lower alkyl group substituted with a lower alkoxy group which may have a silyl group); and Het C is a group represented by any one of the following six formulas: 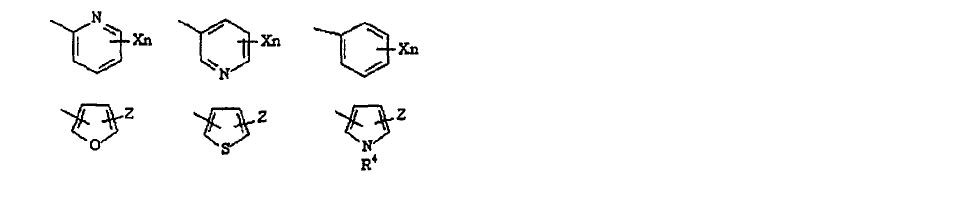 (wherein R⁴ represents a hydrogen atom or a lower alkyl group; X represents a hydrogen atom, a halogen atom, a lower alkyl group, a lower alkyl group substituted with a halogen atom, a lower alkoxy group, or a lower alkoxy group substituted with a halogen atom; Z represents a hydrogen atom, a halogen atom, or a lower alkyl group; and n is from 0 to 3) in the general formula (2).

### Aspect 15

A hydroxime derivative described in aspect 13, wherein Het C is a group represented by any one of the ring structures of the following three formulas: 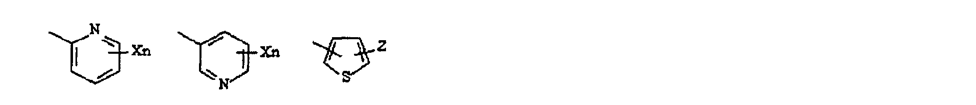 (wherein Z represents a hydrogen atom, a halogen atom, or a lower alkyl group; X represents a hydrogen atom, a halogen atom, a lower alkyl group, or a lower alkoxy group; and n is 0 or 1) in the general formula (2).

### Aspect 16

A hydroxime derivative described in aspect 13, wherein Het C is a group represented by the following formula: 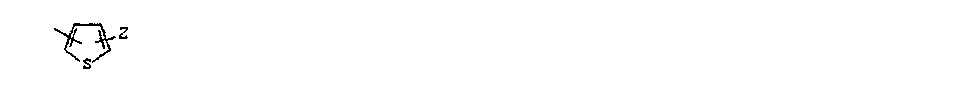 (wherein Z represents a hydrogen atom) in the general formula (2).

### Aspect 17

A hydroxime derivative described in aspect 13, wherein Het C is a group represented by the following formula: 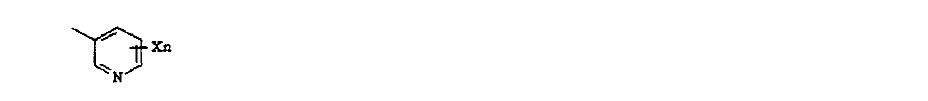 (wherein X represents a hydrogen atom or a halogen atom; and n is 0 or 1) in the general formula (2).

### Aspect 18

A hydroxime derivative described in aspect 14, wherein Het C is a group represented by the following formula: 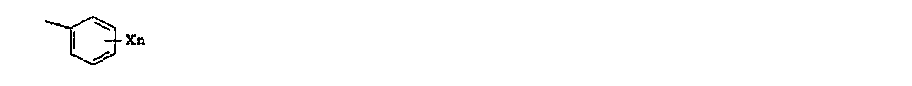 (wherein X represents a hydrogen atom, a halogen atom, a lower alkyl group, a lower alkyl group substituted with a halogen atom, a lower alkoxy group, or a lower alkoxy group substituted with a halogen atom; and n is from 0 to 3) in the general formula (2).

### Aspect 19

A hydroxime derivative described in aspect 14, wherein Het B is a group represented by any one of the following three formulas: 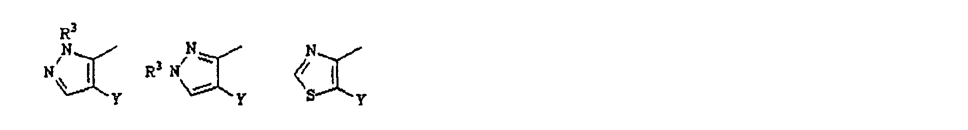 (wherein Y represents a hydrogen atom or a lower alkyl group; and R³ represents a hydrogen atom, a lower alkyl group, or a lower alkyl group substituted with a lower alkoxy group which may have a silyl group); and Het C is a group represented by the following formula: 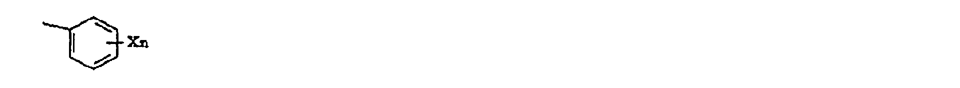 (wherein X represents a hydrogen atom, a halogen atom, a lower alkyl group, or a lower alkyl group substituted with a halogen; and n is 0 or 1) in the general formula (2).

### Aspect 20

A method of preparing a hydroxime derivative represented by the following general formula (2): 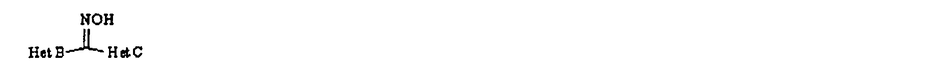 (wherein Het B and Het C are as defined in the general formula (3)), which comprises reacting a ketone compound represented by the following general formula (3): 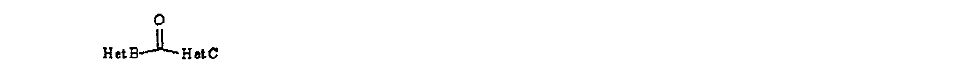 wherein Het B represents a group represented by any one of the following nine formulas: 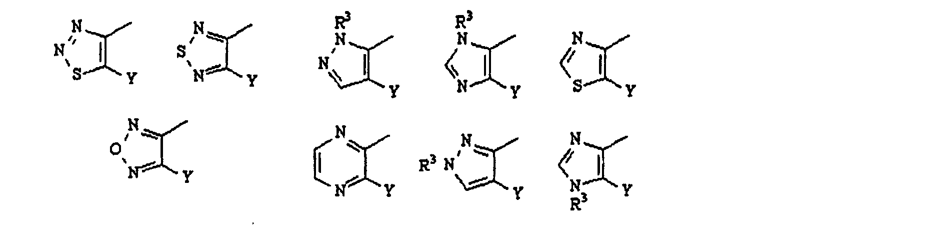 (wherein Y represents a hydrogen atom or a lower alkyl group; and R³ represents a hydrogen atom, a lower alkyl group, or a lower alkyl group substituted with a lower alkoxy group which may have a silyl group); and Het C represents a group represented by any one of the following nine formulas: 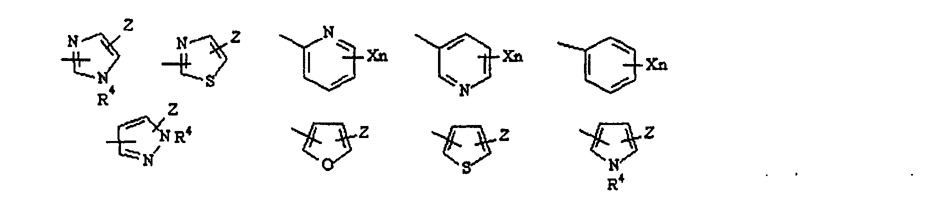 (wherein R⁴ represents a hydrogen atom, a lower alkyl group, or a lower alkyl group substituted with a lower alkoxy group which may have a silyl group; X represents a hydrogen atom, a halogen atom, a lower alkyl group, a lower alkyl group substituted with a halogen atom, a lower alkoxy group, or a lower alkoxy group substituted with a halogen atom; Z represents a hydrogen atom, a halogen atom, or a lower alkyl group; and n is from 0 to 3); with the exception of the case of the following compounds: 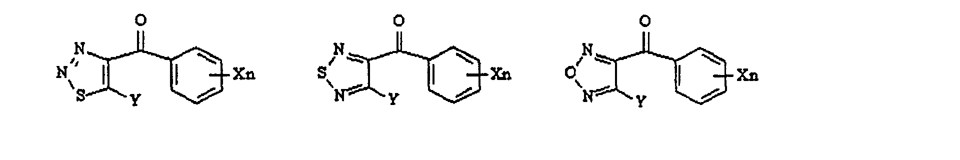 (wherein X represents a hydrogen atom, a halogen atom, a lower alkyl group, a lower alkyl group substituted with a halogen atom, a lower alkoxy group, a lower alkoxy group substituted with a halogen atom, or a cyano group; Y represents a hydrogen atom or a lower alkyl group; and n is 0 from 3), the following compound: 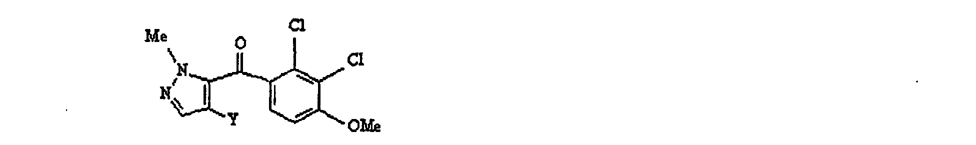 and the following compounds: 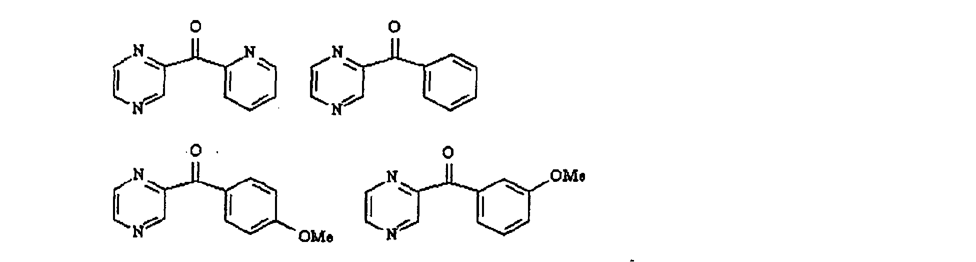 with an oximating agent.

### Aspect 21

A method of preparing a hydroxime derivative described in aspect 20, wherein Het B is a group represented by any one of the ring structures of the following three formulas: 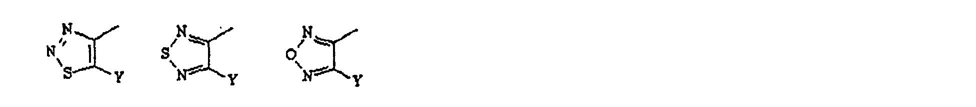 (wherein Y represents a hydrogen atom or a lower alkyl group); and Het C is a group represented by any one of the ring structures of the following eight formulas: 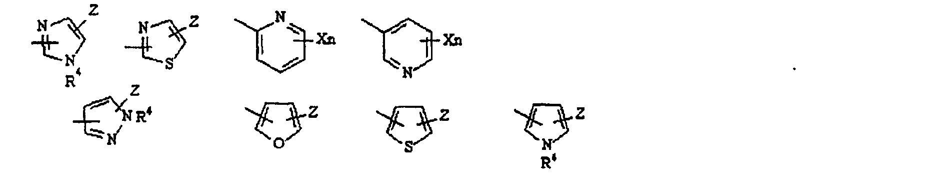 (wherein Z represents a hydrogen atom, a halogen atom, or a lower alkyl group; R⁴ represents a hydrogen atom, a lower alkyl group, or a lower alkyl group substituted with a lower alkoxy group which may have a silyl group; X represents a hydrogen atom, a halogen atom, a lower alkyl group, or a lower alkoxy group; and n is from 0 to 3) in the general formula (3).

### Aspect 22

A method of preparing a hydroxime derivative described in aspect 20, wherein Het B is a group represented by any one of the following six formulas: 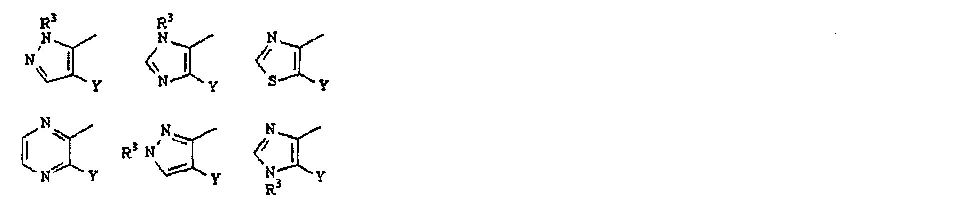 (wherein Y represents a hydrogen atom or a lower alkyl group; and R³ represents a hydrogen atom, a lower alkyl group, or a lower alkyl group substituted with a lower alkoxy group which may have a silyl group); and Het C is a group represented by any one the following six formulas: 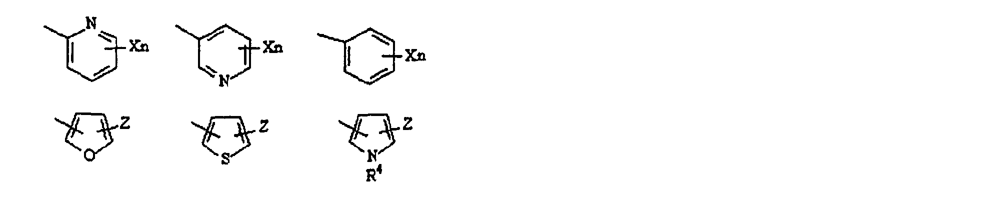 (wherein R⁴ represents a hydrogen atom or a lower alkyl group; X represents a hydrogen atom, a halogen atom, a lower alkyl group, a lower alkyl group substituted with a halogen atom, a lower alkoxy group, or a lower alkoxy group substituted with a halogen atom; Z represents a hydrogen atom, a halogen atom, or a lower alkyl group; and n is from 0 to 3) in the general formula (3).

### Aspect 23

A method of preparing a hydroxime derivative described in aspect 21, wherein Het C is a group represented by any one of the ring structures of the following three formulas: 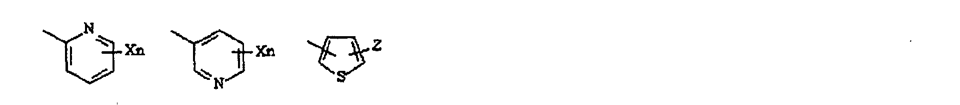 (wherein Z represents a hydrogen atom, a halogen atom, or a lower alkyl group; X represents a hydrogen atom, a halogen atom, a lower alkyl group, or a lower alkoxy group; and n is 0 or 1) in the general formula (3).

### Aspect 24

A method of preparing a hydroxime derivative described in aspect 21, wherein Het C is a group represented by the following formula: 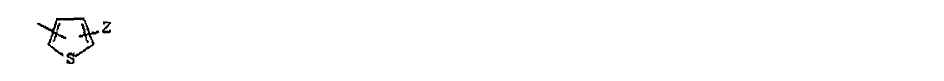 (wherein Z represents a hydrogen atom) in the general formula (3).

### Aspect 25

A method of preparing a hydroxime derivative described in aspect 21, wherein Het C is a group represented by the following formula: 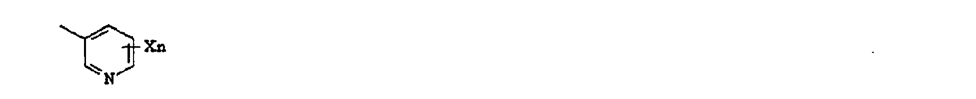 (wherein X represents a hydrogen atom or a halogen atom; and n is 0 or 1) in the general formula (3).

### Aspect 26

A method of preparing a hydroxime derivative described in aspect 22, wherein Het C is a group represented by the following formula: 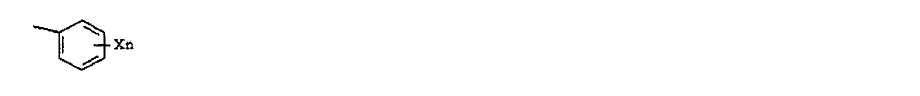 (wherein X represents a hydrogen atom, a halogen atom, a lower alkyl group, a lower alkyl group substituted with a halogen atom, a lower alkoxy group, or a lower alkoxy group substituted with a halogen atom; and n is from 0 to 3) in the general formula (3).

### Aspect 27

A method of preparing a hydroxime derivative described in aspect 22, wherein Het B is a group represented by any one of the following three formulas: 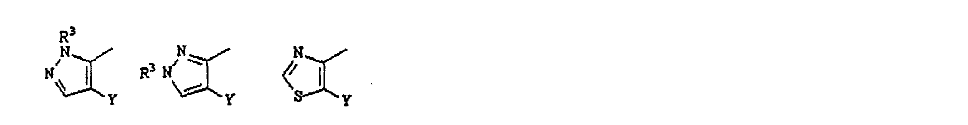 (wherein Y represents a hydrogen atom or a lower alkyl group; and R³ represents a hydrogen atom, a lower alkyl group, or a lower alkyl group substituted with a lower alkoxy group which may have a silyl group); and Het C is a group represented by the following formula: 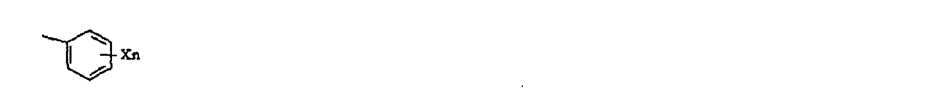 (wherein X represents a hydrogen atom, a halogen atom, a lower alkyl group, or a lower alkyl group substituted with a halogen atom; and n is 0 or 1) in the general formula (3).

### Aspect 28

A method of preparing an oxime derivative represented by the following general formula (1): 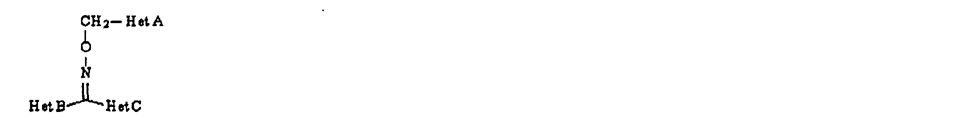 (wherein Het A is as defined in the general formula (4); and Het B and Het C are as defined in the general formula (2)), which comprises reacting a hydroxime compound represented by the following general formula (2): 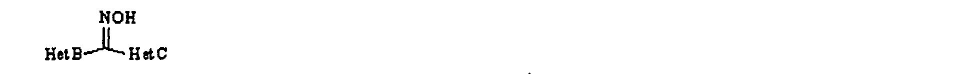 wherein Het B represents a group represented by any one of the following nine formulas: 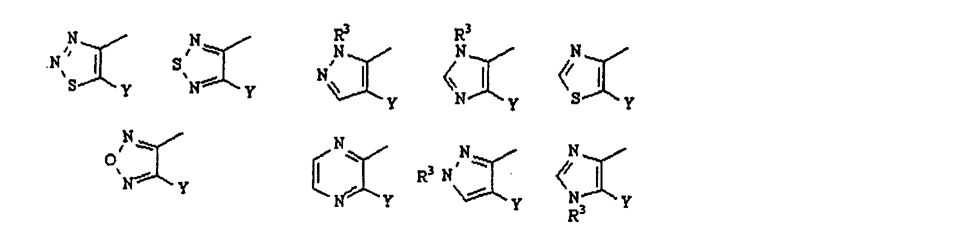 (wherein Y represents a hydrogen atom or a lower alkyl group; and R³ represents a hydrogen atom, a lower alkyl group, or a lower alkyl group substituted with a lower alkoxy group which may have a silyl group); and Het C represents a group represented by any one of the following nine formulas: 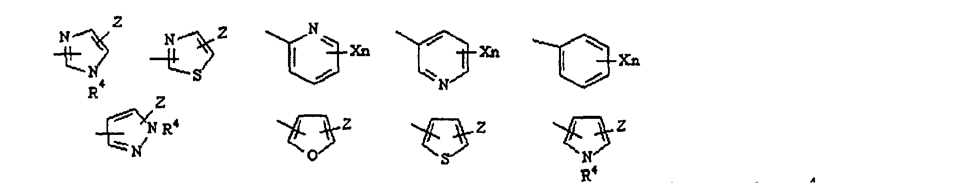 (wherein R⁴ represents a hydrogen atom, a lower alkyl group, or a lower alkyl group substituted with a lower alkoxy group which may have a silyl group; X represents a hydrogen atom, a halogen atom, a lower alkyl group, a lower alkyl group substituted with a halogen atom, a lower alkoxy group, a lower alkoxy group substituted with a halogen atom, or a cyano group; Z represents a hydrogen atom, a halogen atom, or a lower alkyl group; and n is from 0 to 3); with the exception of the case where Het A is a group represented by any one of the following two formulas: 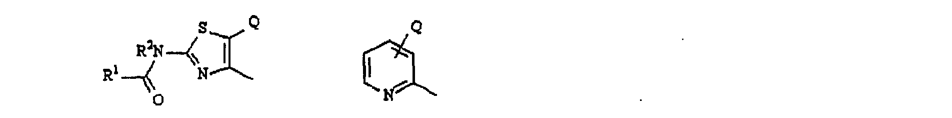 (wherein Q represents a hydrogen atom, a halogen atom, or a lower alkyl group; R¹ represents a hydrogen atom, a lower alkyl group, a lower alkyl group substituted with a halogen atom, a cycloalkyl group, a lower alkenyl group, a lower alkynyl group, an aralkyl group, an aryl group, or an aryl group substituted with one or more substituents selected from the group consisting of halogen atom, lower alkyl group, and lower alkoxy group; and R² represents a hydrogen atom or a lower alkyl group); Het B is a group represented by any one of the ring structures of the following three formulas: 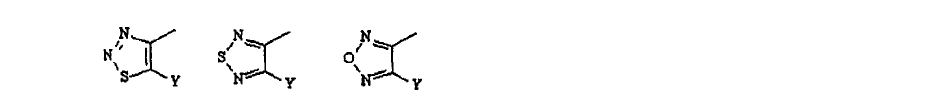 (wherein Y represents a hydrogen atom or a lower alkyl group); and Het C is a group represented by the following formula: 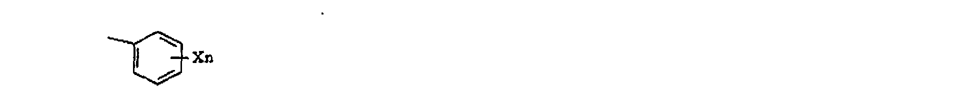 (wherein X represents a hydrogen atom, a halogen atom, a lower alkyl group substituted with a halogen atom, a lower alkoxy group, a lower alkoxy group substituted with a halogen atom, or a cyano group; and n is from 0 to 3), with an alkylating agent represented by the following general formula (4):

Het A-CH₂L

wherein Het A represents a group represented by any one of the following three formulas: 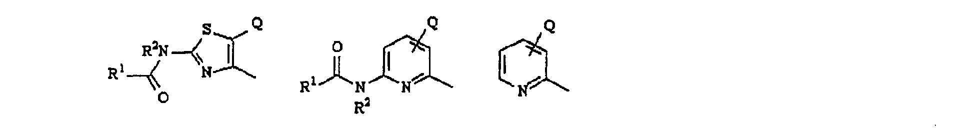 (wherein Q represents a hydrogen atom, a halogen atom, or a lower alkyl group; R¹ represents a hydrogen atom, a lower alkyl group, a lower alkyl group substituted with a halogen atom, a cycloalkyl group, a lower alkenyl group, a lower alkynyl group, an aralkyl group, an aryl group, or an aryl group substituted with one or more substituents selected from the group consisting of halogen atom, lower alkyl group, and lower alkoxy group; and R² represents a hydrogen atom or a lower alkyl group); and L represents a leaving group, in the presence of a base.

### Aspect 29

A method of preparing an oxime derivative described in aspect 28, wherein Het B is a group represented by any one of the ring structures of the following three formulas: 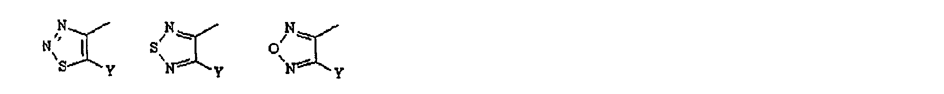 (wherein Y represents a hydrogen atom or a lower alkyl group); and Het C is a group represented by any one of the ring structures of the following eight formulas: 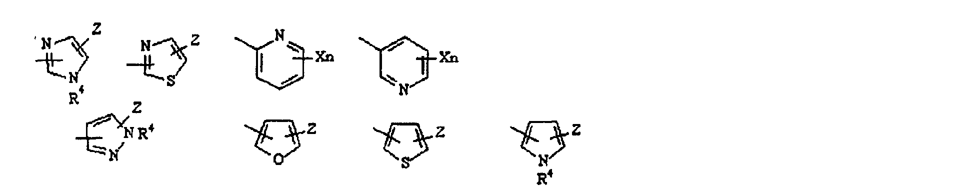 (wherein Z represents a hydrogen atom, a halogen atom, or a lower alkyl group; R⁴ represents a hydrogen atom, a lower alkyl group, or a lower alkyl group substituted with a lower alkoxy group which may have a silyl group; X represents a hydrogen atom, a halogen atom, a lower alkyl group, or a lower alkoxy group; and n is from 0 to 3) in the general formula (2).

### Aspect 30

A method of preparing an oxime derivative described in aspect 28, wherein Het B is a group represented by any one of the ring structures of the following three formulas: 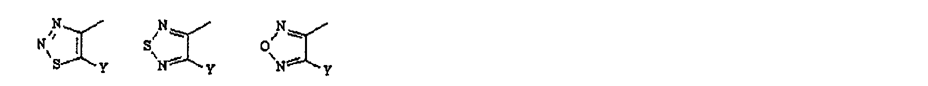 (wherein Y represents a hydrogen atom or a lower alkyl group); Het C is a group represented by any one of the ring structures of the following formula: 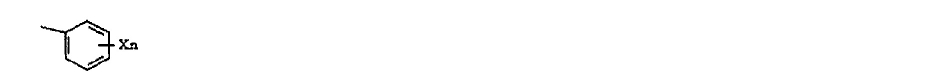 (wherein X represents a hydrogen atom, a halogen atom, a lower alkyl group, a lower alkyl group substituted with a halogen atom, a lower alkoxy group, a lower alkoxy group substituted with a halogen atom, or a cyano group; and n is from 0 to 3) in the general formula (2); and Het A is a group represented by the following formula: 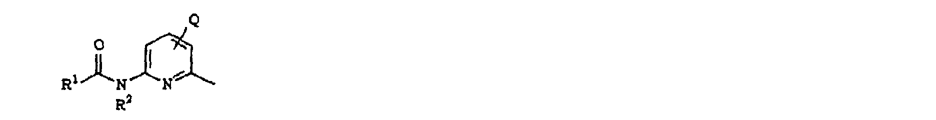 (wherein Q represents a hydrogen atom, a halogen atom, or a lower alkyl group; R¹ represents a hydrogen atom, a lower alkyl group, a lower alkyl group substituted with a halogen atom, a cycloalkyl group, a lower alkenyl group, a lower alkynyl group, an aralkyl group, an aryl group, or an aryl group substituted with one or more substituents selected from the group consisting of halogen atom, lower alkyl group, and lower alkoxy group; and R² represents a hydrogen atom or a lower alkyl group) in the general formula (4).

### Aspect 31

A method of preparing an oxime derivative described in aspect 28, wherein Het B is a group represented by any one of the following six formulas: 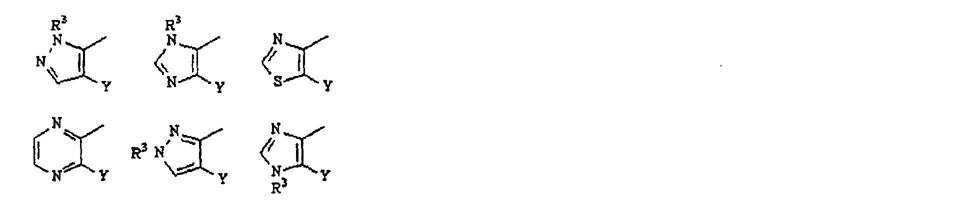 (wherein Y represents a hydrogen atom or a lower alkyl group; and R³ represents a hydrogen atom, a lower alkyl group, or a lower alkyl group substituted with a lower alkoxy group which may have a silyl group); and Het C is a group represented by any one of the following six formulas: 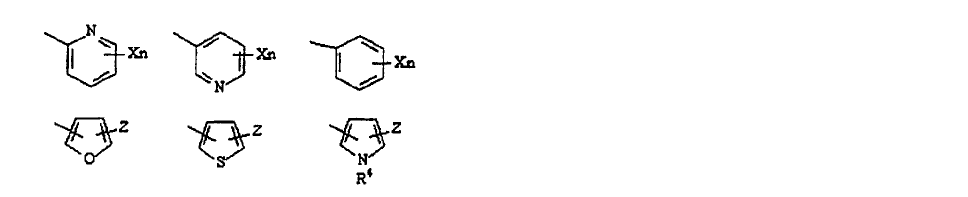 (wherein R⁴ represents a hydrogen atom or a lower alkyl group; X represents a hydrogen atom, a halogen atom, a lower alkyl group, a lower alkyl group substituted with a halogen atom, a lower alkoxy group, or a lower alkoxy group substituted with a halogen atom; Z represents a hydrogen atom, a halogen atom, or a lower alkyl group; and n is from 0 to 3) in the general formula (2).

### Aspect 32

A method of preparing an oxime derivative described in aspect 29, wherein Het C is a group represented by any one of the ring structures of the following three formulas: 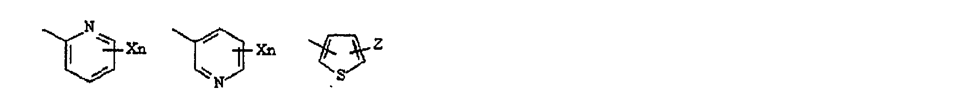 (wherein Z represents a hydrogen atom, a halogen atom, or a lower alkyl group; X represents a hydrogen atom, a halogen atom, a lower alkyl group, or a lower alkoxy group; and n is 0 or 1) in the general formula (2).

### Aspect 33

A method of preparing an oxime derivative described in aspect 29, wherein Het C is a group represented by the following formula: 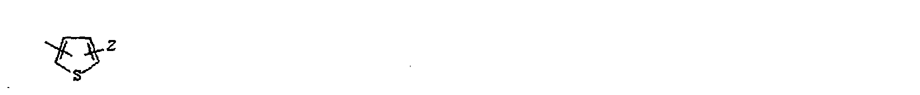 (wherein Z represents a hydrogen atom) in the general formula (2), and Het A is a group represented by the following formula: 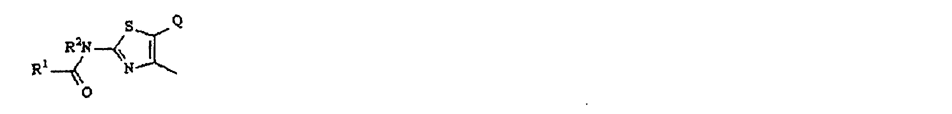 (wherein Q represents a hydrogen atom, a halogen atom, or a lower alkyl group; R¹ represents a lower alkyl group or a cycloalkyl group; and R² represents a hydrogen atom) in the general formula (4).

### Aspect 34

A method of preparing an oxime derivative described in aspect 29, wherein Het C is a group represented by the following formula: 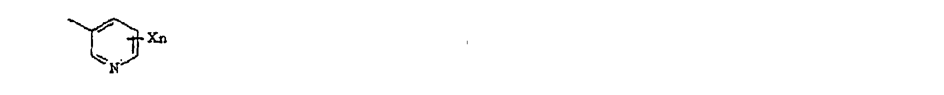 (wherein X represents a hydrogen atom or a halogen atom; and n is 0 or 1) in the general formula (2); and Het A is a group represented by the following formula: 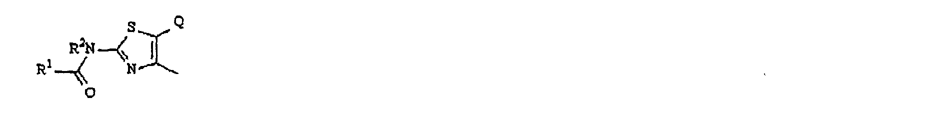 (wherein Q represents a hydrogen atom, a halogen atom, or a lower alkyl group; R¹ represents a lower alkyl group or a cycloalkyl group; and R² represents a hydrogen atom) in the general formula (4).

### Aspect 35

A method of preparing an oxime derivative described in aspect 29, wherein Het C is a group represented by any one of the following three formulas: 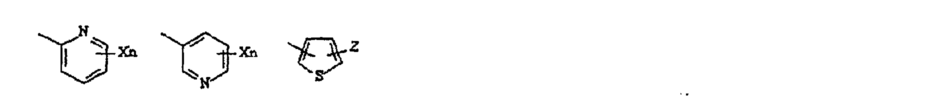 (wherein X represents a hydrogen atom or a halogen atom; Z represents a hydrogen atom; and n is 0 or 1) in the general formula (2); and Het A is a group represented by the following formula: 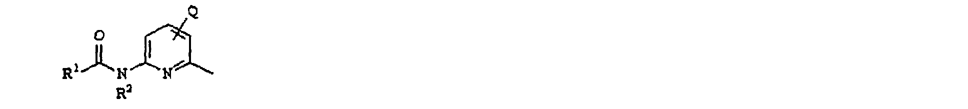 (wherein Q represents a hydrogen atom, a halogen atom, or a lower alkyl group; R¹ represents a lower alkyl group or a cycloalkyl group; and R² represents a hydrogen atom) in the general formula (4).

### Aspect 36

A method of preparing an oxime derivative described in aspect 30, wherein Het C is a group represented by the following formula: 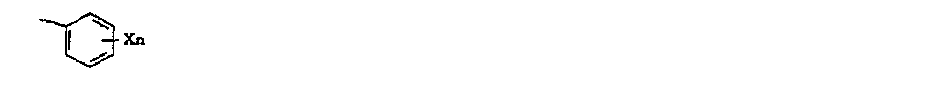 (wherein X represents a hydrogen atom, a halogen atom, a lower alkyl group, a lower alkyl group substituted with a halogen atom, or a cyano group; and n is from 0 to 3) in the general formula (2); and Het A is a group represented by the following formula: 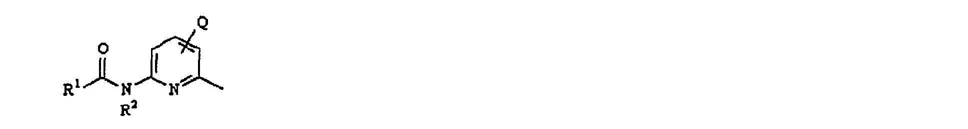 (wherein Q represents a hydrogen atom or a lower alkyl group; R¹ represents a lower alkyl group or a cycloalkyl group; and R² represents a hydrogen atom) in the general formula (4).

### Aspect 37

A method of preparing an oxime derivative described in aspect 31, wherein Het C is a group represented by the following formula: 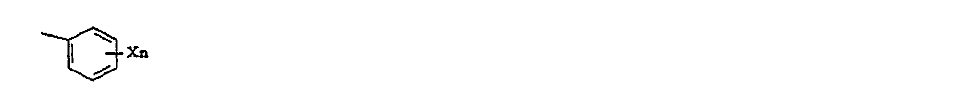 (wherein X represents a hydrogen atom, a halogen atom, a lower alkyl group, a lower alkyl group substituted with a halogen atom, a lower alkoxy group, or a lower alkoxy group substituted with a halogen atom; and n is from 0 to 3) in the general formula (2).

### Aspect 38

A method of preparing an oxime derivative described in aspect 31, wherein Het B is a group represented by any one of the following three formulas: 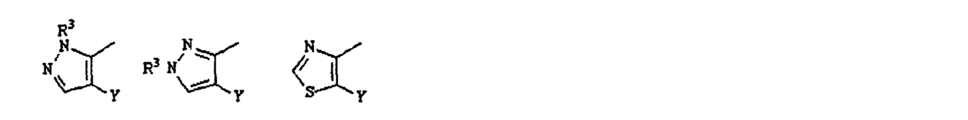 (wherein Y represents a hydrogen atom or a lower alkyl group; and R³ represents a hydrogen atom, a lower alkyl group, or a lower alkyl group substituted with a lower alkoxy group which may have a silyl group); Het C is a group represented by the following formula: 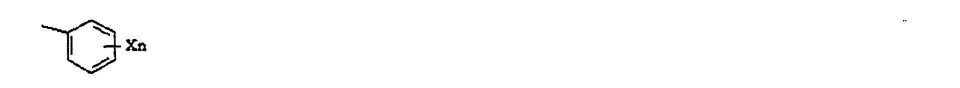 (wherein X represents a hydrogen atom, a halogen atom, a lower alkyl group, or a lower alkyl group substituted with a halogen atom; and n is 0 or 1) in the general formula (2); and Het A is a group represented by the following formula: 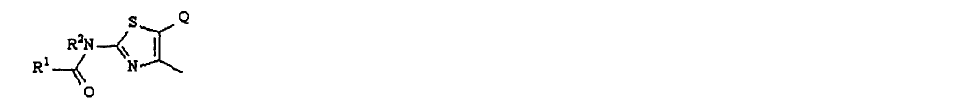 (wherein Q represents a hydrogen atom, a halogen atom, or a lower alkyl group; R¹ represents a lower alkyl group or a cycloalkyl group; and R² represents a hydrogen atom) in the general formula (4).

### Aspect 39

An agricultural chemical comprising an oxime derivative of any one of aspects 1 to 11 as an active ingredient.

### Aspect 40

A plant disease controlling agent comprising the oxime derivative of any one of aspects 1 to 11 as an active ingredient.

### Aspect 41

A plant disease controlling agent described in aspect 40, which is effective against plant diseases caused by filamentous fungi.

### BEST MODE FOR CARRYING OUT THE INVENTION

Het A of the oxime derivative of the present invention represented by the general formula (1): 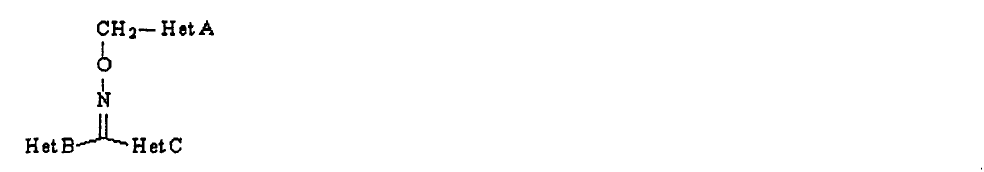 is a group represented by any one of the following three formulas: 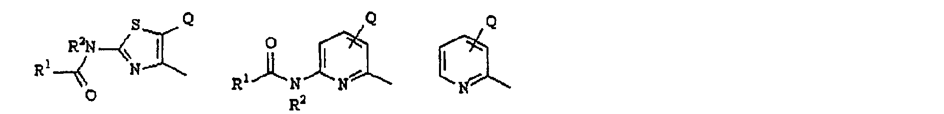 (wherein Q represents a hydrogen atom, a halogen atom, or a lower alkyl group; R¹ represents a hydrogen atom, a lower alkyl group, a lower alkyl group substituted with a halogen atom, a cycloalkyl group, a lower alkenyl group, a lower alkynyl group, an aralkyl group, an aryl group, or an aryl group substituted with one or more substituents selected from the group consisting of a halogen atom, a lower alkyl group, and a lower alkoxy group; and R² represents a hydrogen atom or a lower alkyl group).

Examples of the halogen atom for the Q of Het A include a fluorine atom, a chlorine atom, and a bromine atom. Among these halogen atoms, a chlorine atom is preferred. Examples of the lower alkyl group for the Q of Het A include a straight-chain or branched lower alkyl group having 1 to 6 carbon atoms, and preferably 1 to 4 carbon atoms, such as a methyl group, an ethyl group, an n-propyl group, an isopropyl group, an n-butyl group, an isobutyl group, and a sec-butyl group. Specifically, a methyl group is particularly preferred. The substitution position of the Q may be any position on the carbon atoms. In the case in which Het A is a thiazole ring, a hydrogen atom, a chlorine atom, and a methyl group are particularly preferred as the Q. In the case of a pyridine ring, a hydrogen atom is particularly preferred.

Examples of the lower alkyl group for R¹ of Het A include a straight-chain or branched lower alkyl group having 1 to 6 carbon atoms, and preferably 1 to 4 carbon atoms, such as a methyl group, an ethyl group, an n-propyl group, an isopropyl group, a tert-butyl group, an n-butyl group, an isobutyl group, a sec-butyl group, an n-pentyl group, a neopentyl group, a 1,1-dimethylpropyl group, and a 1-ethylpropyl group. More specifically, a methyl group, an ethyl group, an n-propyl group, an isopropyl group, a tert-butyl group, an n-butyl group, an isobutyl group, and a sec-butyl group are particularly preferred.

Examples of the lower alkyl group substituted with a halogen atom for R¹ of Het A include a halogen-substituted lower alkyl group having 1 to 3 carbon atoms, such as a chloromethyl group, a dichloromethyl group, a trichloromethyl group, a trifluoromethyl group, a pentafluoroethyl group, a heptafluoropropyl group, a chlorodifluoromethyl group, and a 1-bromo-1-methylethyl group.

Examples of the cycloalkyl group for R¹ of Het A include a cycloalkyl group having 1 to 7 carbon atoms, and preferably 3 to 6 carbon atoms, such as a cyclopropyl group, a cyclopentyl group, a cyclohexyl group, and a cycloheptyl group. More specifically, cyclopropyl, cyclopentyl, and cyclohexyl are particularly preferred.

Examples of the lower alkenyl group for R¹ of Het A include a straight-chain or branched lower alkenyl group having 2 to 4 carbon atoms, such as a vinyl group, an allyl group, a 1-propenyl group, and a 1-methyl-2-propenyl group.

Examples of the lower alkynyl group for R¹ of Het A include a lower alkynyl group having 1 to 4 carbon atoms, such as an ethynyl group, a propargyl group, and a 2-butynyl group.

Examples of the aralkyl group substituted for R¹ of Het A include a benzyl group and a phenethyl group.

Examples of the aryl group for R¹ of Het A include a phenyl group.

Examples of the aryl group substituted with one or more substituents selected from halogen atoms, lower alkyl groups, and lower alkoxy groups for R¹ of Het A include a 2-chlorophenyl group, a 3-chlorophenyl group, a 4-chlorophenyl group, a 2,4-dichlorophenyl group, a 3,4-dichlorophenyl group, a 3,5-dichlorophenyl group, a 2,6-dichlorophenyl group, a 2,3-dichlorophenyl group, a 2,4,6-trichlorophenyl group, a 2-fluorophenyl group, a 3-fluorophenyl group, a 4-fluorophenyl group, a 2,4-difluorophenyl group, a 3,4-difluorophenyl group, a 3,5-difluorophenyl group, a 2,6-difluorophenyl group, a 2,3-difluorophenyl group, a 2-methylphenyl group, a 3-methylphenyl group, a 4-methylphenyl group, a 2,4-dimethylphenyl group, a 3,4-dimethylphenyl group, a 3,5-dimethylphenyl group, a 2,6-dimethylphenyl group, a 2,4-dimethylphenyl group, a 2,4,6-trimethylphenyl group, a 2-trifluoromethylphenyl group, a 3-trifluoromethylphenyl group, a 4-trifluoromethylphenyl group, a 2-methoxyphenyl group, a 3-methoxyphenyl group, a 4-methoxyphenyl group, a 2-trifluoromethoxyphenyl group, a 3-trifluoromethoxyphenyl group, a 4-trifluoromethoxyphenyl group, a 2-chlorodifluoromethoxyphenyl group, a 3-chlorodifluoromethoxyphenyl group, and a 4-chlorodifluoromethoxyphenyl group. More specifically, a 4-methylphenyl and a 4-chlorophenyl are particularly preferred.

Preferred examples of R¹ of Het A include a hydrogen atom, a straight-chain or branched lower alkyl group having 1 to 6 carbon atoms, and a cycloalkyl group having 3 to 6 carbon atoms.

Examples of the lower alkyl group for R² of Het A include a lower alkyl group having 1 to 6 carbon atoms, and preferably 1 to 4 carbon atoms, such as a methyl group and an ethyl group. More specifically, a methyl group is particularly preferred. A hydrogen atom is particularly preferred as R².

In the general formulas (1), (2), and (3), Het B represents a 1,2,3-thiadiazol-4-yl group, a 1,2,5-thiazol-3-yl group, or a 1,2,5-oxadiazol-3-yl group, or a lower alkylsubstituted derivative or an optionally substituted five- to six-membered nitrogen-containing aromatic heterocyclic ring thereof, which is represented by any one of the ring structures of the following nine formulas: 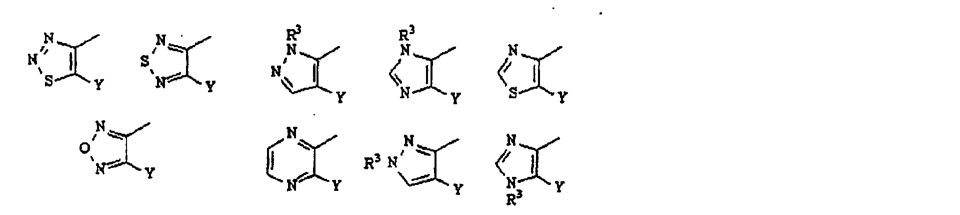 (wherein Y represents a hydrogen atom or a lower alkyl group; and R³ represents a hydrogen atom, a lower alkyl group, or a lower alkyl group substituted with a lower alkoxy group which may have a silyl group).

Examples of the lower alkyl group for Y of Het B include a lower alkyl group having 1 to 6 carbon atoms, and preferably 1 to 4 carbon atoms, such as a methyl group, an ethyl group, an n-propyl group, or an isopropyl group. More specifically, a methyl group is particularly preferred.

Examples of the lower alkyl group for R³ of Het B include a lower alkyl group having 1 to 6 carbon atoms, and preferably 1 to 4 carbon atoms, such as a methyl group, an ethyl group, an n-propyl group, an isopropyl group, or an n-butyl group. More specifically, a methyl group is particularly preferred.

The lower alkyl group substituted with a lower alkoxy group which may have a silyl group for R³ of Het B is a lower alkyl group having 1 to 6 carbon atoms, and preferably 1 to 4 carbon atoms, which is substituted with a lower alkoxy group having 1 to 4 carbon atoms, or a lower alkyl group having 1 to 6 carbon atoms, and preferably 1 to 4 carbon atoms, which is substituted with a lower alkoxy group having 1 to 4 carbon atoms which has a silyl group. The "silyl group" in the present invention is a silyl group which has 1 to 3 hydrogen atoms or 1 to 3 lower alkyl groups having 1 to 4 carbon atoms as substituents. Specific examples thereof include a methoxyethyl group, a 2-methoxyethyl group, a 1-ethoxyethyl group, a 2-trimethylsilylethoxymethyl group, a tert-butoxymethyl group, a 3-methoxypropyl group, a 1-(isopropoxy)ethyl group, a 2-ethoxyethyl group, a 1-methyl-1-methoxyethyl group, a 3-ethoxypropyl group, and a benzoyloxymethyl group. Among these groups, a 2-trimethylsilylethoxymethyl group is more preferred.

A methyl group and a 2-trimethylsilylethoxymethyl group are particularly preferred as R³.

In the general formulas (1), (2), and (3), Het C represents a group which is represented by any one of the ring structures of the following nine formulas: 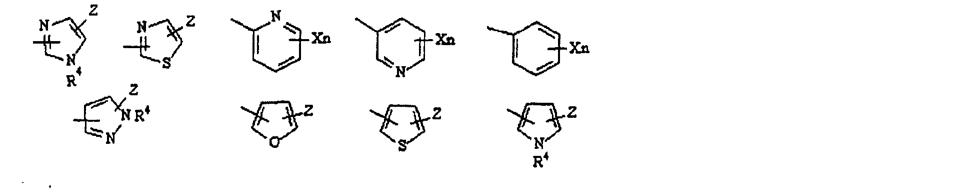 (wherein R⁴ represents a hydrogen atom, a lower alkyl group, a lower alkyl group substituted with a lower alkoxy group which may have a silyl group, or a benzoyloxymethyl group; X represents a hydrogen atom, a halogen atom, a lower alkyl group, a lower alkyl group substituted with a halogen atom, a lower alkoxy group, a lower alkoxy group substituted with a halogen atom, or a cyano group; Z represents a hydrogen atom, a halogen atom, or a lower alkyl group; and n is from 0 to 3).

Examples of the lower alkyl group for R⁴ of Het C include a lower alkyl group having 1 to 6 carbon atoms, and preferably 1 to 4 carbon atoms, such as a methyl group, an ethyl group, an n-propyl group, an isopropyl group, and an n-butyl group. More specifically, a methyl group is particularly preferred.

The lower alkyl group substituted with a lower alkoxy group which may have a silyl group for R³ of Het C is a lower alkyl group having 1 to 6 carbon atoms, and preferably 1 to 4 carbon atoms, which is substituted with a lower alkoxy group having 1 to 4 carbon atoms, or a lower alkyl group having 1 to 6 carbon atoms, and preferably 1 to 4 carbon atoms, which is substituted with a lower alkoxy group having 1 to 4 carbon atoms which has a silyl group. Examples of the silyl group include silyl groups which has 1 to 3 hydrogen atoms or 1 to 3 lower alkyl groups having 1 to 4 carbon atoms as substituents. Specific examples thereof include a methoxyethyl group, a 2-methoxyethyl group, a 1-ethoxyethyl group, a 2-trimethylsilylethoxymethyl group, a tert-butoxymethyl group, a 3-methoxypropyl group, a 1-(isopropoxy)ethyl group, a 2-ethoxyethyl group, a 1-methyl-1-methoxyethyl group, and a 3-ethoxypropyl group. Among these groups, a 2-trimethylsilylethoxymethyl group is more preferred.

Preferred examples of R⁴ of Het C include a methyl group and a 2-trimethylsilylethoxymethyl group. Among these groups, a 2-trimethylsilylethoxymethyl group is particularly preferred.

Examples of the halogen atom X of Het C include a fluorine atom, a chlorine atom, and a bromine atom. Among these halogen atoms, a chlorine atom is preferred.

Examples of the lower alkyl group for X of Het C include a lower alkyl group having 1 to 6 carbon atoms, and preferably 1 to 4 carbon atoms, such as a methyl group and an ethyl group. More specifically, a methyl group is particularly preferred.

Examples of the lower alkyl group substituted with a halogen atom for X of Het C include a trifluoromethyl group, a difluoromethyl group, and a difluorochloromethyl group. Furthermore, a trifluoromethyl group is preferred.

Examples of the lower alkoxy group for X of Het C include a lower alkoxy group having 1 to 4 carbon atoms such as a methoxy group, an ethoxy group, and an n-propoxy group. Furthermore, a methoxy group is preferred.

Examples of the lower alkoxy group substituted with a halogen atom for X of Het C include a difluoromethoxy group and a trifluoromethoxy group.

n represents an integer of 3 or less, that is, an integer of 0 to 3. In the case in which n is 2 or 3, X may be the same or different.

X of Het C is preferably a hydrogen atom, a lower alkyl group having 1 to 2 carbon atoms, a fluoroalkyl group having 1 carbon atom, or a halogen atom, and n is 0 or 1, particularly preferably.

Preferred examples of the halogen atom for Z of Het C include a chlorine atom, a bromine atom, and a fluorine atom. Among these halogen atoms, a chlorine atom is preferred. Preferred examples of the lower alkyl group for X of Het C include a lower alkyl group having 1 to 6 carbon atoms, and preferably 1 to 4 carbon atoms, such as a methyl group and an ethyl group.

Z of Het C is preferably a hydrogen atom or a chlorine atom. Furthermore, a hydrogen atom is particularly preferred.

The substitution position of the substituents X and Z may be any position on the carbon atoms.

Het A of the alkylating agent represented by the general formula (4):

Het A-CH₂L

of the present invention is as defined in the general formula (1). Examples of the leaving group L include halogen atoms such as a chlorine atom, a bromine atom, and an iodine atom, and sulfonyloxy groups such as a methanesulfonyloxy group, a p-toluenesulfonyloxy group and a trifluoromethanesulfonyl group. Among these groups, a halogen atom such as a chlorine atom, a bromine atom, and an iodine atom is preferred.

In the present invention, there is excluded the case where Het A is a group represented by any one of the following two formulas: 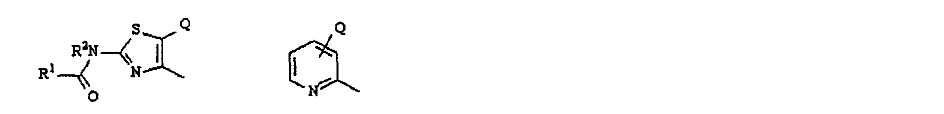 (wherein Q represents a hydrogen atom, a halogen atom, or a lower alkyl group; R¹ represents a hydrogen atom, a lower alkyl group, a lower alkyl group substituted with a halogen atom, a cycloalkyl group, a lower alkenyl group, a lower alkynyl group, an aralkyl group, an aryl group, or an aryl group substituted with one or more substituents selected from the group consisting of halogen atom, lower alkyl group, and lower alkoxy group; and R² represents a hydrogen atom or a lower alkyl group); and Het B is a group represented by any one of the ring structures of the following three formulas: 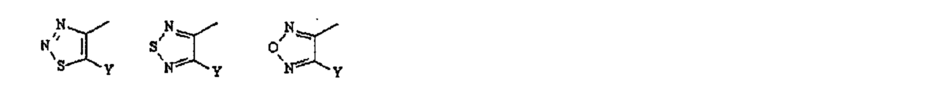 (wherein Y represents a hydrogen atom or a lower alkyl group); and Het C is a group represented by the following formula: 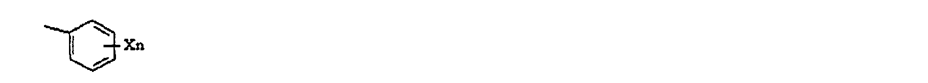 (wherein X represents a hydrogen atom, a halogen atom, a lower alkyl group substituted with a halogen atom, a lower alkoxy group, a lower alkoxy group substituted with a halogen atom, or a cyano group; and n is from 0 to 3).

In the general formula (2), there is excluded the case of the following compounds: 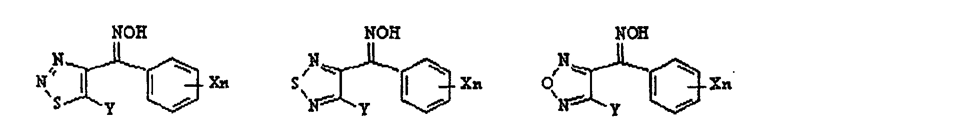 (wherein X represents a hydrogen atom, a halogen atom, a lower alkyl group, a lower alkyl group substituted with a halogen atom, a lower alkoxy group, a lower alkoxy group substituted with a halogen atom, or a cyano group; and n is 0 or 1), the following compound: 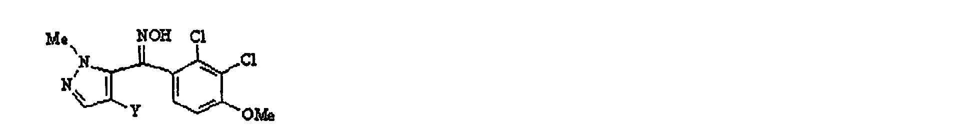 and the following compounds:

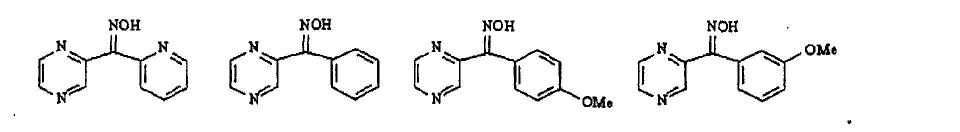

A hydroxime derivative of the general formula (2) in which Het B is a group represented by any one of the ring structures of the following three formulas: 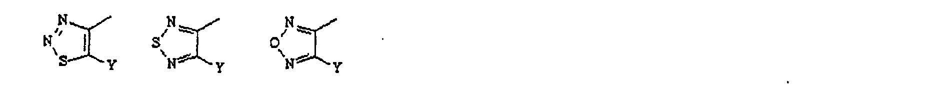 (wherein Y represents a hydrogen atom or a lower alkyl group), and Het C is a group represented by any one of the following formula: 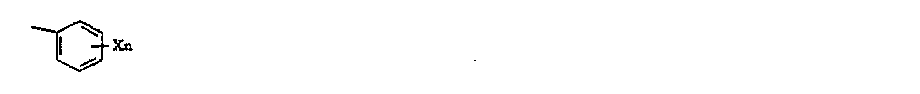 (wherein X represents a hydrogen atom, a halogen atom, a lower alkyl group, a lower alkyl group substituted with a halogen atom, a lower alkoxy group, or a lower alkoxy group substituted with a halogen atom; and n is from 0 to 3) is a hydroxime derivative of the general formula (5).

Examples of the lower alkyl group for Y of Het B in the general formula (5) include a lower alkyl group having 1 to 6 carbon atoms, and preferably 1 to 4 carbon atoms, such as a methyl group, an ethyl group, an n-propyl group, and an isopropyl group. More specifically, a methyl group is preferred.

Examples of the halogen atom for X in the general formula (5) include a chlorine atom, a bromine atom, and a fluorine atom. Examples of the lower alkyl group include a straight-chain or branched lower alkyl group having 1 to 6 carbon atoms, and preferably 1 to 4 carbon atoms, such as a methyl group, an ethyl group, an n-propyl group, an isopropyl group, an n-butyl group, an isobutyl group, a tert-butyl group, and sec-butyl group. Examples of the lower alkyl group substituted with a halogen atom include a trifluoromethyl group, a difluoromethyl group, and a difluorochloromethyl group.

Examples of the lower alkoxy group for X in the general formula (5) include a lower alkoxy group having 1 to 4 carbon atoms, such as a methoxy group, an ethoxy group, an n-propoxy group, an isopropoxy group, an n-butoxy group, an isobutoxy group, a sec-butoxy group, or a tert-butoxy group. Examples of the lower alkoxy group substituted with a halogen atom include a difluoromethoxy group and a trifluoromethoxy group.

The substitution position of X in the general formula (5) is not specifically limited, and n represents an integer of 1 to 3. In the case in which n is 2 or 3, X may be the same or different. Preferred examples of X in the general formula (5) include a lower alkyl group having 1 to 2 carbon atoms, a fluoroalkyl group having 1 carbon atom, a halogen atom, and a cyano group. Among these groups, a trifluoromethyl group, a fluorine atom or a chlorine atom is particularly preferred.

The stereostructure of the oxime moiety, which exists in the oxime derivative represented by the general formula (1) and the hydroxime derivative represented by the general formula (2) includes (E) or (Z) isomer, and these stereoisomers are included in the present invention. The synthesized product is usually obtained in the form of a mixture of (E) and (Z) isomers, each of which can be isolated by separation and purification.

The (Z) isomer is particularly superior in plant disease controlling activity to the (E) isomer. However, in the oxime derivatives represented by the general formulas (1) and (2), both the (E) isomer and the (Z) isomer exist in fixed ratio in the form of a mixture since the (Z) isomer is converted into the (E) isomer by light in a natural environment. The stable ratios of the (E) and (Z) isomers vary according to the type of compound.

The method of preparing the oxime derivative represented by the general formula (1) in the present invention will now be described. For example, the oxime derivative represented by the general formula (1) can be prepared by the following methods, but the method of preparing the compound is not limited to the following methods.

The oxime derivative represented by the general formula (1) can be prepared by reacting a hydroxime derivative represented by the following general formula (2): 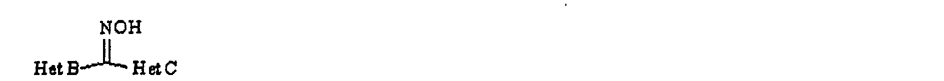 which is obtained by reacting a ketone compound represented by the following general formula (3): 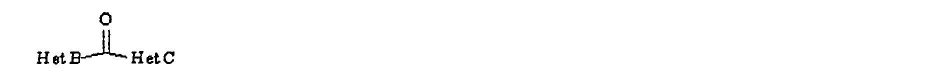 (wherein Het B and Het C are as defined in the general formula (1)) with an oximating agent, with an alkylating agent represented by the following general formula (4):

Het A-CH₂L

in the presence of a base.

The ketone compound represented by the general formula (3) is a raw material used in the preparation method of the present invention and can be synthesized, for example, from ethyl 5-methyl(1,2,3-thiadiazoyl-4-yl)carboxylate (Acta Pharm. Suecica, Vol. 10, page 297 (1973)), 5-methyl(1,2,3-thiazoyl-4-yl)carboxylic acid (Acta Pharm. Suecica, Vol. 10, page 297 (1973)), 3-formyl-4-methyl-1,2,5-oxadiazole (J. Org. Chem., Vol. 32, page 3865 (1967)), and 3-formyl-4-methyl-1,2,5-thiadiazole (Heterocyclic Chem., Vol. 22, page 325 (1985)) by a method described in the literature (for example, New Experimental Chemistry Lecture 14, Synthesis and Reaction II of Organic Compounds, 1997, Maruzen; C. A. Buehler, D. E. Pearson, Survey of Organic Syntheses, John Wiley and Sons, New York, page 532, 1976).

Examples of the oximating agent used in this reaction include hydroxylamine salts such as hydroxylamine hydrochloride, hydroxylamine sulfate, and hydroxylamine acetate. The amount of the oximating agent is usually from 1 to 10 mol, and preferably from 1 to 2 mol, per mol of ketone.

When using the hydroxylamine salt, the reaction is carried out in the presence of a base. Examples of the base include inorganic bases such as sodium hydroxide, potassium hydroxide, sodium carbonate, potassium carbonate, cesium carbonate, and sodium hydrogencarbonate; and organic bases such as pyridine, triethylamine, tripropylamine, tributylamine, diisopropylethylamine, trioctylamine, N,N-dimethylaniline, N-methylpyrrolidine, N-methylmorpholine, N-ethylmorpholine, and 1,8-diazabicyclo[5.4.0]undeca-7-ene. The base is used in an equimolar amount or more relative to the amount of the hydroxylamine salt.

This reaction can be carried out in the presence or absence of a solvent. The solvent is not specifically limited as long as it is inert in the reaction, and examples thereof include hydrocarbon solvents such as pentane, hexane, heptane, petroleum ether, benzene, toluene, and xylene; halogen solvents such as dichloromethane, 1,2-dichloroethane, chloroform, and carbon tetrachloride; alcohol solvents such as methanol, ethanol, propanol, 2-propanol, and trifluoroethanol; nitrile solvents such as acetonitrile and propionitrile; ether solvents such as diethyl ether, diisopropyl ether, dimethoxyethane, dioxane, tetrahydrofuran, and diglyme; aprotic polar solvents such as N,N-dimethylformamide, N,N-dimethylacetamide, and dimethyl sulfoxide; water; and a mixed solvent of two or more of these solvents. Among these solvents, methanol, ethanol, dioxane, tetrahydrofuran, and a mixed solvent of these solvents and water are preferred. The proportion of water is from 1 to 99%, and preferably from 10 to 90%.

The temperature of this reaction is within a range from-78 to 250°C, and preferably from -10 to 120°C. The reaction time varies according to the amount of the reaction reagent and the reaction temperature, but is within a range from 30 minutes to 24 hours. After the completion of the reaction, the product can be easily purified by distillation, recrystallization, or column chromatography, if necessary.

In the reaction between the hydroxime derivative (2) and the alkylating agent (4) in the presence of the base, the amount of the alkylating agent is usually within a range from 1 to 5 mol, and preferably from 1 to 2 mol, per mol of the hydroxime derivative.

Examples of the base include inorganic bases such as sodium hydroxide, potassium hydroxide, sodium carbonate, potassium carbonate, cesium carbonate, and sodium hydrogencarbonate; organic bases such as pyridine, triethylamine, tripropylamine, tributylamine, diisopropylethylamine, trioctylamine, N,N-dimethylaniline, N-methylpyrrolidine, N-methylmorpholine, N-ethylmorpholine, and 1,8-diazabicyclo[5.4.0]undeca-7-ene; alkoxides such as sodium methoxide, sodium ethoxide, potassium tert-butoxide, and sodium isopentanate; alkali metal hydrides such as sodium hydride and potassium hydride; and organic lithiums such as methyllithium, butyllithium, and phenyllithium. The amount of the base is usually within a range from 1 to 10 mol, and preferably from 1 to 3 mol, per mol of the hydroxime derivative.

This reaction can be carried out in the presence or absence of a solvent. The solvent is not specifically limited as long as it is inert in the reaction, and examples thereof include hydrocarbon solvents such as pentane, hexane, heptane, petroleum ether, benzene, toluene, and xylene; halogen solvents such as dichloromethane, 1,2-dichloroethane, chloroform, and carbon tetrachloride; alcohol solvents such as methanol, ethanol, propanol, 2-propanol, and trifluoroethanol; nitrile solvents such as acetonitrile and propionitrile; ether solvents such as diethyl ether, diisopropyl ether, dimethoxyethane, dioxane, tetrahydrofuran, and diglyme; aprotic polar solvents such as N,N-dimethylformamide, N,N-dimethylacetamide, and dimethyl sulfoxide; water; and a mixed solvent of two or more of these solvents. Among these solvents, dioxane, tetrahydrofuran, acetonitrile, N,N-dimethylformamide, N,N-dimethylacetamide, and dimethyl sulfoxide are preferred.

The temperature of this reaction is within a range from-78 to 250°C, and preferably from -10 to 100°C. The reaction time varies according to the amount of the reaction reagent and the reaction temperature, but is within a range from 30 minutes to 24 hours. After the completion of the reaction, the product can be easily purified by distillation, recrystallization, or column chromatography, if necessary.

Specific structures of the oxime derivative represented by the general formula (1) prepared by the method described above are as shown in Tables 1 to 60. In the tables, Het A, Het B, Het C, R¹, R², R³, R⁴, Q, X, Y, Z, and n are as defined in the general formula (1), and the abbreviations Me, Et, Pr, Bu, and Ph respectively denote a methyl group, an ethyl group, a propyl group, a butyl group, and a phenyl group.

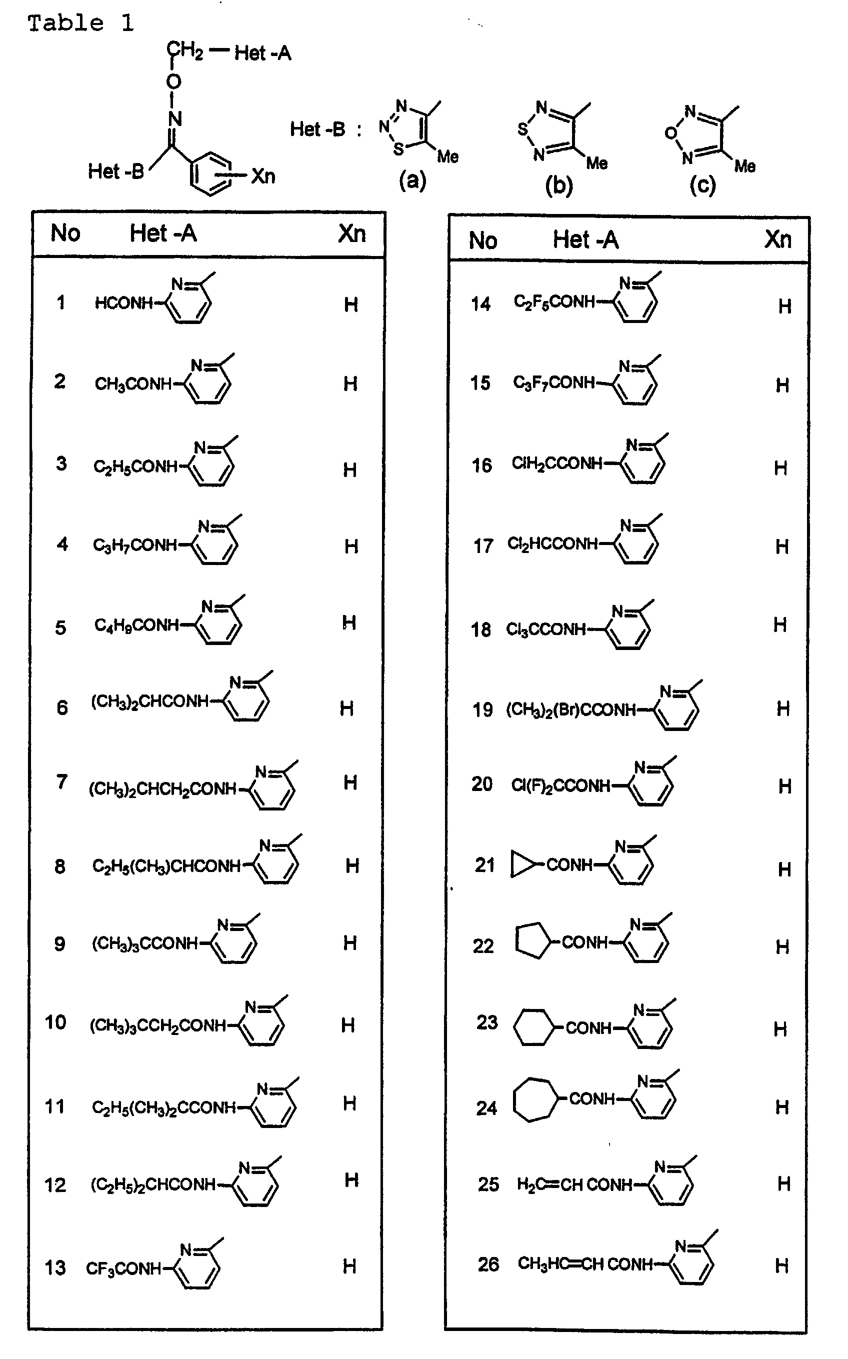

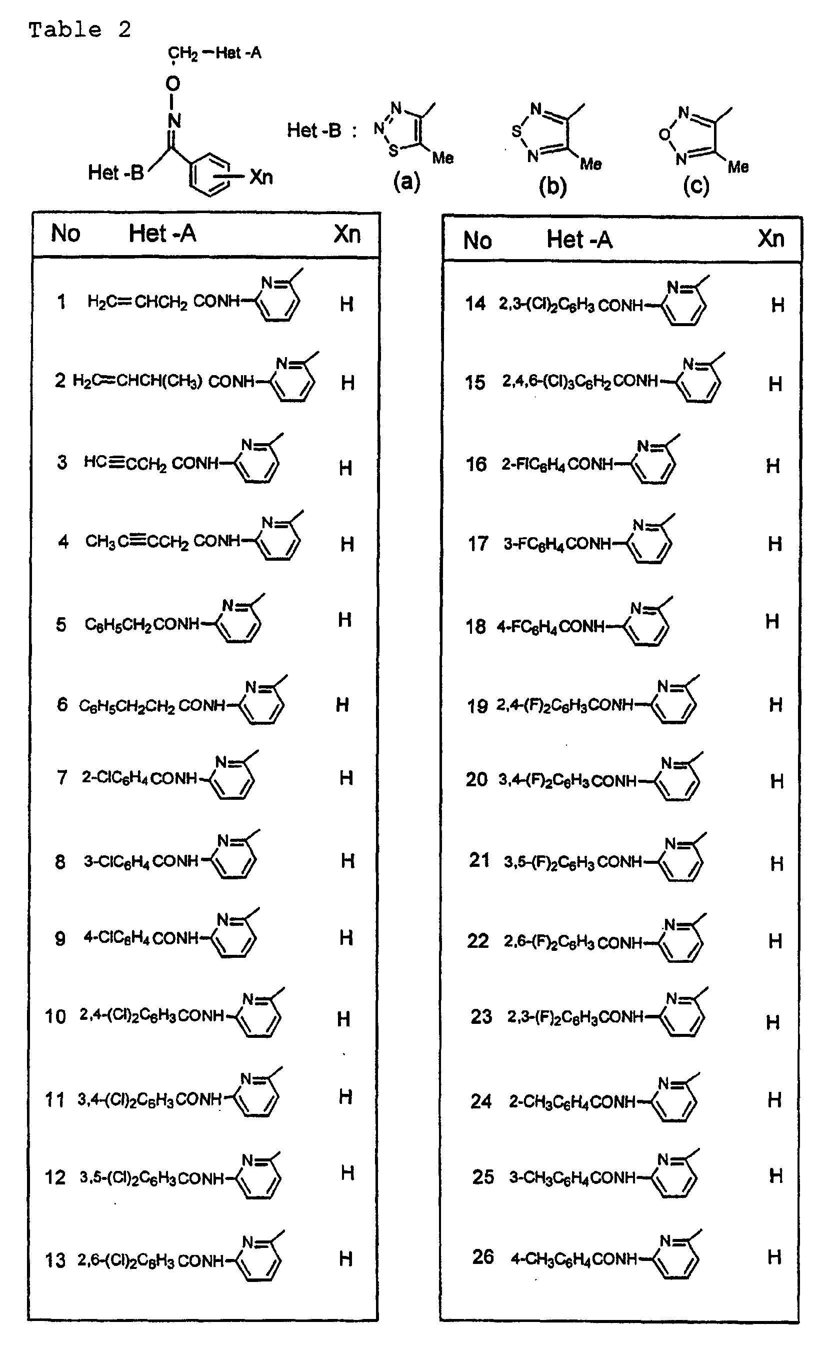

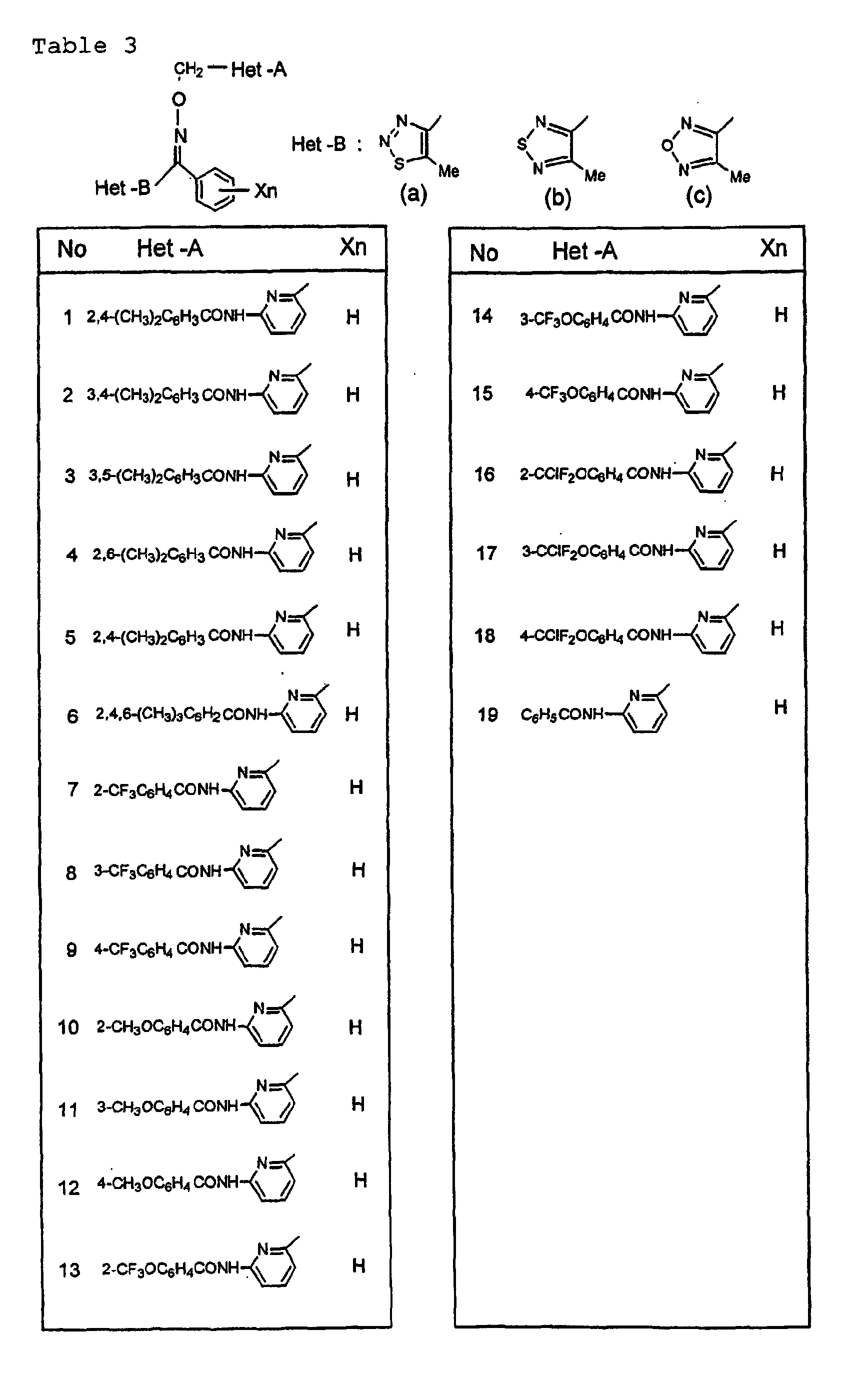

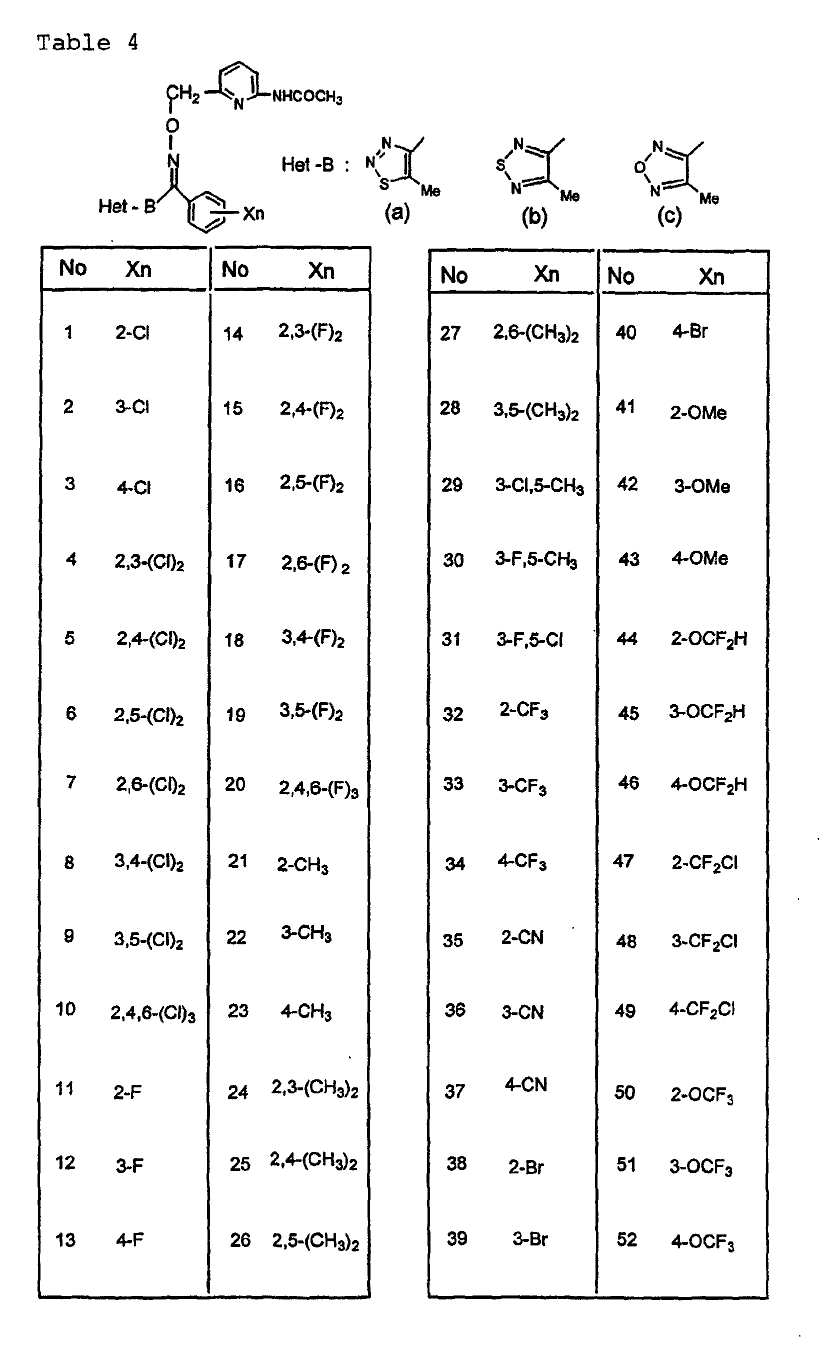

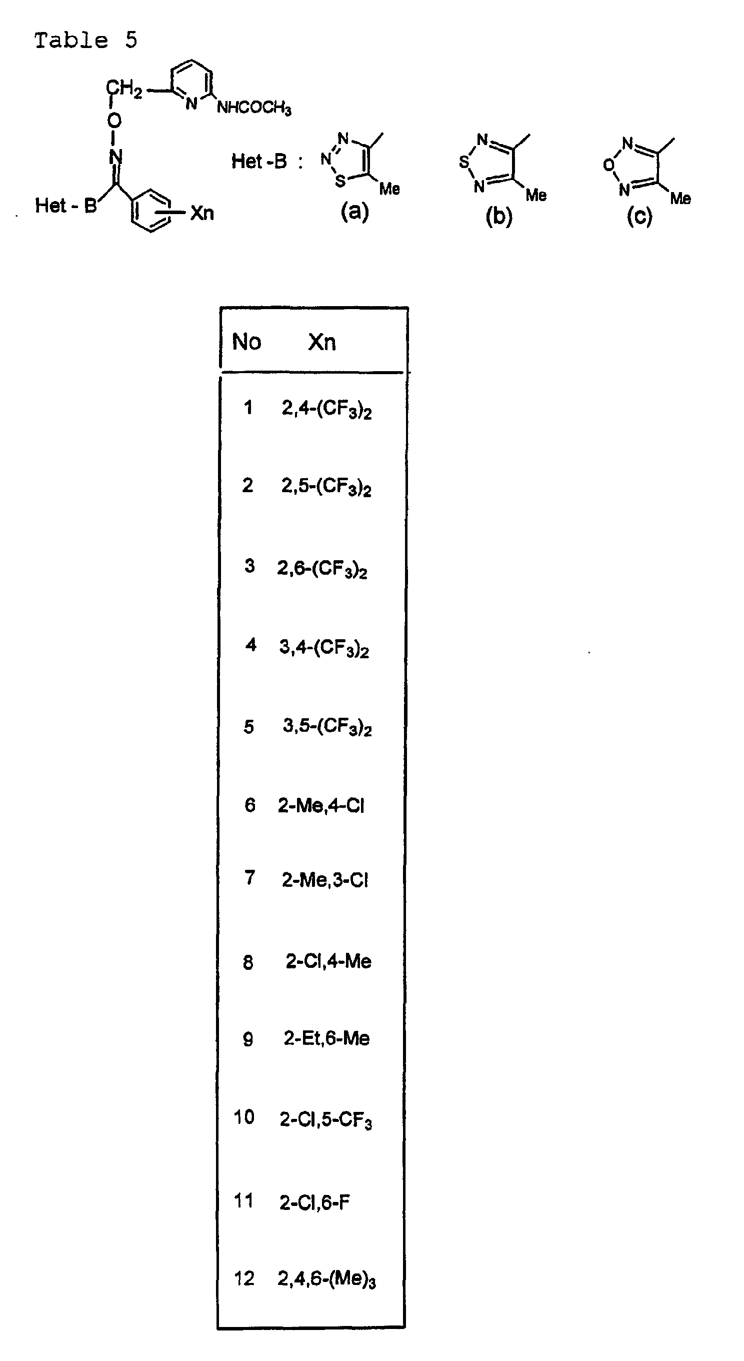

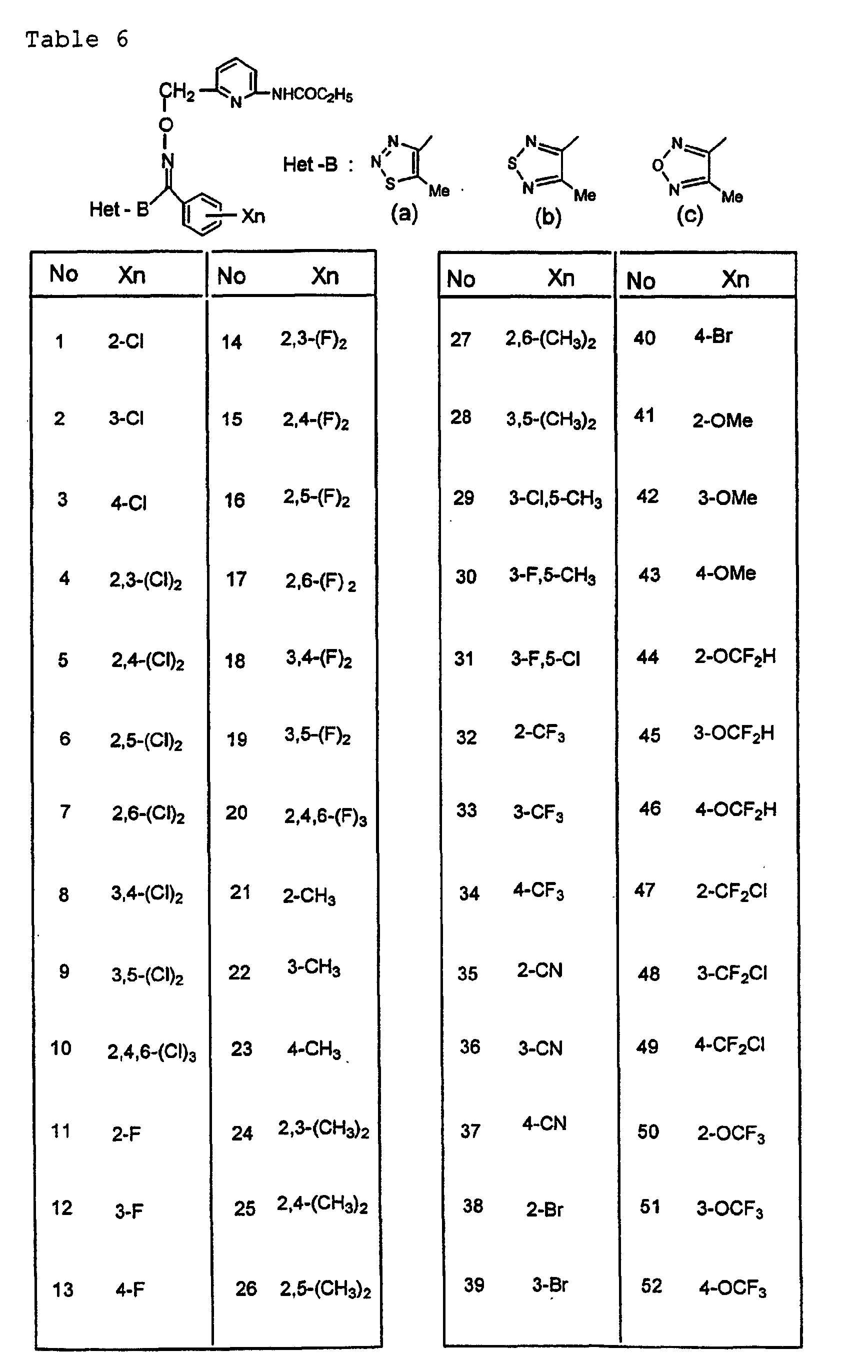

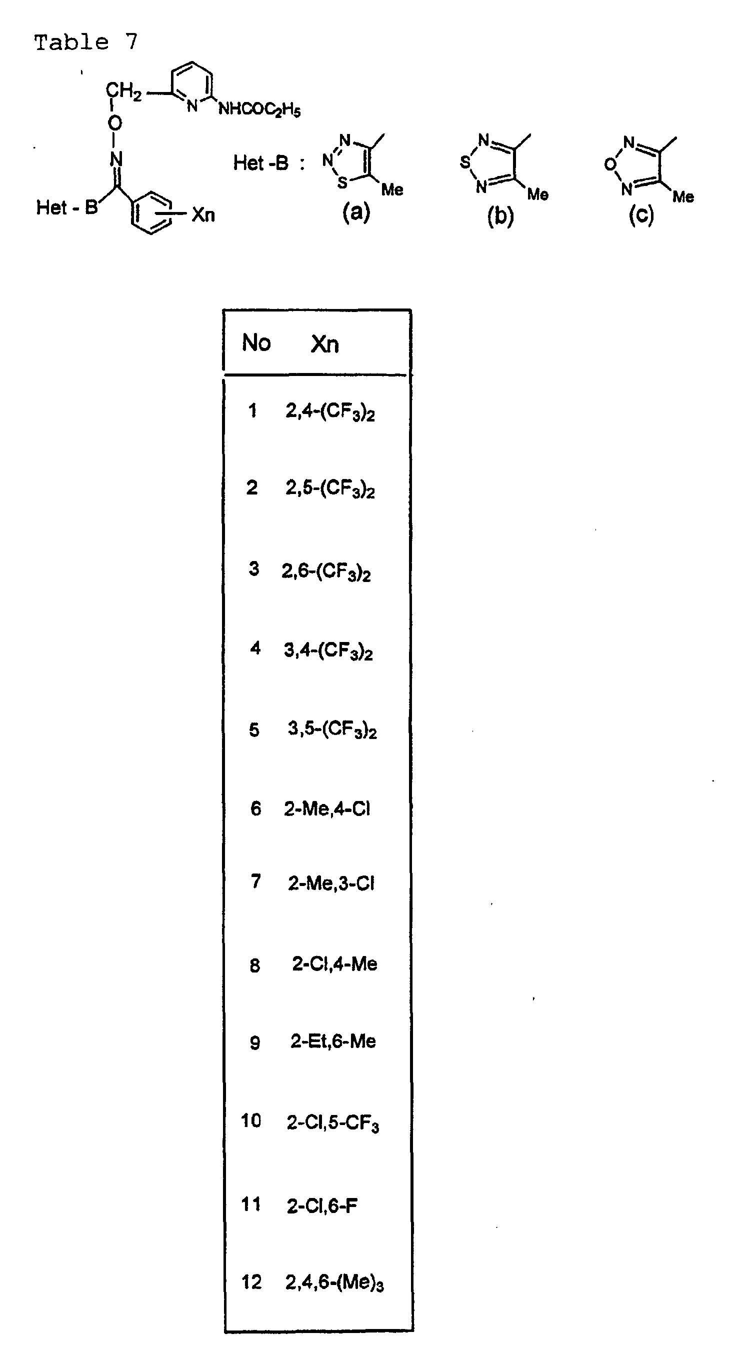

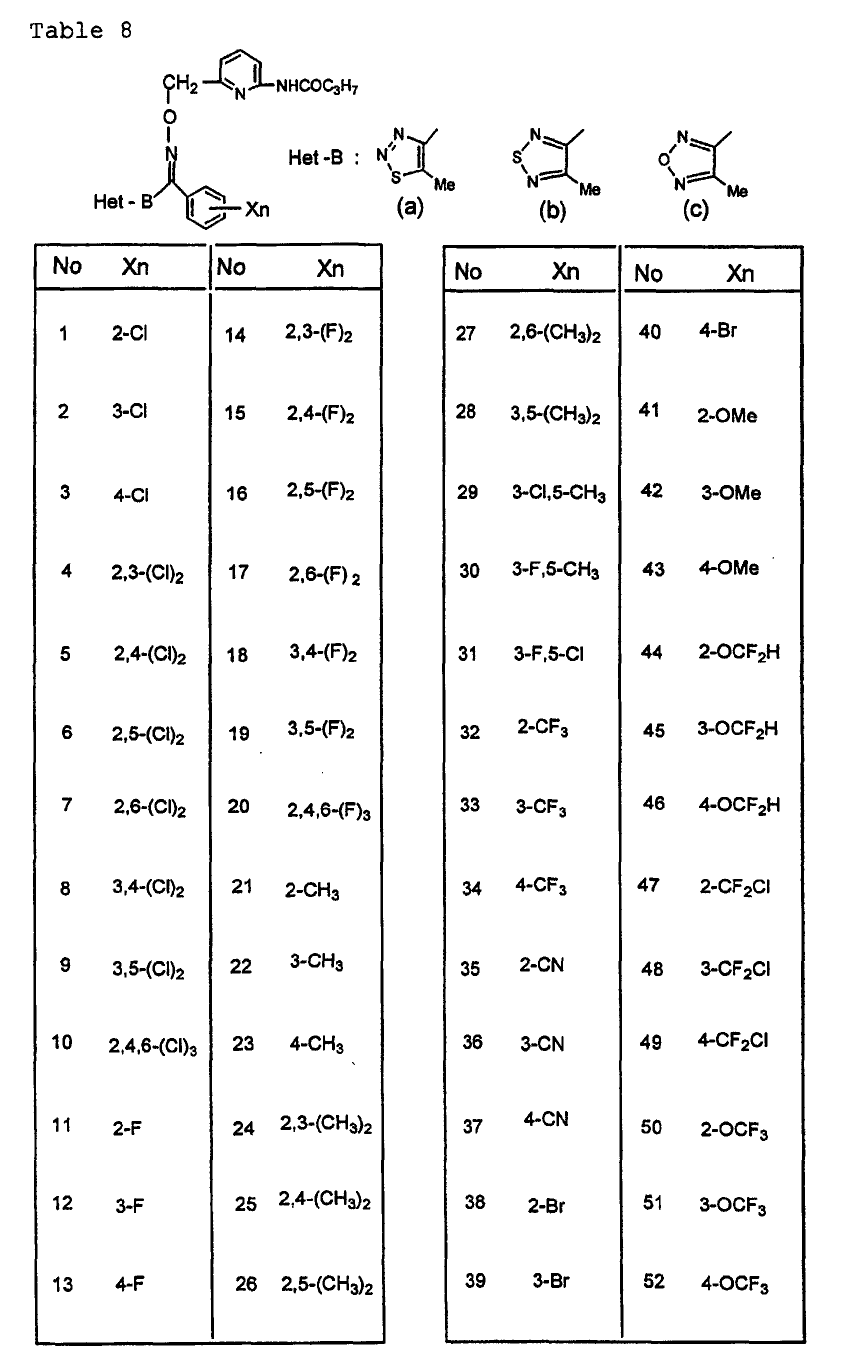

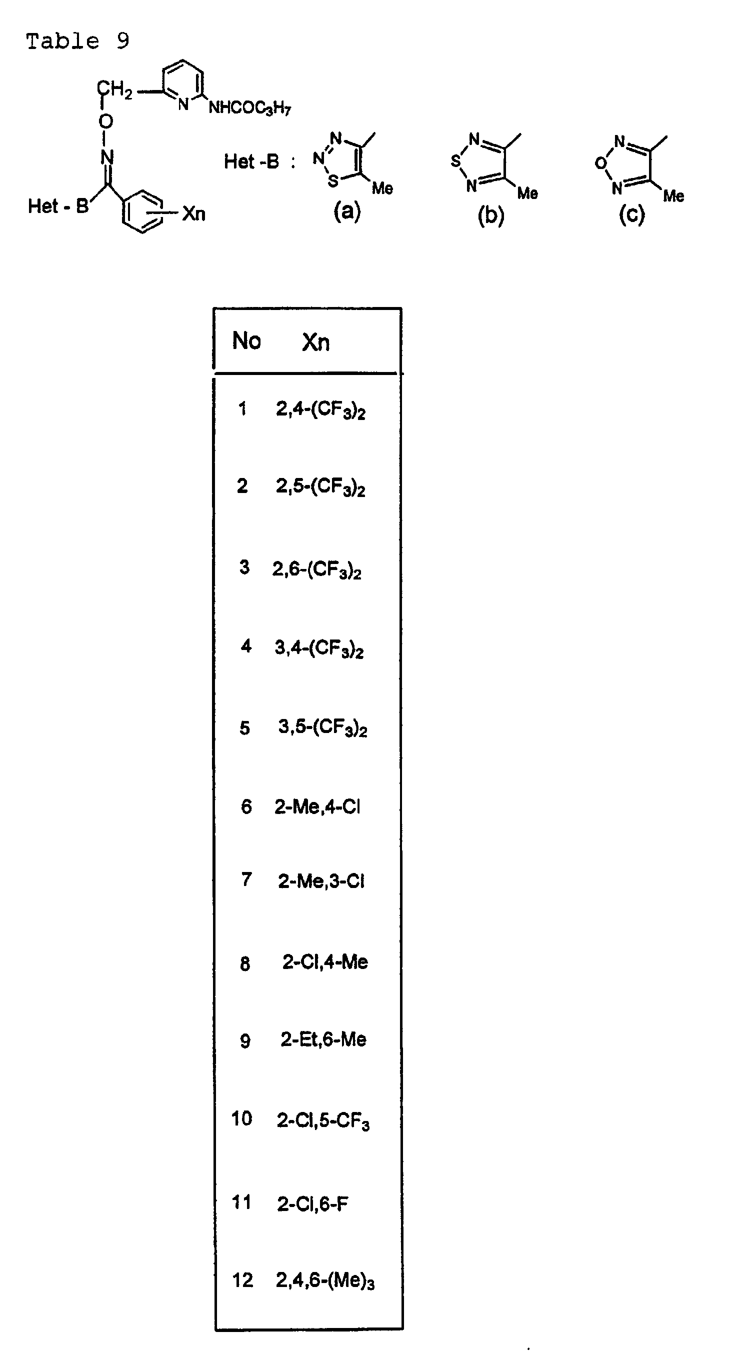

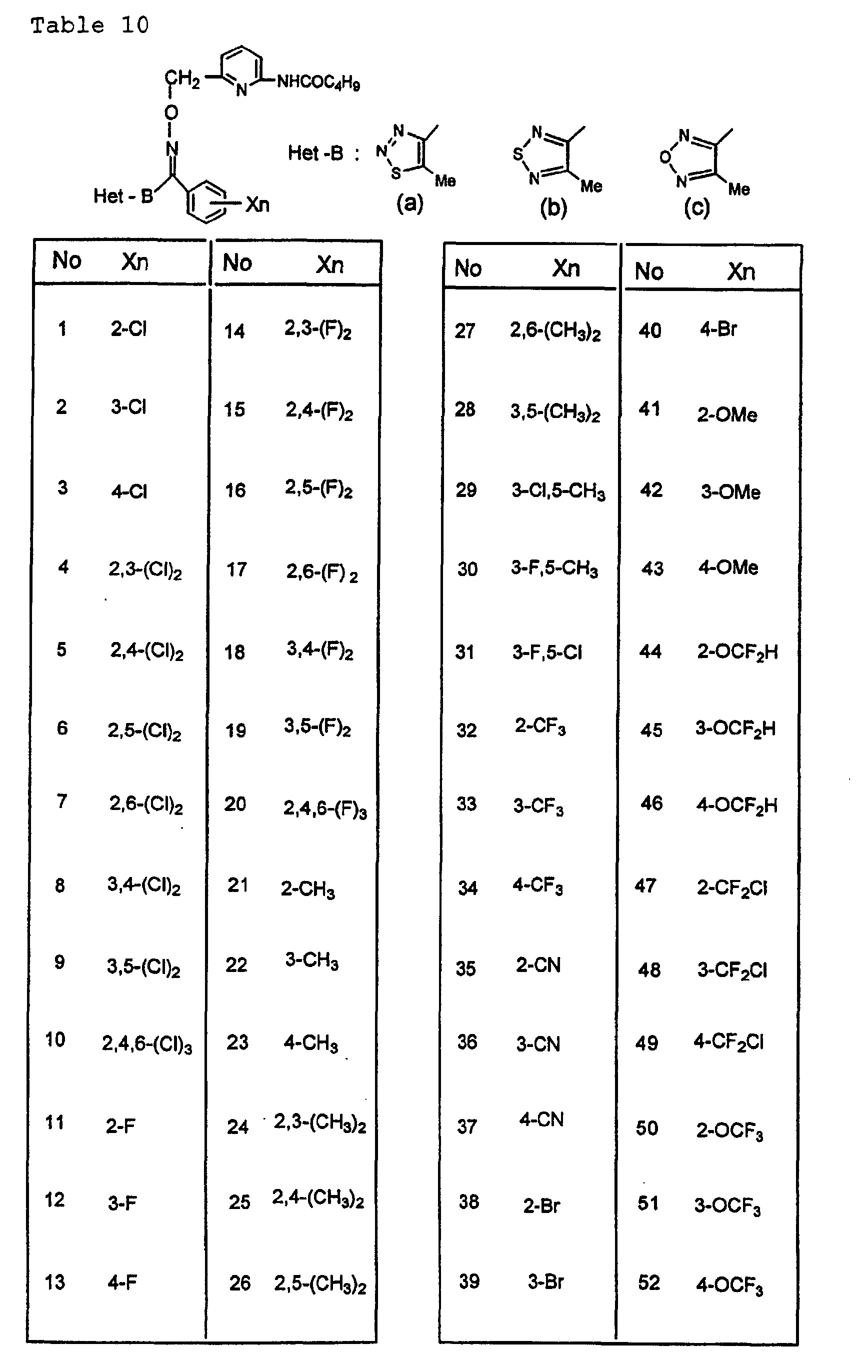

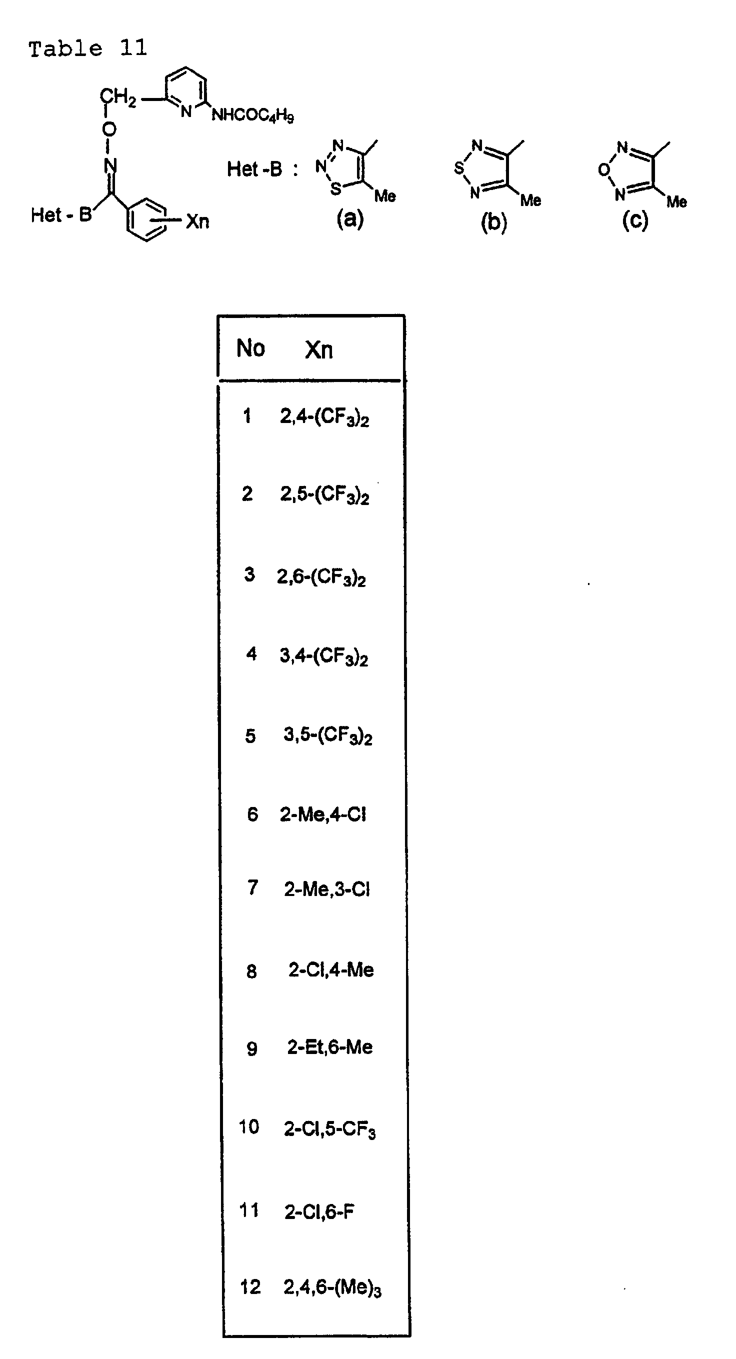

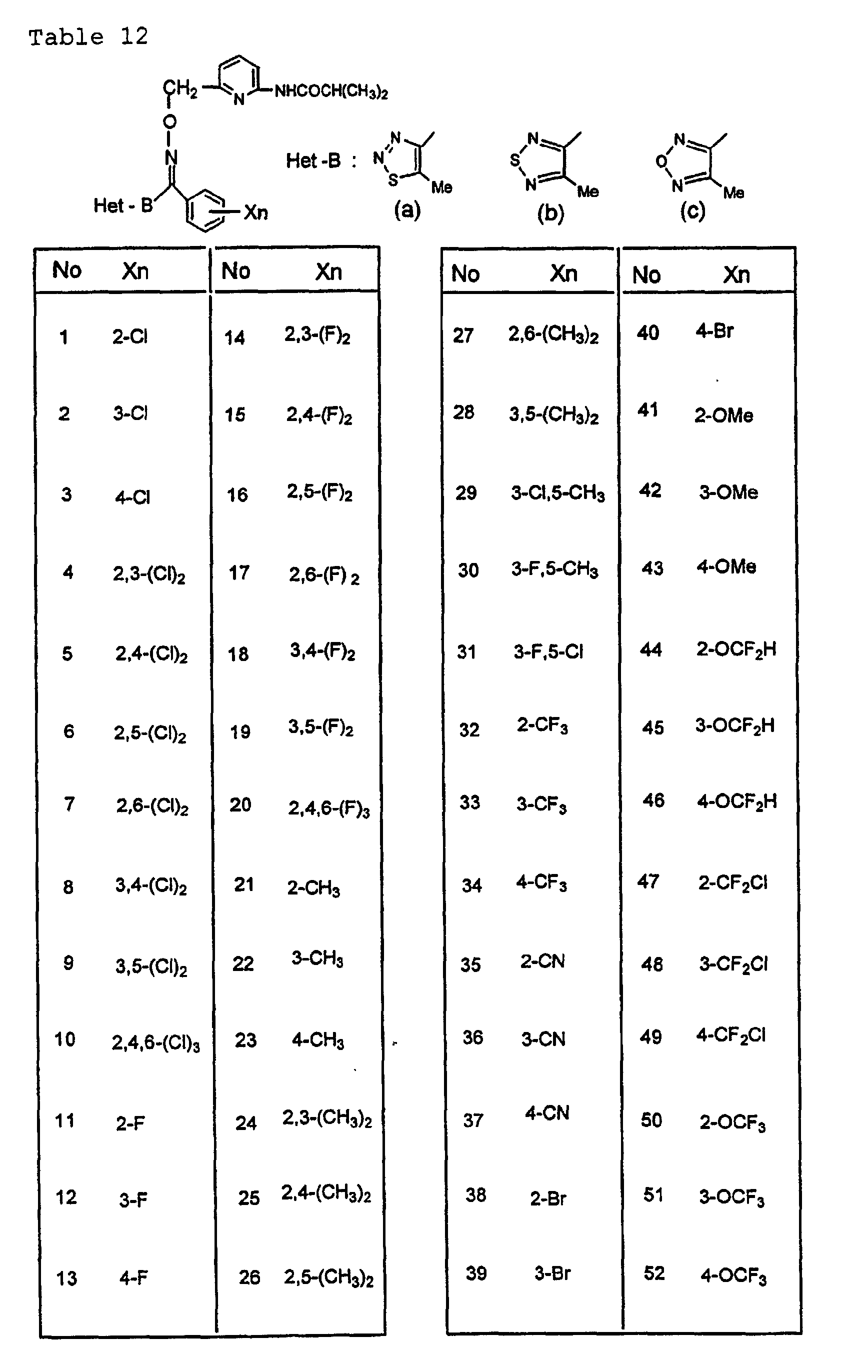

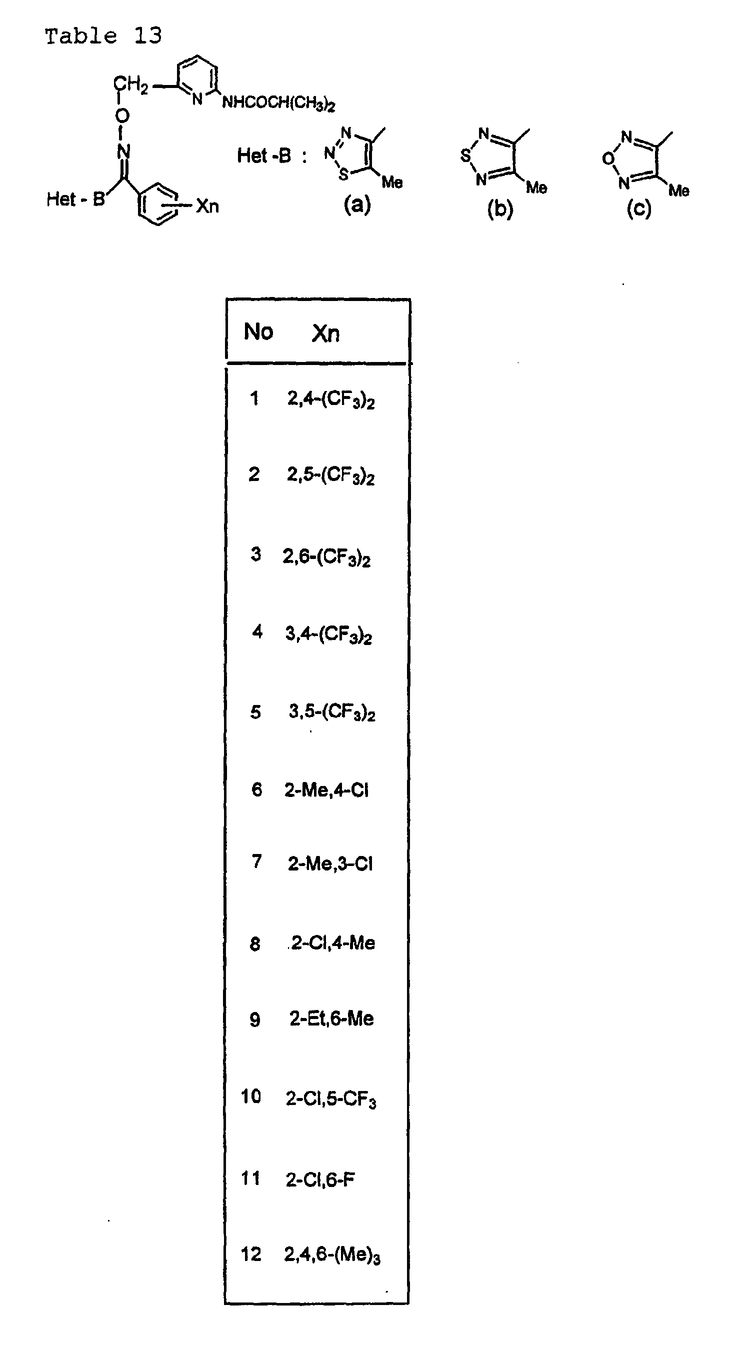

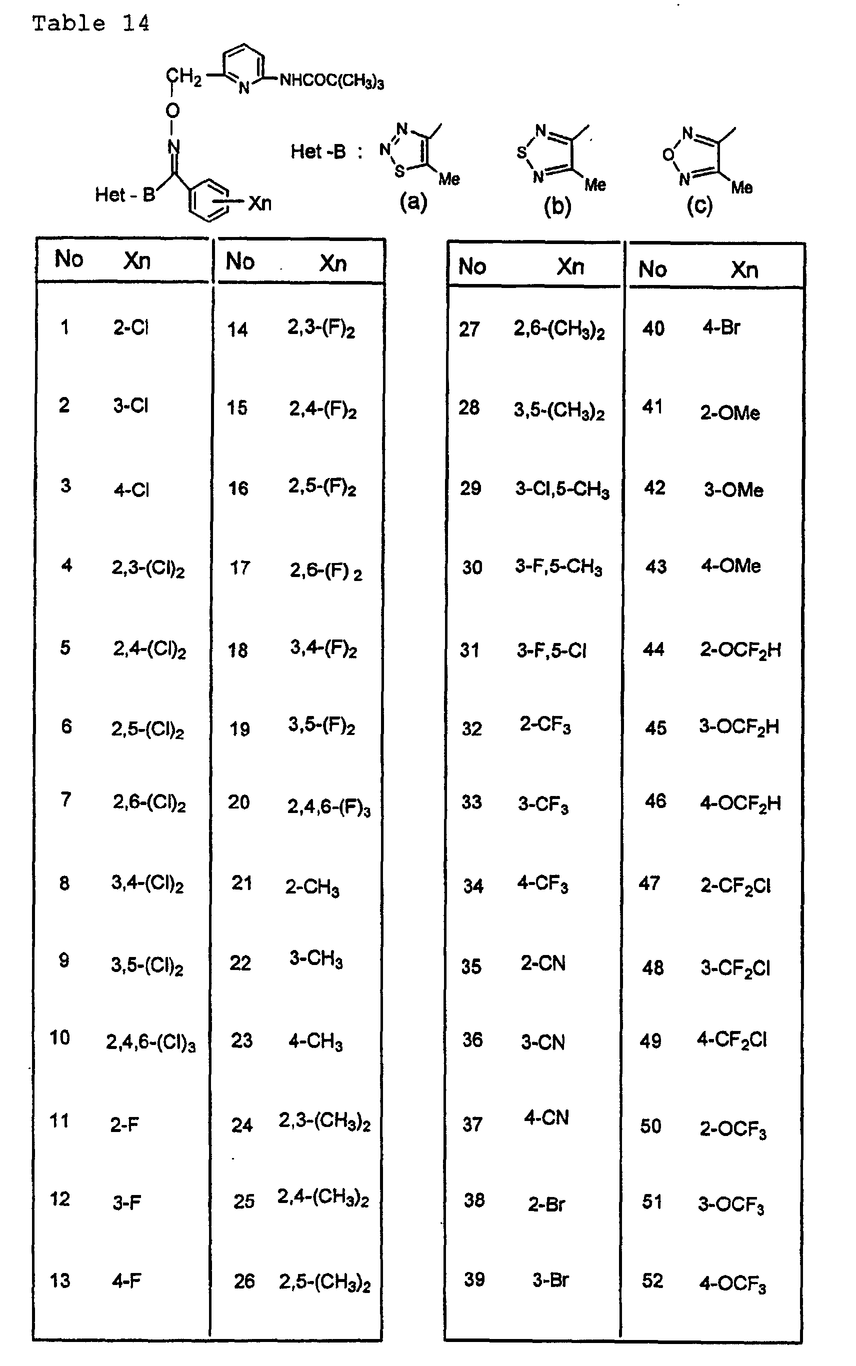

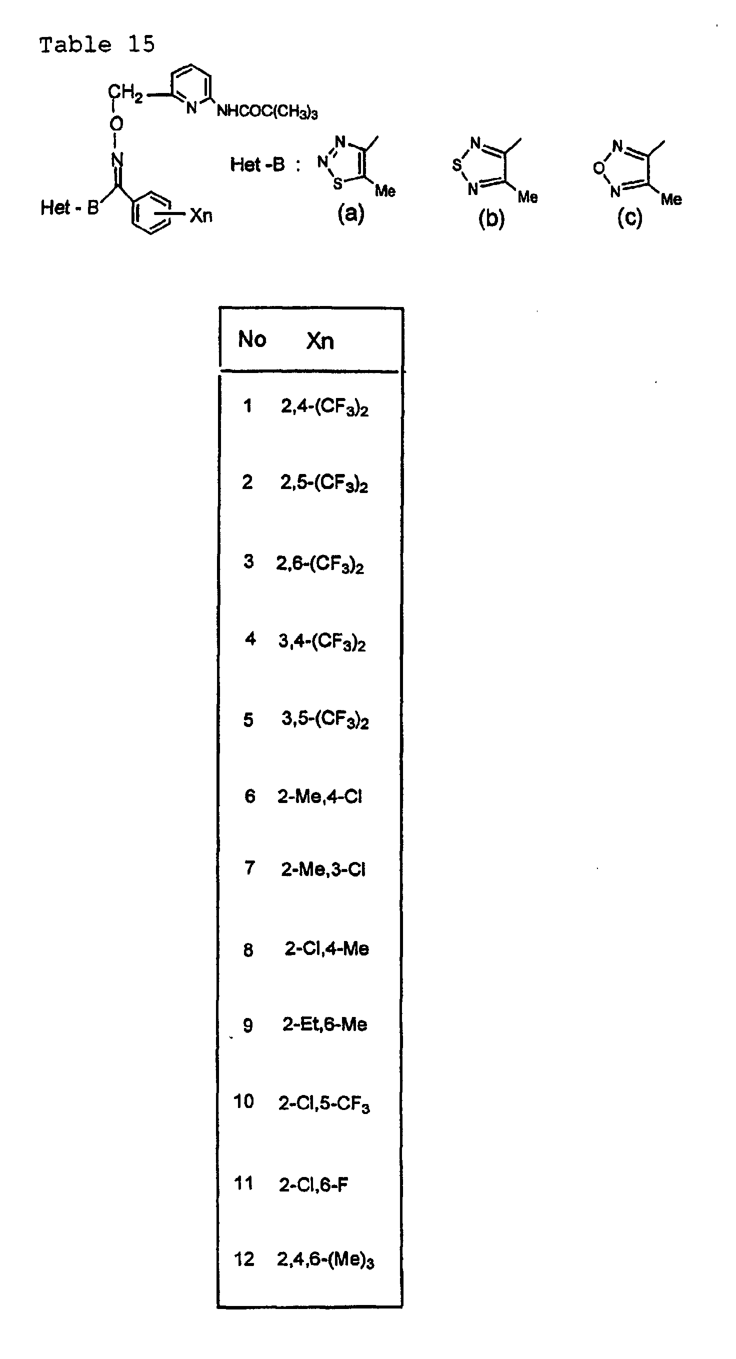

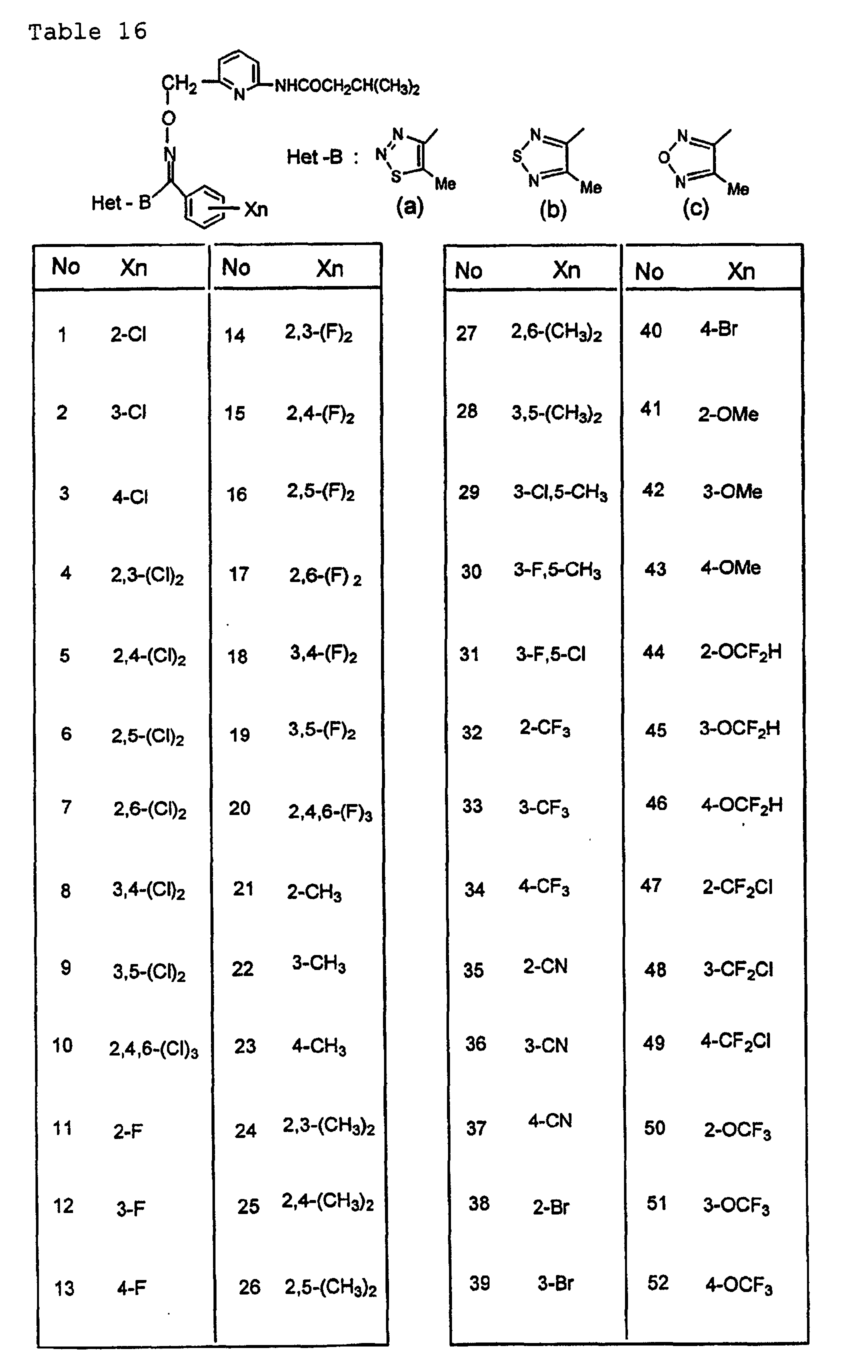

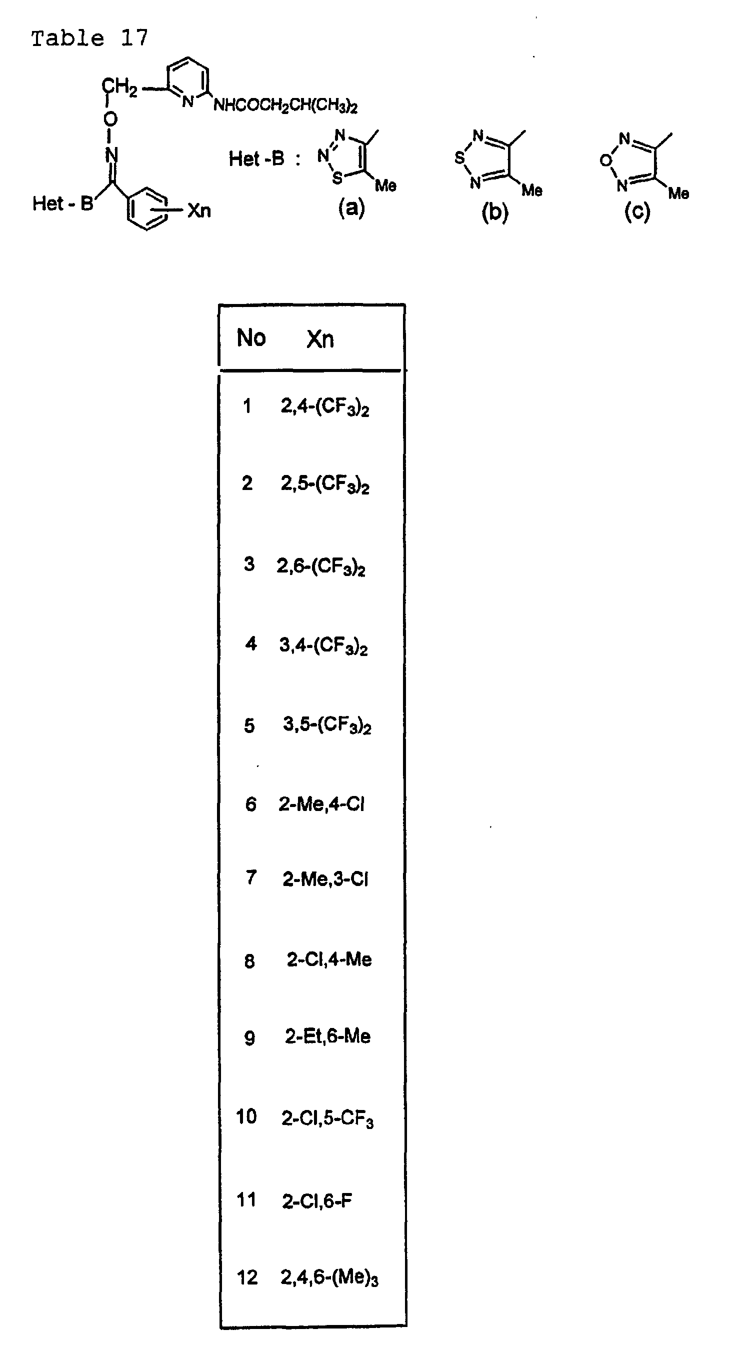

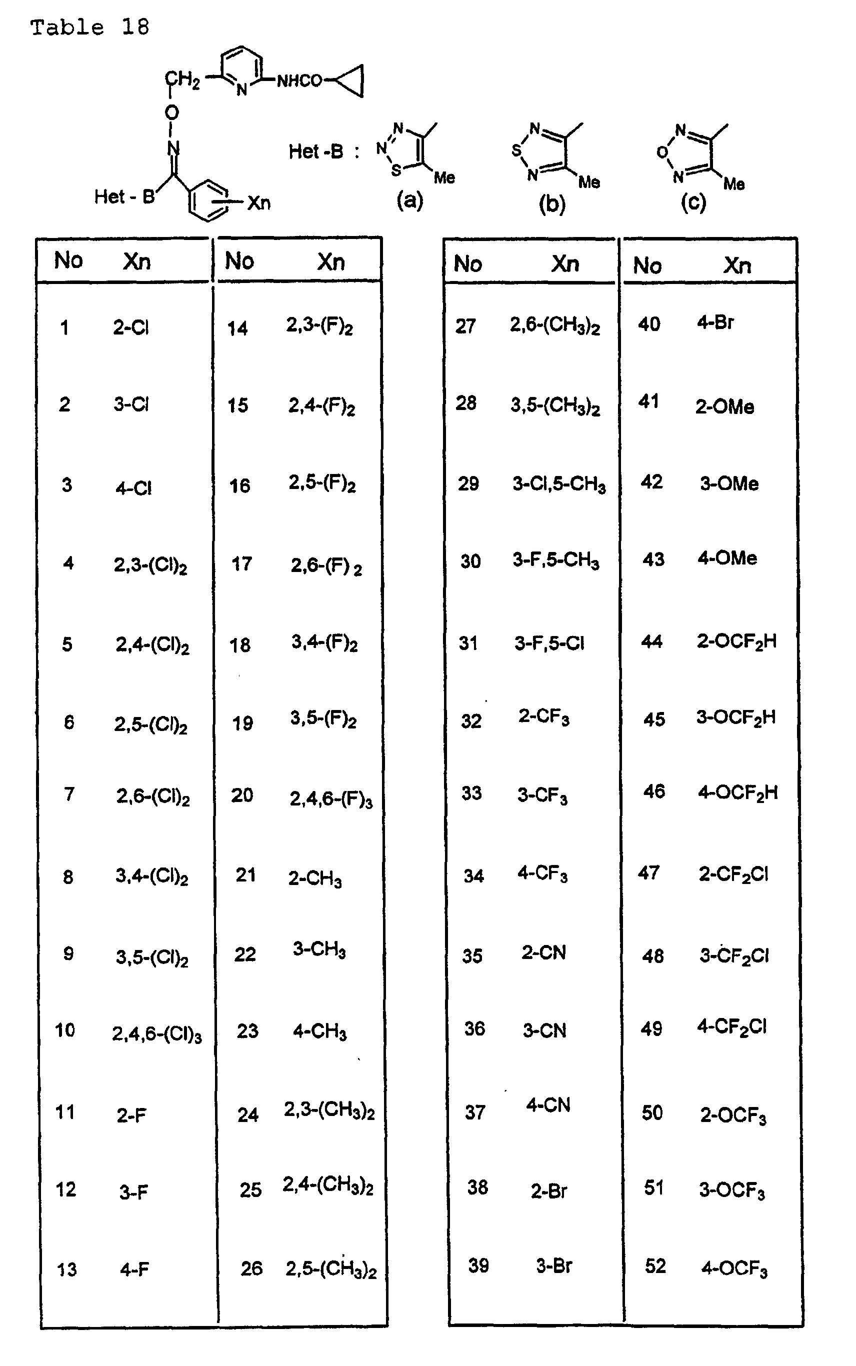

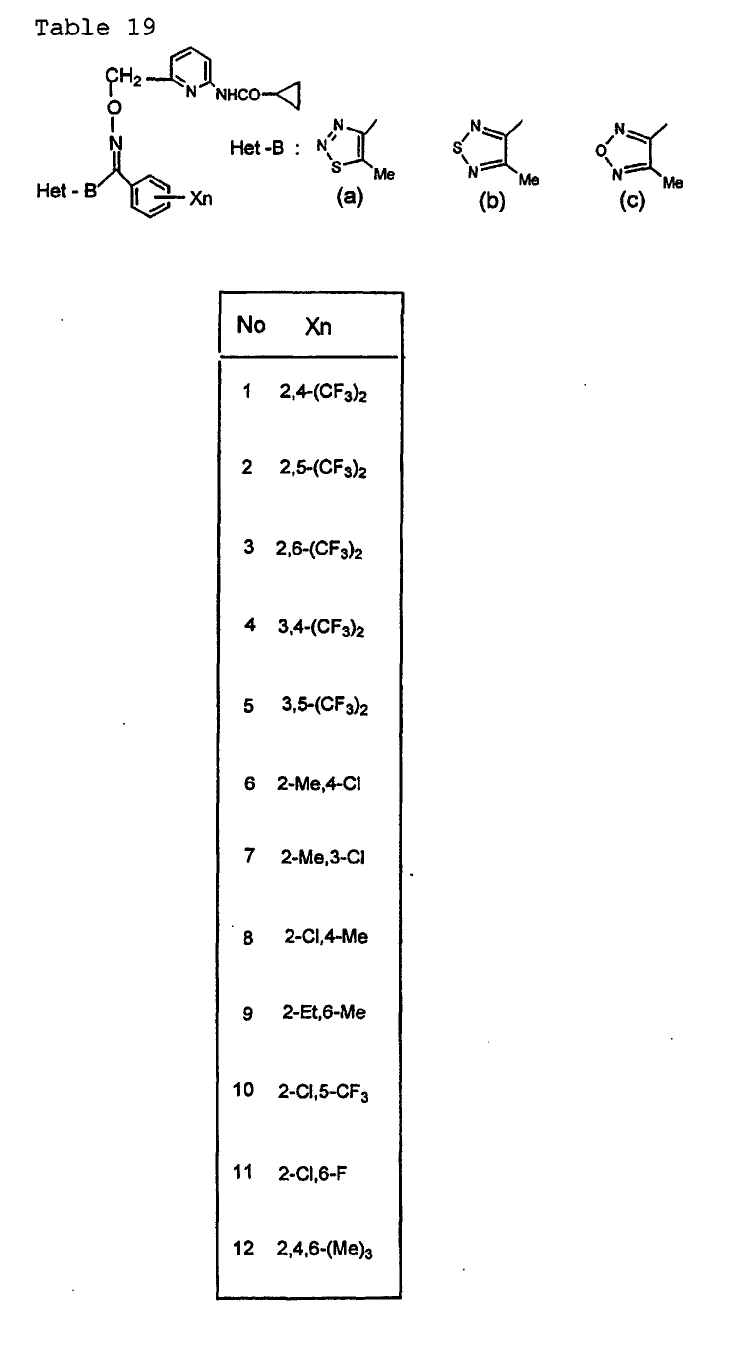

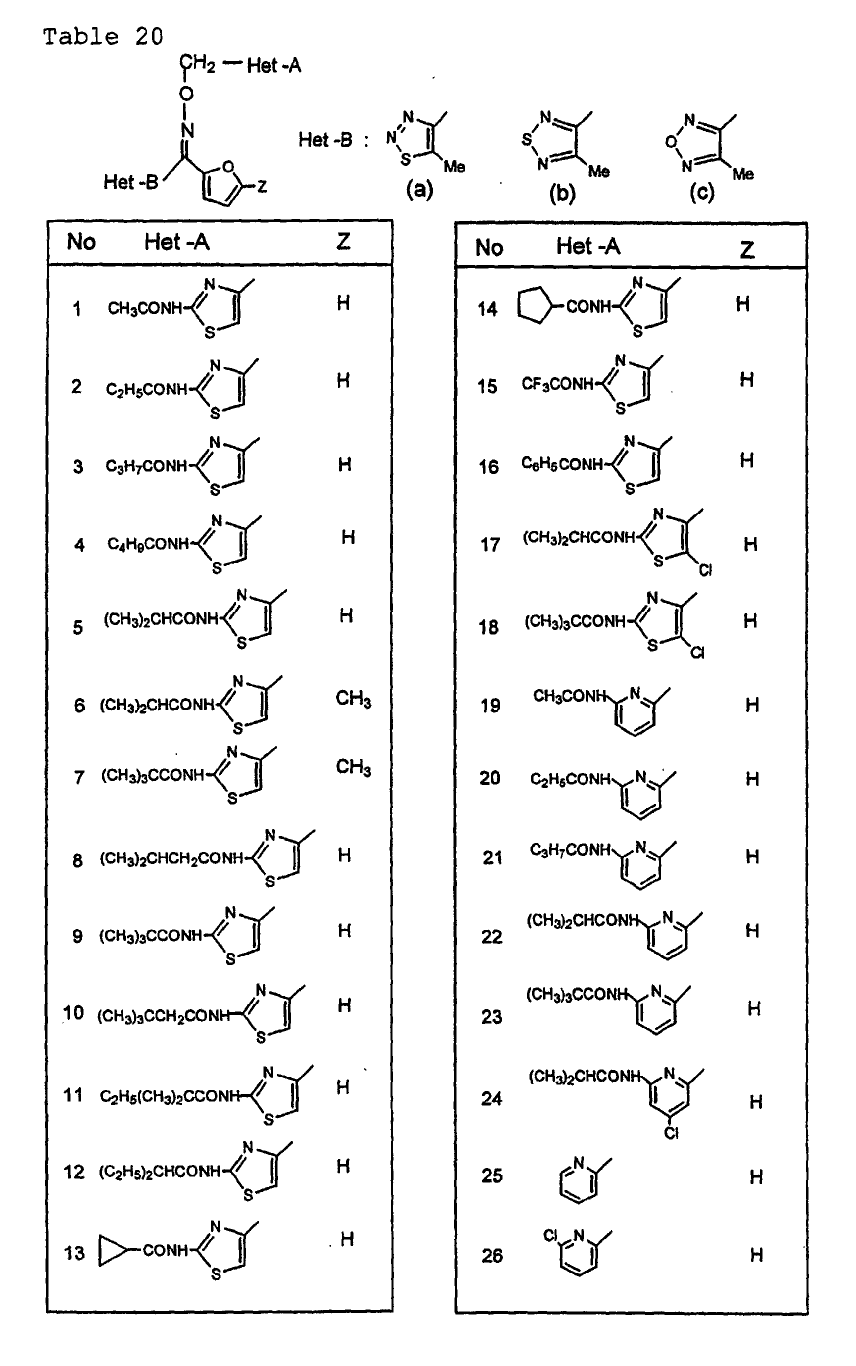

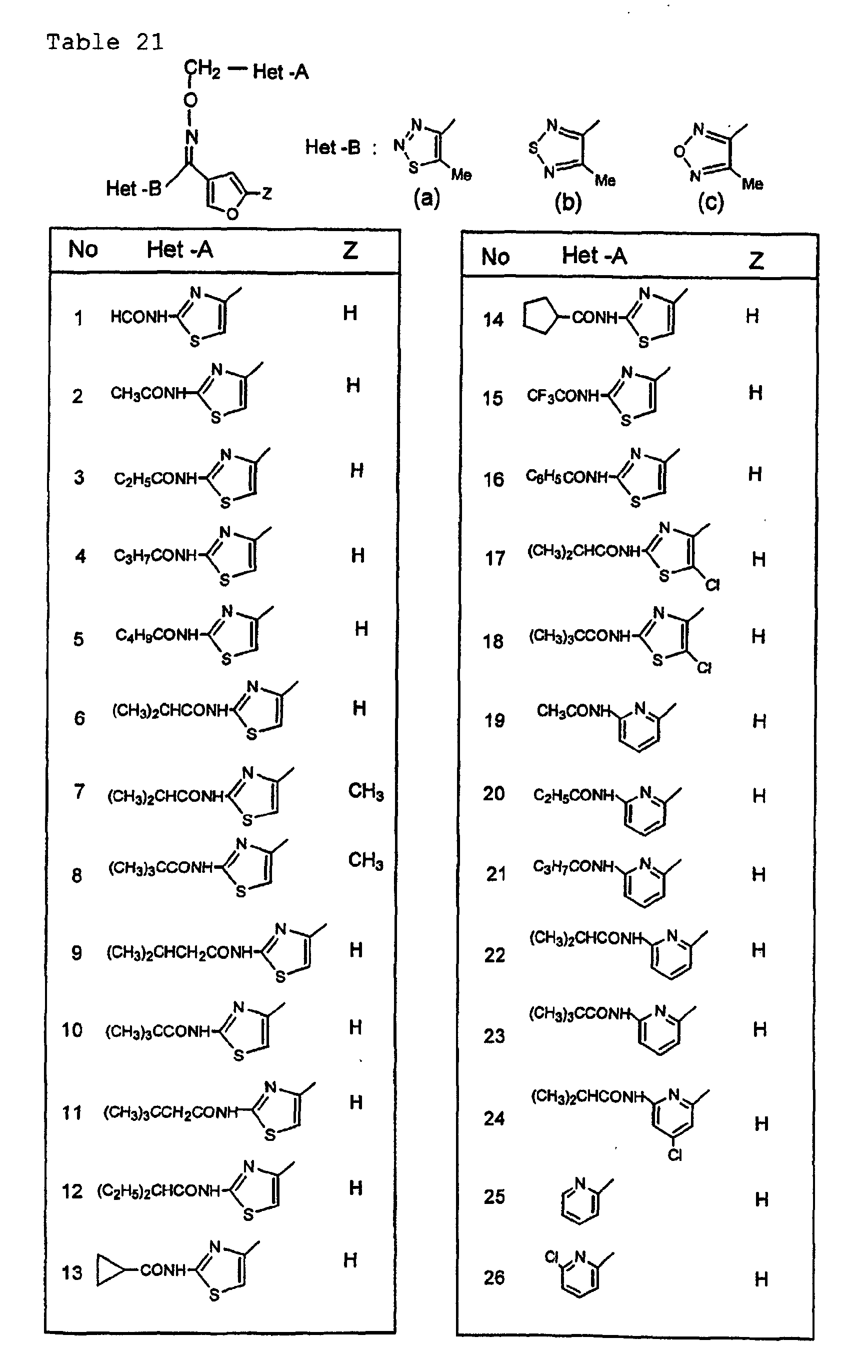

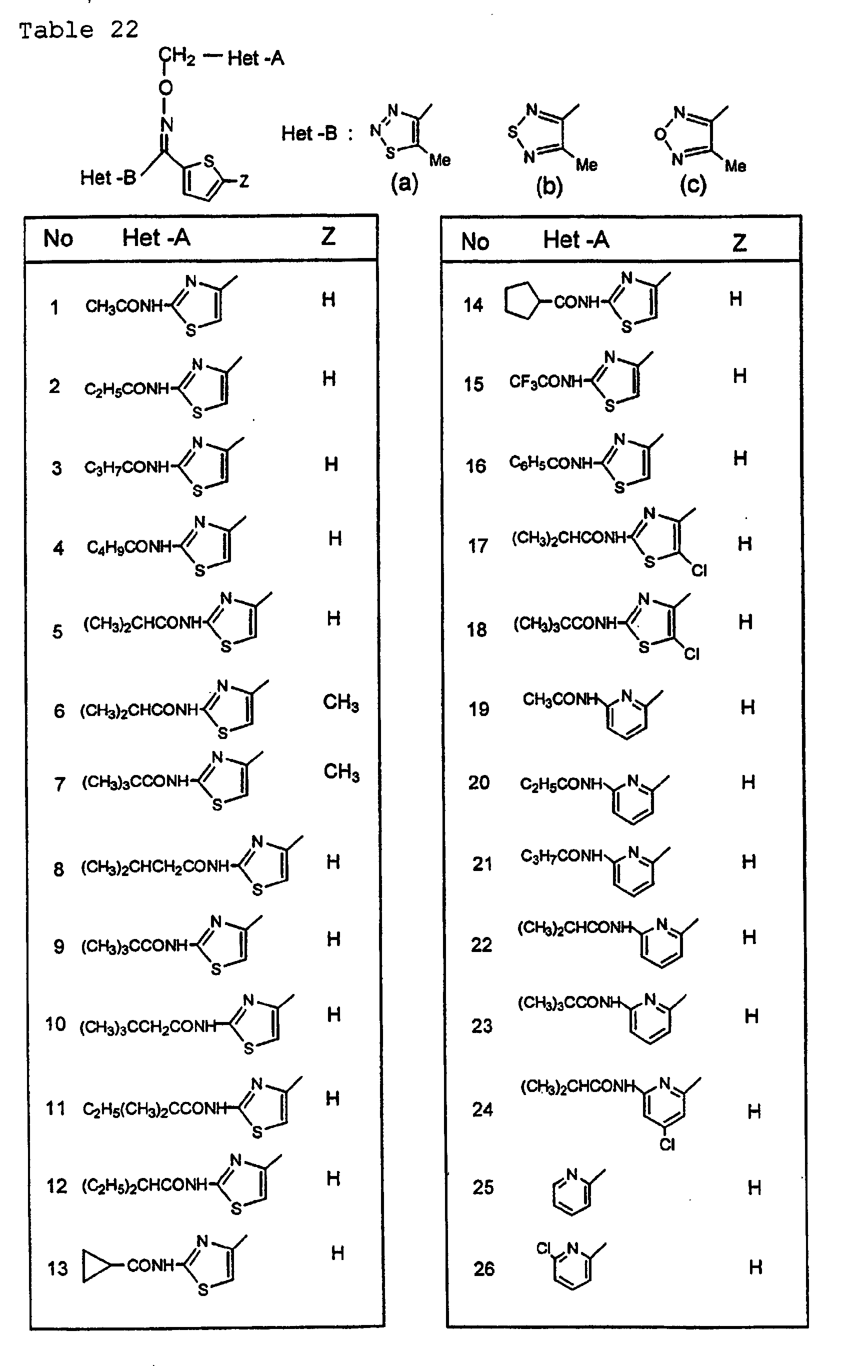

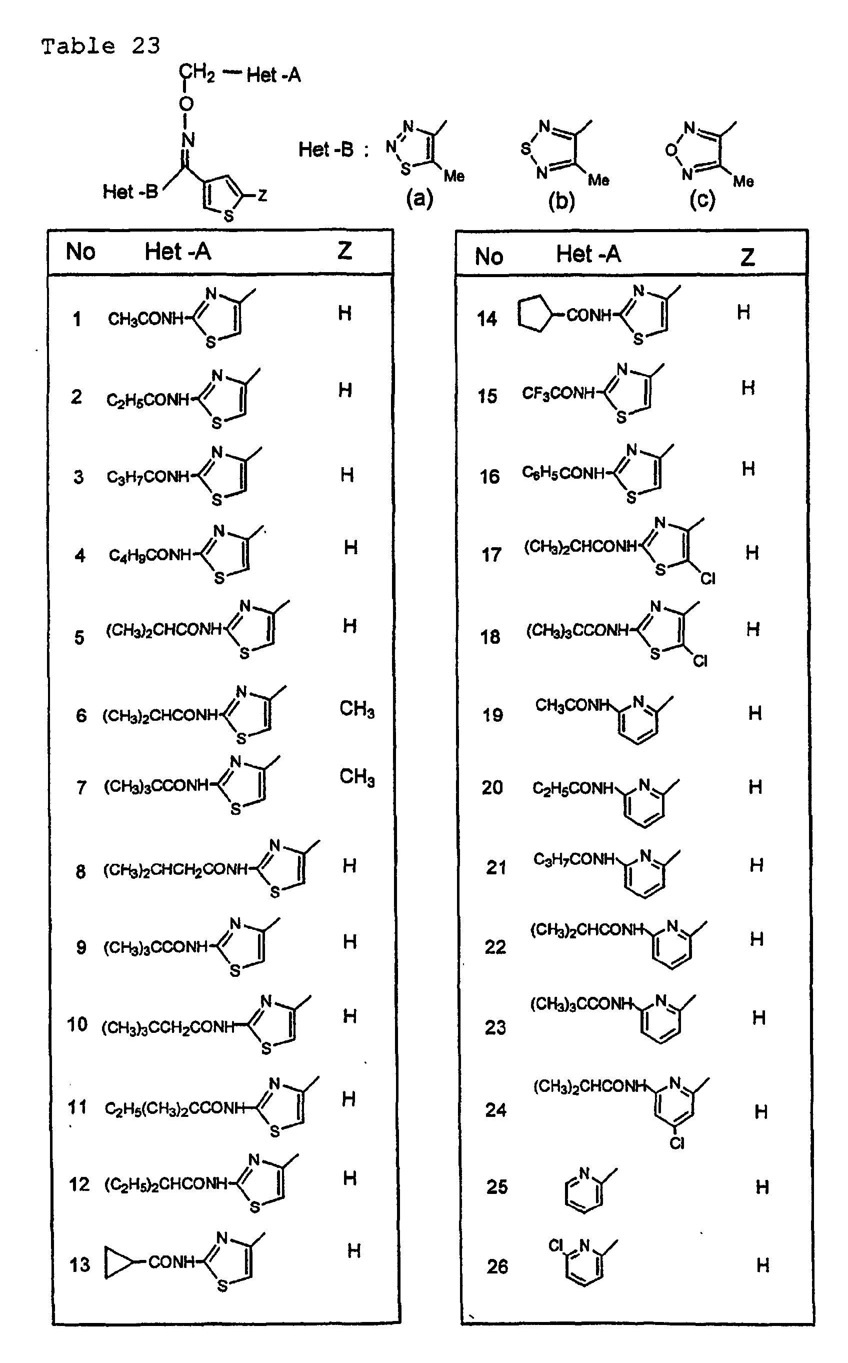

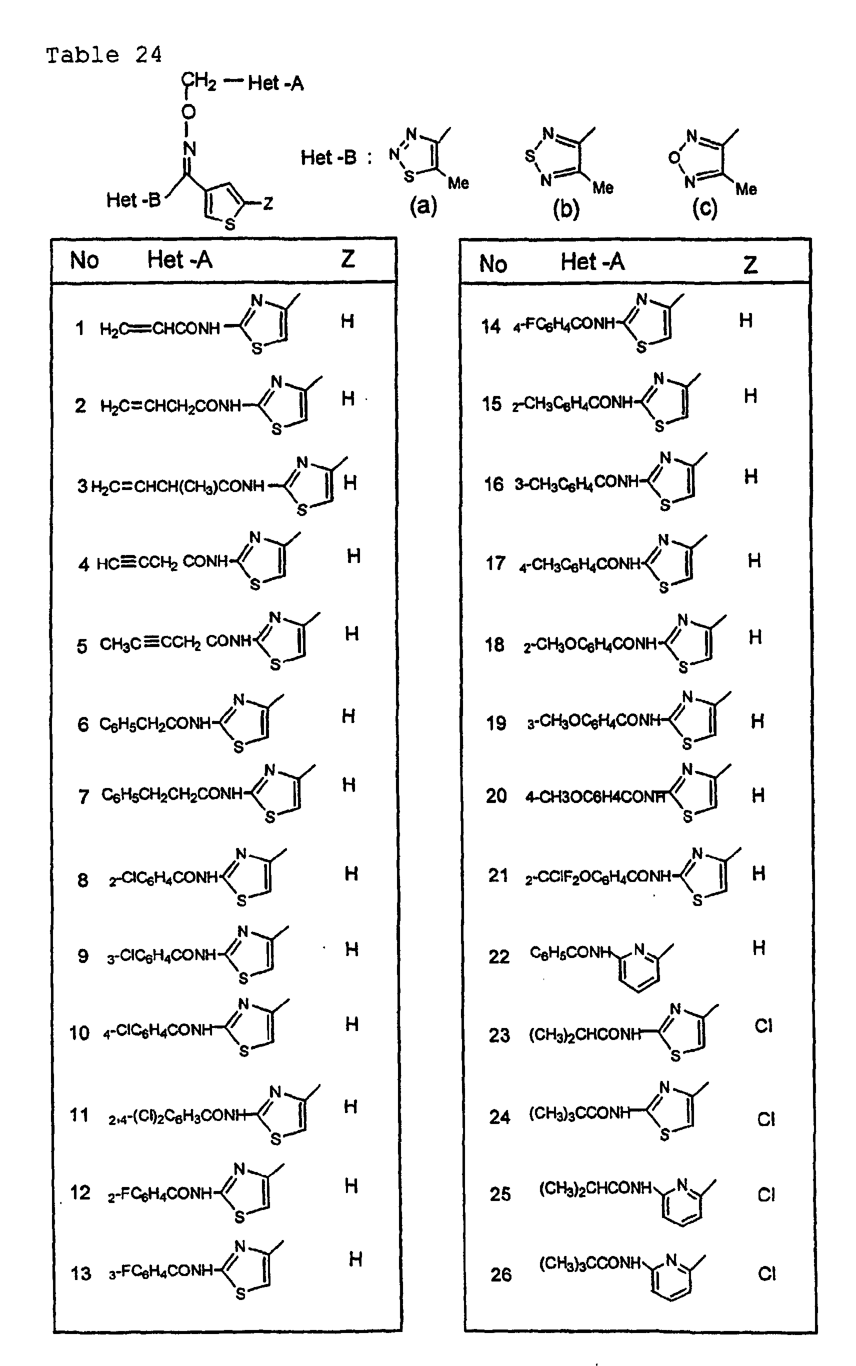

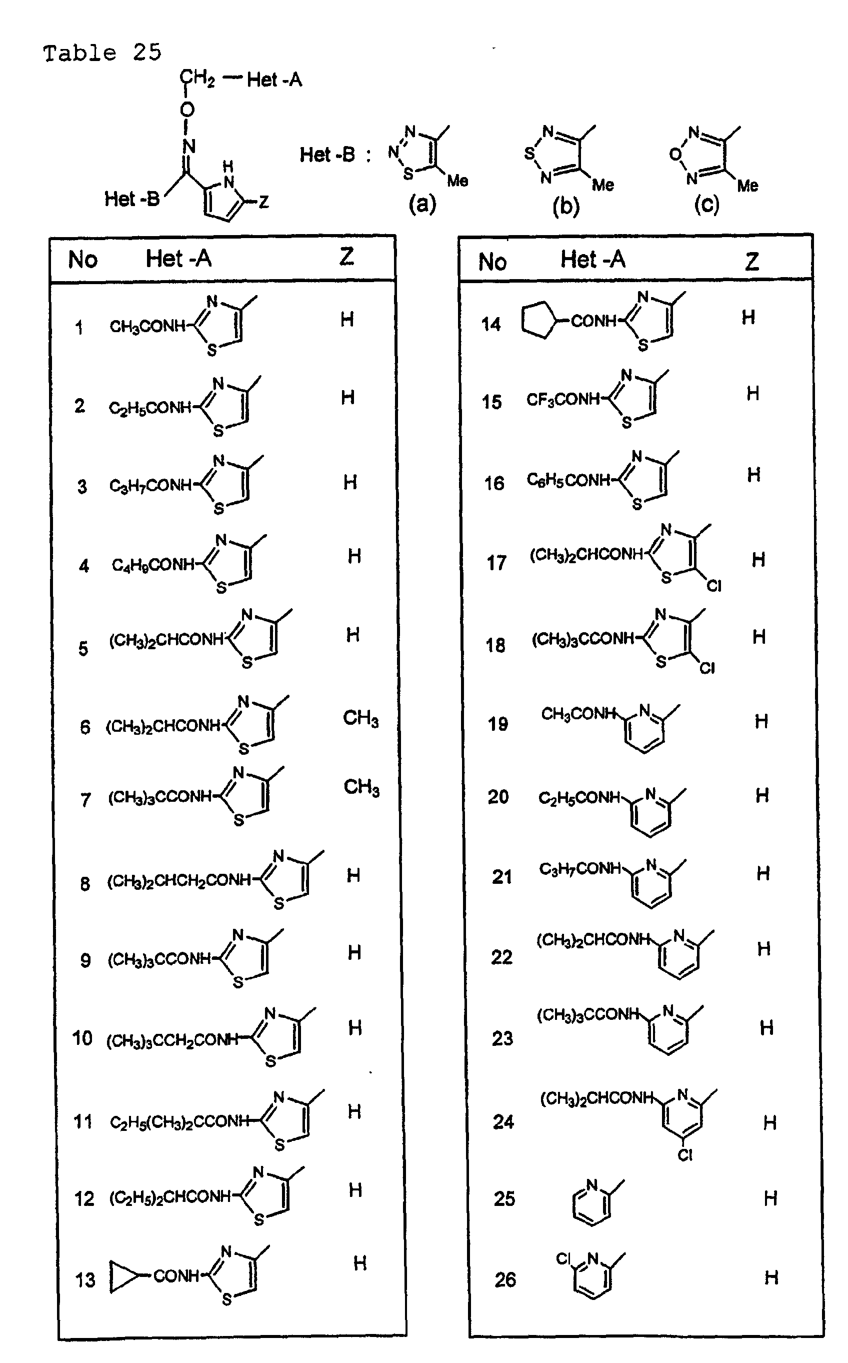

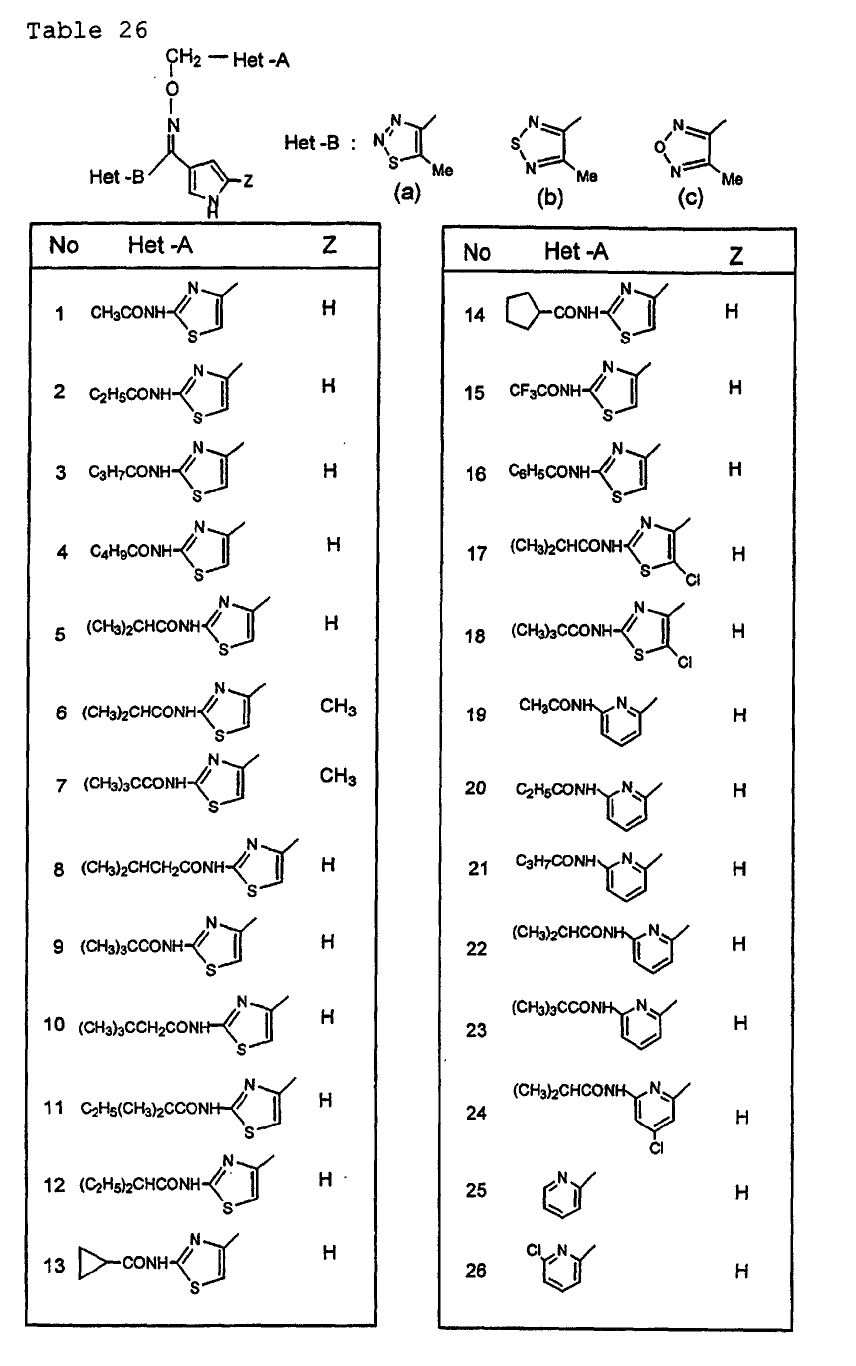

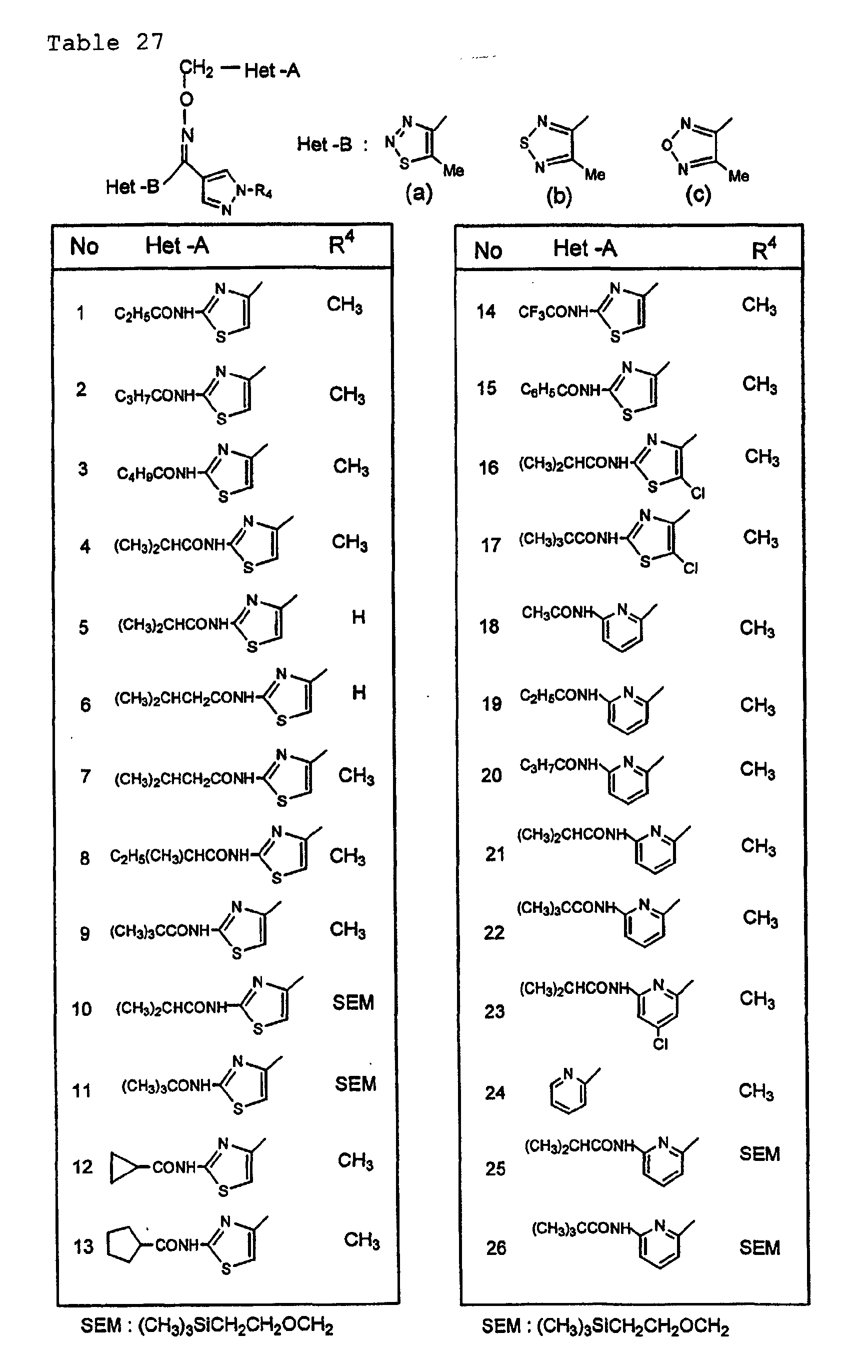

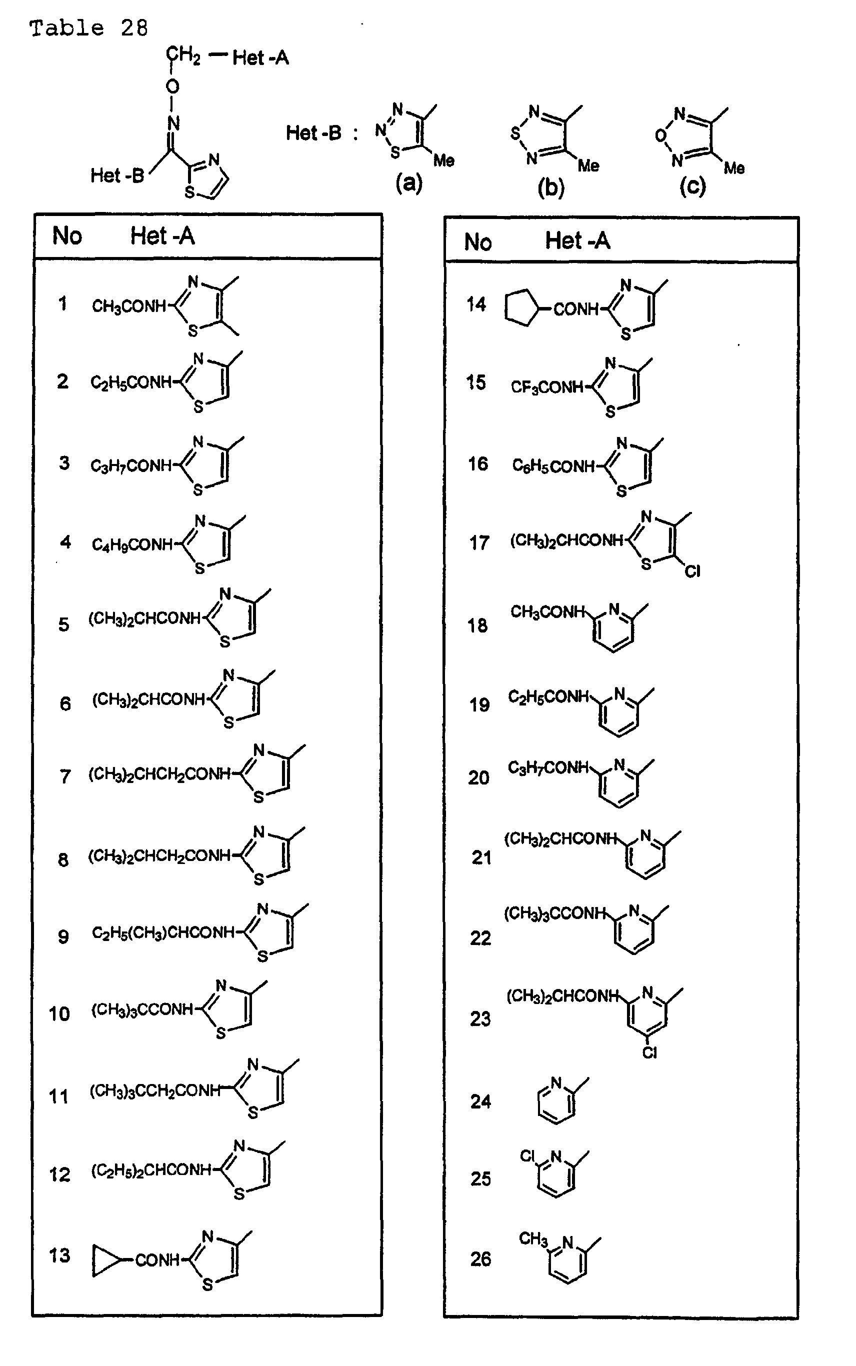

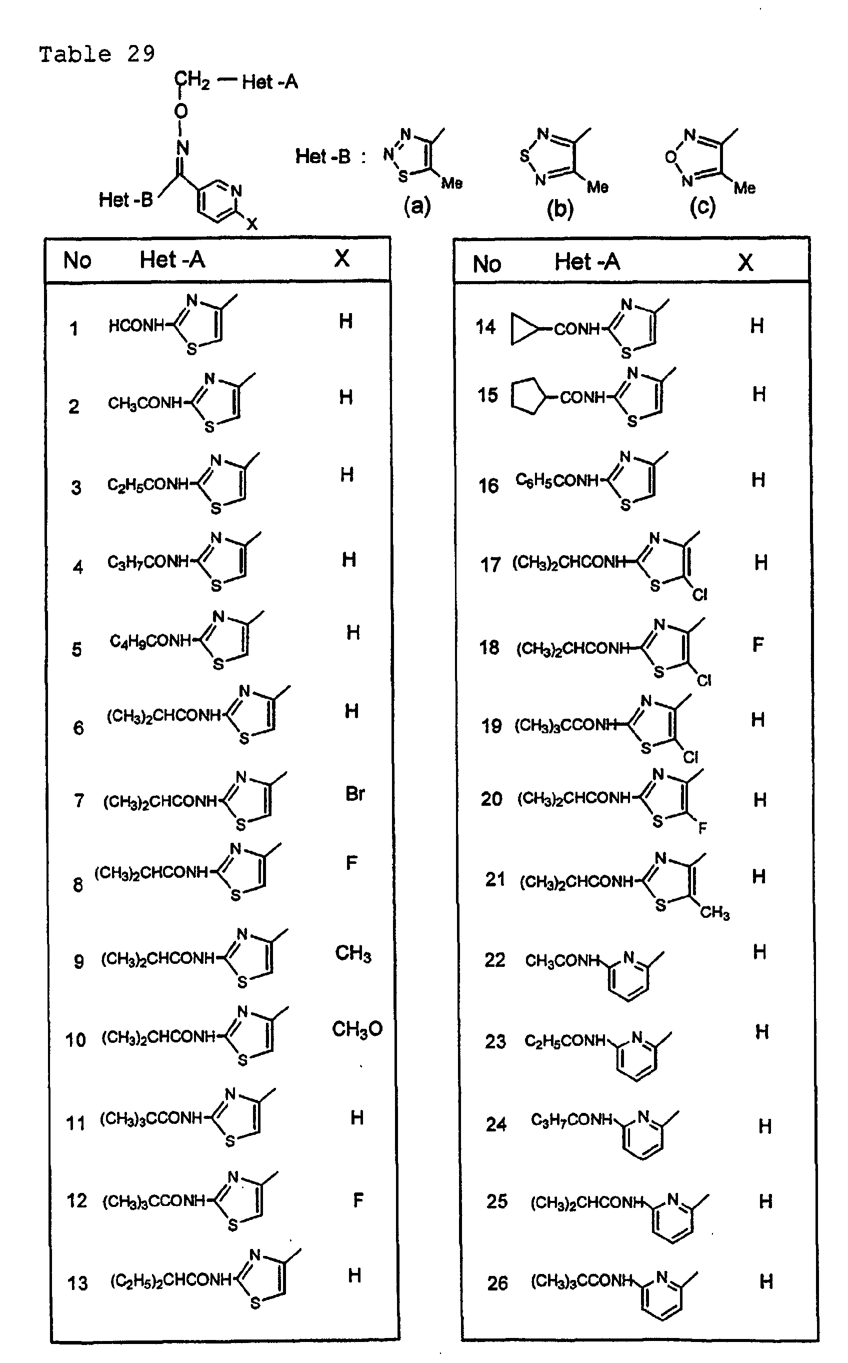

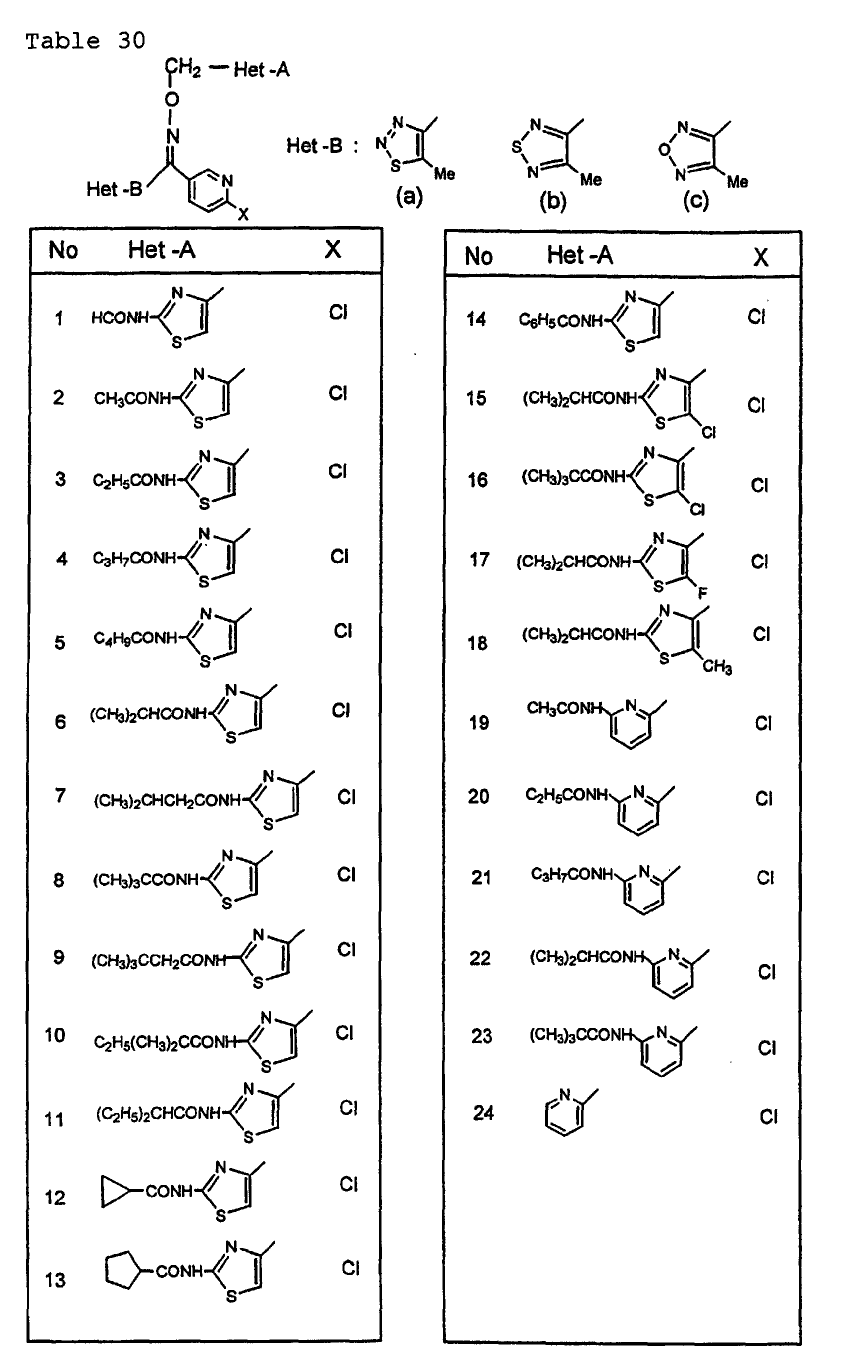

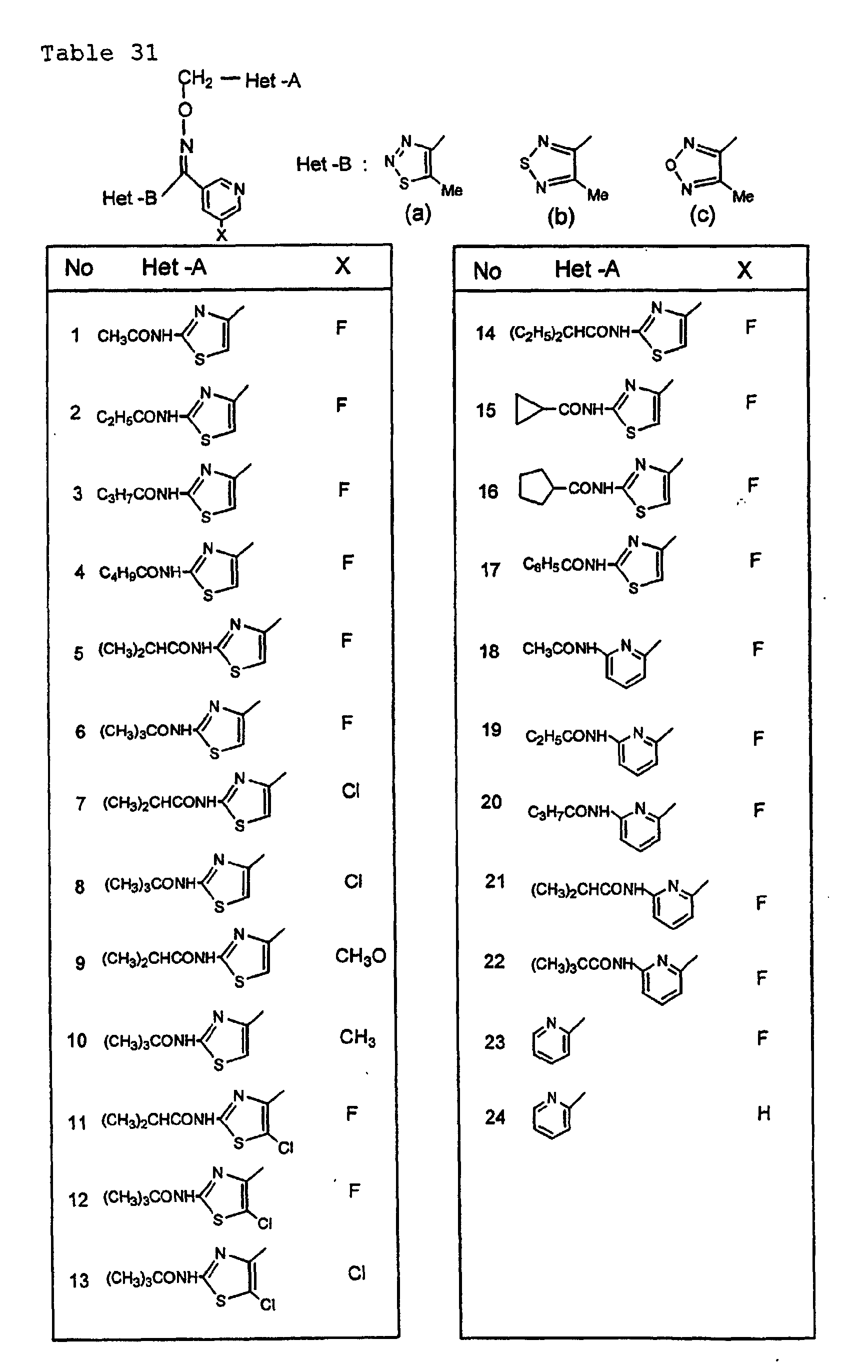

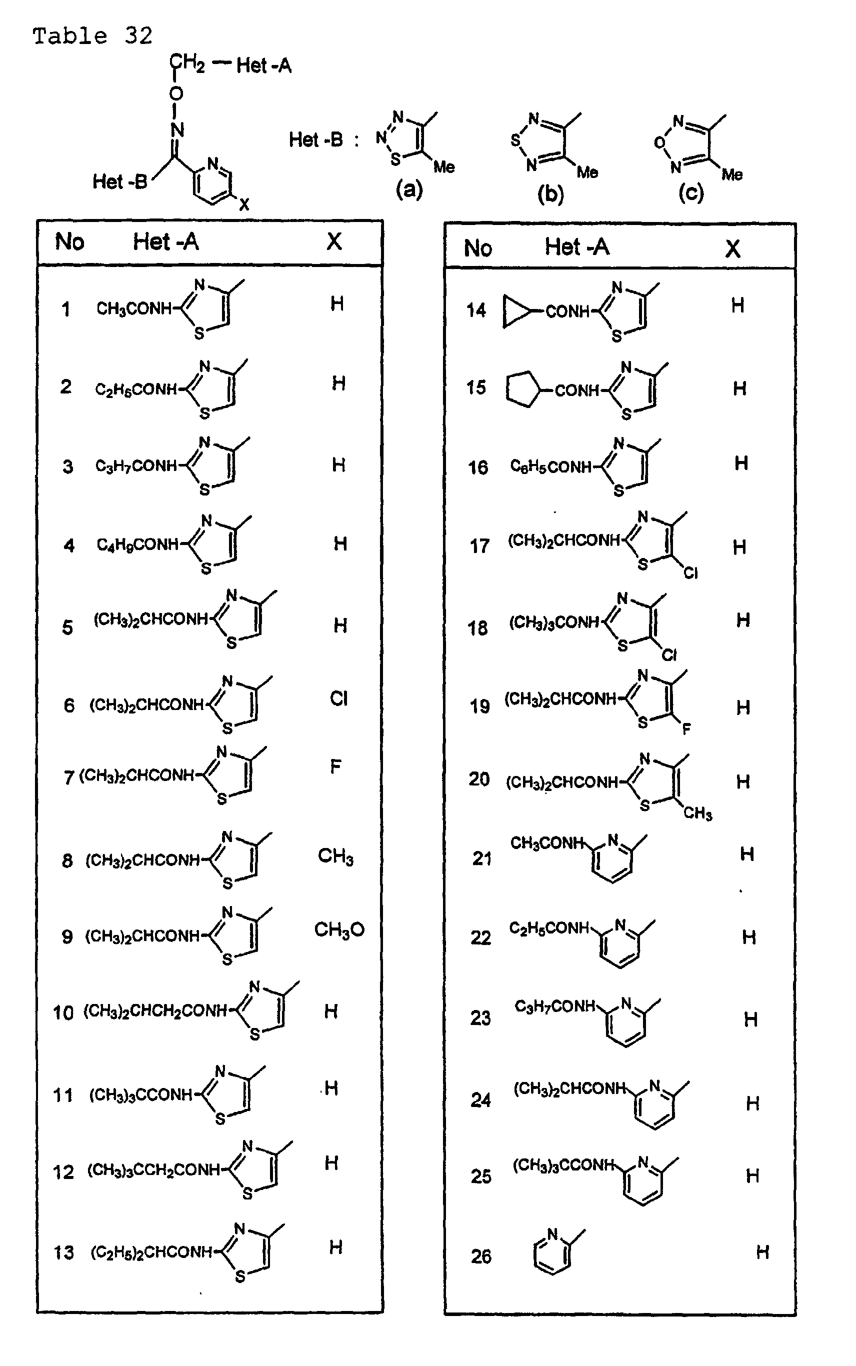

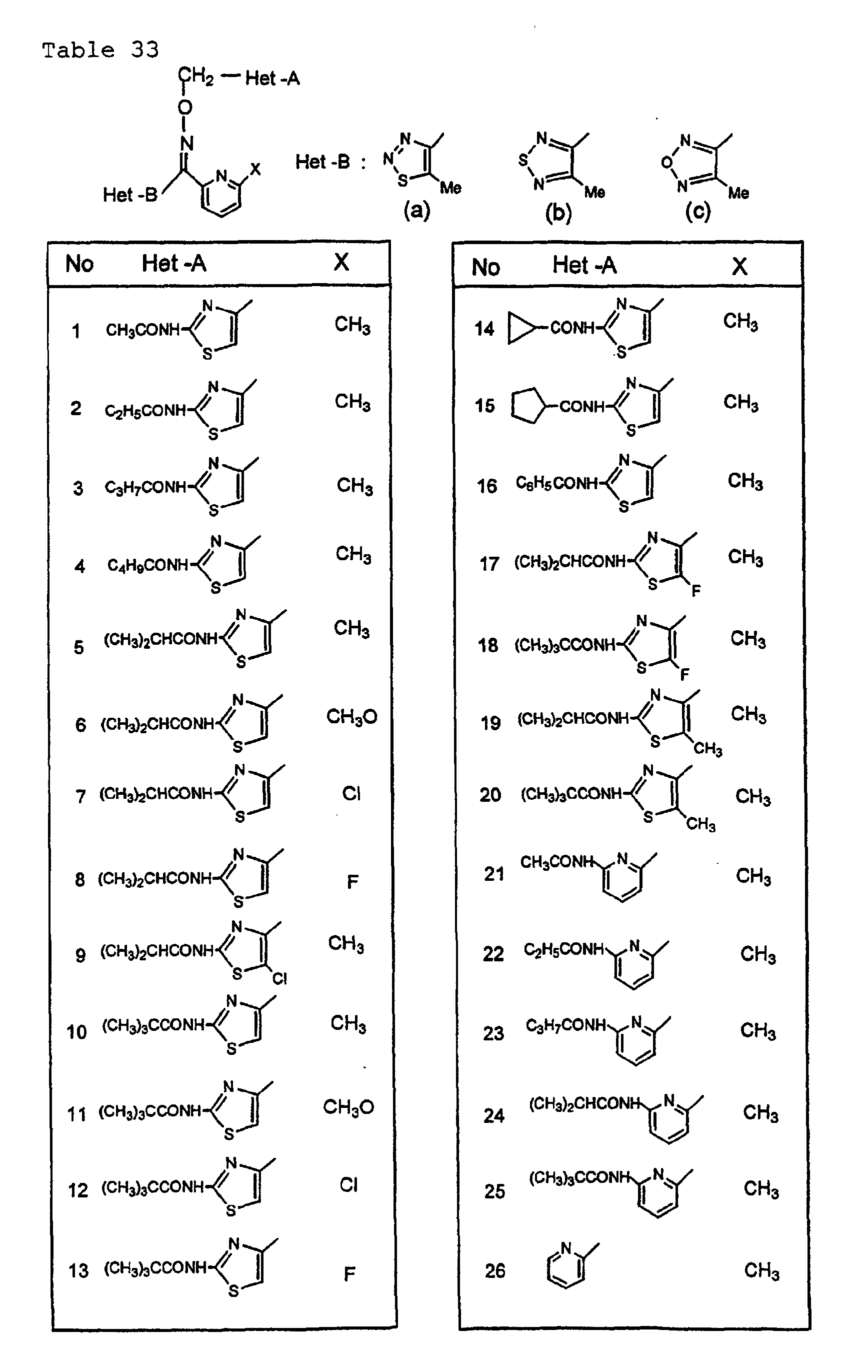

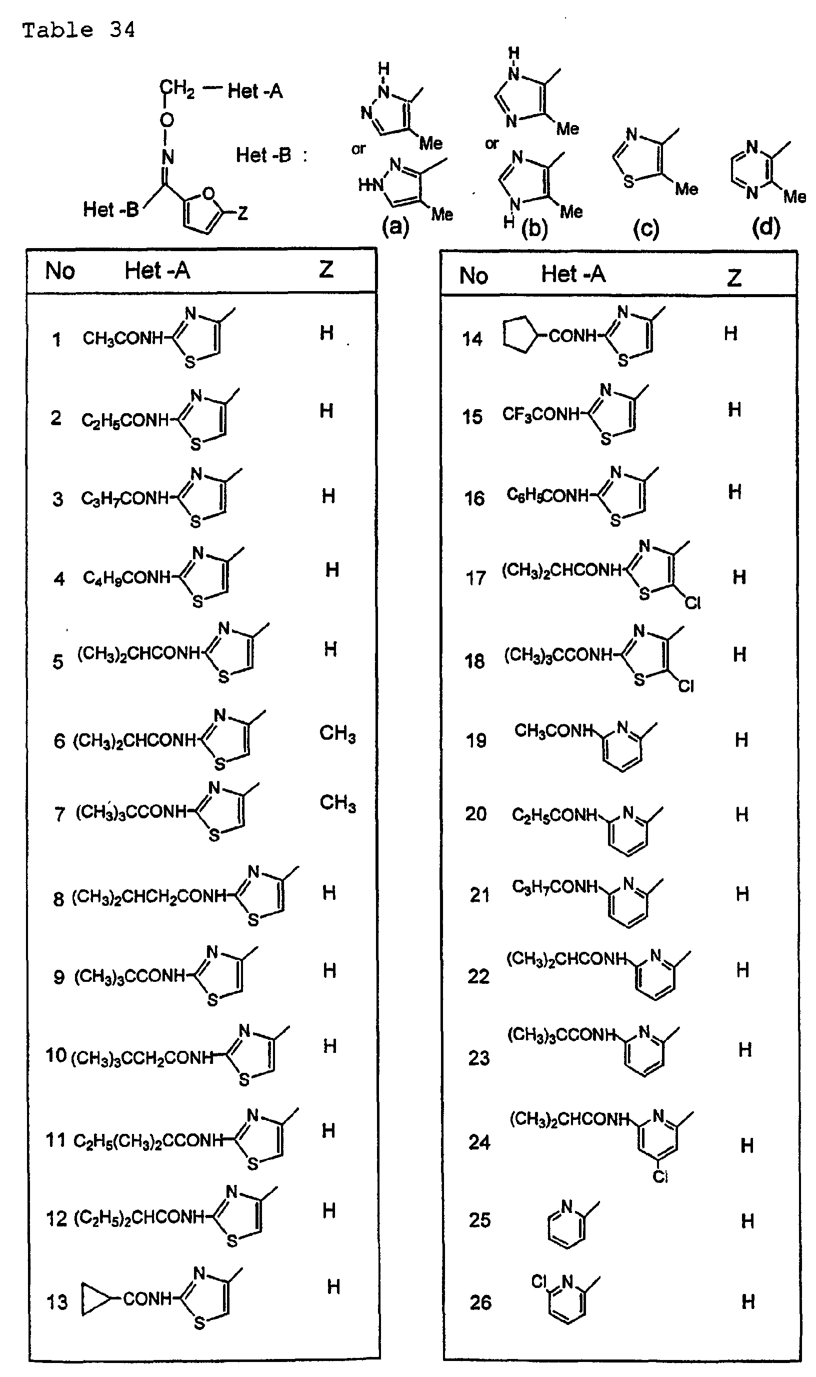

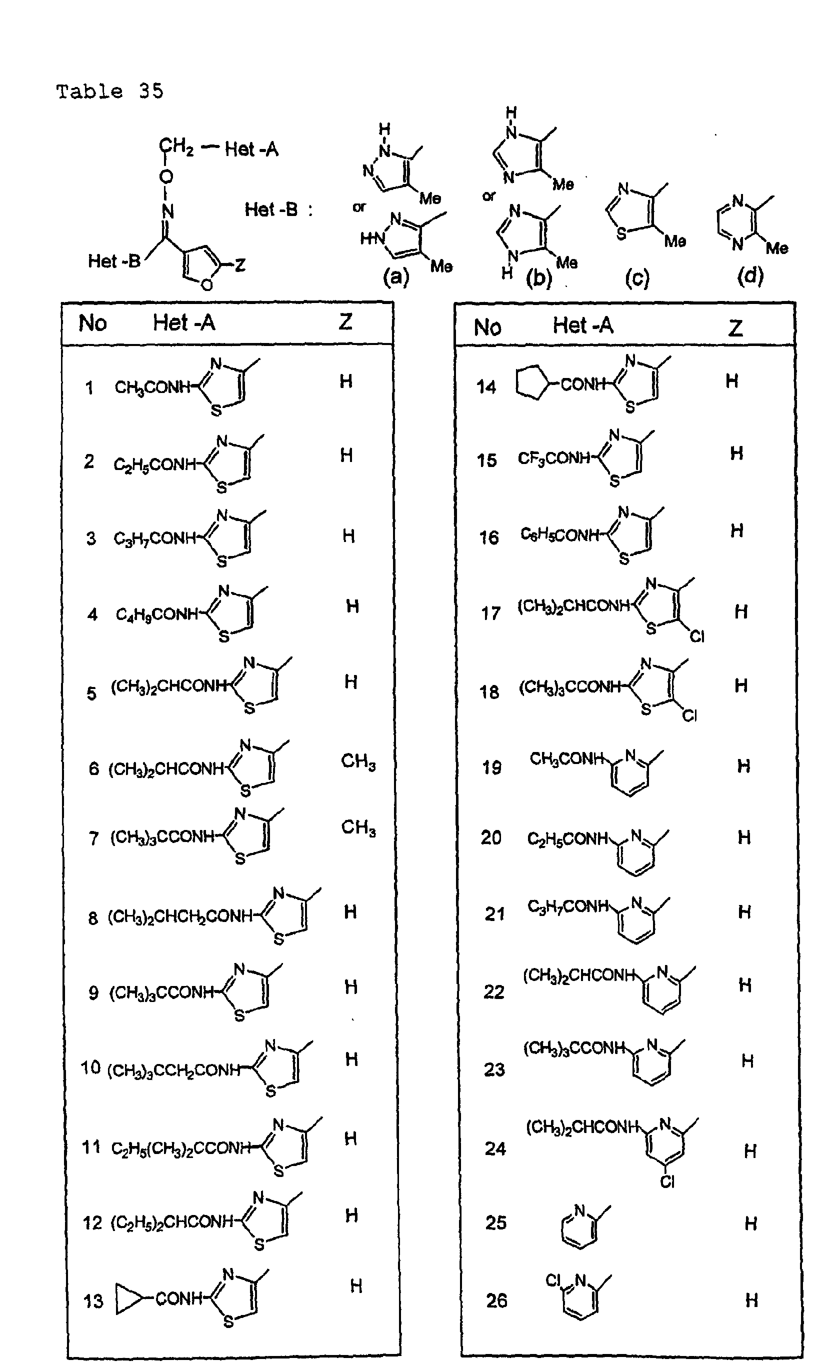

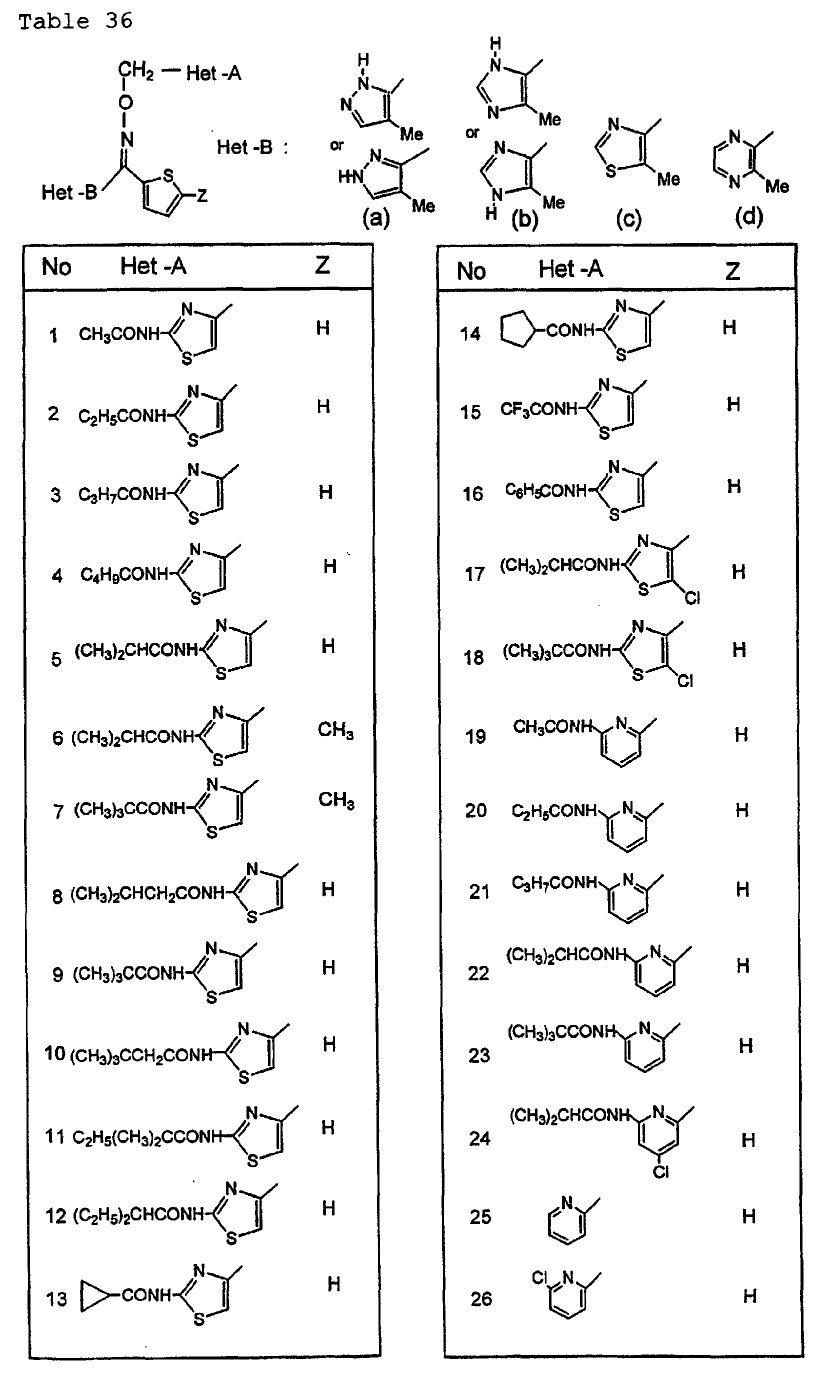

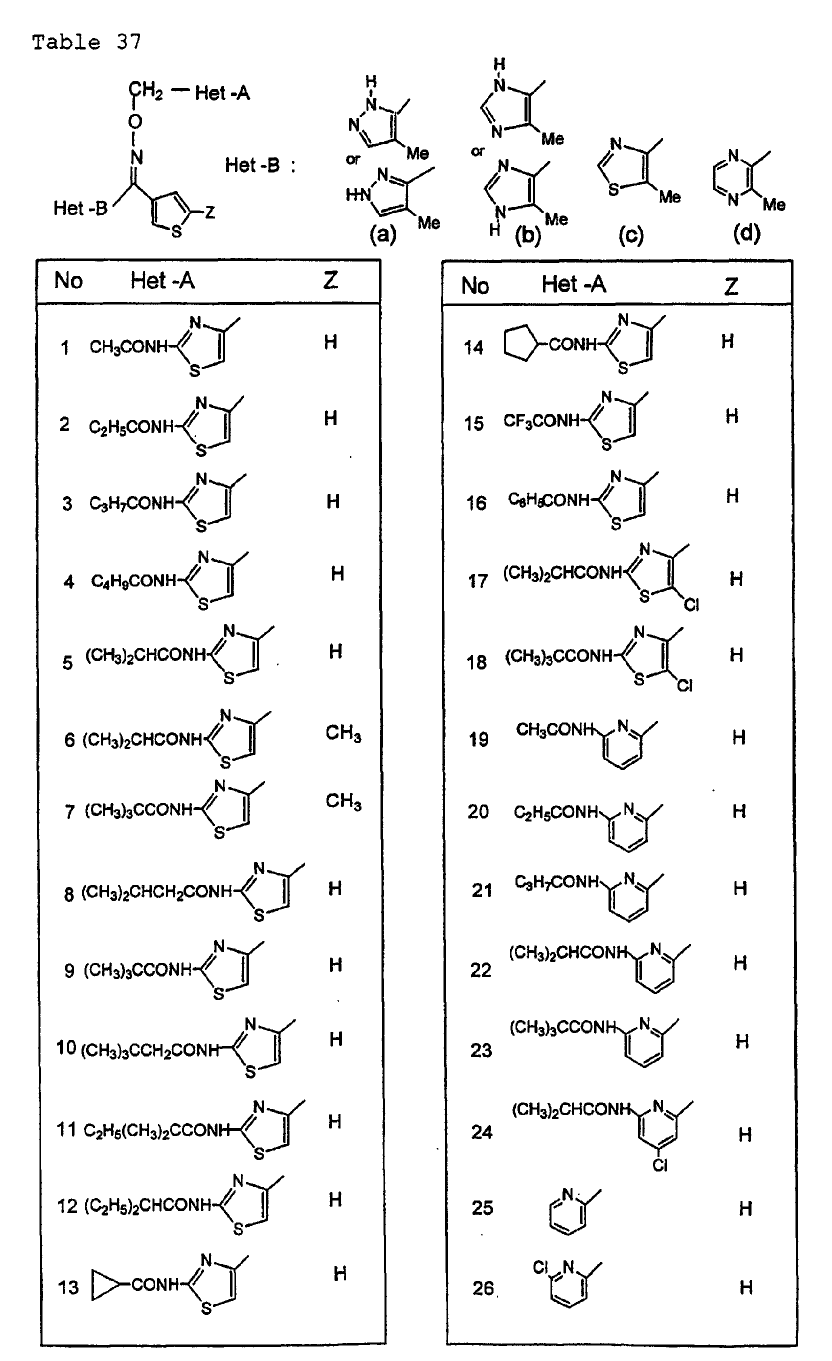

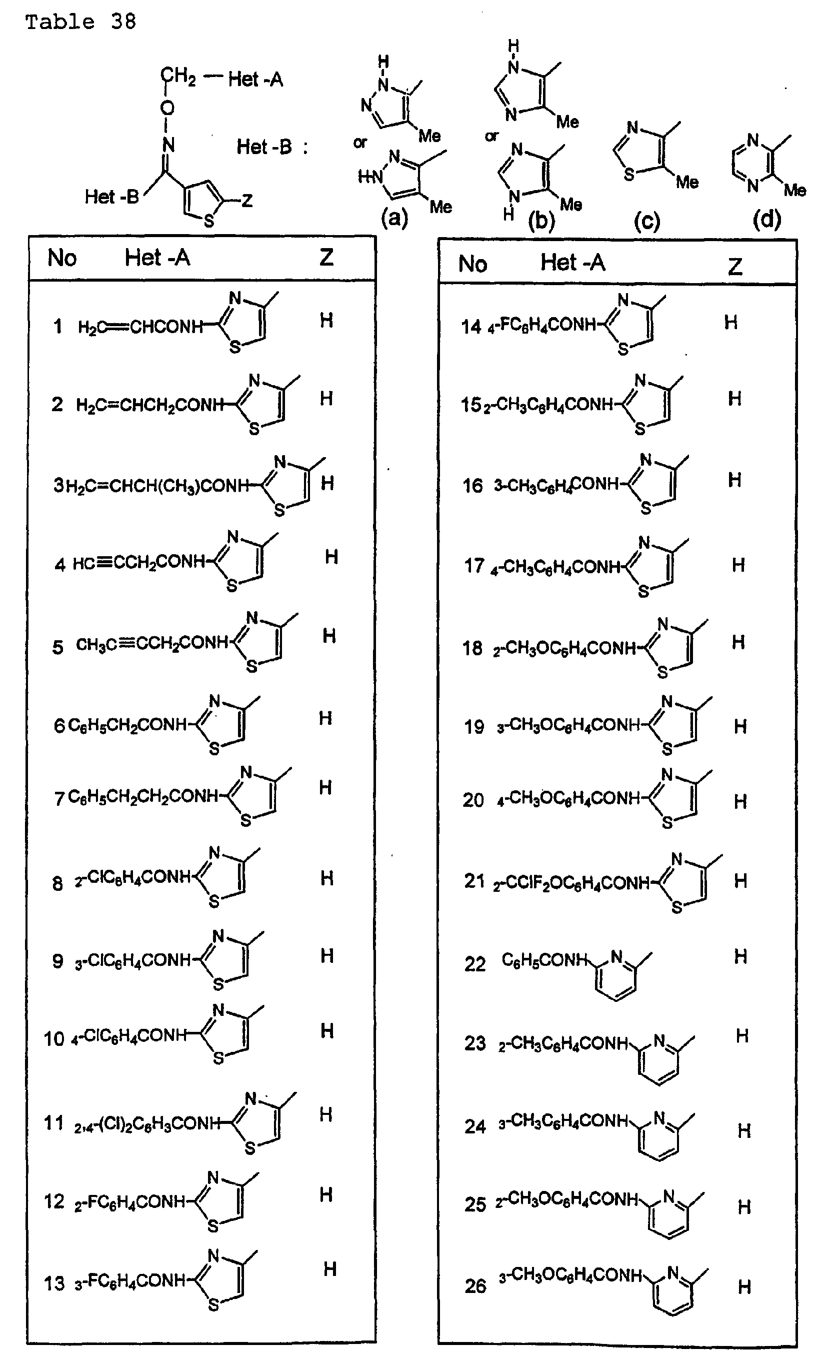

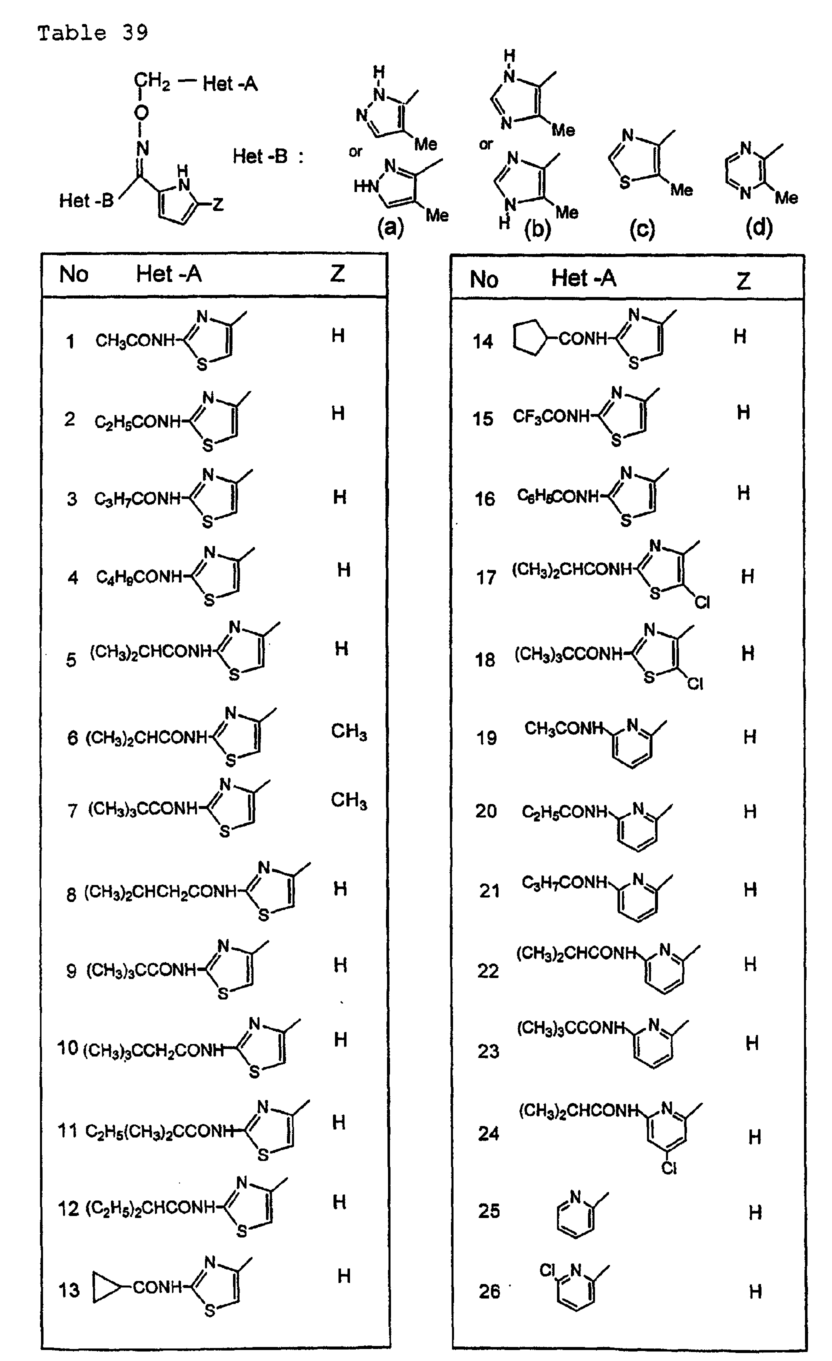

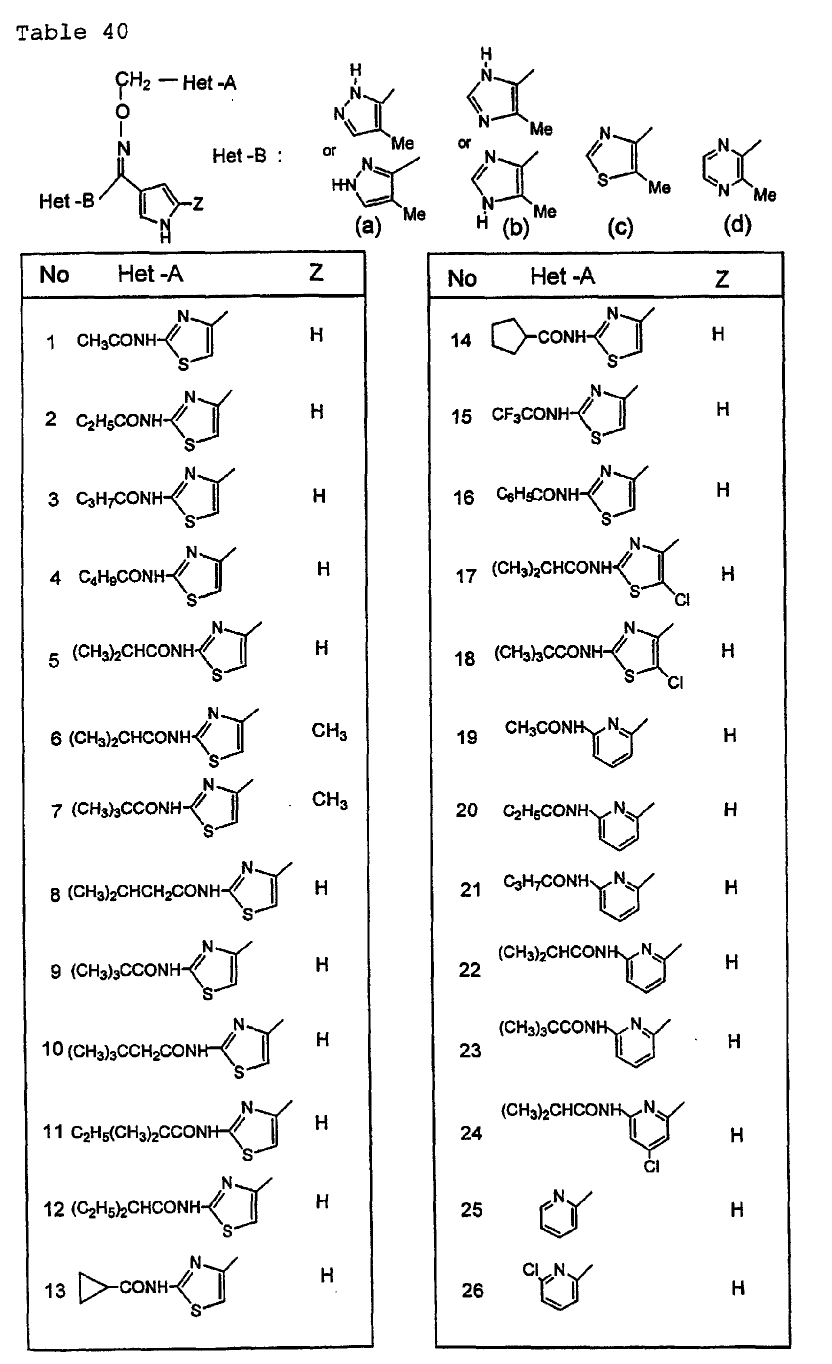

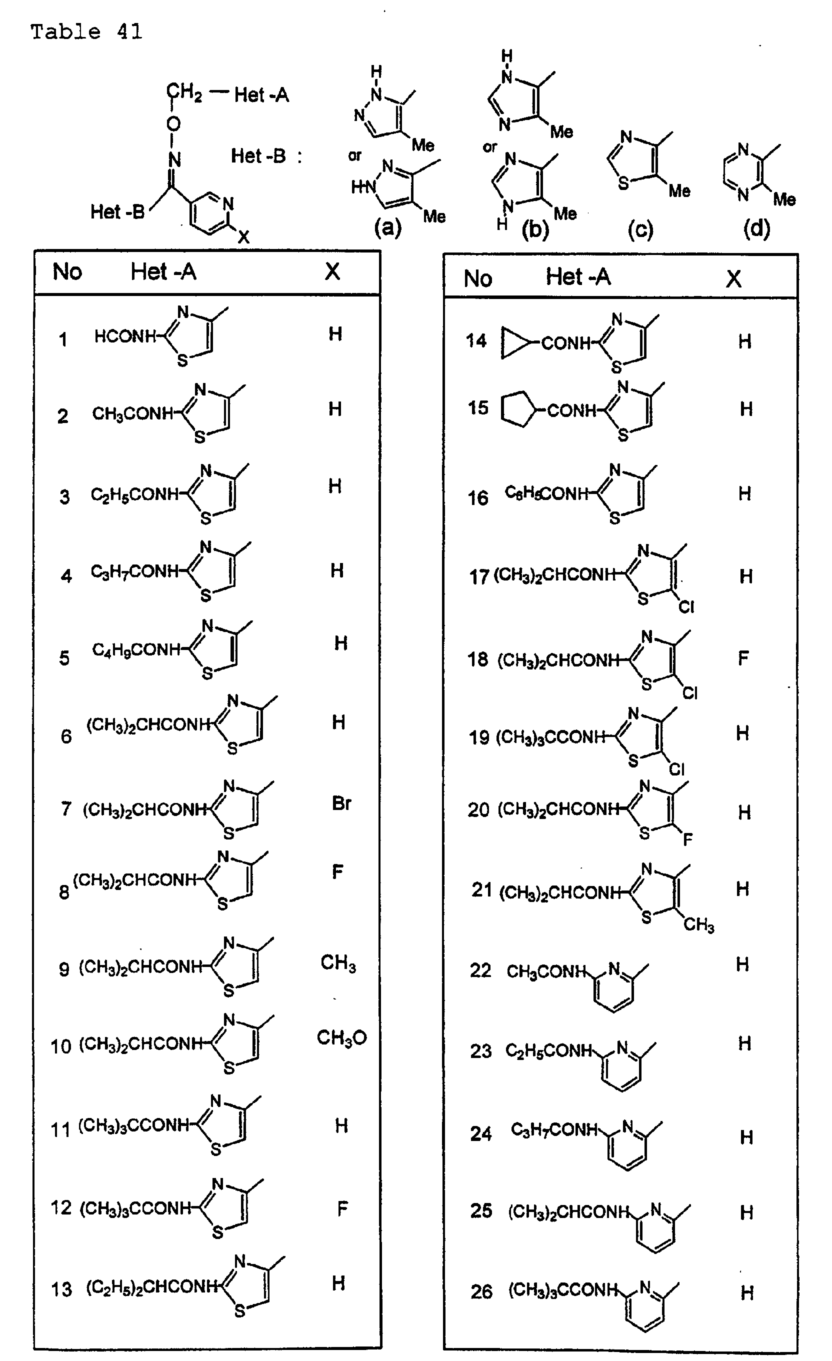

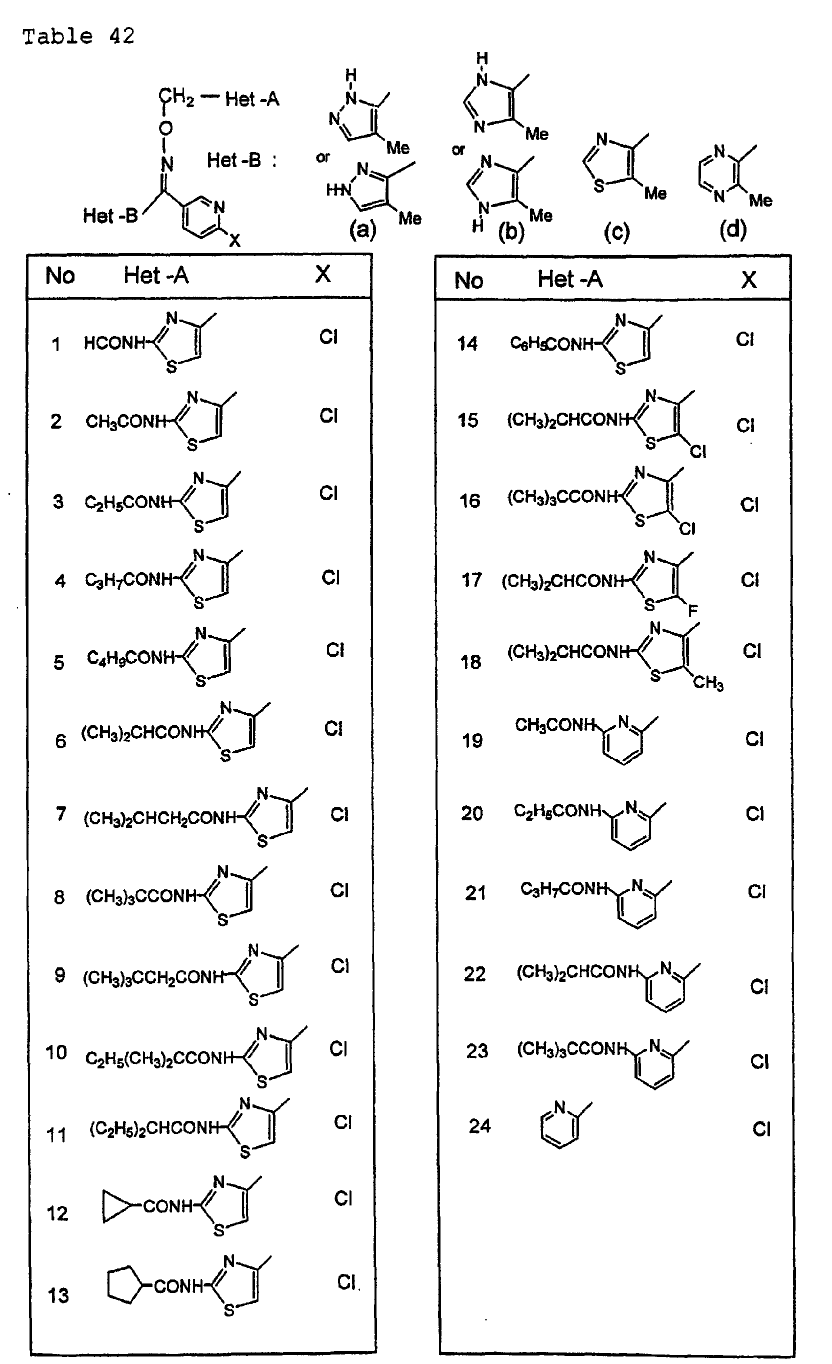

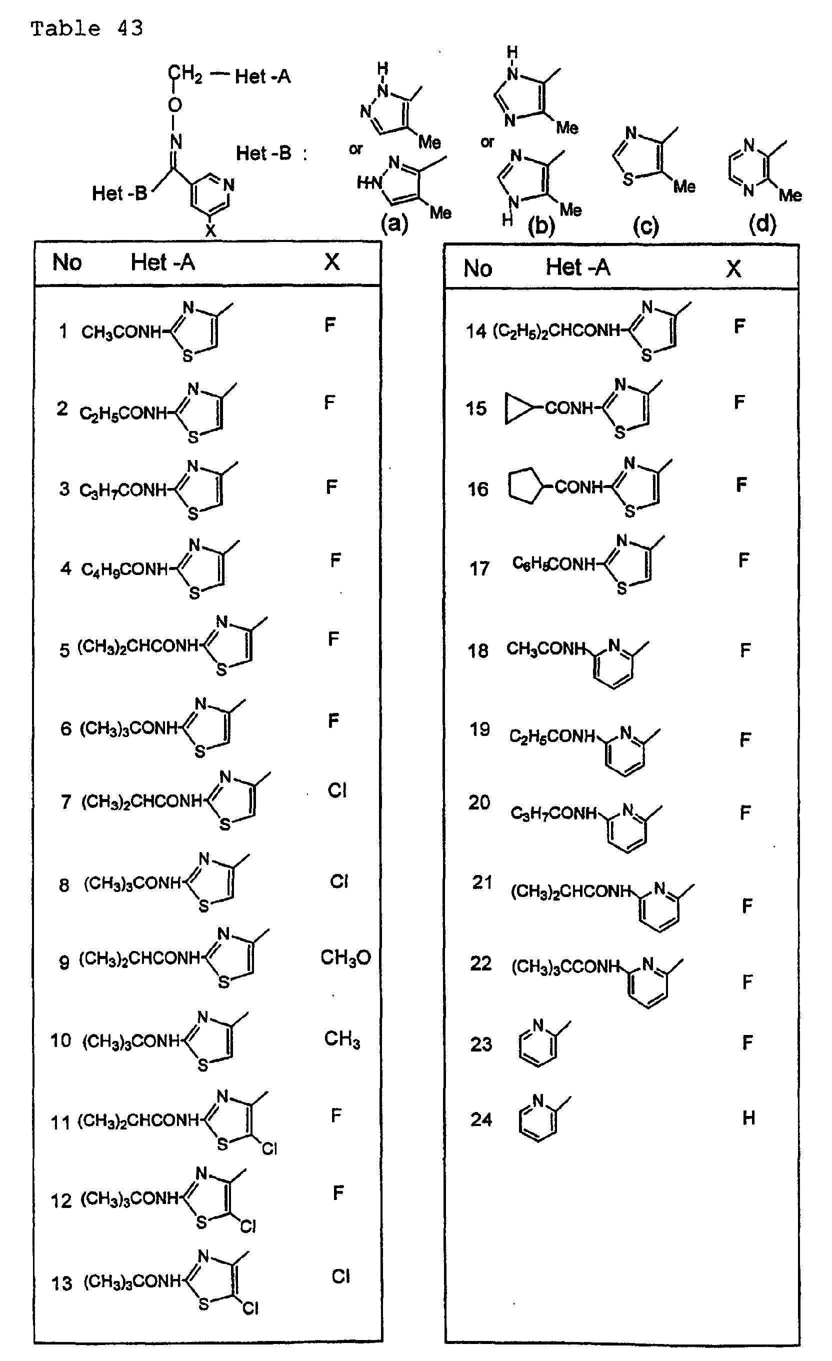

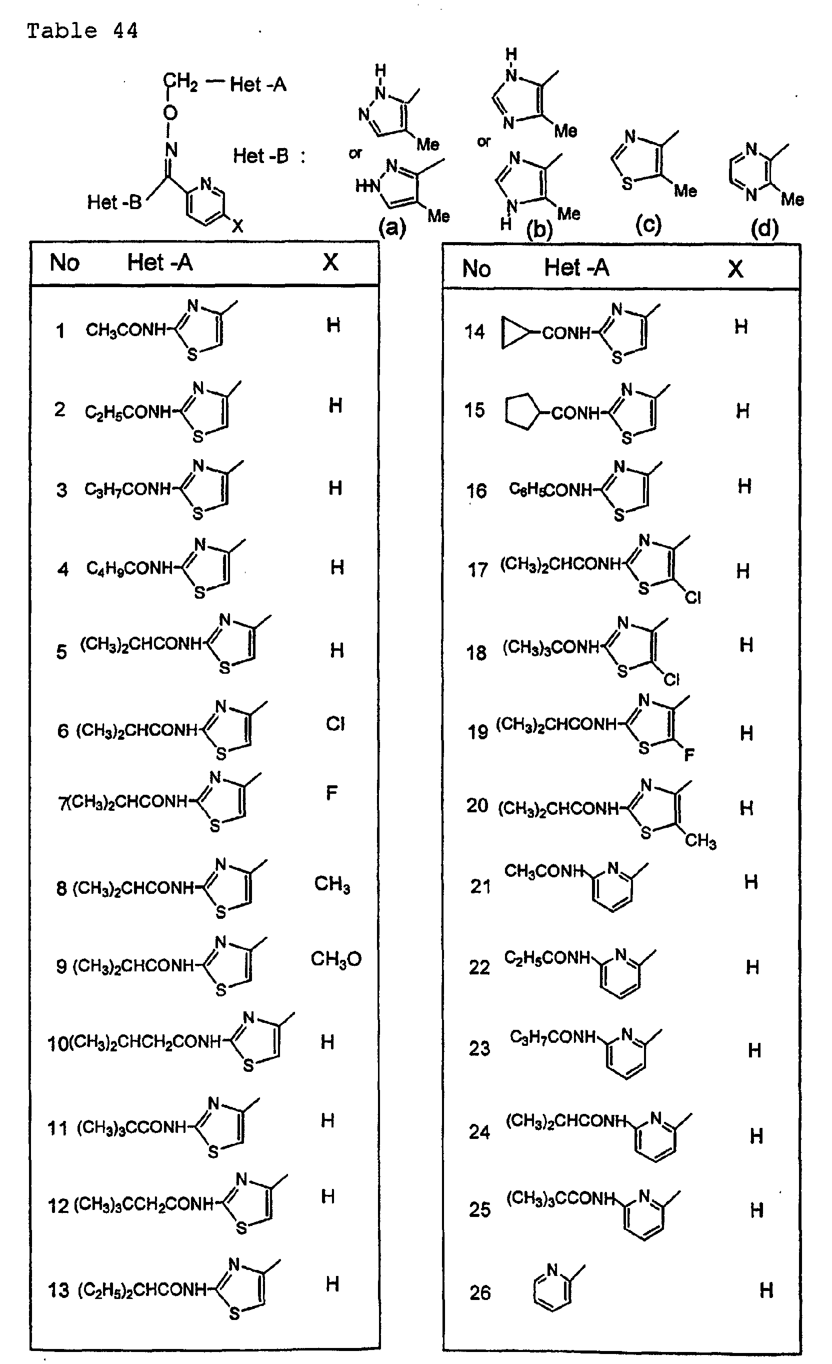

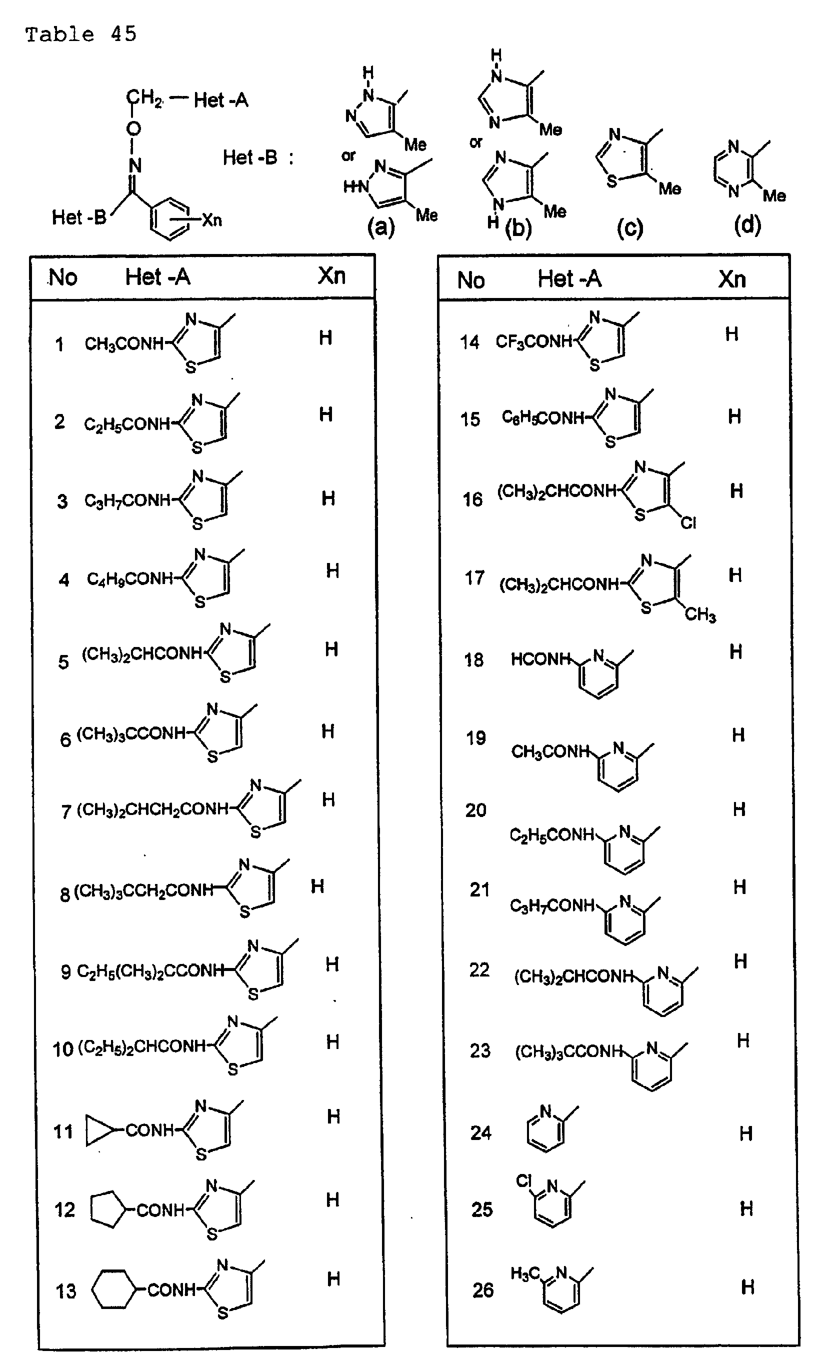

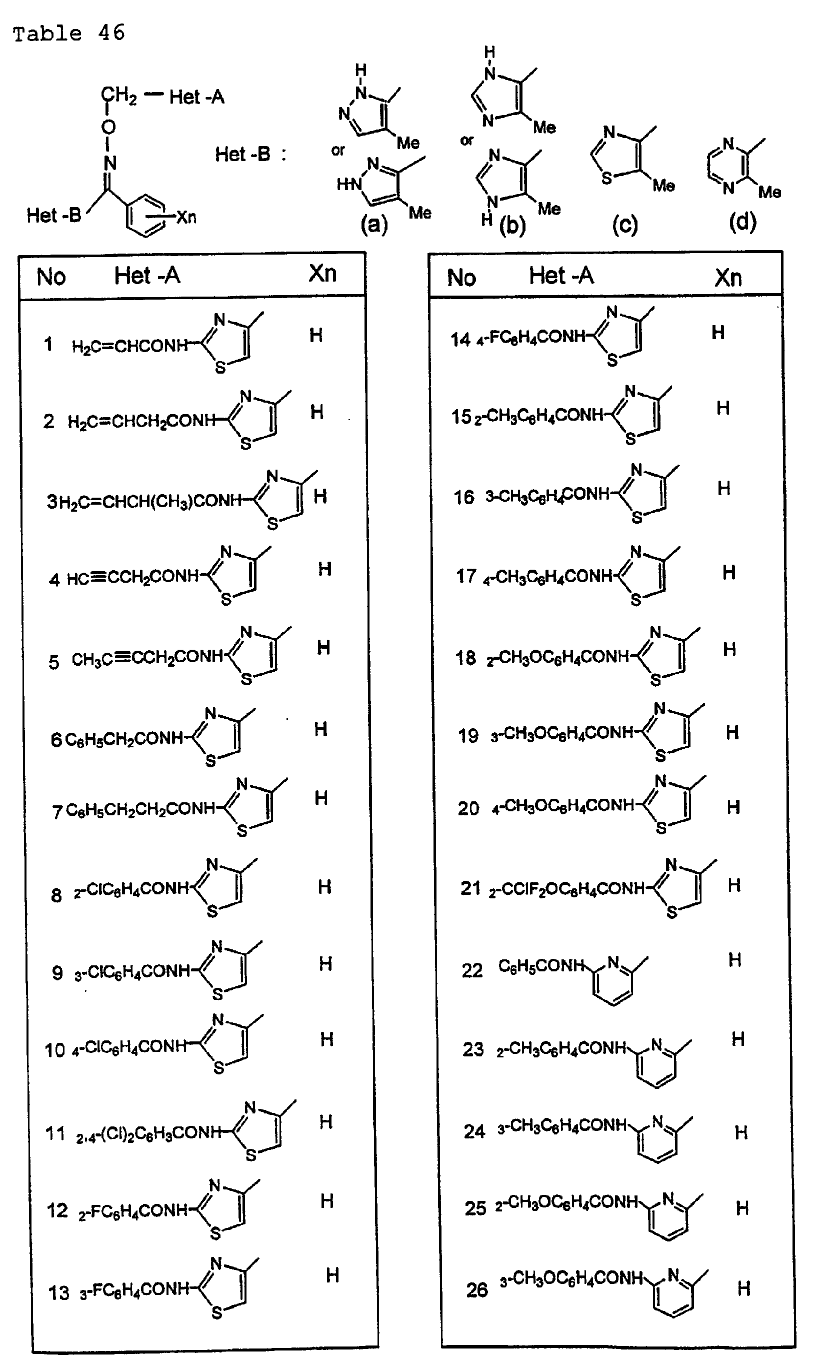

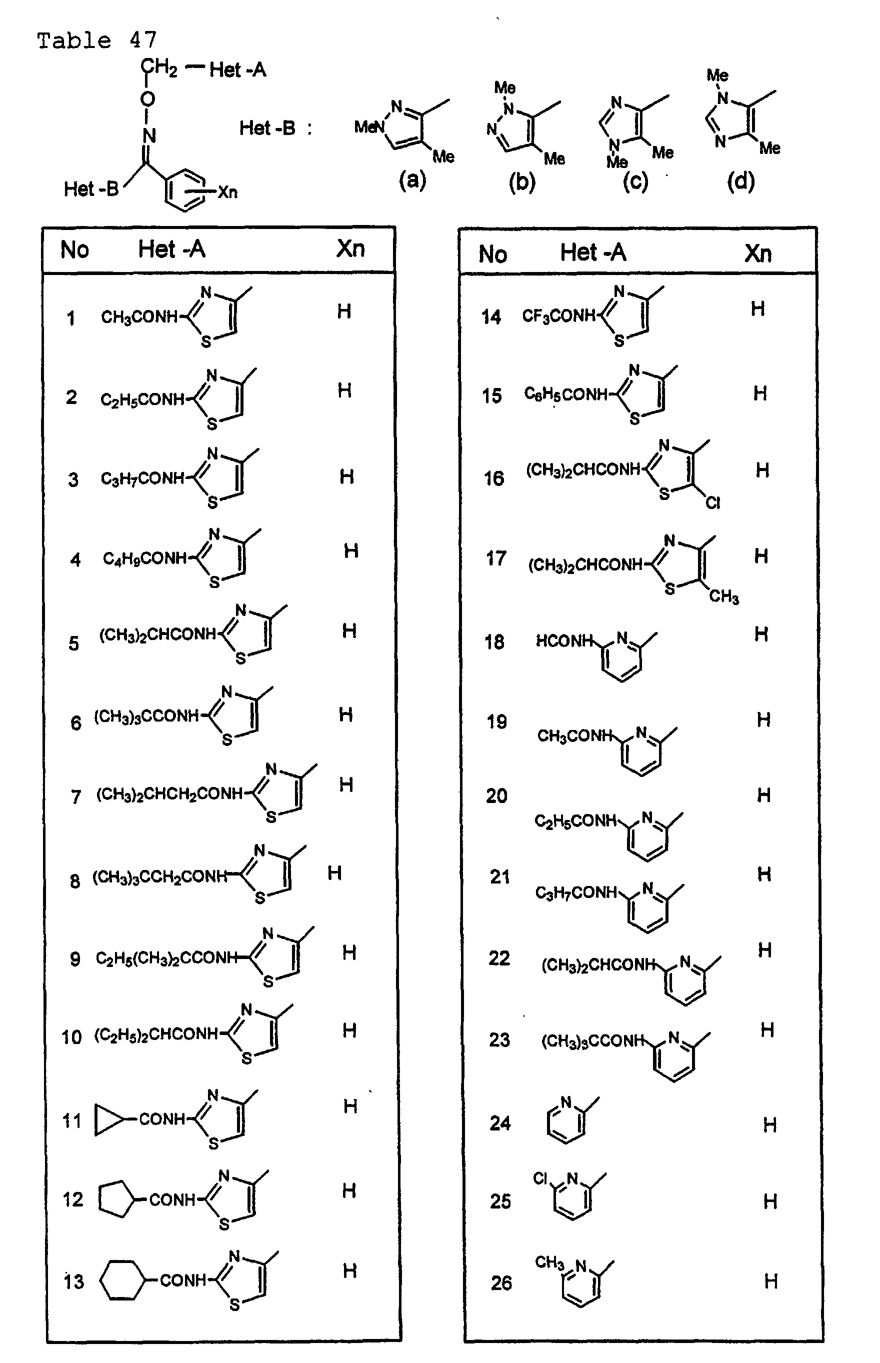

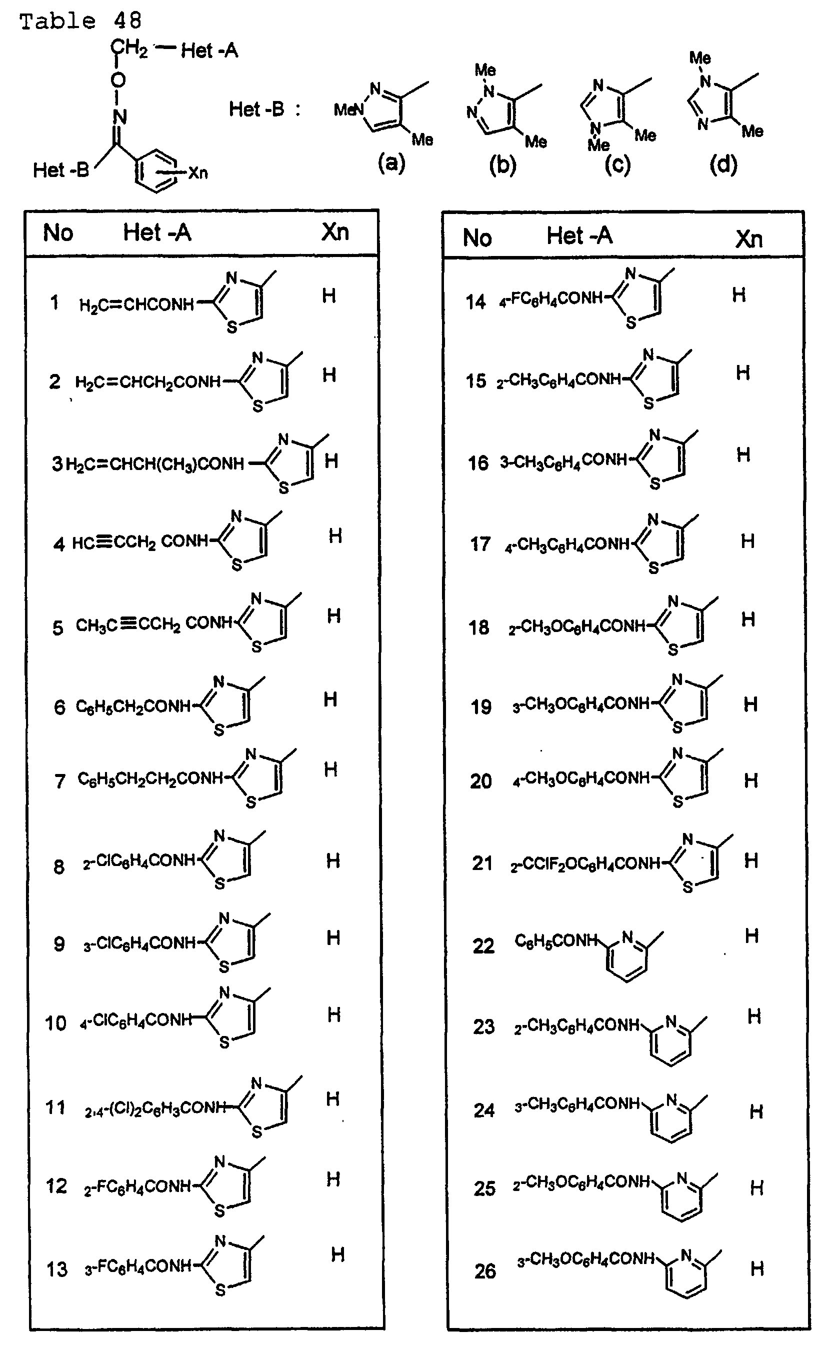

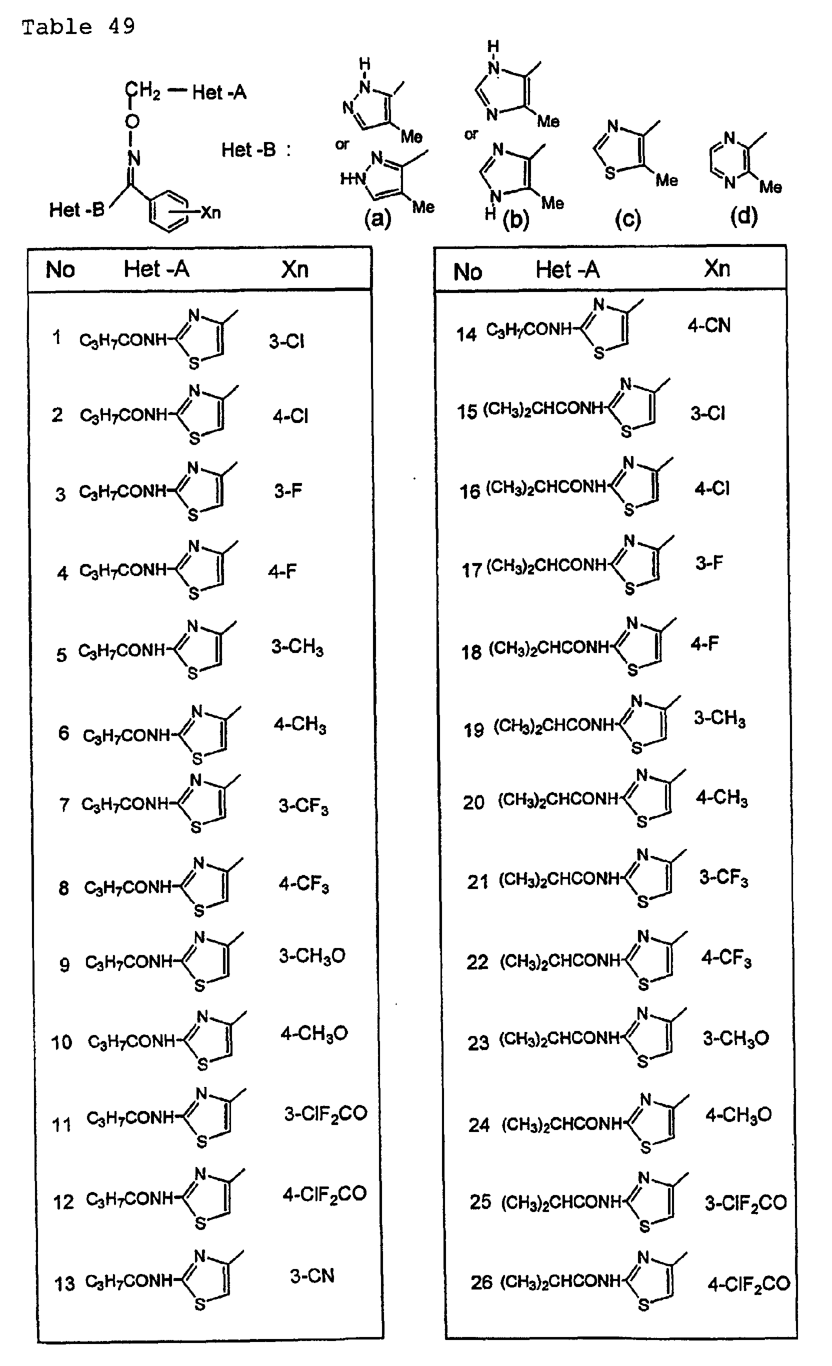

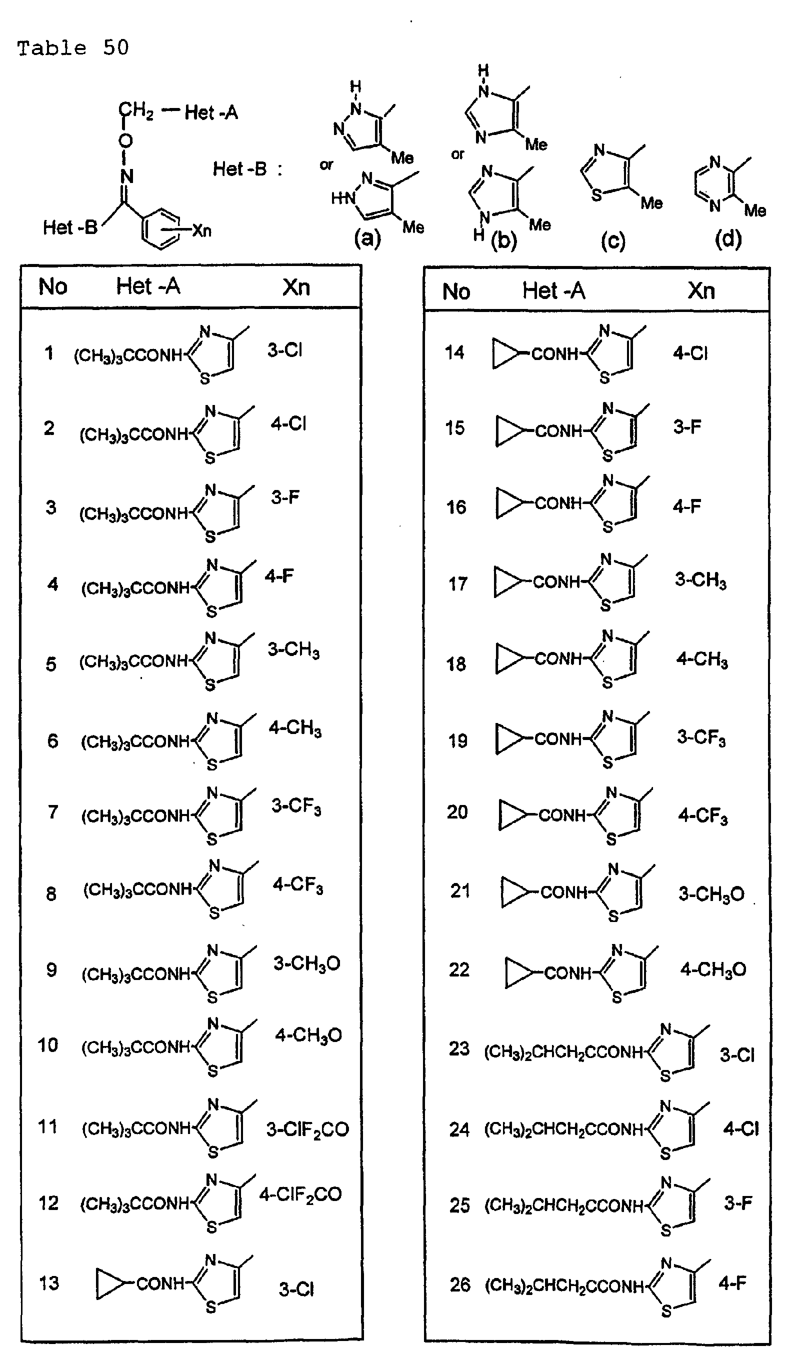

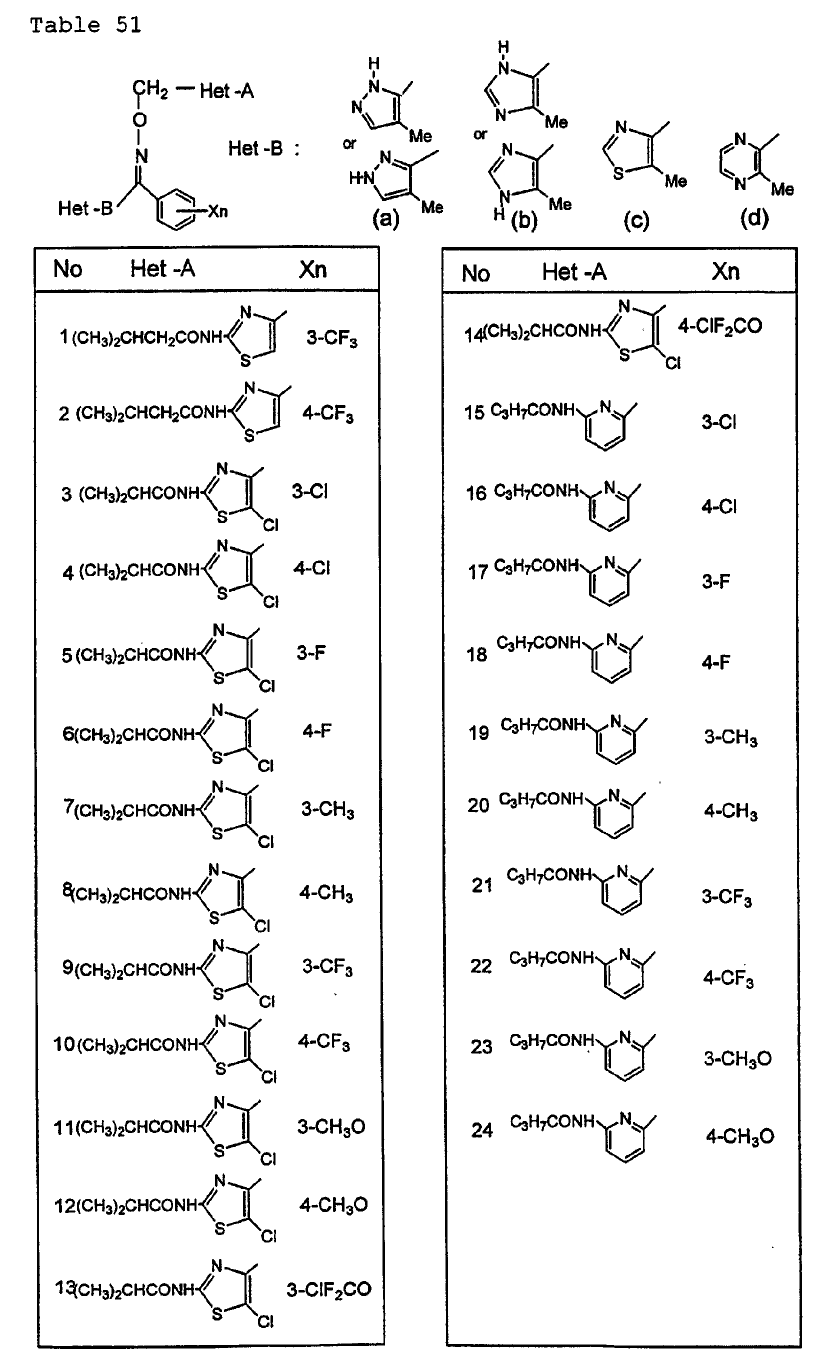

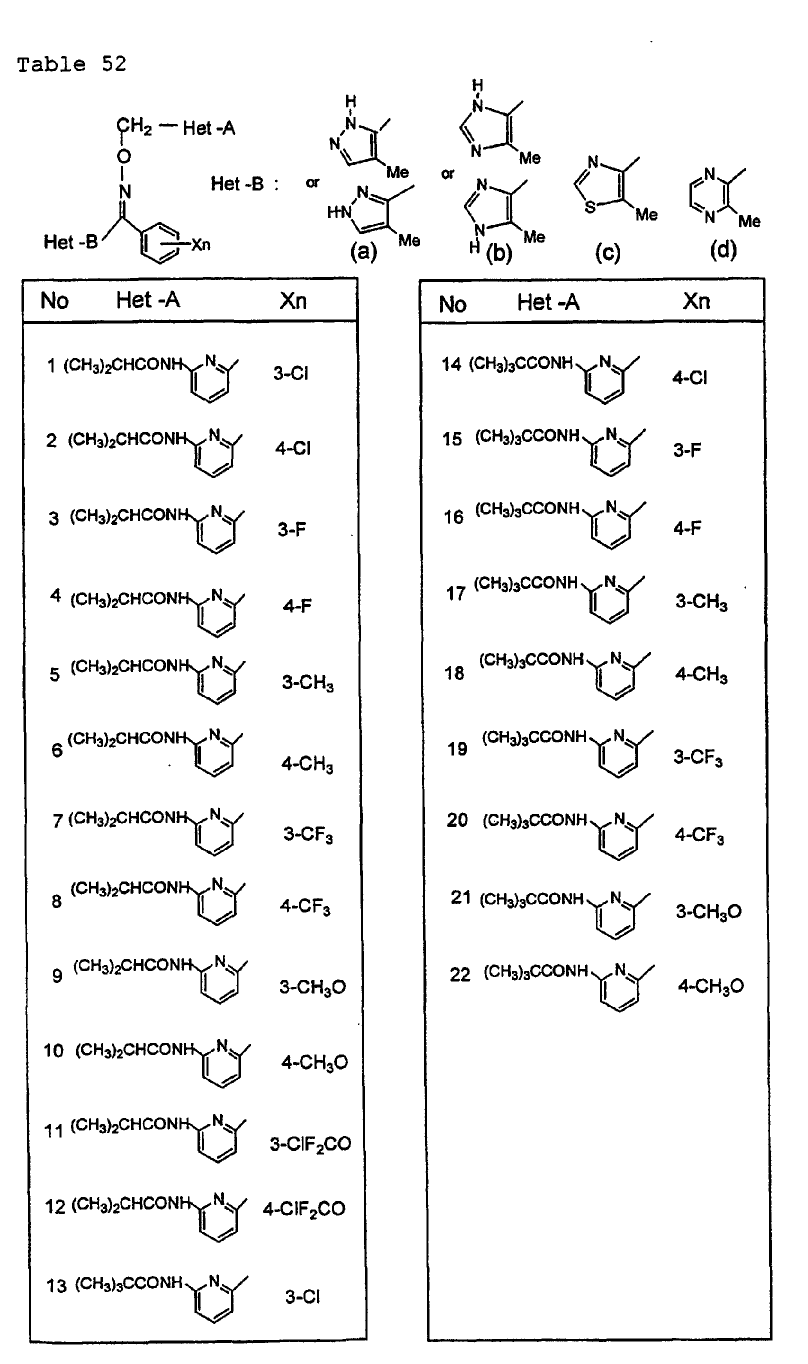

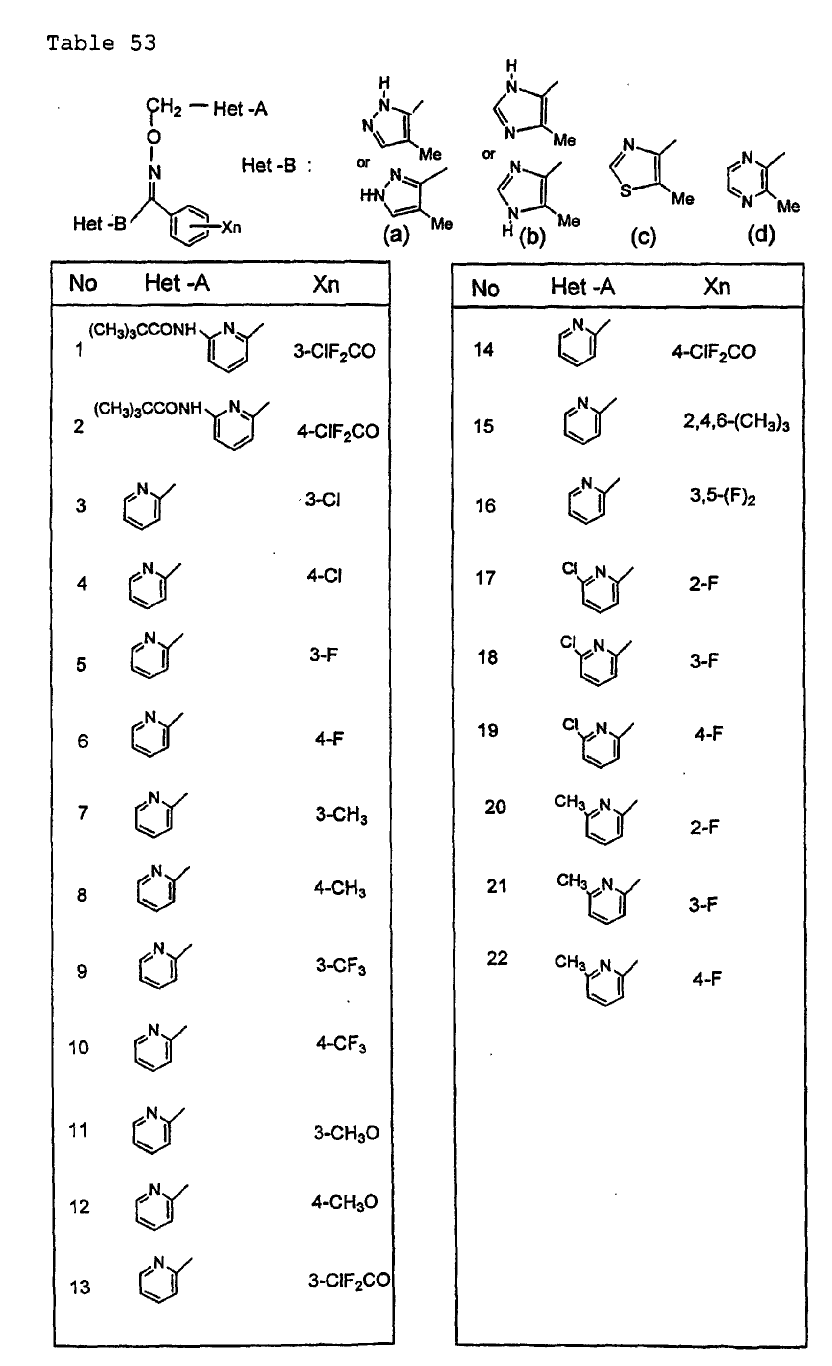

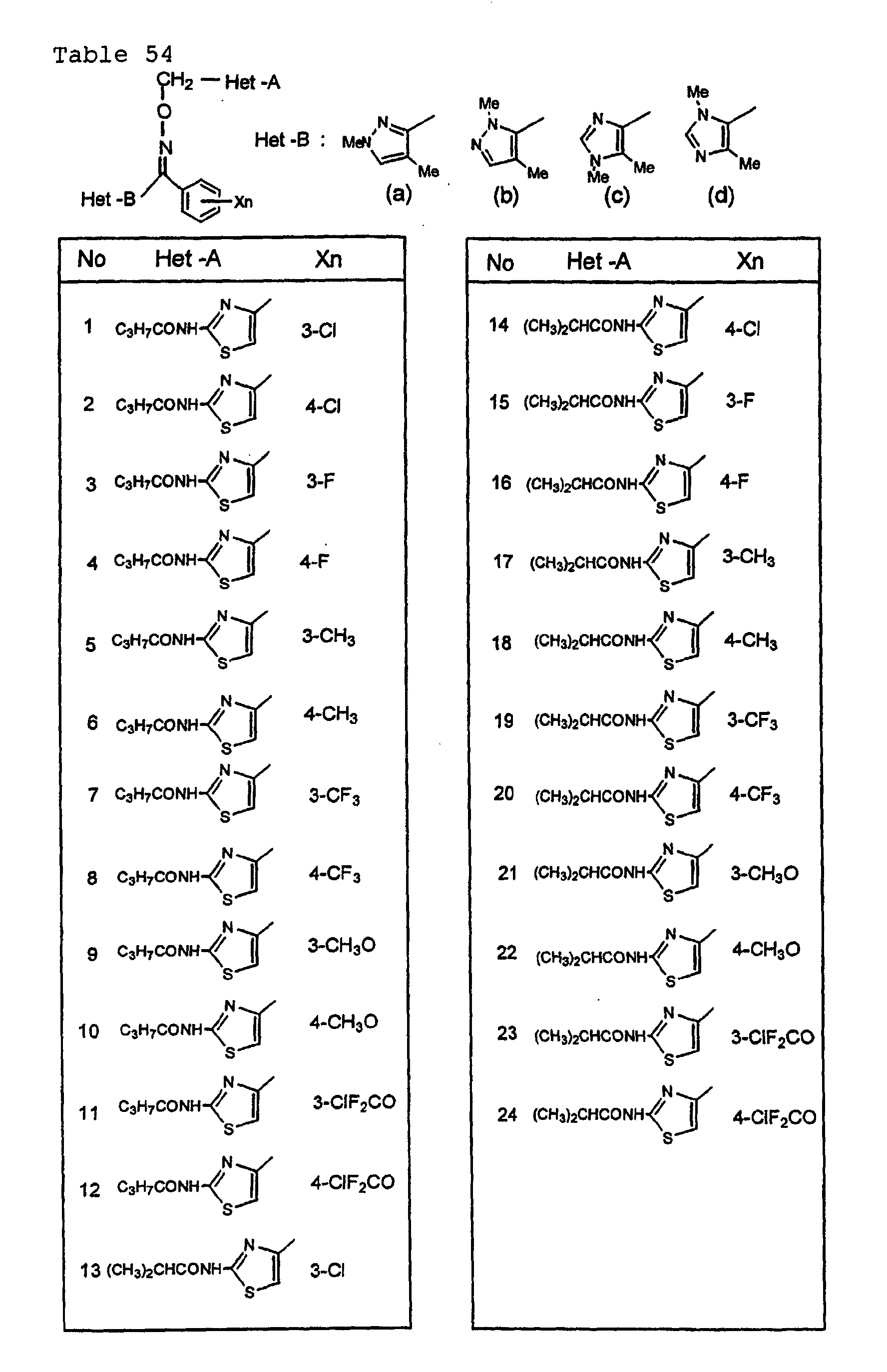

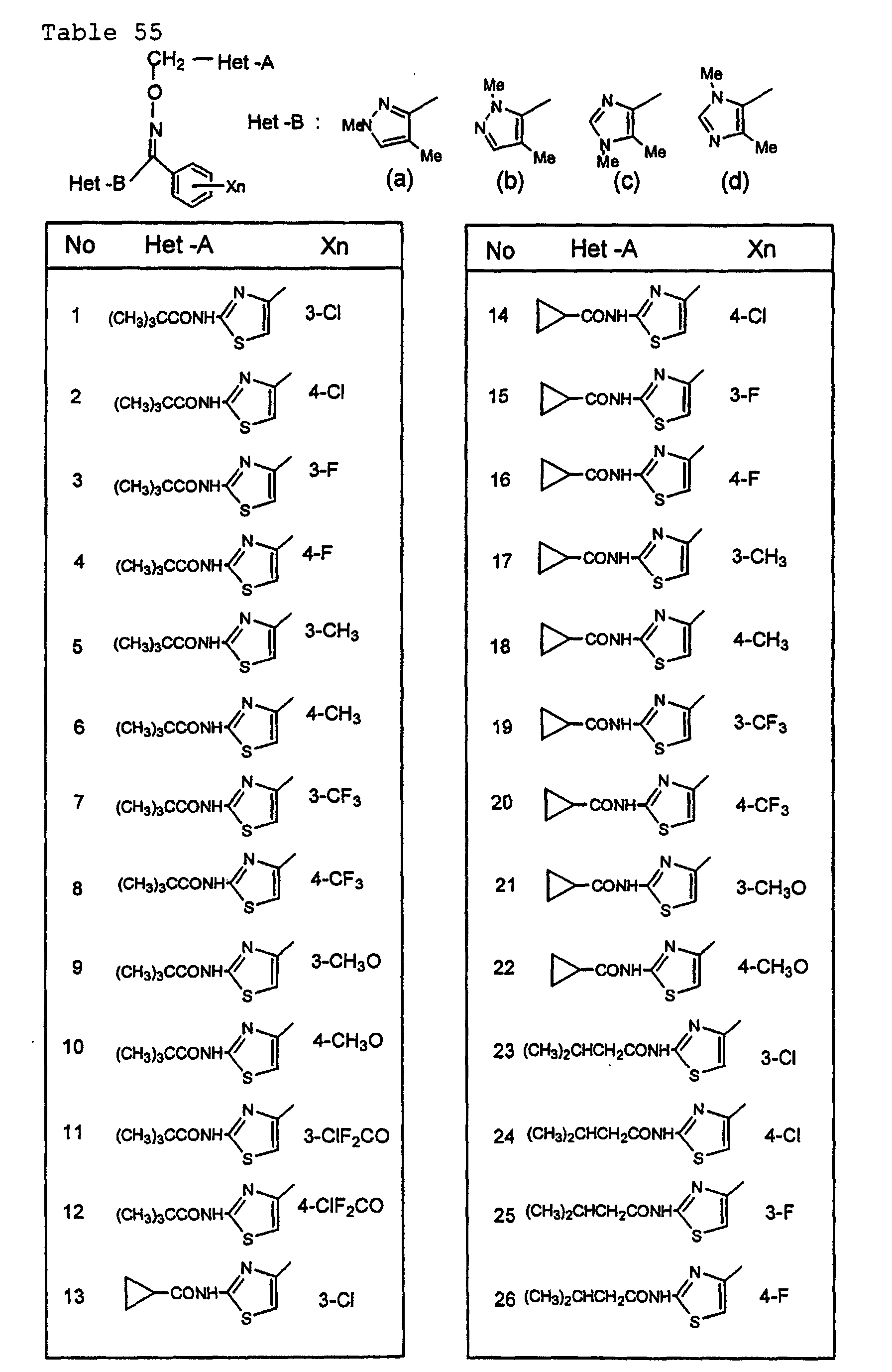

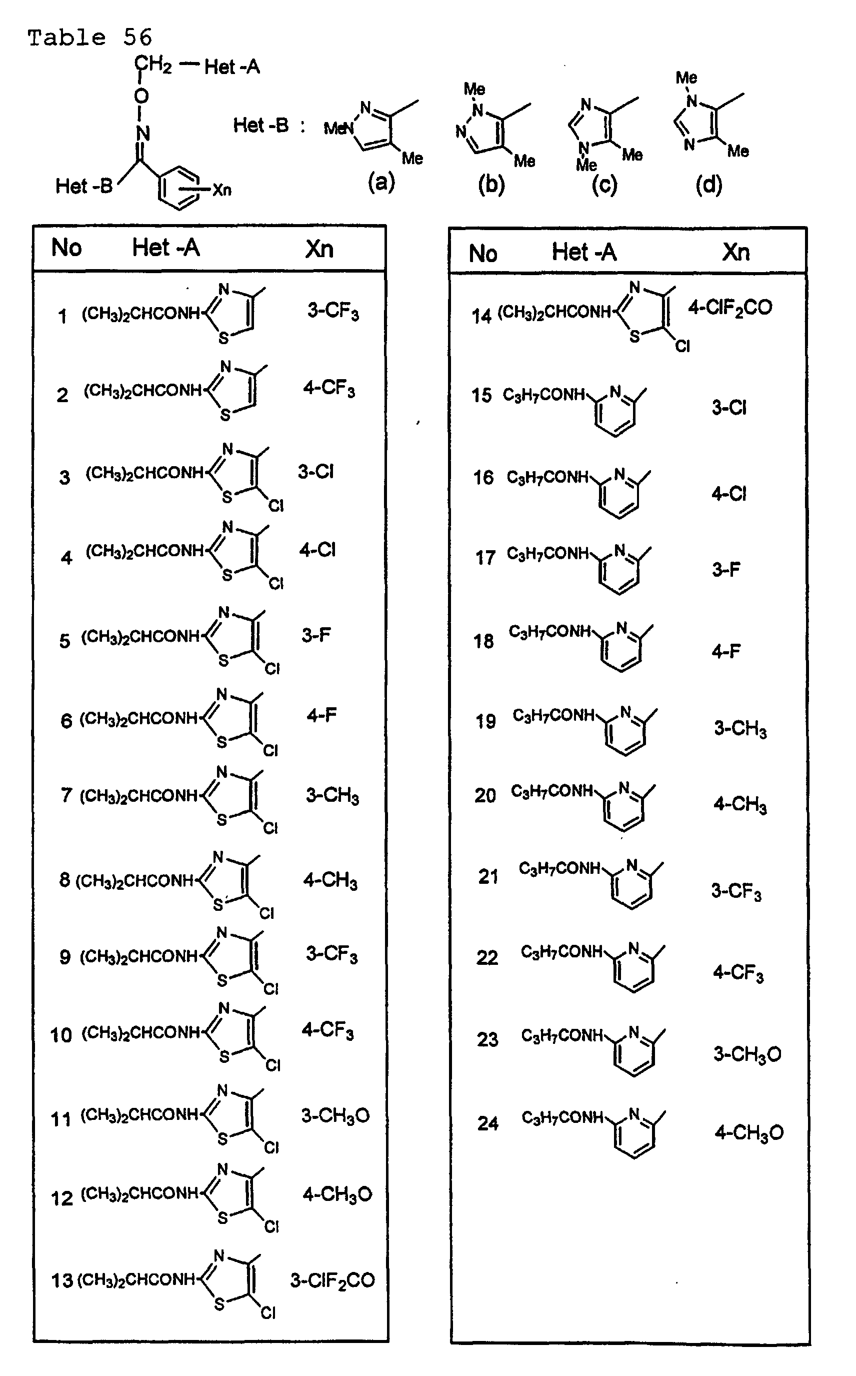

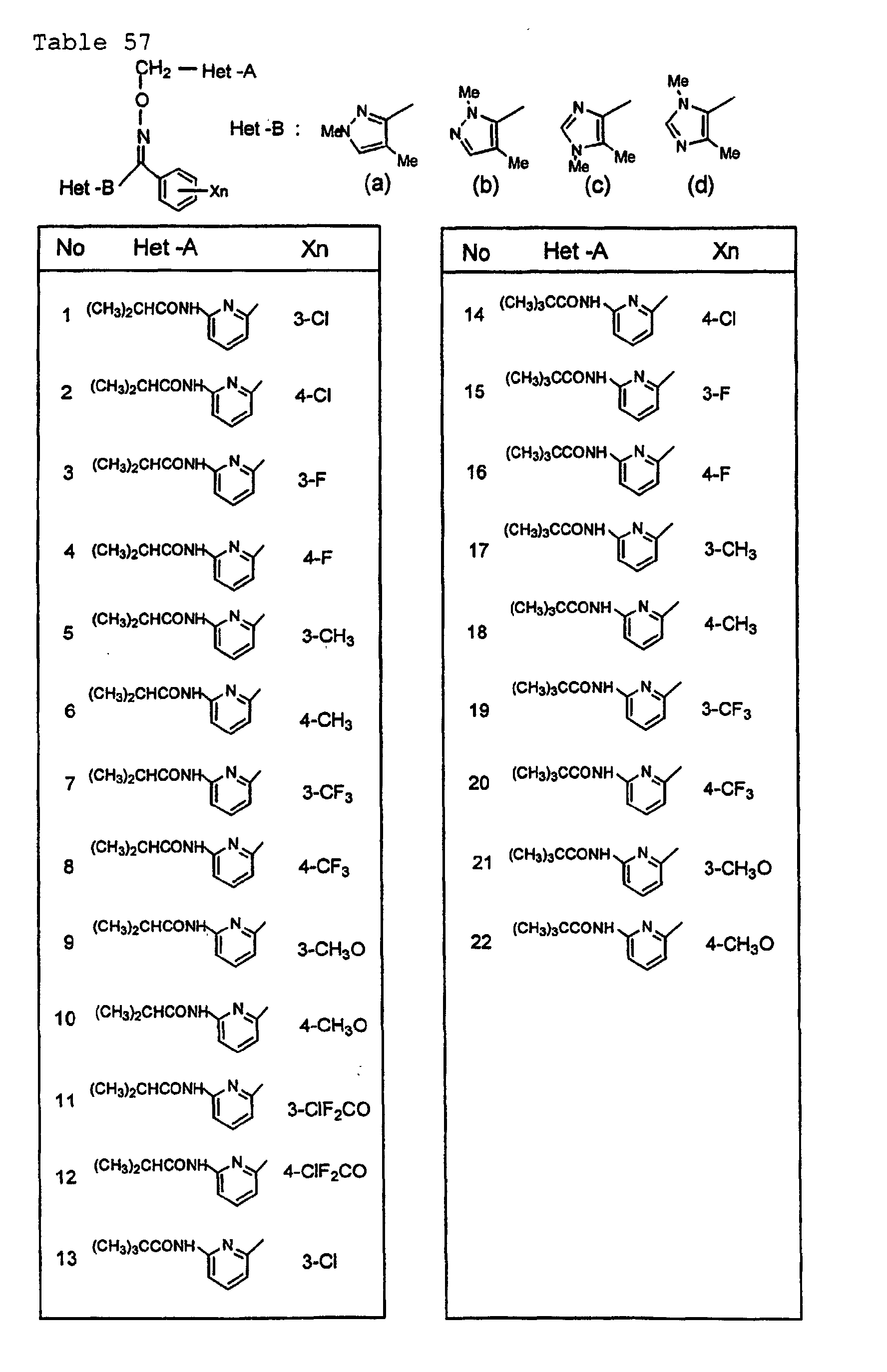

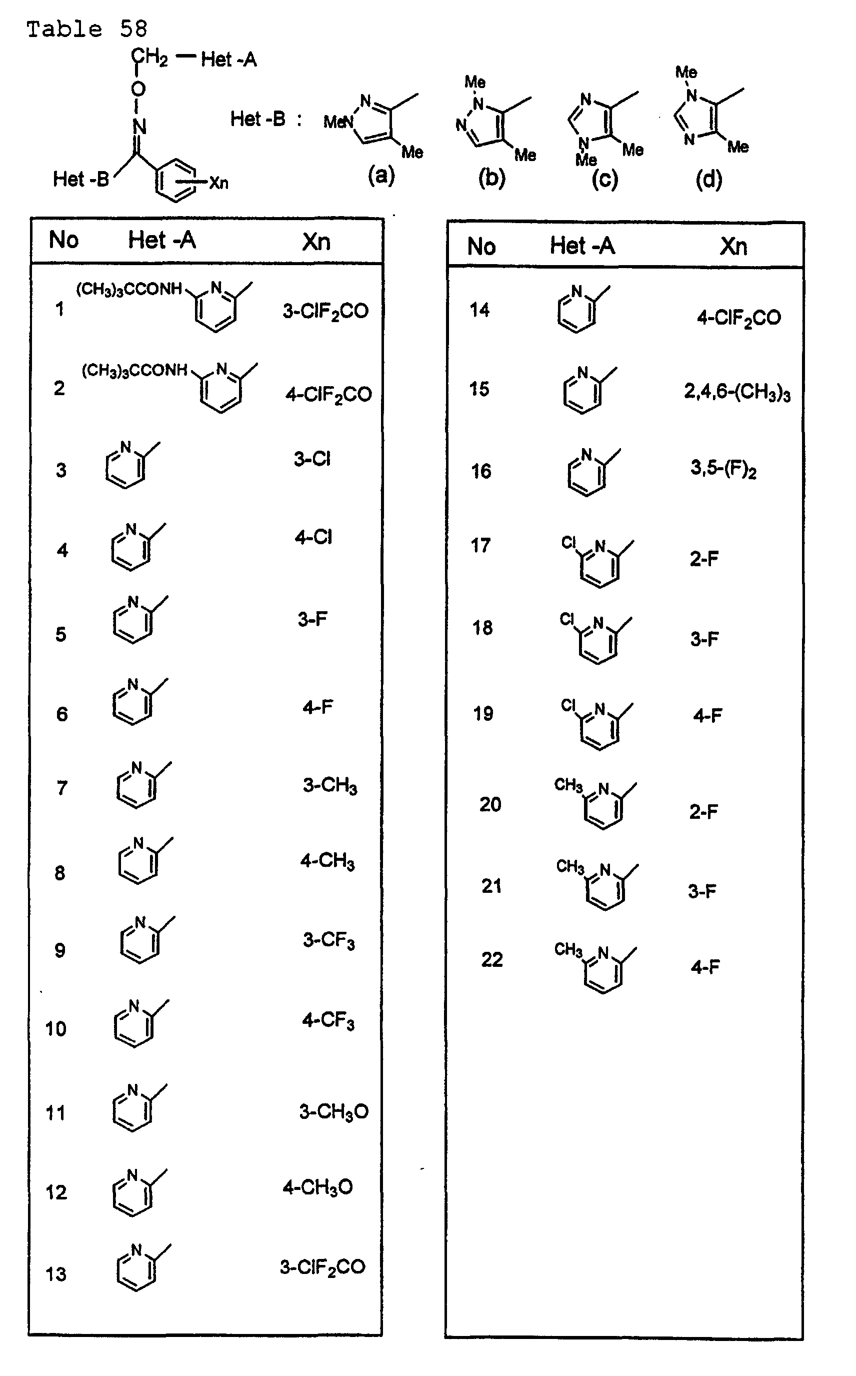

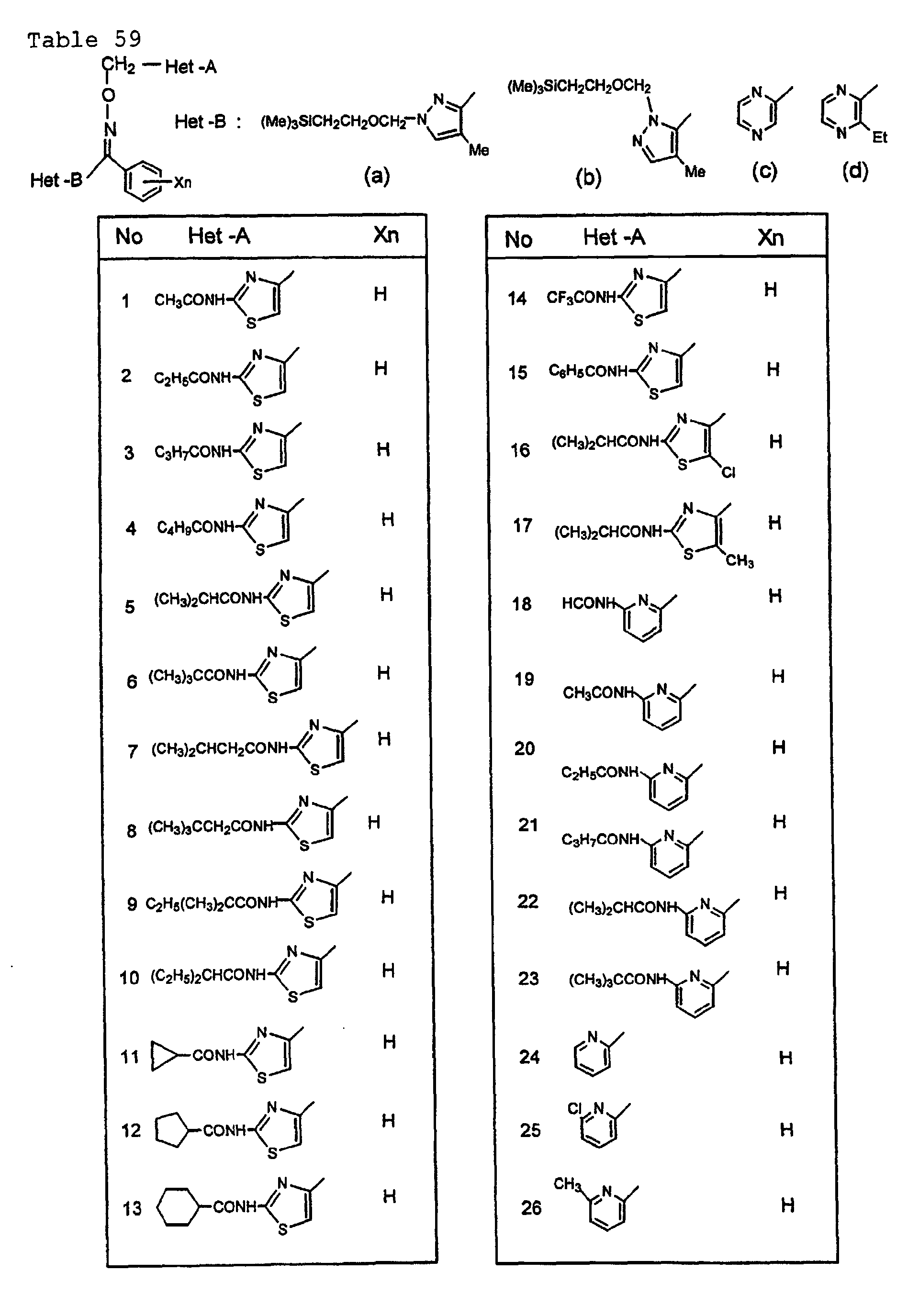

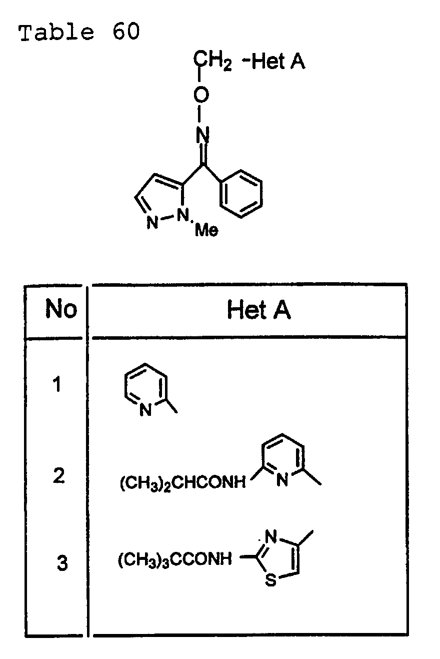

Specific structures of the hydroxime derivative represented by the general formula (2) prepared by the method described above are as shown in Tables 61 to 71. In the tables, Het B, Het C, R³, X, Y, Z, and n are as defined in the general formula (2), and the abbreviations Me, Et, Pr, Bu, and Ph respectively denote a methyl group, an ethyl group, a propyl group, a butyl group, and a phenyl group.

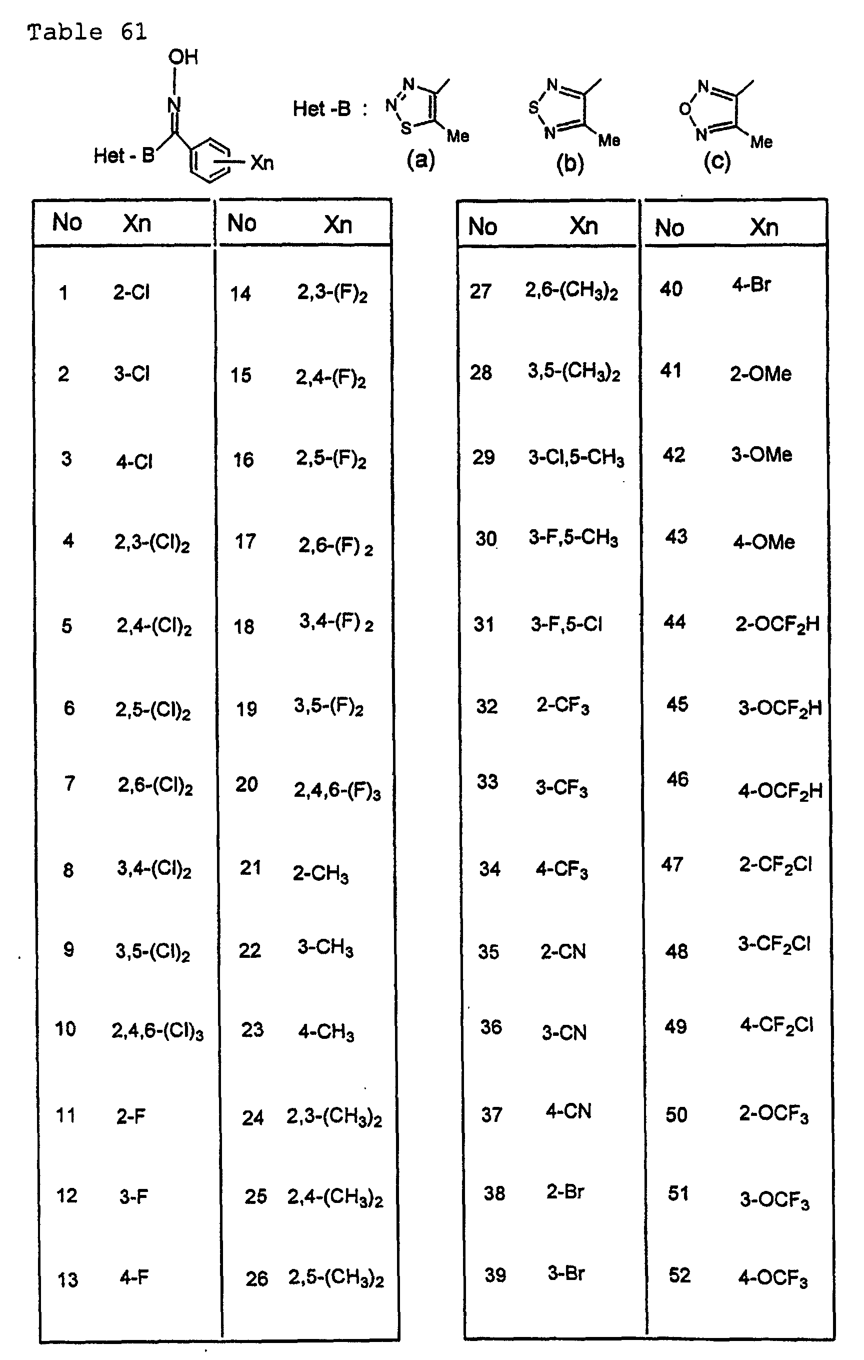

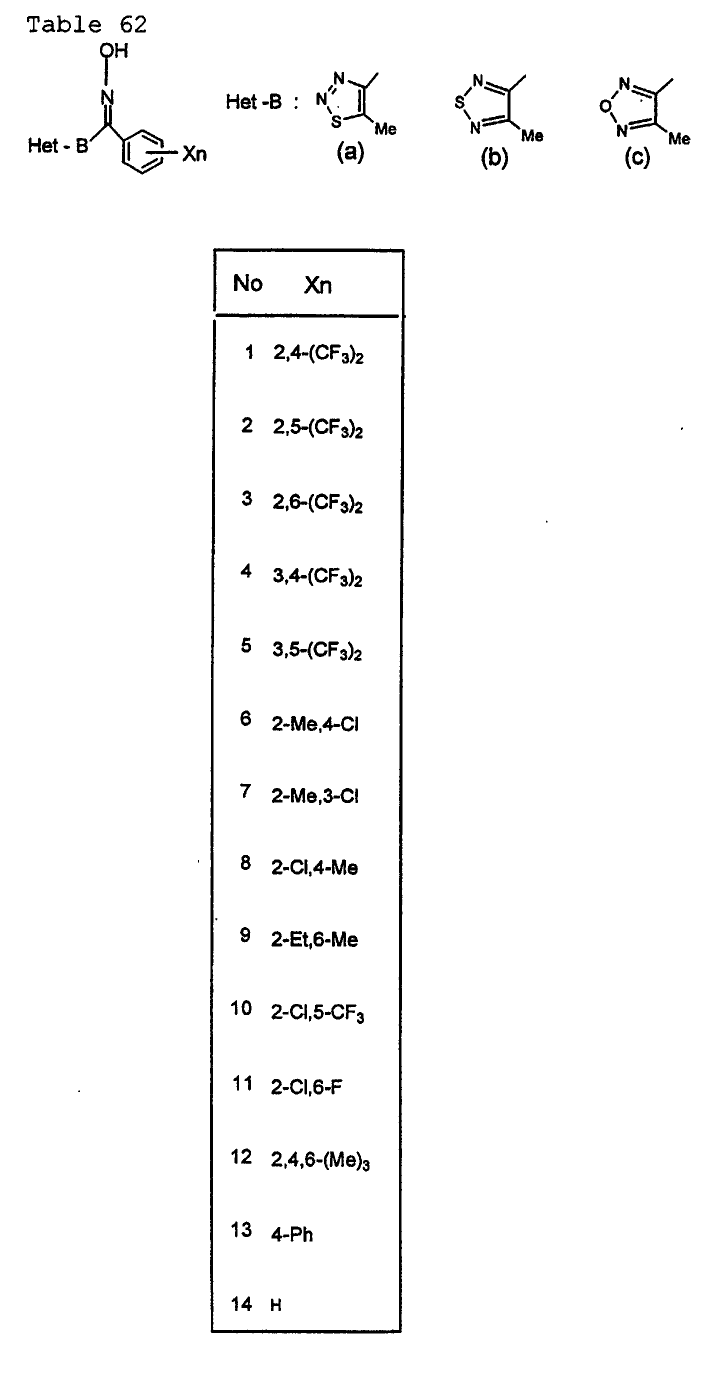

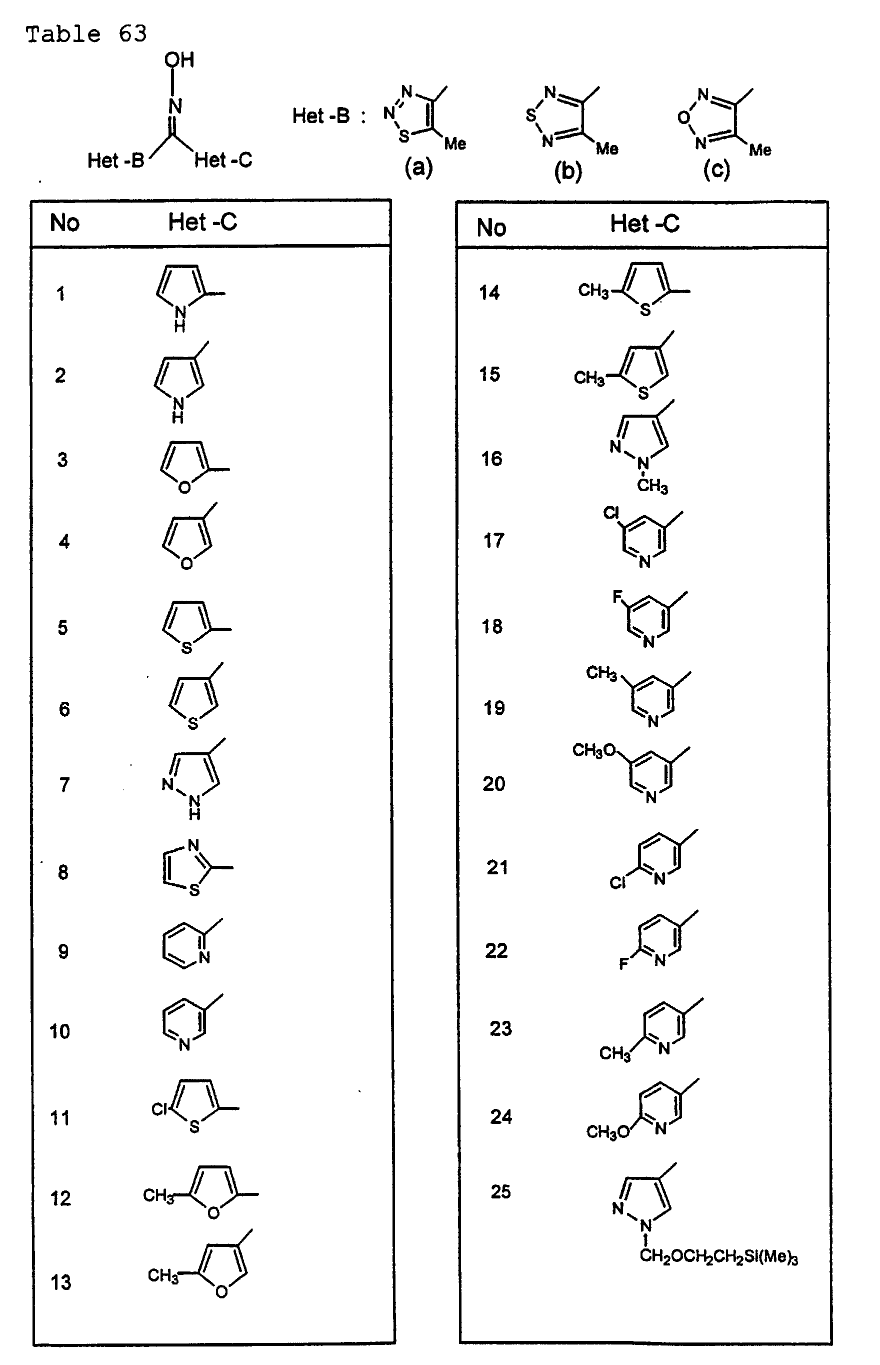

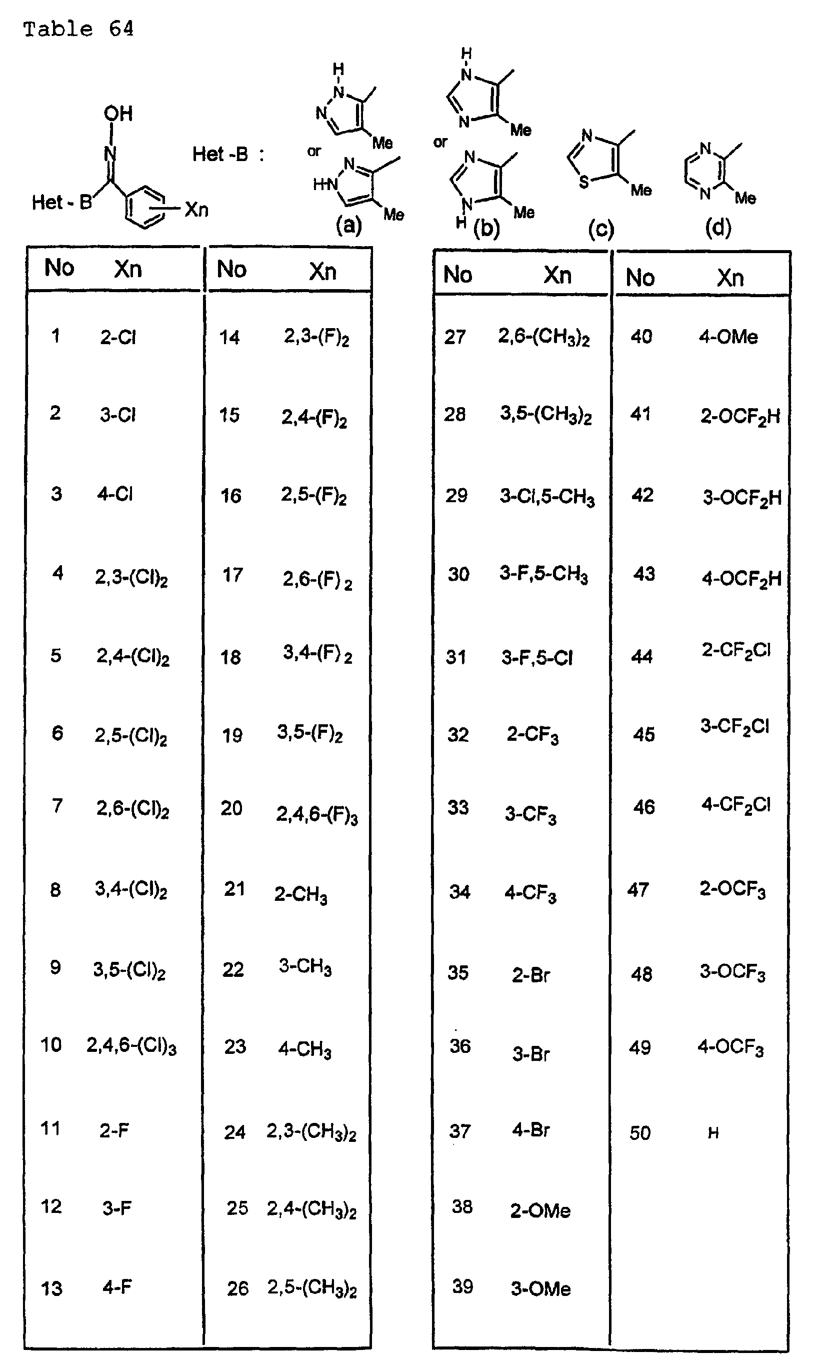

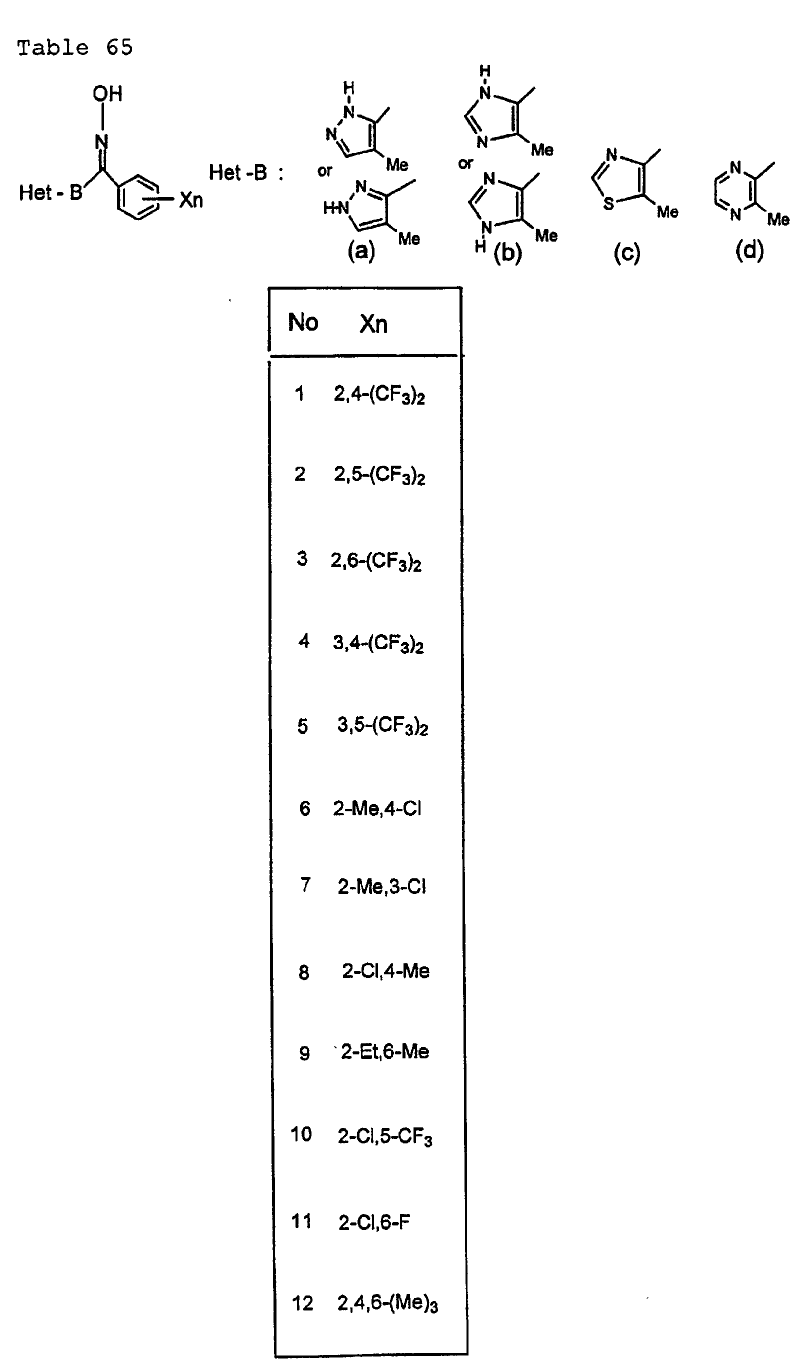

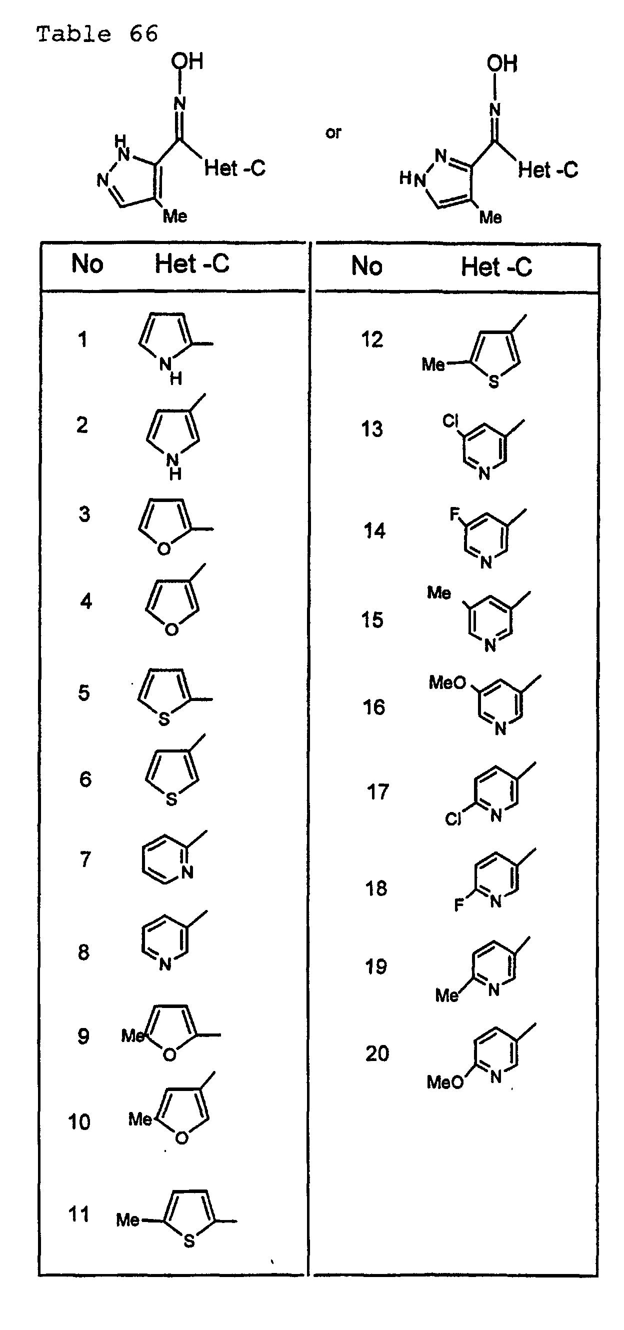

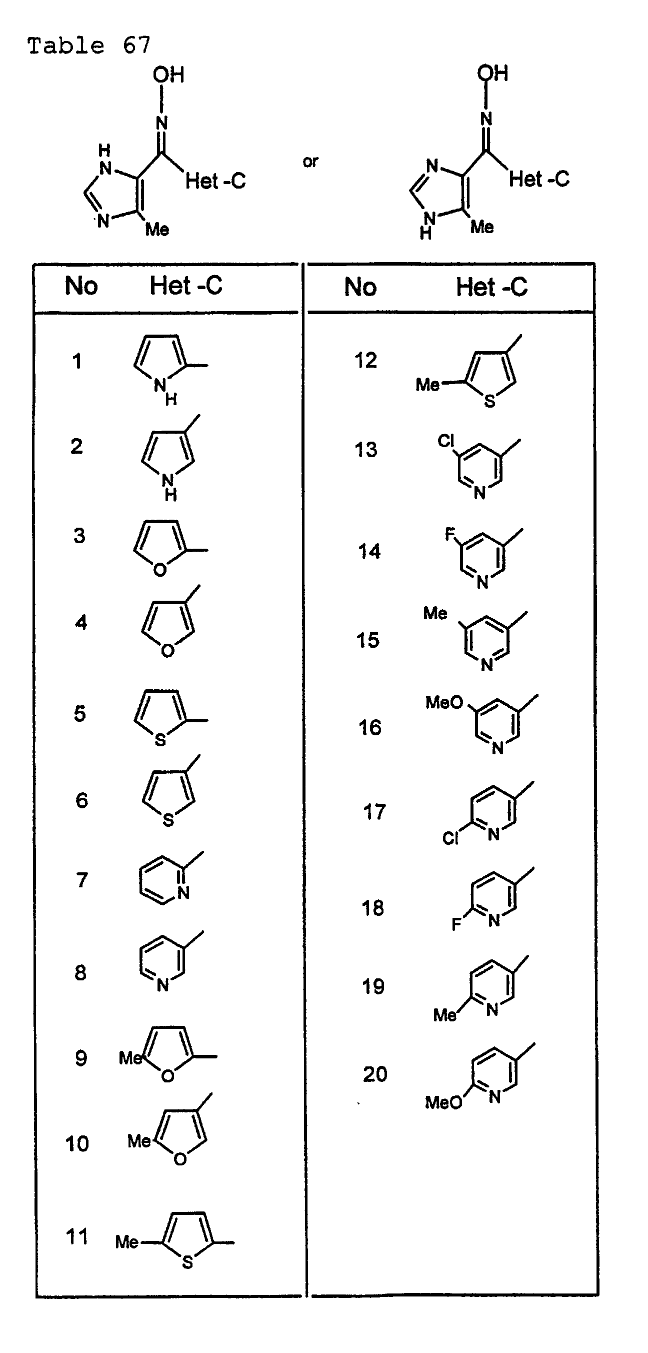

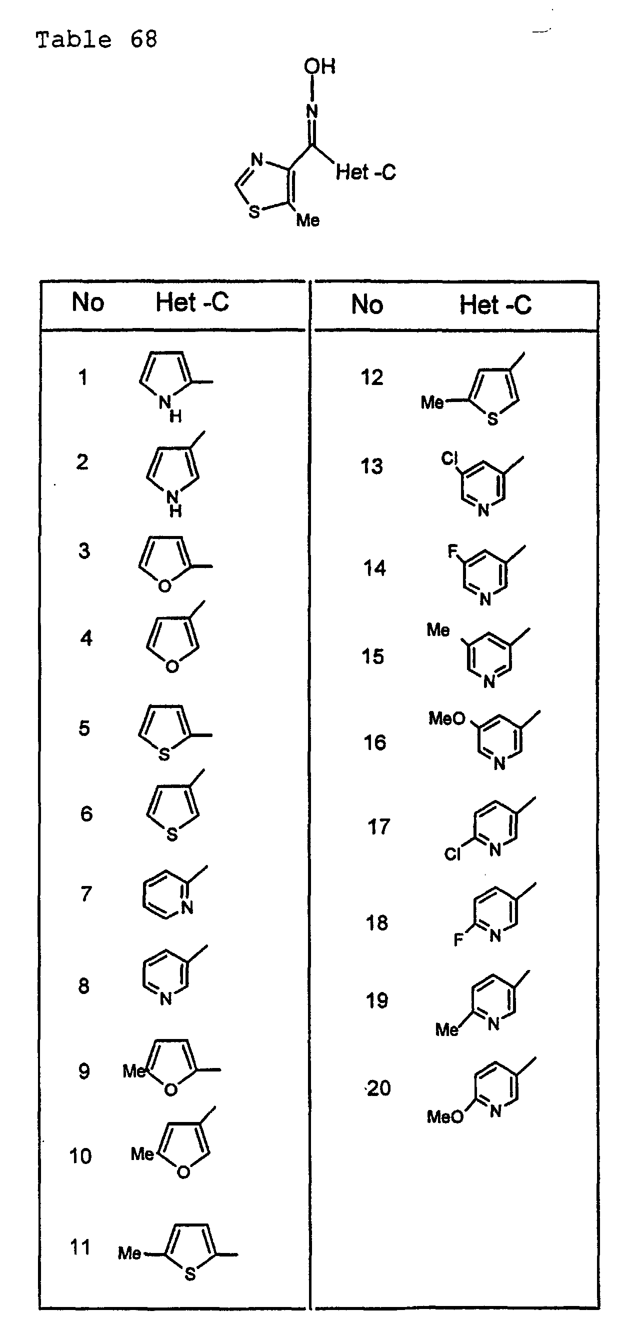

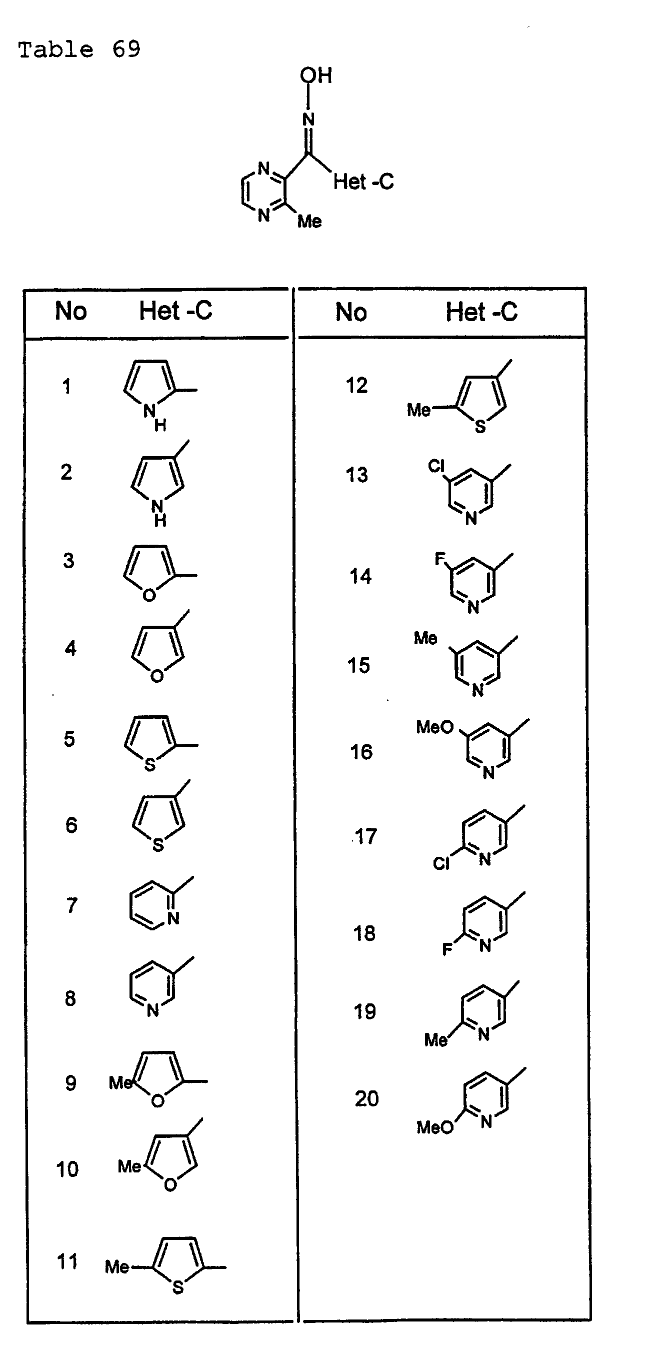

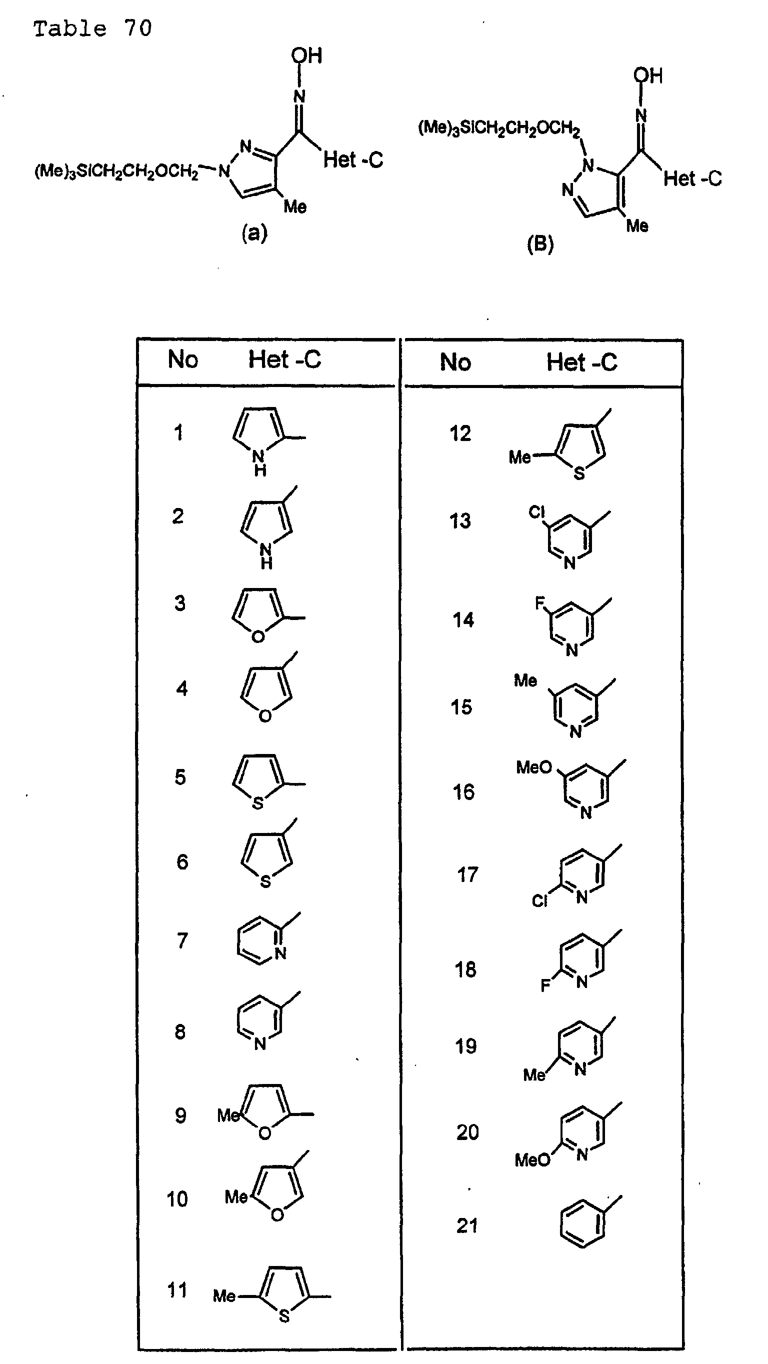

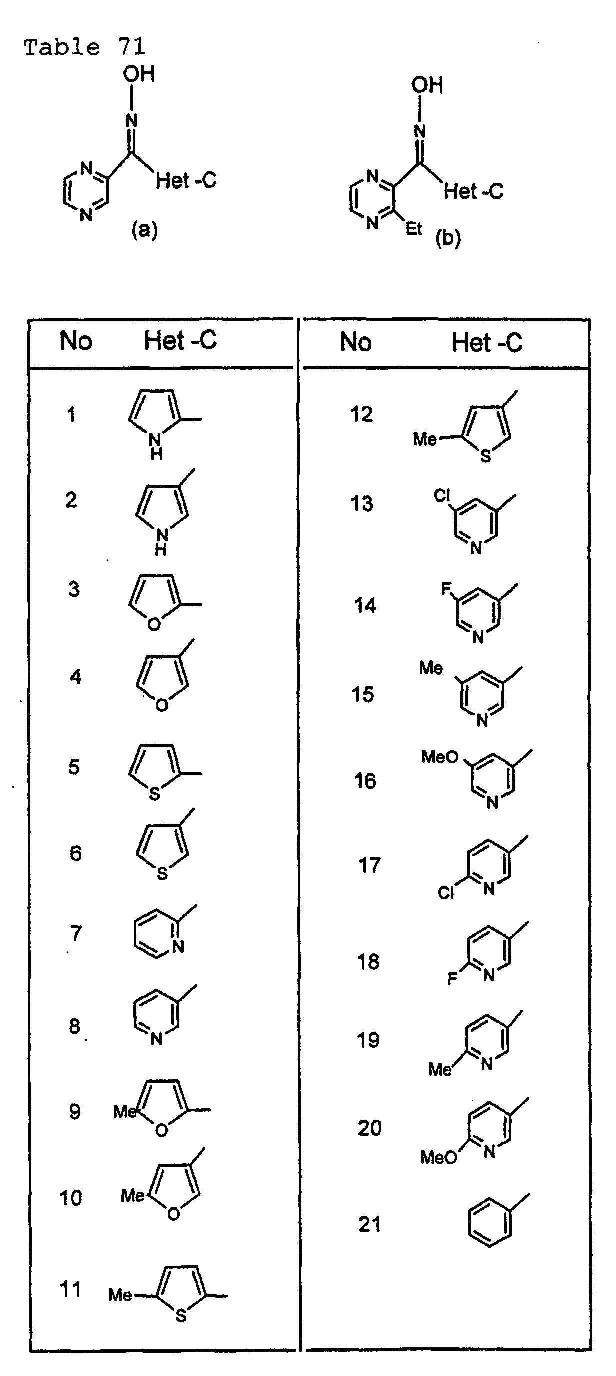

The agricultural chemical containing the oxime derivative as an active ingredient, especially the plant disease controlling agent, is effective against various plant diseases caused by pathogenic plant viruses, bacteria, and filamentous fungi. Plant diseases caused by filamentous fungi include, for example, various plant diseases caused by Oomycetes and plant diseases caused by *Pyricularia oryzae.*

These diseases are downy mildew and late blight or Phytophthora rot, etc., in various plants, and the agricultural chemical of the present invention has excellent control effect on various plant diseases caused by Oomycetes, for example, downy mildew (*Plasmopara viticola)* on grape; downy mildew (*Pseudoperonospora cubensis)* and phytophthora rot (*Phytophthora melonis*) on cucurbitaceous plants; Phytophthora rot (*Photophthora capsici)* and late blight *(Phytophthora inrestans*) on tomato; downy mildew (*Peronospora brassicae*) on cruciferous plants; downy mildew (*Peronospora destructor)* on welsh onion; downy mildew (*Peronospora spinaciae*) on spinach; downy mildew (*Peronospora manshurica)* on soy bean; downy mildew (*Peronospora viciae)* on broad bean; black shank (*Phytophthora nicotianae* var. *nicotianae)* on tobacco; late blight (*Phytophthora infestans)* on potato; downy mildew (*Pseudoperonospora humuli*) on hops; root rot wilt (*Phytophthora cinnamomi*) on pineapple, phytophthora blight (*Phytophthora capsici*) on bell pepper; red stele (*Phytophthora fragariae*) on strawberry; damping-off (caused by bacteria of the genus *Pythium*) on various plants; and phythium rot (*Pythium aphandidermatum*) on bentgrass; and rice blast (*Pyricularia oryzae*) on rice.

The agricultural chemical is also effective against powdery mildew (*Spaerotheca fuliginea*) and damping-off (*Rhizoctonia solani*) on cucumber; alternaria leaf spot (*Alternaria mali*) on apple; and fusarium wilt (*Fusarium oxysporum*) on cucumber.

Although this chemical can be used alone as an active ingredient, the active ingredient is typically mixed with publicly known conventional solid and liquid carriers used in the formulation of agricultural chemicals, and auxiliaries such as dispersants, diluents, emulsifiers, spreaders, and thickeners to give formulations in the form of wettable powders, liquid formulations, oil solutions, dusts, granules, and sols (flowables).

Examples of the solid and liquid carriers include talc, clay, bentonite, kaolin, diatomaceous earth, montmorillonite, mica, vermiculite, gypsum, calcium carbonate, white carbon, wood flour, starch, alumina, silicate, dextrin, waxes, alcohols (e.g., methyl alcohol, ethyl alcohol, n-propyl alcohol, isopropyl alcohol, n-butyl alcohol, ethylene glycol, etc.), petroleum fraction (e.g., petroleum ether, kerosine, solvent naphtha, etc.), aliphatic or alicyclic hydrocarbons (e.g., n-hexane, cyclohexane, etc.), aromatic hydrocarbons (e.g., benzene, toluene, xylene, ethylbenzene, chlorobenzene, cumene, methylnaphthalene, etc.), halogenated hydrocarbons (e.g., chloroform, dichloromethane, etc.), ethers (e.g., isopropyl ether, ethylene oxide, tetrahydrofuran, etc.), ketones (e.g., acetone, methyl ethyl ketone, cyclohexanone, methyl isobutyl ketone, etc.), esters (e.g., ethyl acetate, butyl acetate, ethylene glycol acetate, amyl acetate, etc.), acid amides (e.g., dimethylformamide, diethylacetanilide, etc.), nitriles (e.g., acetonitrile, propionitrile, acrylonitrile, etc.), sulfoxides (e.g., dimethyl sulfoxide, etc.), and alcohol ethers (e.g., ethylene glycol monomethyl ether, ethylene glycol monoethyl ether, etc.).

Examples of the auxiliary include nonionic surfactants (e.g., polyoxyethylene alkyl ether, polyoxyethylene alkyl ester, polyoxyethylene alkyl phenyl ether, polyoxyethylene sorbitan alkyl ester, sorbitan alkyl ester, etc.), anionic surfactants (e.g., alkylbenzene sulfonate, alkyl sulfosuccinate, polyoxyethylene alkyl sulfonate, aryl sulfonate, etc.), cationic surfactants (e.g., alkylamines, polyoxyethylene alkylamines, quaternary ammonium salts, etc.), amphoteric surfactants (e.g., alkylaminoethylglycine, alkyldimethylbetaine, etc.), polyvinyl alcohol, hydroxypropylcellulose, carboxymethylcellulose, gum arabic, tragacanth gum, xanthan gum, polyvinyl acetate, gelatin, casein, and sodium alginate.

Furthermore, this chemical can be mixed with agricultural chemicals such as publicly known conventional fungicides, herbicides, plant growth regulators, insecticides and acaricides, and fertilizers. The content of this chemical varies according to the type of the formulation, the method of application, and other conditions, but is usually within a range from 0.5 to 95% (by weight), and preferably from 2 to 70% (by weight).

The method of applying this chemical includes, for example, application to plants (stem and leaf application), application to growing soil of plants (soil application), application to the surface of the water in a paddy field (water surface application), and application to seeds (seed treatment).

The amount of this chemical to be applied varies according to the kind of plan and the type of damage. In the case of the stem and leaf application, a solution containing an active ingredient in the concentration within a range of 1 to 10000 ppm, preferably from 10 to 1000 ppm, is preferably applied in the amount within a range from 50 to 300 liters per 10 ares. In the case of the soil application and water surface application, the active ingredient is preferably applied in the amount within a range from 0.1 to 1000 g, and particularly preferably from 10 to 100 g, per 10 ares. In the case of the seed treatment, the active ingredient is preferably applied in the amount within a range from 0.001 to 50 g per 1 kg of seeds.

The following Preparation Examples, Formulation Examples, and Test Examples further illustrate the present invention, although the present invention is not limited to these Examples. The compound No. shows (No. of table) - (No. of Het B) - (compound No. in table).

### Preparation Example 1

To an acetonitrile (20 ml) solution of 3-(3-methyl-1,2,5-thiadiazol-4-yl)pyridinemethaneoxime (150 mg, 0.68 mmol, E:Z = 1:4), potassium carbonate (160 mg, 1.15 mmol)and 2-picolyl chloride (149 mg, 0.87 mmol) were added, and then the mixture was heated and refluxed for 7 hours. After adding water to the reaction solution, the reaction solution was extracted with ethyl acetate, washed with water, and then dried over magnesium sulfate. The solvent was distilled off, and the residue was purified by column chromatography to obtain (Z)-3-(3-methyl-1,2,5-thiadiazol-4-yl)pyridylmethanone 0-(2-pyridyl)methyloxime (157 mg) and (E)-3-(3-methyl-1,2,5-thiadiazol-4-yl)pyridylmethanone O-(2-pyridyl)methyloxime (47 mg) (compound No. 31-(b)-24).
(Z)-3-(3-methyl-1,2,5-thiadiazol-4-yl)pyridylmethanone O-(2-pyridyl)methyloxime
¹H-NMR(CDCl₃): δ2.50(s, 3H), 5.39(s, 2H), 7.10-7.48(m, 3H), 7.69(t, j=7.7Hz, 1H), 7.81(d, j=7.9Hz, 1H), 8.48-8.70(m, 3H). MS(m/z) : 312(M⁺+1).

(E)-3-(3-methyl-1,2,5-thiadiazol4-yl)pyridylmethanone O-(2-pyridyl)methyloxime
¹H-NMR(CDCl₃): δ2.66(s, 3H), 5.42(s, 2H), 7.13-7.51(m, 3H), 7.70(t, j=7.6Hz, 1H), 7.85(d, j=7.9Hz, 1H), 8.60(d, j=4.8Hz, 1H), 8.67(d,j=4.95Hz,1H), 8.75(d, j=2.2Hz, 1H).
MS(m/z): 312(M⁺+1).

### Preparation Example 2

To DMF (6 ml), 60% sodium hydrate (80 mg, 2 mmol) was added. After ice cooling, DMF (6 ml) of 2-(3-methyl-1,2,5-oxadiazol-4-yl)pyridinemethanoneoxime (160 mg, 0.78 mmol, E:Z = 1:4) was added, followed by stirring for 30 minutes. To the reaction solution, a DMF (6 ml) solution of 4-chloromethyl-2-isopropionylaminothiazole (222 mg, 1.02 mmol) was added, followed by stirring at room temperature for 21 hours. After the reaction solution was concentrated under reduced pressure, ethyl acetate/aqueous ammonium chloride was added to the residue, and the solution was extracted. The extract was washed with water and was then dried over magnesium sulfate. The solvent was distilled off, and the residue was purified by column chromatography to obtain (Z)-2-(3-methyl-1,2,5-oxadiazol-4-yl)pyridylmethanone O-(2-isopropionylaminothiazol-4-yl)methyloxime (135 mg) (compound No. 32-(c)-5).
¹H-NMR(CDCl₃) : δ1.28(d, j=6.9Hz, 6H), 2.28(s, 3H), 2.43-2.80(m, 1H), 5.29(s, 2H), 6.92(s, 1H), 7.75(t, j=7.7Hz, 1H), 7.94(d, j=8.0Hz, 1H), 8.53(d, j=4.8Hz, 1H), 8.83-9.09(brd, 1H).
MS(m/z): 386(M⁺).

### Preparation Example 3

To DMF (2 ml), 60% sodium hydrate (19 mg, 0.47 mmol) was added. After ice cooling, 3-(3-methyl-1,2,5-thiadiazol-4-yl)pyridinemethanoneoxime (44 mg, 0.2 mmol, E:Z = 1:2.3) was added, followed by stirring for 30 minutes. To the reaction solution, a DMF (1 ml) solution of 2-bromomethyl-6-isopropionylaminopyridine (57 mg, 0.22 mmol) was added, followed by stirring at room temperature for 3 days. After the reaction solution was concentrated under reduced pressure, ethyl acetate/aqueous sodium hydrogencarbonate was added to the residue, and the solution was extracted. The extract was washed with water and was then dried over magnesium sulfate. The solvent was distilled off, and the residue was purified by column chromatography to obtain (Z)-3-(3-methyl-1,2,5-thiadiazol-4-yl)pyridylmethanone 0-(6-isopropionylaminopyridin-2-yl)methyloxime (74 mg) (compound No. 29-(b)-25).
¹H-NMR(CDCl₃) : δ1.27(d, j=6.9Hz, 6H), 2.49(s, 3H), 2.56(m, 1H), 5.25(s, 2H), 7.02(d, j=7.6Hz, 1H), 7.30(dd, j=4.8, 8.0Hz, 1H), 7.71(t, j=8.0Hz, 1H), 7.80(dt, j=1.9, 8.1Hz, 1H), 7.94(brs, 1H), 8.17(d, j=8.1Hz, 1H), 8.63(dd, j=1.4, 7.6Hz, 1H), 8.63(d, j=4.8Hz, 1H).
MS(m/z): 396(M⁺).

### Preparation Example 4

To an acetonitrile (7.5 ml) solution of (Z)-2-(5-methyl-1,2,3-thiadiazol-4-yl)thienylmethanoneoxime (130 mg, 0.58 mmol, E:Z = 1:4), potassium carbonate (140 mg, 0.99 mmol) and 2-picolyl chloride (91 mg, 0.72 mmol) were added, and then the mixture was heated and refluxed for 16 hours. After adding water to the reaction solution, the reaction solution was extracted with ethyl acetate, was washed with water, and was then dried over magnesium sulfate. The solvent was distilled off, and the residue was purified by column chromatography to obtain (Z)-2-(5-methyl-1,2,3-thiadiazol-4-yl)thienylmethanone O-(2-pyridyl)methyloxime (163 mg)(compound No. 22-(a)-25).
¹H-NMR(CDCl₃) : δ2.56(s, 3H), 5.36(s, 2H), 6.85(dd, j=1.1, 3.7Hz, 1H), 6.99(dd, j=3.7, 5.1Hz, 1H), 7.20(m, 1H), 7.33(d,j=8.5Hz, 1H), 7.36(dd, j=1.1, 5.1Hz, 1H), 7.67(ddd, j=1.6, 7.7, 7.7Hz, 1H), 8.56(m, 1H).
MS(m/z): 316(M⁺).

### Preparation Example 5

3-(5-methyl-1,2,3-thiadiazol-4-yl)thienylmethanone (180 mg, 0.86 mmol) and hydroxylamine hydrochloride (147 mg, 2.11 mmol) were dissolved in pyridine (4 ml), followed by stirring at 60°C for 17 hours. After the reaction solution was concentrated under reduced pressure, ethyl acetate/dilute hydrochloric acid was added to the residue, and the solution was extracted. The extract was washed in turn with dilute hydrochloric acid, water, and saturated aqueous sodium chloride, and was then dried over magnesium sulfate. The solvent was distilled off, and the residue was purified by column chromatography to obtain (Z)-3-(5-methyl-1,2,3-thiadiazol-4-yl)thienylmethanoneoxime (136 mg) and (E)-3-(5-methyl-1,2,3-thiadiazol-4-yl)thienylmethanoneoxime (57 mg) (compound No.36-(a)-6).
(Z)-3-(5-methyl-1,2,3-thiadiazol-4-yl)thienylmethanoneoxime ¹H-NMR(CDCl₃): δ2.60(s, 3H), 7.18(dd,j=1.3; 3.0Hz,1H), 7.36(dd, j=3.0, 5.1Hz, 1H), 7.49(dd, j=1.3, 5.1Hz, 1H), 7.90(brd, 1H).
MS(m/z): 225(M⁺).
(E)-3-(5-methyl-1,2,3-thiadiazol4-yl)thienylmethanoneoxime ¹H-NMR(CDCl₃) : δ2.64(s, 3H), 7.33(dd,j=3.0, 5.1Hz, 1H), 7.42(dd, j=1.3,5.1Hz, 1H), 8.03(dd, j=1.3, 3.0Hz, 1H), 9.2(brd, 1H).
MS(m/z):225(M⁺)

### Preparation Example 6

4-chlorophenyl(5-methyl-1,2,3-thiadiazol-4-yl)methanone (3.24 g, 13.6 mmol) and hydroxylamine hydrochloride (2.32 g, 33.4 mmol) were dissolved in pyridine (60 ml), followed by stirring at 70°C for 6.5 hours. After the reaction solution was concentrated under reduced pressure, ethyl acetate/dilute hydrochloric acid was added to the residue, and the solution was extracted. The extract was washed in turn with dilute hydrochloric acid, water, and saturated aqueous sodium chloride, and was then dried over magnesium sulfate. The solvent was distilled off, and the residue was purified by column chromatography to obtain (Z)-4-chlorophenyl(5-methyl-1,2,3-thiadiazol-4-yl)methanoneoxime (2.35 g) and (E)-4-chlorphenyl(5-methyl-1,2,3-thiadiazol-4-yl)methanoneoxime (1.14 g) (compound No. 34-(a)-3).
(Z)-4-chlorophenyl(5-methyl-1,2,3-thiadiazol-4-yl)methanoneoxime
¹H-NMR(CDCl₃): δ2.57(s, 3H), 7.34(d, j=8.9Hz, 2H), 7.43(d, j=8.9Hz, 2H), 8.04(s, 1H).
MS(m/z) : 253(M⁺).
(E)-4-chlorophenyl(5-methyl-1,2,3-thiadiazol-4-
yl)methanoneoxime
¹H-NMR(CDCl₃) : δ2.70(s, 3H), 7.43(d, j=8.9Hz, 2H), 7.50(d, j=8.9Hz, 2H), 8.13(s, 1H).
MS(m/z): 253(M⁺).

### Preparation Example 7

4-fluorophenyl(3-methyl-1,2,5-thiadiazol-4-yl)methanone (1.0 g, 4.5 mmol)and hydroxylamine hydrochloride (0.44 g, 6.3 mmol) were dissolved in ethanol (10 ml) and water (5 ml), and potassium carbonate (0.37 g, 2.7 mmol) was added, followed by stirring at 90°C for 16 hours. After the reaction solution was concentrated under reduced pressure, ethyl acetate/water was added to the residue, and the solution was extracted. The extract was washed in turn with dilute hydrochloric acid, water, and saturated aqueous sodium chloride, and was then dried over magnesium sulfate. The solvent was distilled off, and the residue was purified by column chromatography to obtain a mixture (E:Z = 1:3.7, 1.13 g) (compound No. 34-(b)-13) of (Z)-4-fluorophenyl(3-methyl-1,2,5-thiadiazol-4-yl)methanoneoxime and (E)-4-fluorophenyl (3-methyl-1,2,5-thiadiazol-4-yl)methanoneoxime.
¹H-NMR(CDCl₃) : δ2.53(Z), 2.70(E) (s, 3H), 6.90-7.21(m, 2H), 7.22-7.60(m, 2H), 8.52-8.82(brd, 1H).
MS(m/z): 237 (M⁺).

### Preparation Example 8

4-fluorophenyl (3-methyl-1,2,5-oxadiazol-4-yl)methanone (752 mg, 3.65 mmol) and an aqueous 50% hydroxylamine solution (730 mg, 11.0 mmol)were dissolved in ethanol (10 ml) and water (2 ml), followed by stirring at 80°C for 16 hours. After the reaction solution was concentrated under reduced pressure, the residue was extracted with ethyl acetate. The extract was washed with saturated aqueous sodium chloride, and was then dried over magnesium sulfate. The solvent was distilled off, and the residue was purified by column chromatography to obtain (Z)-4-fluorophenyl(3-methyl-1,2,5-oxadiazol-4-yl)methanoneoxime (557 mg) (compound No. 34-(c)-13).
¹H-NMR(CDCl₃): δ2.38(Z)(s, 3H), 7.00-7.18(m, 2H), 7.46-7.64(m, 2H), 7.90(s, 1H).
MS(m/z): 221(M⁺).

### Preparation Example 9

To DMF (1 ml), 60% sodium hydrate (20 mg, 0.51 mmol) was added. After ice cooling, (Z)-4-fluorophenyl(3-methyl-1,2,5-thiadiazol-4-yl)methanoneoxime (48 mg, 0.2 mmol) was added, followed by stirring for 10 minutes. To the reaction solution, a DMF (1 ml) solution of 2-bromomethyl-6-isopropionylaminopyridine (52 mg, 0.20 mmol) was added, followed by stirring at room temperature for 18 hours. After the reaction solution was concentrated under reduced pressure, ethyl acetate/water was added to the residue, and the solution was extracted. The extract was washed with water and was then dried over magnesium sulfate. The solvent was distilled off, and the residue was purified by column chromatography to obtain (Z)-4-fluorophenyl(3-methyl-1,2,5-thiadiazol-4-yl)methanone O-(6-isopropionylaminopyridin-2-yl)methyloxime (36 mg) (compound No. 12-(b)-13)
¹H-NMR(CDCl₃): δ1.21(d, j=7.0Hz, 6H), 2.47(s, 3H), 2.54(m, 1H), 5.21(s, 2H), 7.03(dd, j=8.2, 9.0Hz, 2H), 7.05(d, j=7.2Hz, 1H), 7.41(dd, j=5.4, 9.0Hz, 2H), 7.69(dd, j=7.2, 8.2Hz, 1H),7.86(brs, 1H), 8.15(d, j=8.2Hz, 1H).
MS (m/z) : 414(M⁺+1).

### Preparation Example 10

To DMF (1 ml), 60% sodium hydrate (18 mg, 0.46 mmol) was added. After ice cooling, (Z)-4-fluorophenyl(3-methyl-1,2,5-oxadiazol-4-yl)methanoneoxime (41 mg, 0.18 mmol) was added, followed by stirring for 10 minutes. To the reaction solution, a DMF (1 ml) solution of 2-bromomethyl-6-tert-butyroylaminopyridine (50 mg, 0.18 mmol) was added, followed by stirring at room temperature for 18.5 hours. After the reaction solution was concentrated under reduced pressure, ethyl acetate/water was added to the residue, and the solution was extracted. The extract was washed with water and was then dried over magnesium sulfate. The solvent was distilled off, and the residue was purified by column chromatography to obtain (Z)-4-fluorophenyl(3-methyl-1,2,5-oxadiazol-4-yl)methanone O-(6-tert-butyroylaminopyridin-2-yl)methyloxime (22 mg) (compound No. 14-(c)-13).
¹H-NMR(CDCl₃): δ1.33(s, 9H), 2.33(s,3H), 5.25(s, 2H), 7.01(d, j=7.6Hz, 1H), 7.06(dd, j=7.6, 8.2Hz, 1H), 7.52(dd, j=5.3, 8.9Hz, 2H), 7.70(dd, j=7.6, 8.2Hz, 1H), 7.98(brs, 1H), 8.18(d, j=8.2Hz, 1H).
MS(m/z) : 41.2(M⁺+1).

### Preparation Example 11

To a pyridine (8 ml) solution of 1-(2-trimethylsilylethoxymethyl)-4-methylpyrazol-3-yl)phenylmethanone (0.30 g, 0.95 mmol), hydroxylamine hydrochloride (0.17 g, 2.4 mmol) was added, followed by stirring at 70°C for 6.5 hours. After the reaction solution was concentrated under reduced pressure, the residue was dissolved in ethyl acetate, and the solution was washed in turn with dilute hydrochloric acid and saturated aqueous sodium bicarbonate, and was then dried over magnesium sulfate. The solvent was distilled off, and the residue was purified by column chromatography to obtain a mixture Z/E = 60/40, 0.31 g) (compound No. 70-(a)-21) of (Z)-(1-(2-trimethylsilylethoxymethyl)-4-methylpyrazol-3-yl)phenylmethanoneoxime and(E)-(1-(2-trimethylsilylethoxymethyl)-4-methylpyrazol-3-yl)phenylmethanoneoxime.
¹H-NMP(CDCl₃): δ-0.03, 0.00(s, 9H), 0.88, 0.92(m, 2H), 1.90, 2.03(s, 3H), 3.53, 3.59(m, 2H), 5.33, 5.44(s, 2H), 7.30-7.55(m, 6H), 7.72, 8.98(brd, 1H).

### Preparation Example 12

A DMF (4 ml) solution of a mixture (0.10 g, 0.30 mmol) of (Z)-(1-(2-trimethylsilylethoxymethyl)-4-methylpyrazol-3-yl)phenylmethanoneoxime and (E)-(1-(2-trimethylsilylethoxymethyl)-4-methylpyrazol-3-yl)phenylmethanoneoxime was ice-cooled, and sodium hydride (30 mg, 0.75 mmol) and 4-chloromethyl-2-isopropionylaminothiazol (82 mg, 0.38 mmol) were added with stirring. The reaction solution was cooled to room temperature and was then stirred for 3 hours. Saturated aqueous ammonium chloride was added to the reaction solution, and the solution was extracted with ethyl acetate. The extract was washed with saturated aqueous sodium chloride and was then dried over magnesium sulfate. The solvent was distilled off, and the residue was purified by column chromatography to obtain a mixture (Z/E = 65/35, 110 mg) (compound No. 59-(a)-5) of (Z)-(1-(2-trimethylsilylethoxymethyl)-4-methylpyrazol-3-yl)phenylmethanone O-(2-isopropylaminothiazol-4-yl)methyloxime and (E)-(1-(2-trimethylsilylethoxymethyl)-4-methylpyrazol-3-yl)phenylmethanone O-(2-isopropylaminothiazol-4-yl)methyloxime.
¹H-NMR(CDCl₃): δ-0.03, 0.00(s, 9H), 0.87, 0.90(m, 2H), 1.24(d, j=7.0Hz, 6H), 1.94, 2.03(s, 3H), 2.59(seq, j=7.0Hz, 1H), 3.51, 3.55(m, 2H), 5.20, 5.24(s, 2H), 5.31, 5.40(s, 2H), 6.85,6.88(s, 1H), 7.25-7.40(m, 3H), 7.40-7.56(m, 3H), 9.30(brd, 1H).
MS(m/z): 513(M⁺).

### Preparation Example 13

To a pyridine (10 ml) solution of 4-fluorophenyl(3-methylpyrazin-2-yl)methanone (0.58 g, 2.7 mmol) and hydroxylamine hydrochloride (0.47 g, 6.7 mmol) was added, followed by stirring at 60°C for 5 hours. After the reaction solution was concentrated, the residue was dissolved in ethyl acetate. The solution was washed in turn with dilute hydrochloric acid, saturated aqueous sodium bicarbonate, and saturated aqueous sodium chloride, and was then dried over magnesium sulfate. The solvent was distilled off, and the residue was purified by column chromatography to obtain (Z)-4-fluorophenyl(3-methylpyrazin-2-yl)methanoneoxime (0.43 g) and (E)-4-fluorophenyl(3-methylpyrazin-2-yl)methanoneoxime (0.13 g, compound No. 64-(d)-13).
(Z)-4-fluorophenyl(3-methylpyrazin-2-yl)methanoneoxime ¹H-NMR(CDCl₃): δ2.52(s, 3H), 7.04(m, 2H), 7.42(m, 2H), 8.18(brd, 1H), 8.56(s, 2H).
(E)-4-fluorophenyl(3-methylpyrazin-2-yl)methanoneoxime H-NMR(CDCl₃): δ2.58(s, 3H), 7.09(m, 2H), 7.56(m, 2H), 8.48(d, j=2.6Hz, 1H), 8.50(d, j=2.6Hz, 1H), 9.86(brd, 1H).

### Preparation Example 14

A DMF (4 ml) solution of (Z)-4-fluorophenyl(3-methylpyrazin-2-yl)methanoneoxime (90 mg, 0.39 mmol) was ice-cooled, and sodium hydride (47 mg, 1.2 mmol) and 4-chloormethyl-2-tert-butyroylaminothiazole (100 mg, 0.43 mmol) were added with stirring. The reaction solution was cooled to room temperature and was then stirred for 3.5 hours. Saturated aqueous ammonium chloride was added to the reaction solution, and the solution was extracted with ethyl acetate. The extract was washed with saturated aqueous sodium chloride and was then dried over magnesium sulfate. The solvent was distilled off, and the residue was purified by column chromatography to obtain (Z)-4-fluorophenyl(3-methylpyrazin-2-yl)methanone O-(2-tert-butyroylaminothiazol-4-yl)methyloxime (123 mg, compound No. 50-(d)-4).
¹H-NMR(CDCl₃) : δ1.32(s, 9H), 2.44(s, 3H), 5.19(s, 2H), 6.84(s, 1H), 7.03(m, 2H), 7.42(m, 2H), 8.52(s, 2H), 8.94(brd, 1H).

Physicochemical data such as ¹H-NMR spectrum and mass spectrum of the oxime derivatives and hydroxime derivatives prepared in the same manner as in these Preparation Examples are summarized in Tables 72 to 91. Regarding the indication of the compounds in the tables, for example, the compound 1-(a)-1 represents a compound No. 1 in Table 1 in which Het B is (a).

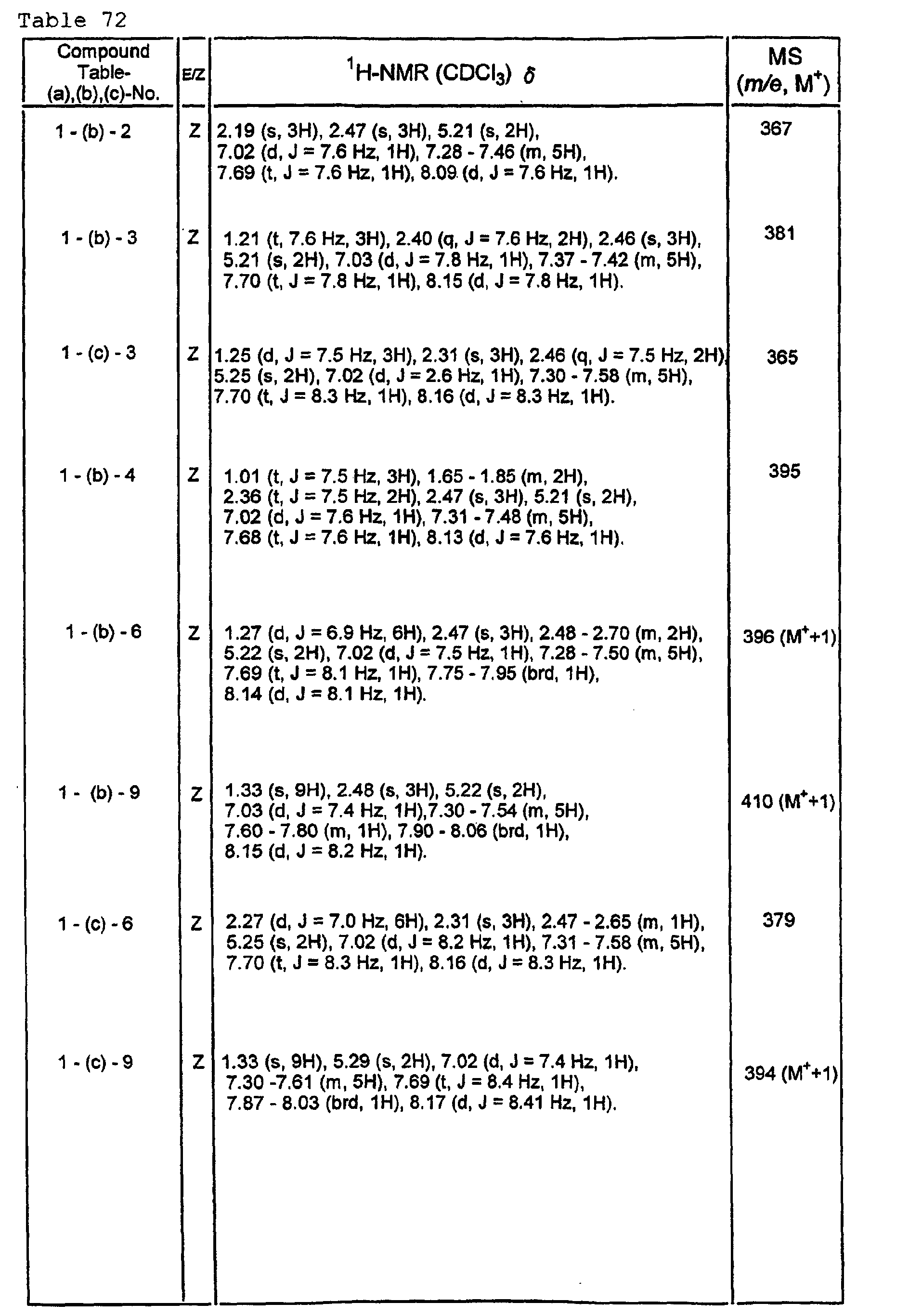

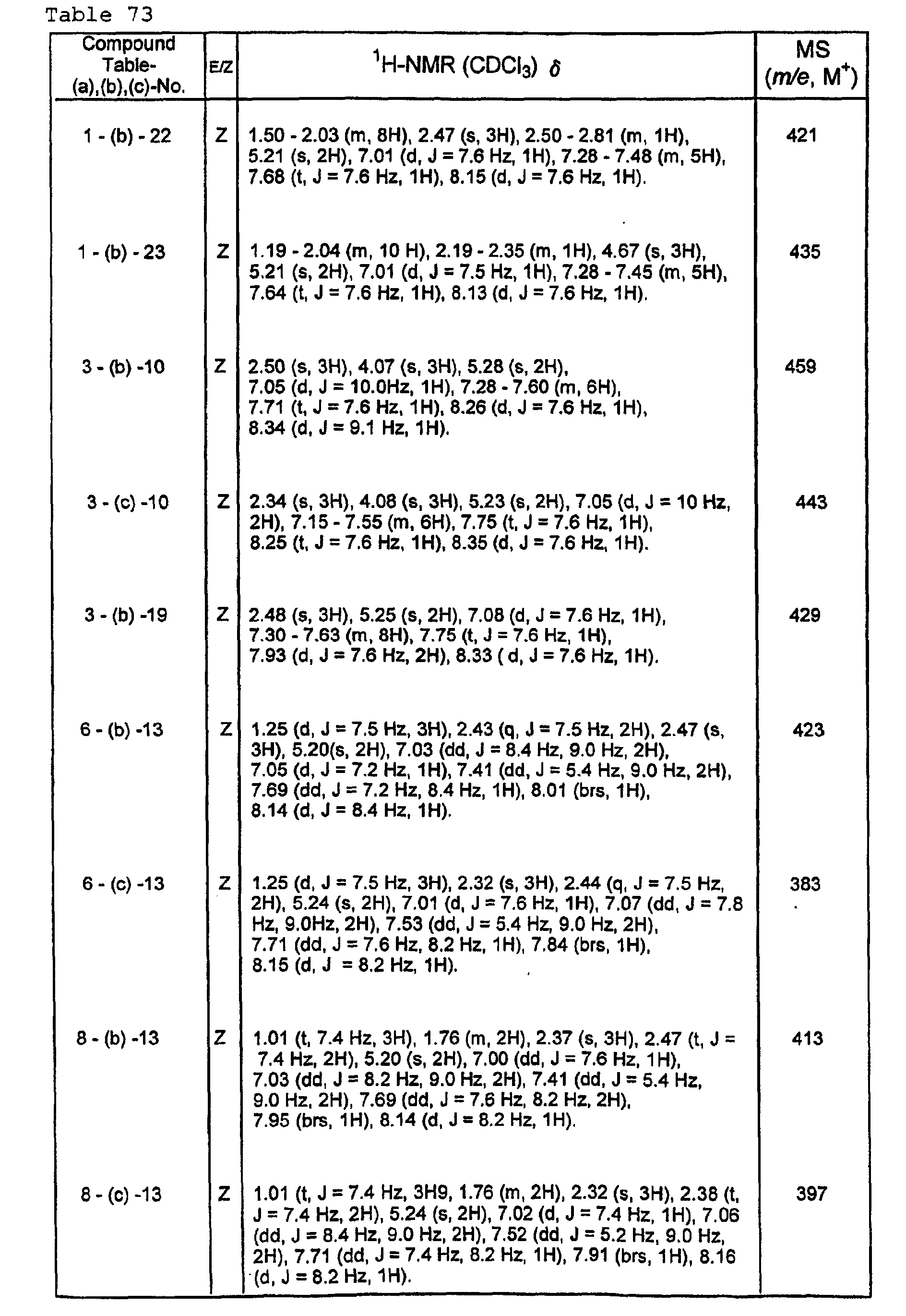

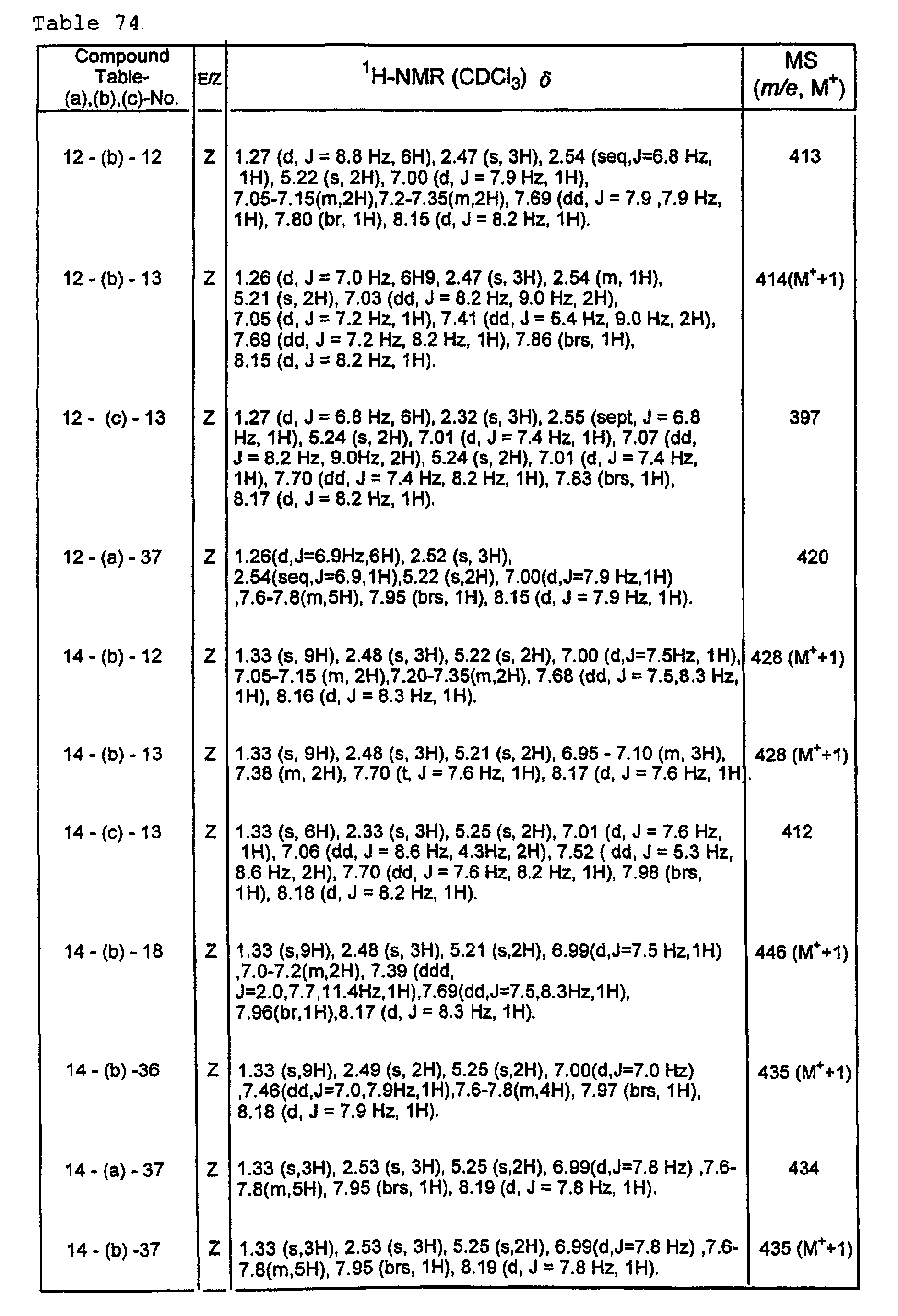

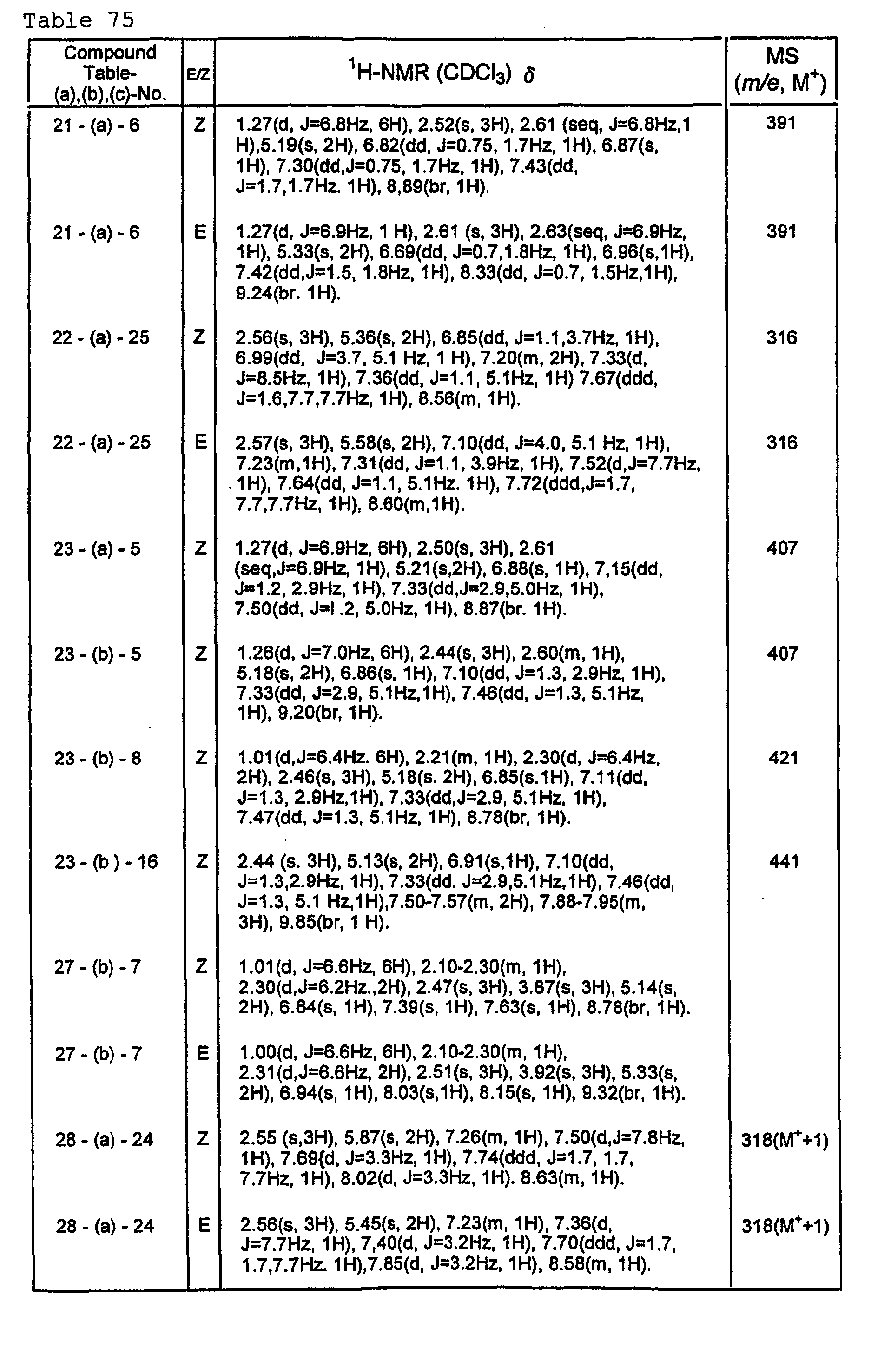

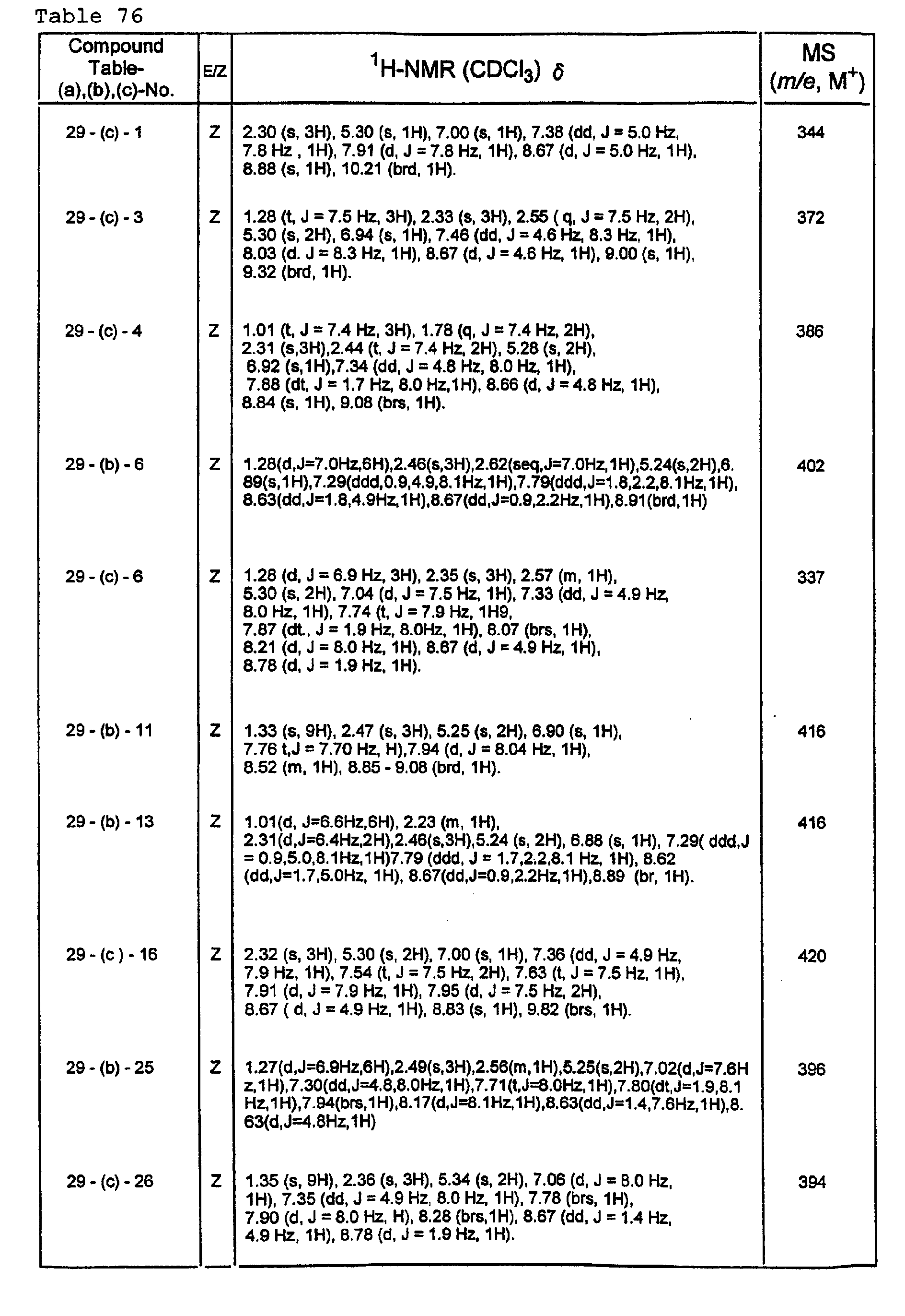

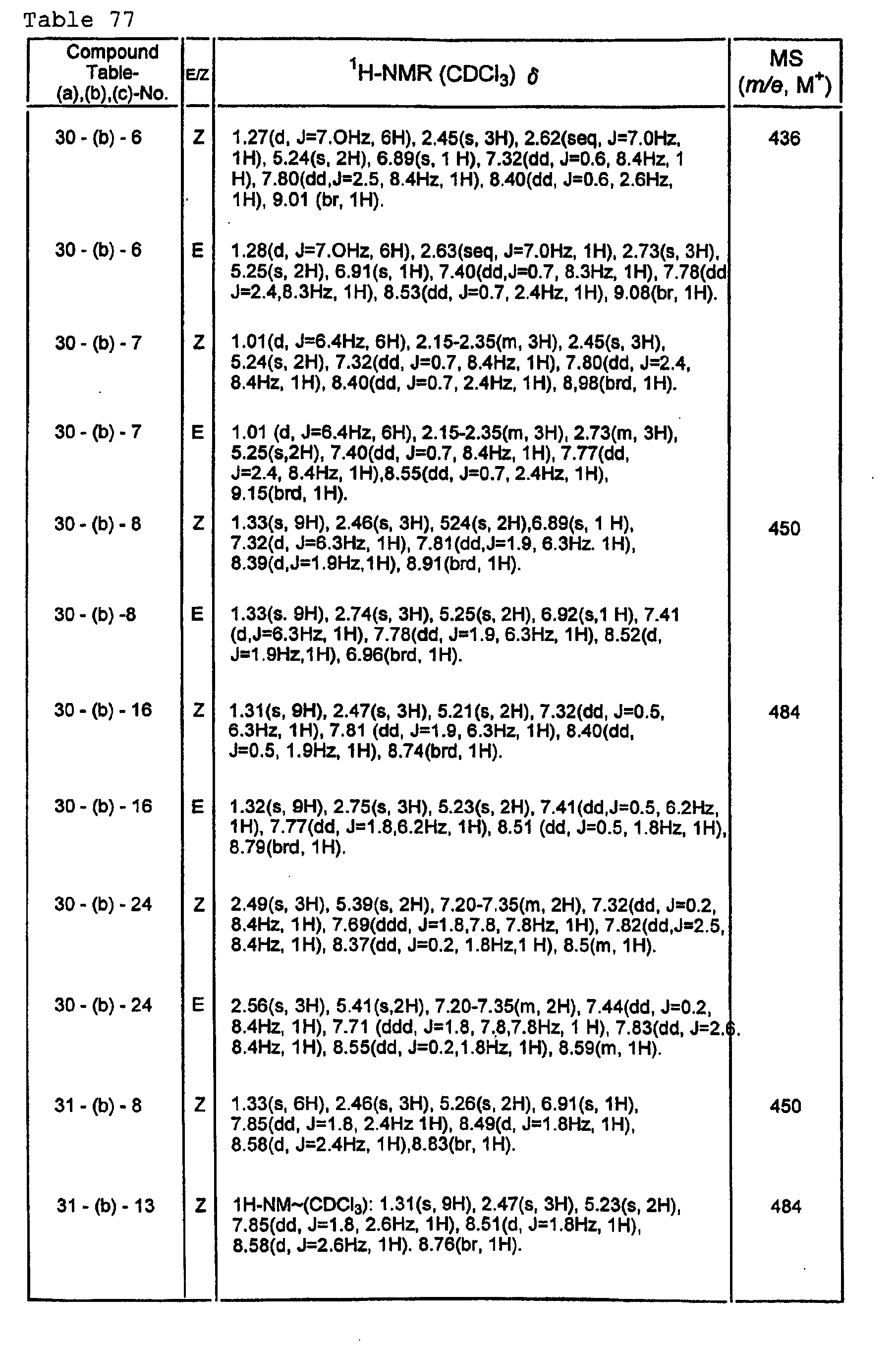

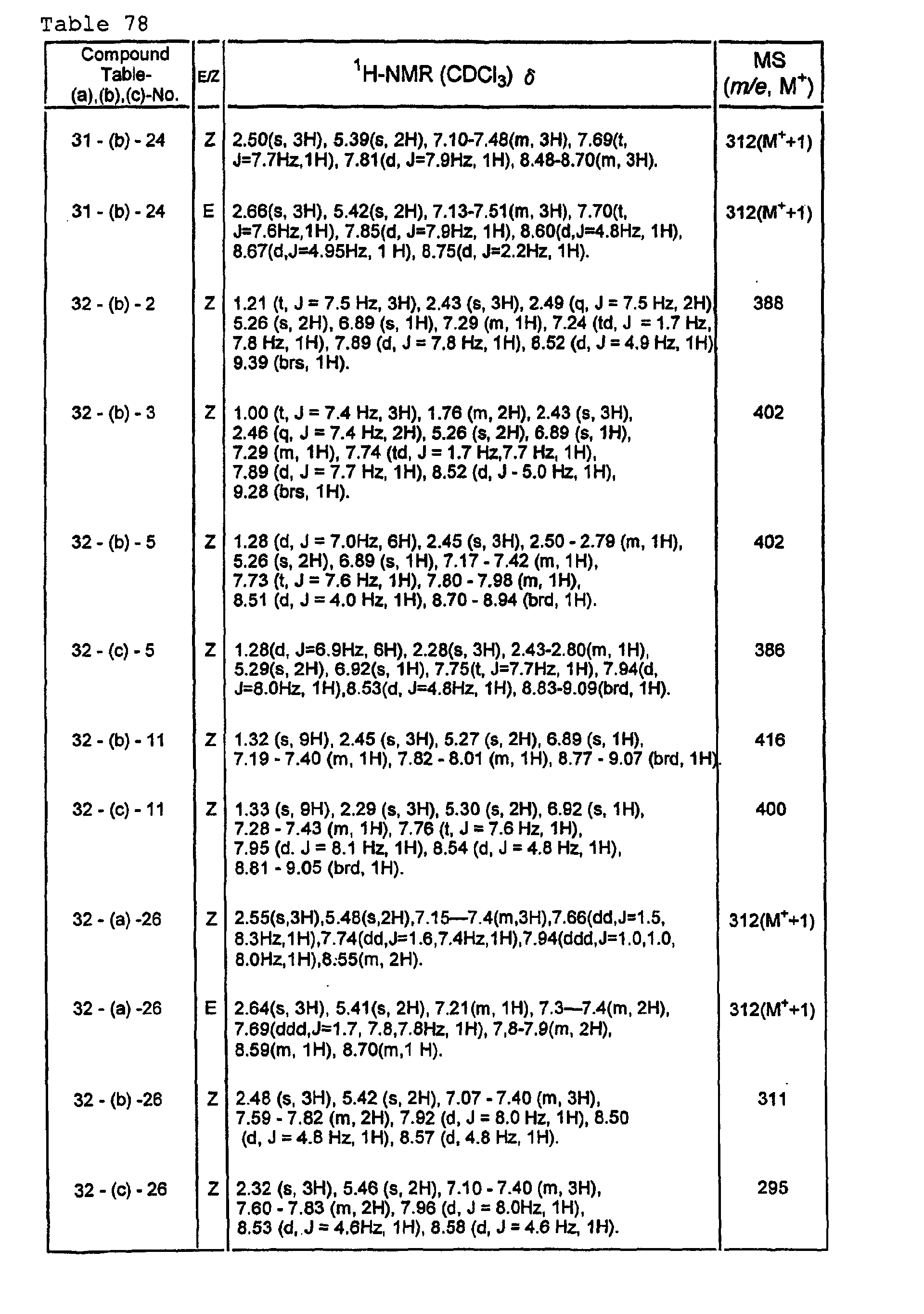

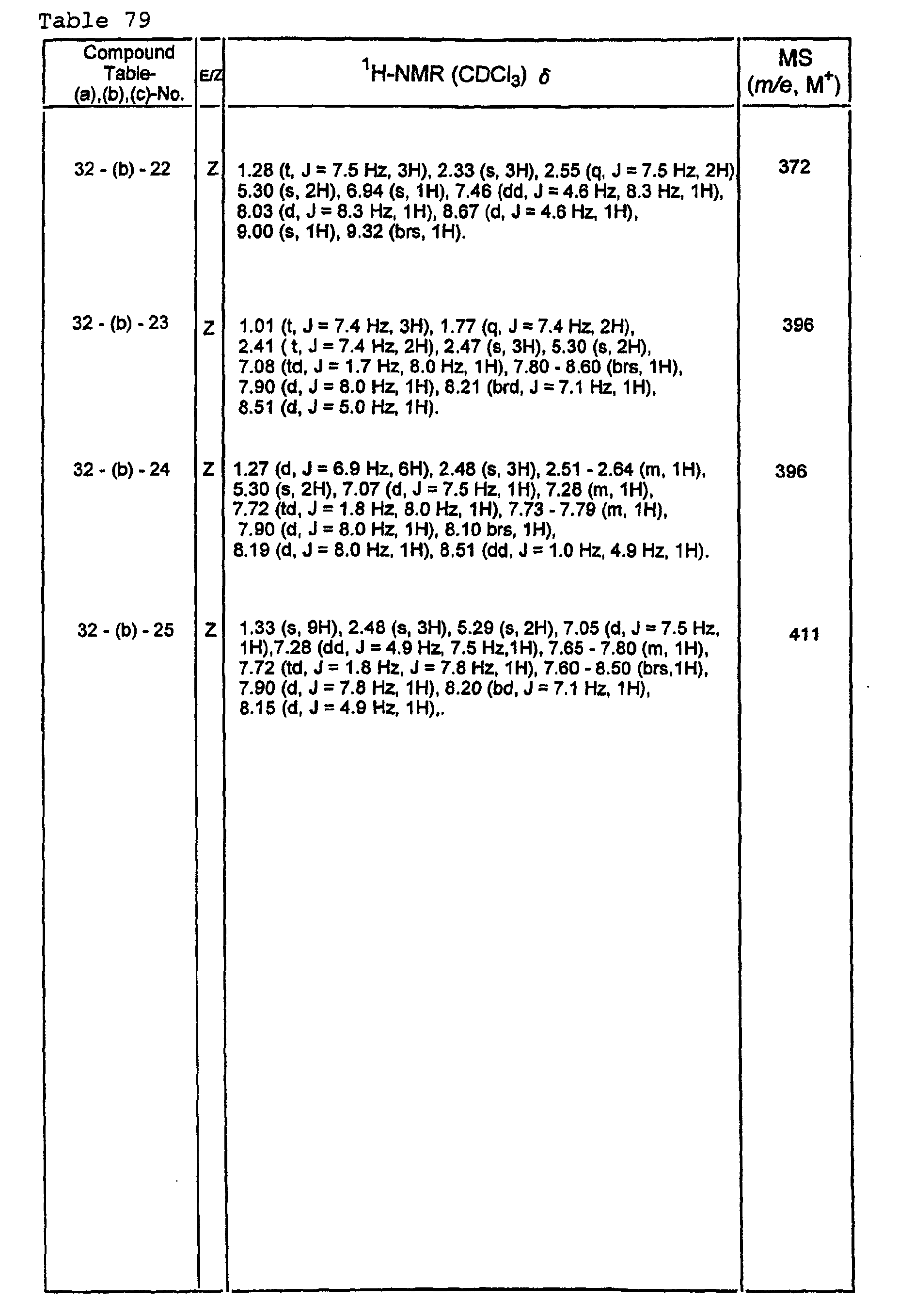

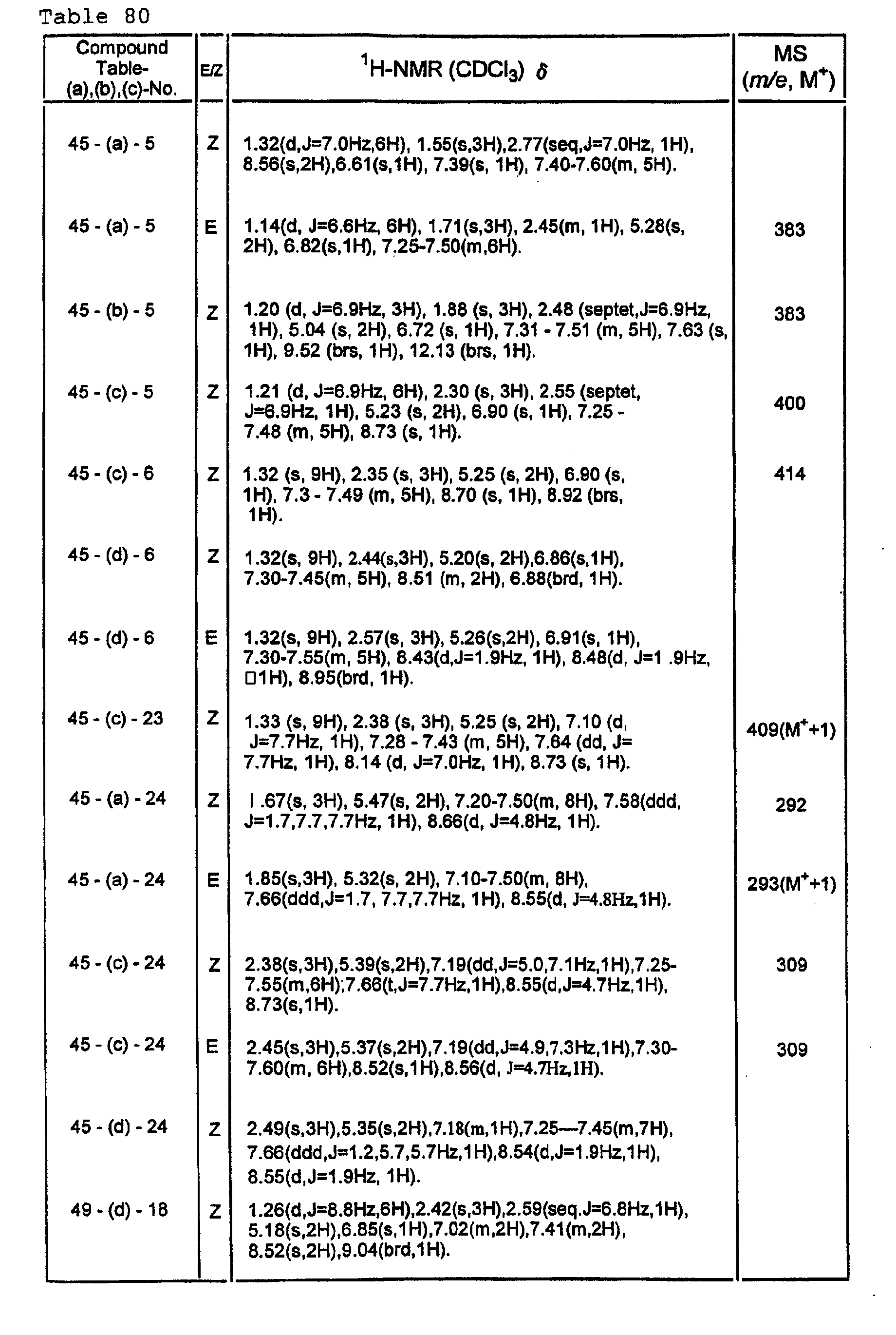

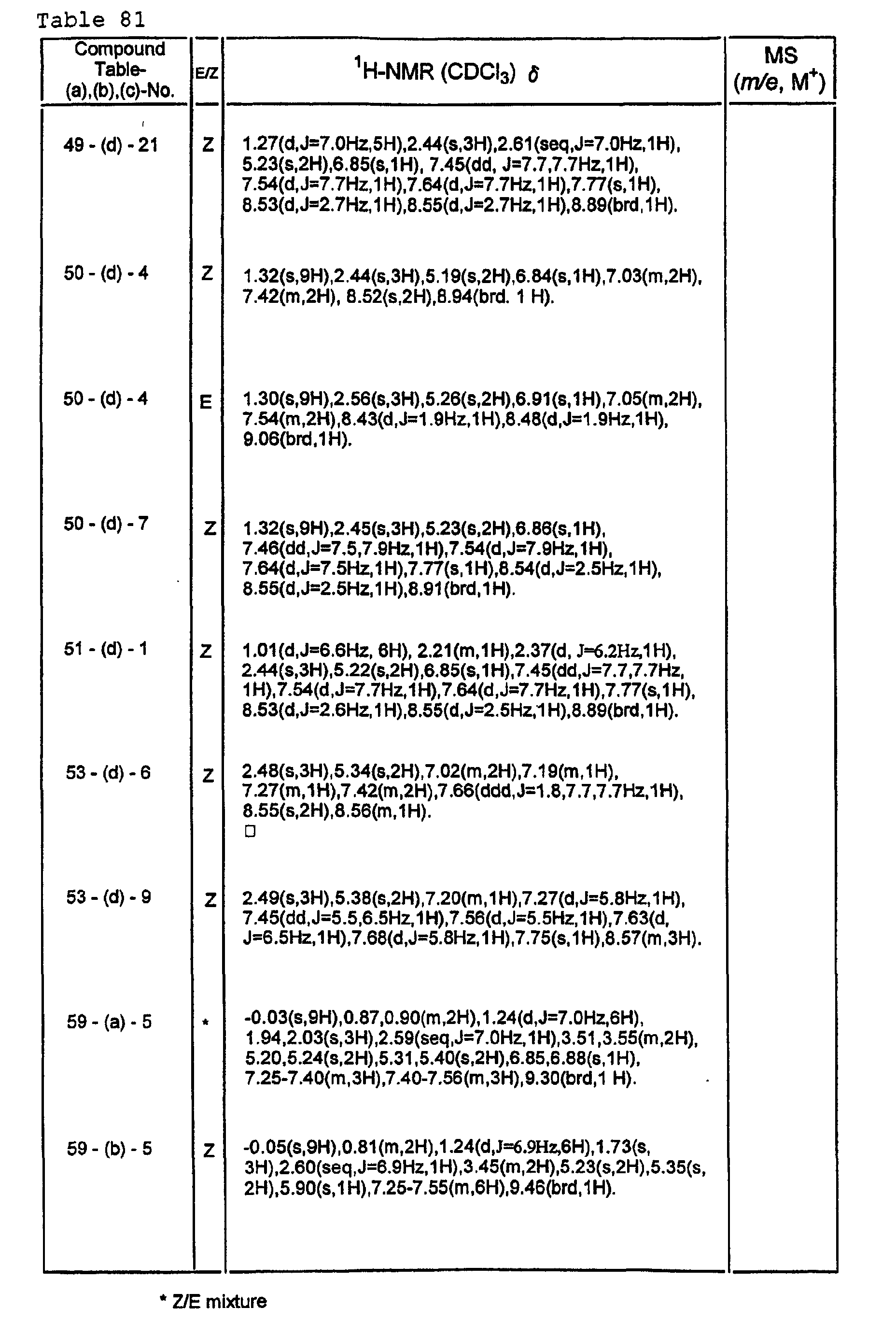

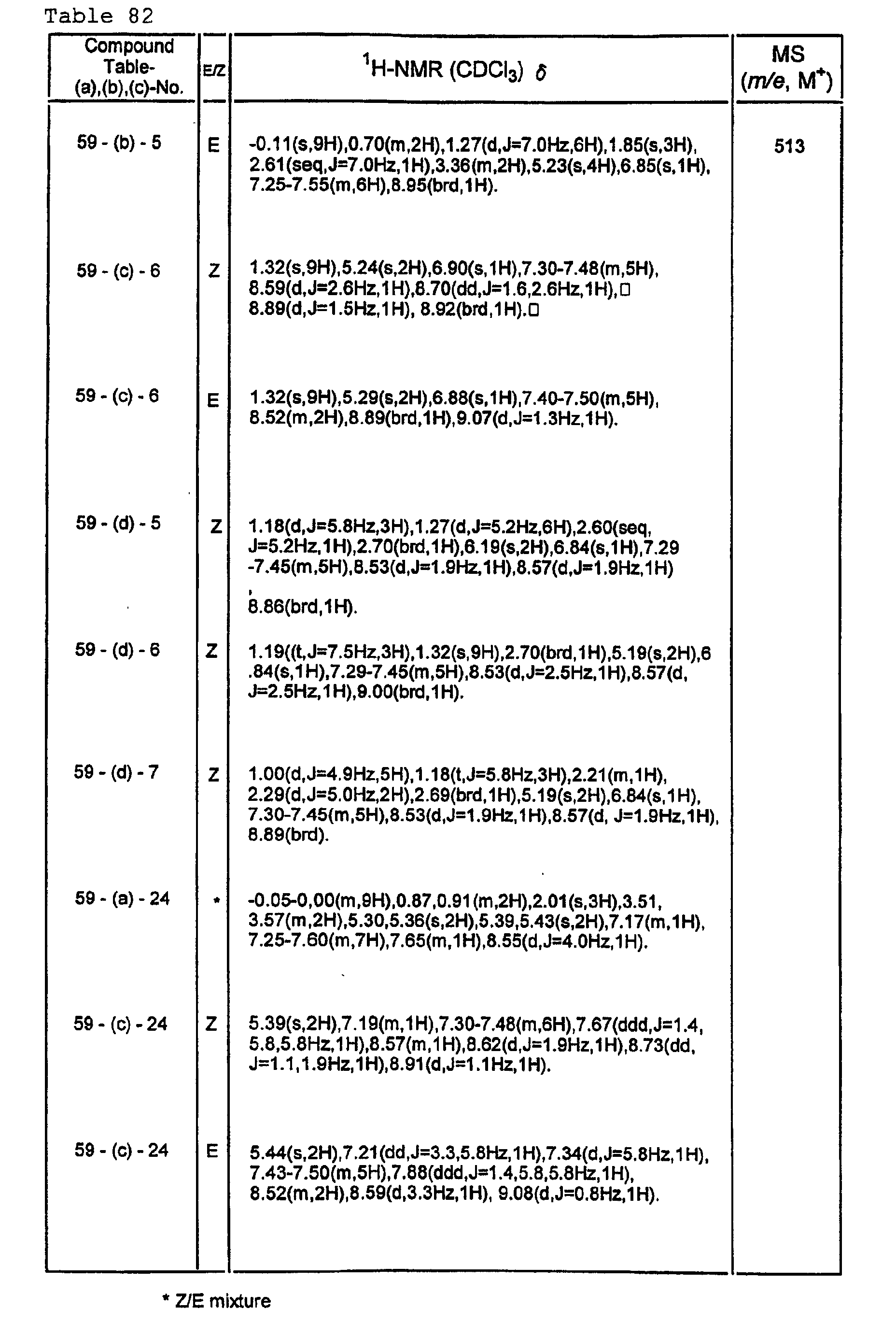

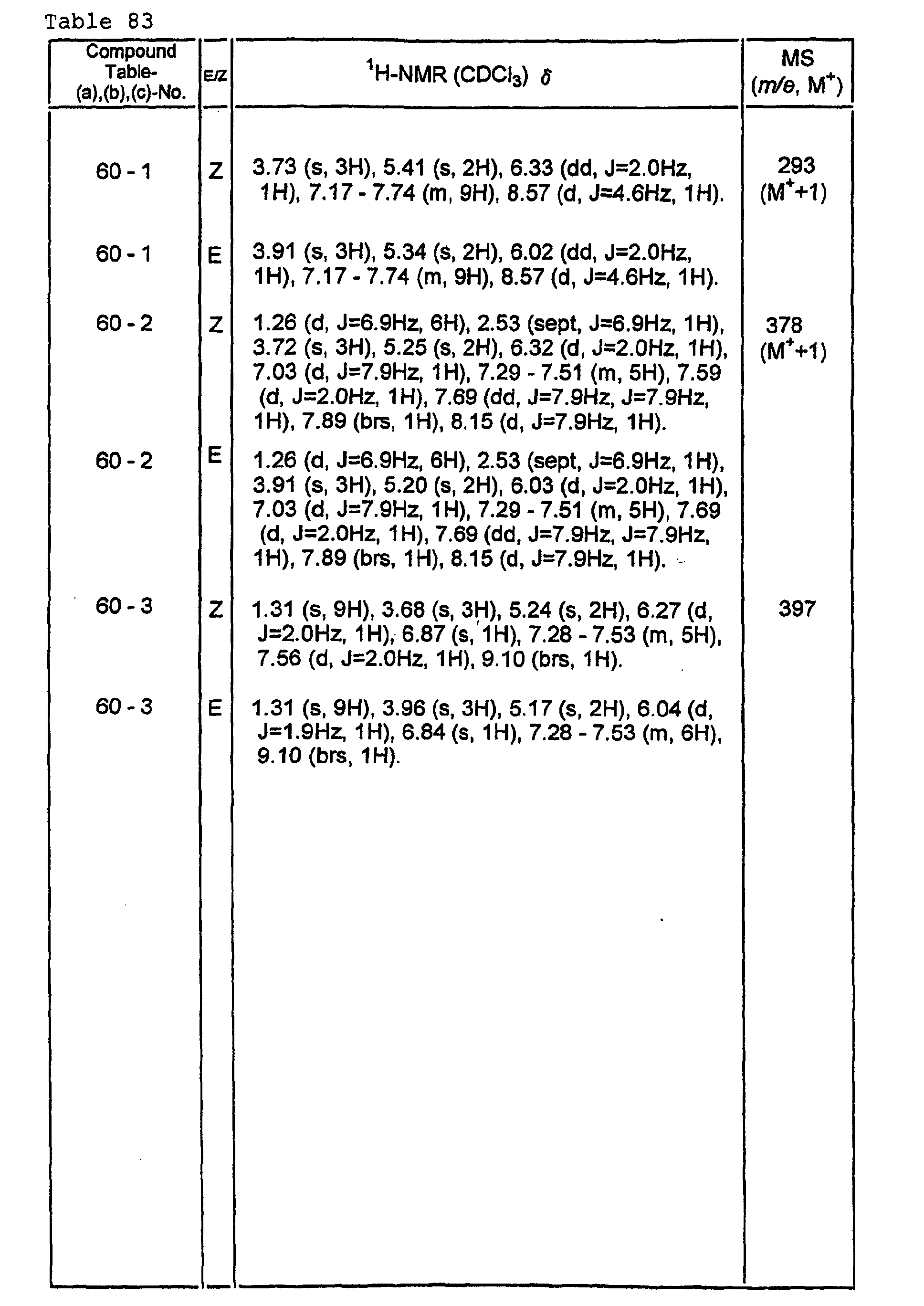

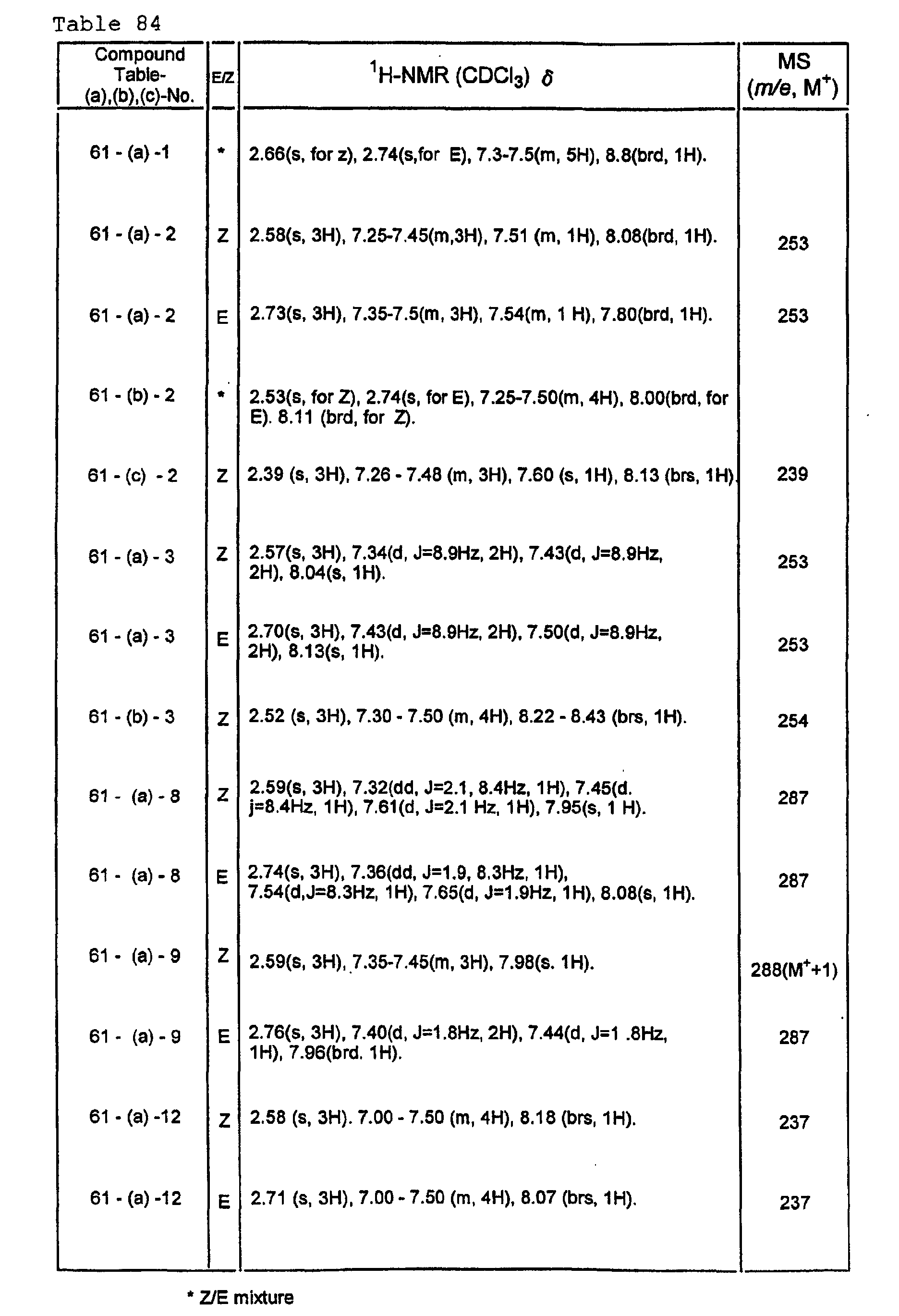

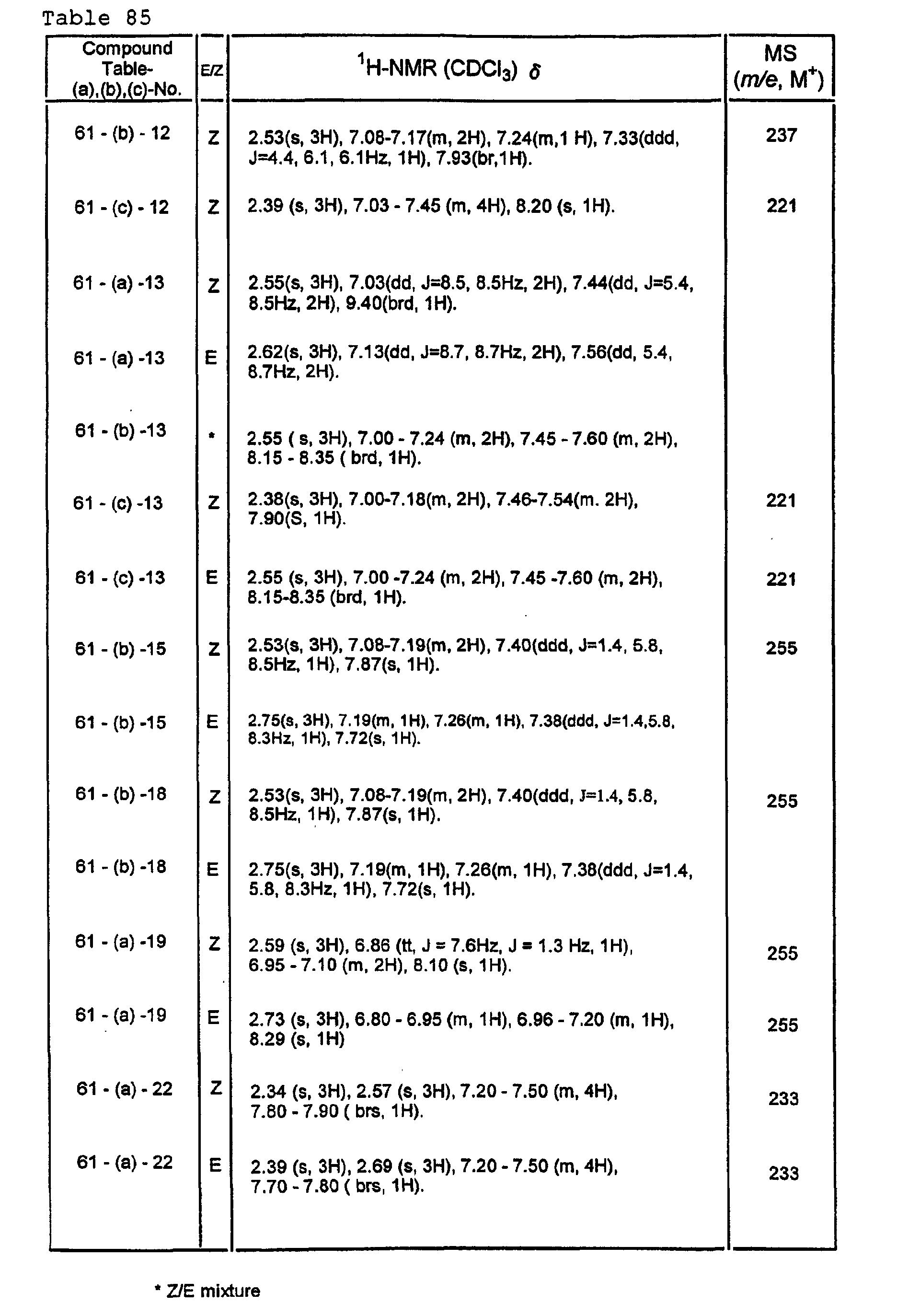

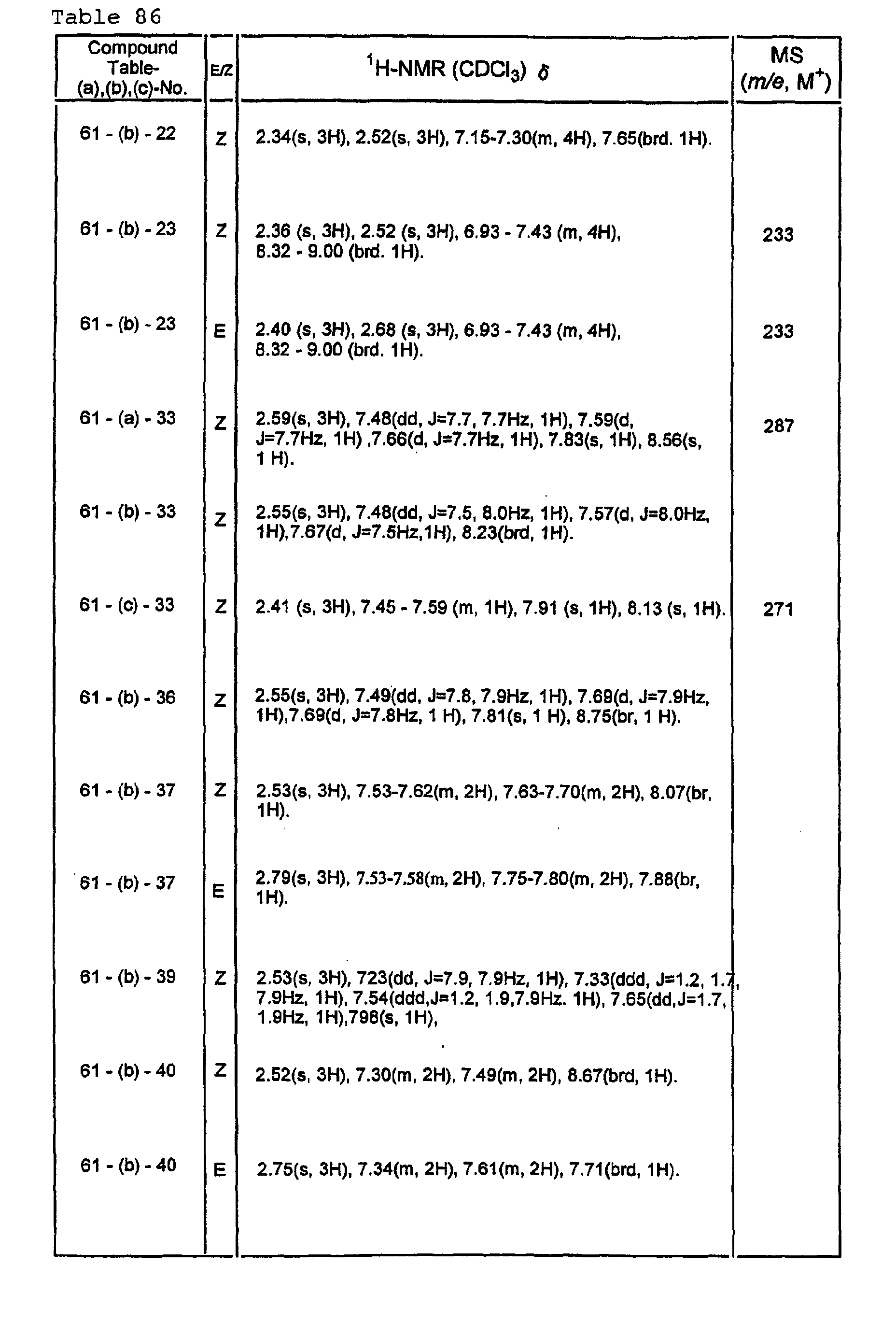

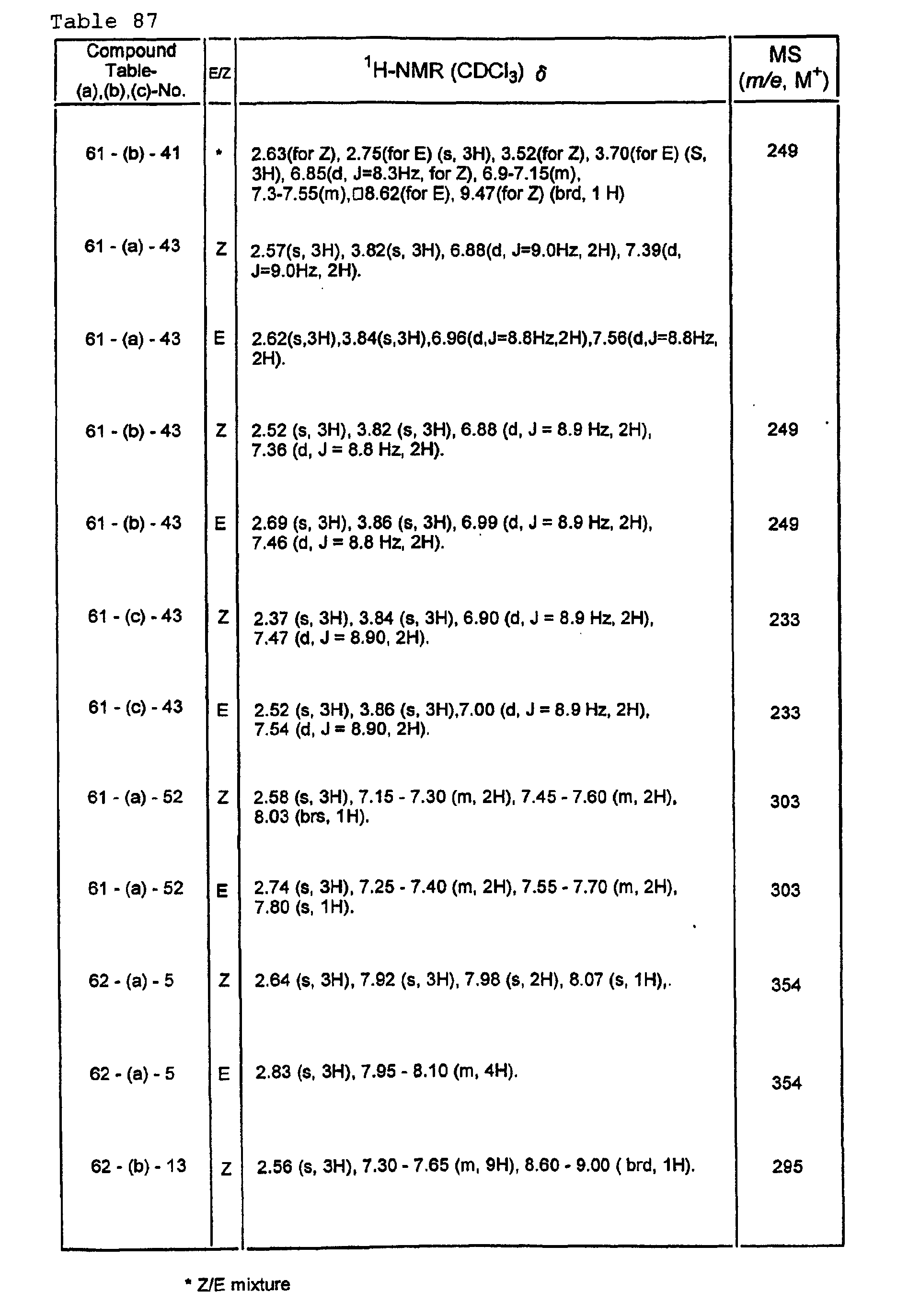

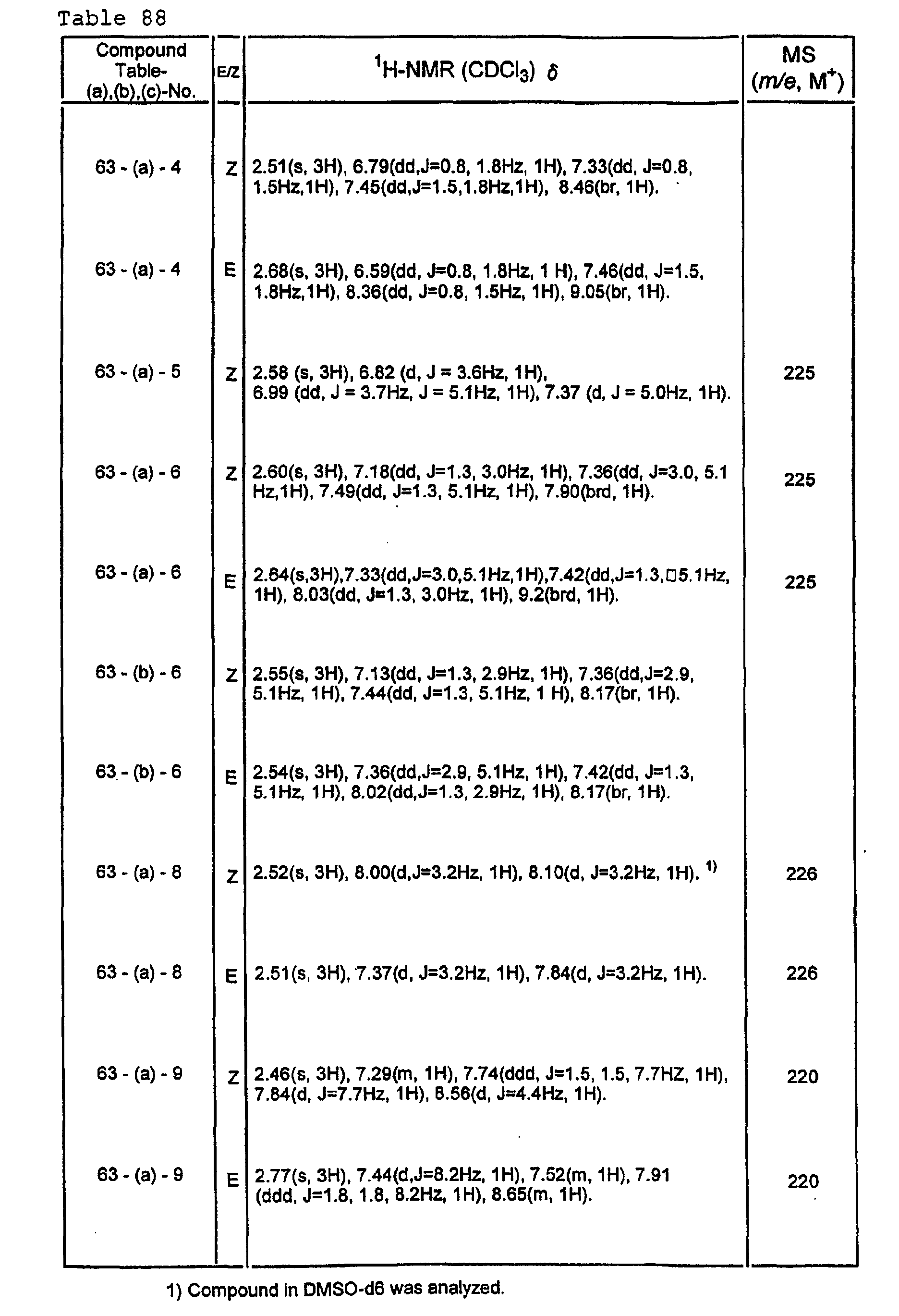

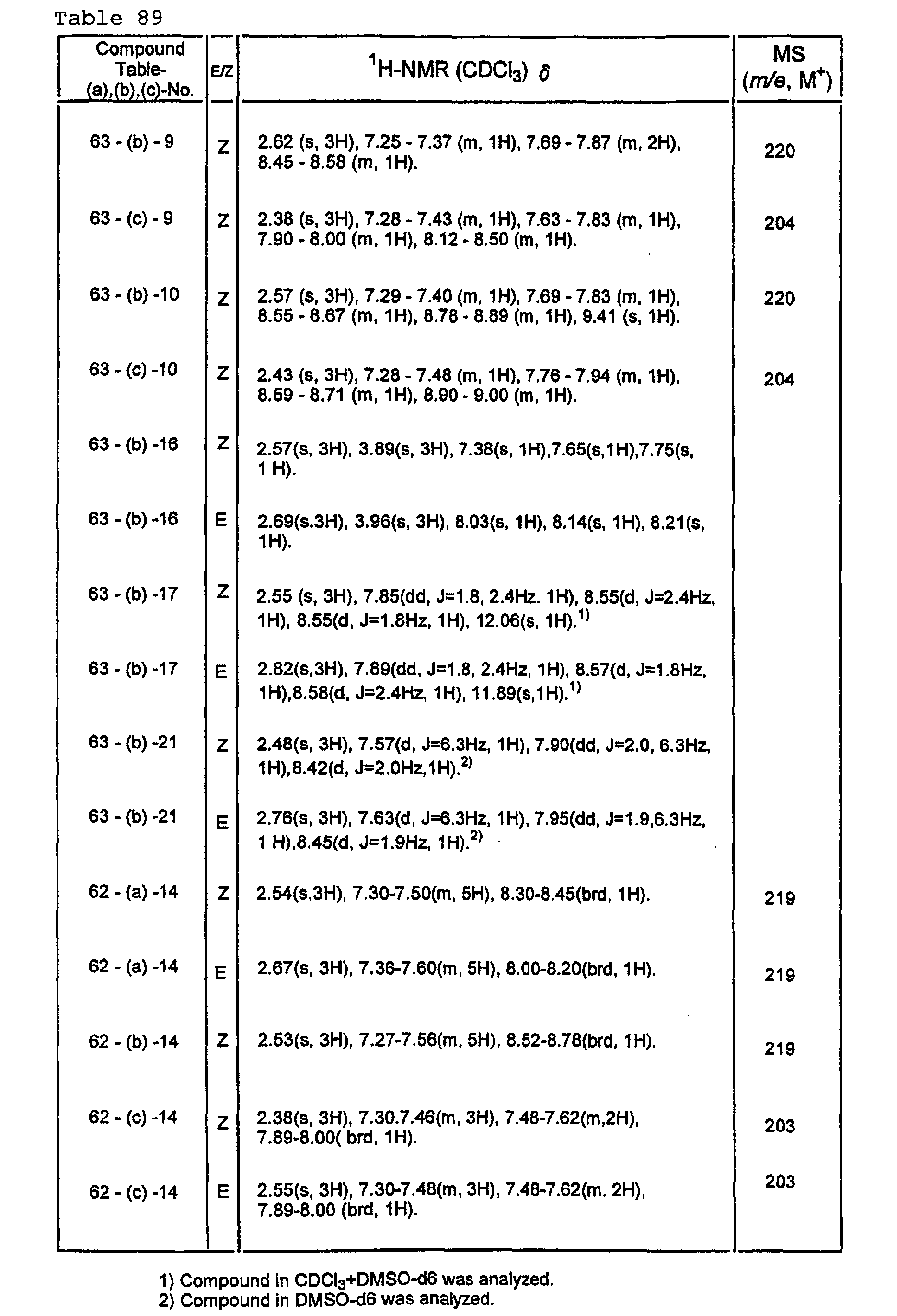

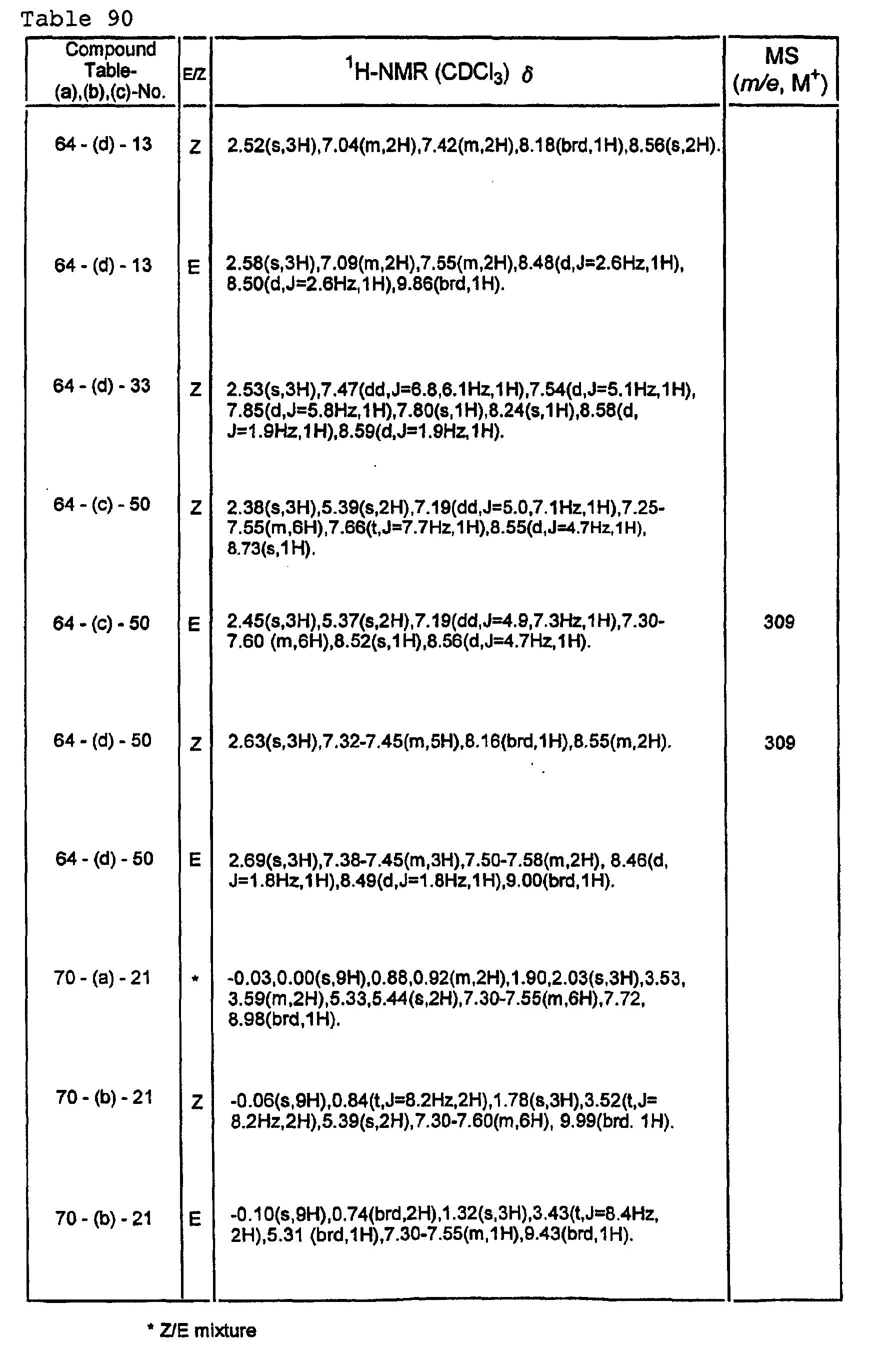

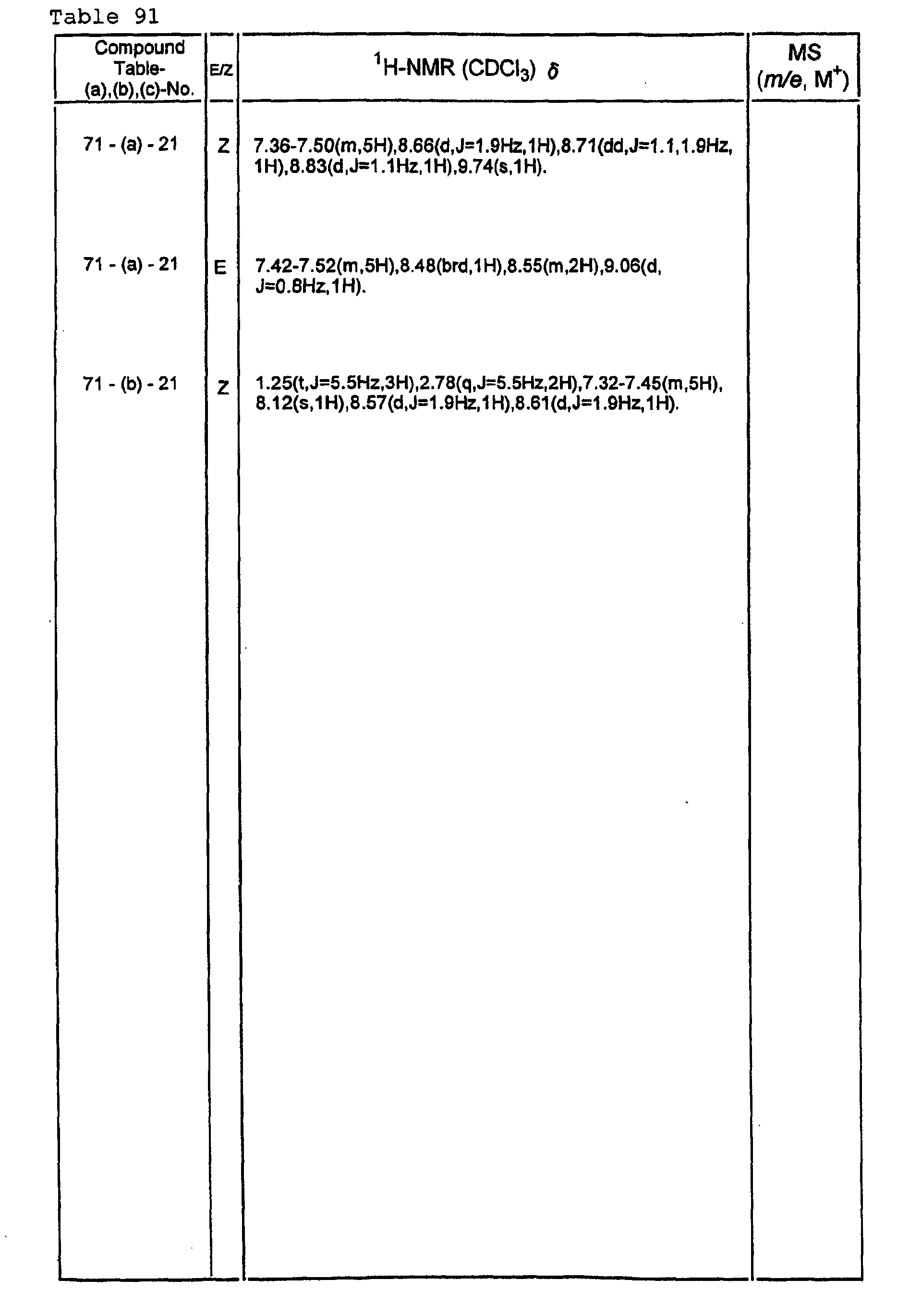

Formulation Examples using the compound of the present invention will now be described. The compounds used in the Formulation Examples and control tests are mixtures of (Z) and (E) isomers unless otherwise specified.

### (Formulation Example 1) Powders

2 parts by weight of each of the oxime derivatives of the present invention was mixed with 98 parts by weight of clay, and each mixture was ground to yield powders.

### (Formulation Example 2) Wettable powders

20 parts by weight of each of the oxime derivatives of the present invention was mixed with 68 parts of clay, 8 parts by weight of white carbon, and 4 parts by weight of polyoxyethylene nonyl phenyl ether, and each mixture was ground to yield wettable powders.

### (Formulation Example 3) Granules

5 parts by weight of each of the oxime derivatives of the present invention was mixed with 90 parts of a mixture of bentonite and talc in a mixing ratio of 1:1 and 5 parts of sodium alkylbenzenesulfonatem, and each mixture was molded to yield granules.

The following Test Examples show that the present invention is suited for use as a plant disease controlling agent. The control effect was evaluated by the following procedure. The pathopoiesis state of the test plant on examination, that is, the degree of colonies and lesions of leaves and stems is visually observed in a pot test, while the radius of colonies is measured in an antimicrobial activity test. The results were judged according to the following six rank criteria. Samples where neither colonies nor lesions were observed are rated "5", sample where colonies and lesions were observed at about 10% are rated "4", sample where colonies and lesions were observed at about 30% are rated "3", sample where colonies and lesions were observed at about 50% are rated "2", sample where colonies and lesions were observed at about 70% are rated "1", and sample where colonies and lesions were observed at about 70% or more are rated "0", respectively.

### (Test Example 1) Test of rice blast controlling effect (pot test)

Rice plants (Aichi Asahi, 3-4 leaf stage) cultivated in plastic pots 9 cm in diameter had their stems and leaves sprayed with wettable powders prepared according to the method of Preparation Example 2 diluted with water containing a surfactant (0.02%) to a prescribed concentration (active ingredient concentration of 250 mg/L). After air drying, a spore suspension of *Pyricularia oryzae* was spray-inoculated onto the plants, and inoculated pots were placed in a constant humidity chamber maintained at 25°C for 48 hours and were then transferred to a greenhouse to allow disease to develop. After 7 days had passed since inoculation, disease severity was determined. The results are shown in Table 92.

**Table 92**

| Test compounds | Control effect |
|---|---|
| 23-(a)- 5 | 4 |
| 23-(b)- 8 | 4 |
| 45-(c)-24 | 4 |
| 59-(a)- 5 | 5 |
| 59-(a)-24 | 5 |

### (Test Example 2) Test of blight controlling effect (pot test)

Potato plants (Hofuku, 3-4 leaf stage) cultivated in plastic pots 9 cm in diameter had their stems and leaves sprayed with wettable powders prepared according to the method of Preparation Example 2 diluted with water containing a surfactant (0.02%) to a prescribed concentration (active ingredient concentration of 250 mg/L). After air drying, a spore suspension of *Phytophthora infestans* was spray-inoculated onto the plants, and inoculated pots were placed in a constant humidity chamber maintained at 20°C for 24 hours and were then transferred to a greenhouse to allow disease to develop. After 5 days had passed since inoculation, disease severity was determined. The results are shown in Table 93.

**Table 93**

| Test compounds | Control effect |
|---|---|
| 1-(b)-4 | 5 |
| 12-(b)-12 | 5 |
| 23-(a)- 5 | 5 |
| 45-(b)-5 | 4 |
| 45-(c)-5 | 4 |

### (Test Example 3) Test of control effect on Alternaria blotch and Fusarium wilt (antimicrobial activity test)

Wettable powders prepared according to the method of Preparation Example 2 were diluted with water containing a surfactant (0.02%) to a prescribed concentration (active ingredient concentration of 250 mg/L). The resulting solution was mixed with a potato dextrose agar medium in a plastic petri dish 5 cm in diameter to prepare a medium containing 50 mg/L of the sample. Colonies of *Alternaria mali* and *Fusarium oxysporum* punched out using a cork borer were spray-inoculated onto the medium and were cultured at 24°C for 48 hours. After the growth of bacteria, the radii of colonies were measured, and the growth inhibition ratio was calculated. The results are shown in Table 94 (Alternaria blotch) and Table 95 (Fusarium wilt).

**Table 94**

| Test compounds | Control effect |
|---|---|
| 32-(a)-26 | 5 |

**Table 95**

| Test compounds | Control effect |
|---|---|
| 32-(a)-26 | 5 |

### (Test Example 4) Test of grape downy mildew controlling effect

Wettable powders prepared according to the method of Preparation Example 2 were charged in a test tube and were then diluted with water to a prescribed concentration (active ingredient concentration of 5 mg/L). Three grape leaf disks (Neomuscat) punched out using a cork borer 10 mm in diameter was put in a test solution. After 30 minutes, leaf disks were taken out and placed with the undersurface of the leaf up on a filter paper in a plastic petri dish. After allowing the filter paper to absorb moisture, a spore suspension of *Plasmopara viticola* was spray-inoculated onto the leaf disks. After sealing the plastic petri dish, disease development was allowed to progress in a growth cabinet (25°C, 5000 Lx, 14 hours, 18°C dark conditions, 10 hours, humidity of 70%). After seven days, disease severity was determined. The results are shown in Table 96.

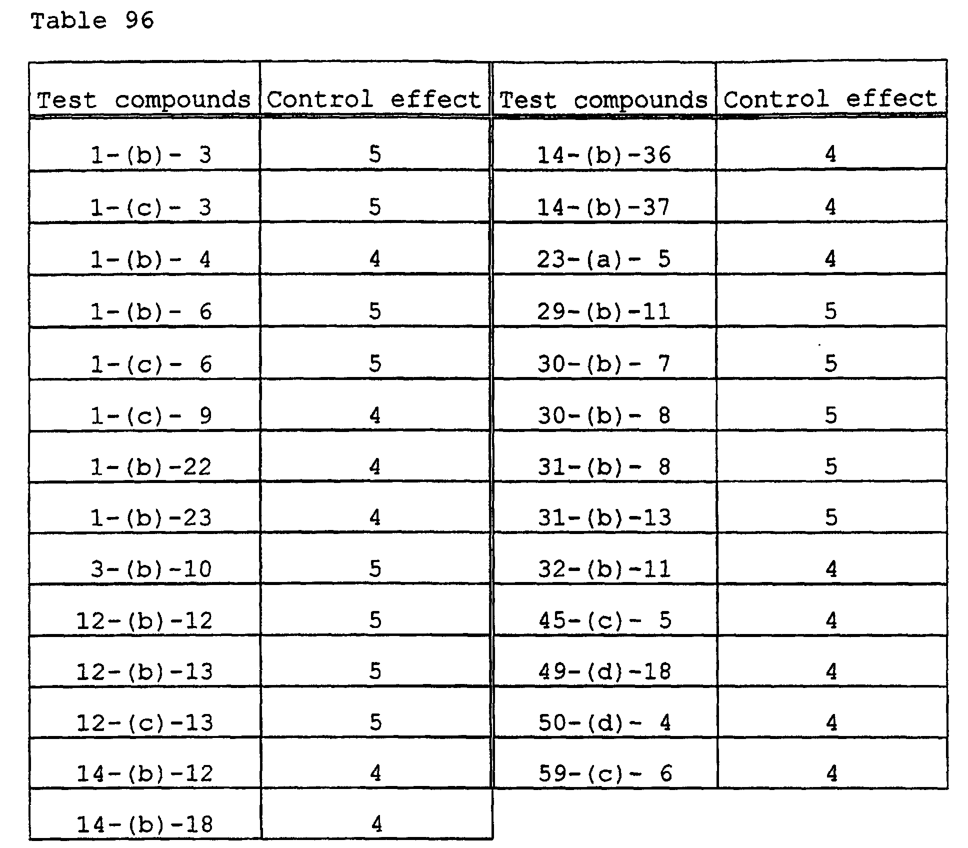

### INDUSTRIAL APPLICABILITY

The present invention can provide a novel oxime derivative which is less likely to cause chemical injury to useful plants and also has sufficient chemical efficacy, a method of preparing the same, and an agricultural chemical containing the same as an active ingredient, especially a plant disease controlling agent.

## Claims

1. An oxime derivative represented by the following general formula (1): 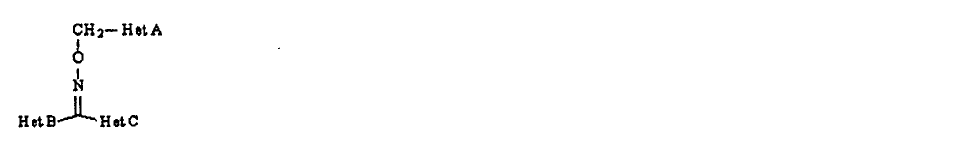 wherein Het A represents a group represented by any one of the following three formulas: 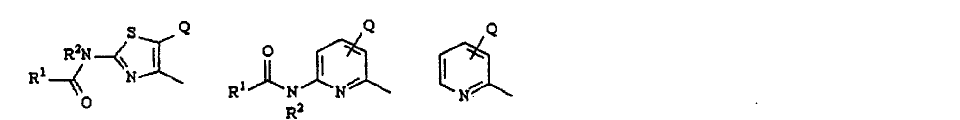 (wherein Q represents a hydrogen atom, a halogen atom, or a lower alkyl group; R¹ represents a hydrogen atom, a lower alkyl group, a lower alkyl group substituted with a halogen atom, a cycloalkyl group, a lower alkenyl group, a lower alkynyl group, an aralkyl group, an aryl group, or an aryl group substituted with one or more substituents selected from the group consisting of halogen atom, lower alkyl group, and lower alkoxy group; and R² represents a hydrogen atom or a lower alkyl group); Het B represents a group represented by any one of the following nine formulas: 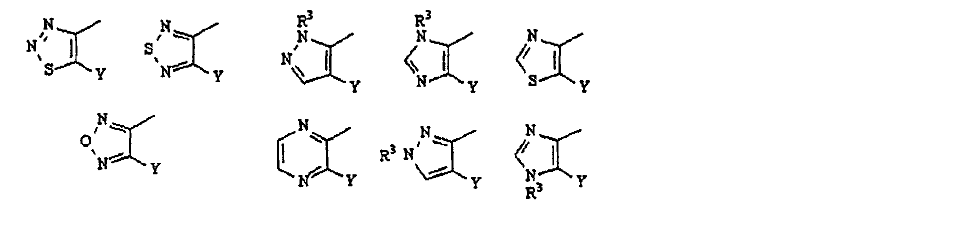 (wherein Y represents a hydrogen atom or a lower alkyl group; and R³ represents a hydrogen atom, a lower alkyl group, or a lower alkyl group substituted with a lower alkoxy group which may have a silyl group); and Het C represents a group represented by any one of the following nine formulas: 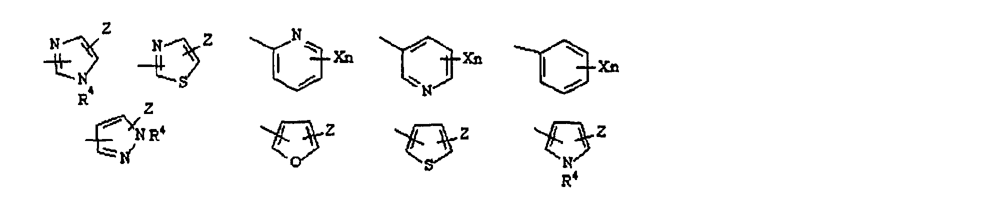 (wherein R⁴ represents a hydrogen atom, a lower alkyl group, or a lower alkyl group substituted with a lower alkoxy group which may have a silyl group; X represents a hydrogen atom, a halogen atom, a lower alkyl group, a lower alkyl group substituted with a halogen atom, a lower alkoxy group, a lower alkoxy group substituted with a halogen atom, or a cyano group; Z represents a hydrogen atom, a halogen atom, or a lower alkyl group; and n is from 0 to 3); with the exception of the case where Het A is a group represented by any one of the following two formulas: 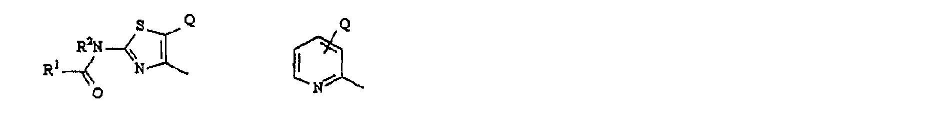 (wherein Q represents a hydrogen atom, a halogen atom, or a lower alkyl group; R¹ represents a hydrogen atom, a lower alkyl group, a lower alkyl group substituted with a halogen atom, a cycloalkyl group, a lower alkenyl group, a lower alkynyl group, an aralkyl group, an aryl group, or an aryl group substituted with one or more substituents selected from the group consisting of halogen atom, lower alkyl group, and lower alkoxy group; and R² represents a hydrogen atom or a lower alkyl group); Het B is a group represented by any one of the ring structures of the following three formulas: 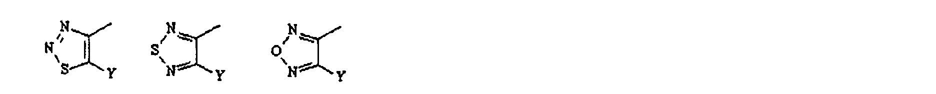 (wherein Y represents a hydrogen atom or a lower alkyl group); and Het C is a group represented by the following formula: 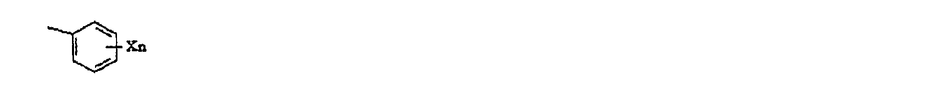 (wherein X represents a hydrogen atom, a halogen atom, a lower alkyl group, a lower alkyl group substituted with a halogen atom, a lower alkoxy group, a lower alkoxy group substituted with a halogen atom, or a cyano group; and n is from 0 to 3).

2. An oxime derivative represented by the following general formula (1) : 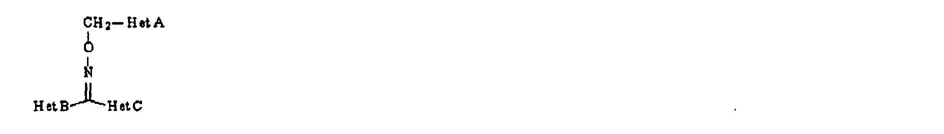 wherein Het A represents a group represented by any one of the following three formulas: 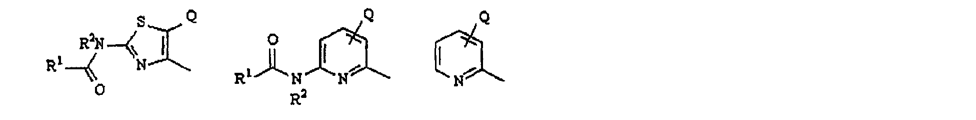 (wherein Q represents a hydrogen atom, a halogen atom, or a lower alkyl group; R¹ represents a hydrogen atom, a lower alkyl group, a lower alkyl group substituted with a halogen atom, a cycloalkyl group, a lower alkenyl group, a lower alkynyl group, an aralkyl group, an aryl group, or an aryl group substituted with one or more substituents selected from the group consisting of halogen atom, lower alkyl group, and lower alkoxy group; and R² represents a hydrogen atom or a lower alkyl group); Het B represents a group represented by any one of the ring structures of the following three formulas: 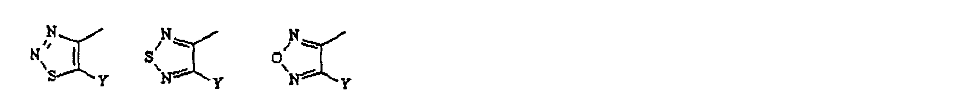 (wherein Y represents a hydrogen atom or a lower alkyl group); and Het C represents a group represented by any one of the ring structures of the following eight formulas: 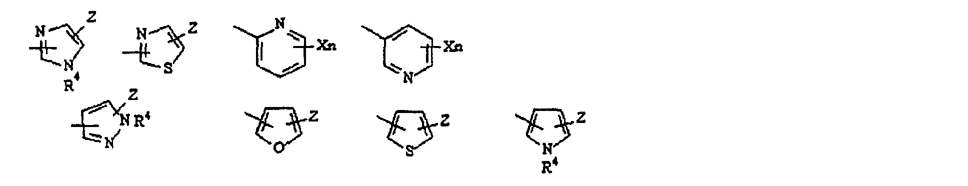 (wherein Z represents a hydrogen atom, a halogen atom, or a lower alkyl group; R⁴ represents a hydrogen atom, a lower alkyl group, or a lower alkyl group substituted with a lower alkoxy group which may have a silyl group; X represents a hydrogen atom, a halogen atom, a lower alkyl group, or a lower alkoxy group; and n is from 0 to 3).

3. An oxime derivative represented by the following general formula (1) : 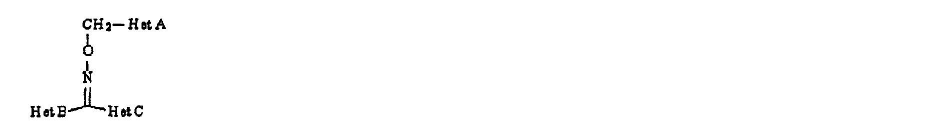 wherein Het A represents a group represented by the following formula: 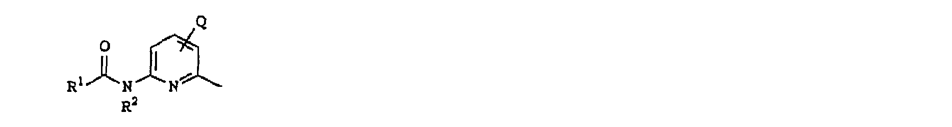 (wherein Q represents a hydrogen atom, a halogen atom, or a lower alkyl group; R¹ represents a hydrogen atom, a lower alkyl group, a lower alkyl group substituted with a halogen atom, a cycloalkyl group, a lower alkenyl group, a lower alkynyl group, an aralkyl group, an aryl group, or an aryl group substituted with one or more substituents selected from the group consisting of halogen atom, lower alkyl group, and lower alkoxy group; and R² represents a hydrogen atom or a lower alkyl group); Het B represents a group represented by any one of the ring structures of the following three formulas: 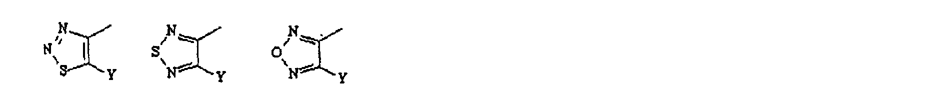 (wherein Y represents a hydrogen atom or a lower alkyl group); and Het C represents a group represented by the following formula: 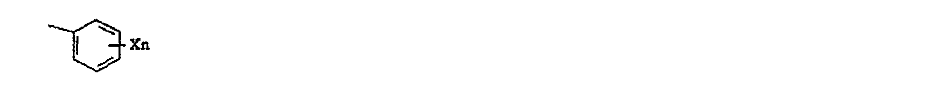 (wherein X represents a hydrogen atom, a halogen atom, a lower alkyl group, a lower alkyl group substituted with a halogen atom, a lower alkoxy group, a lower alkoxy group substituted with a halogen atom, or a cyano group; and n is from 0 to 3).

4. An oxime derivative represented by the following general formula (1): 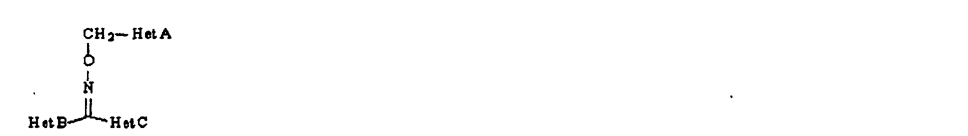 wherein Het A represents a group represented by any one of the following three formulas: 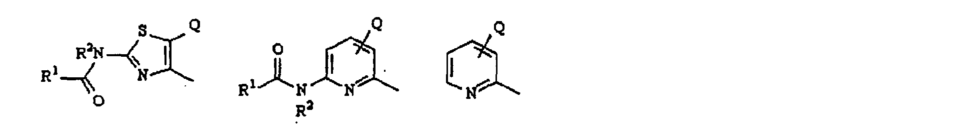 (wherein Q represents a hydrogen atom, a halogen atom, or a lower alkyl group; R¹ represents a hydrogen atom, a lower alkyl group, a lower alkyl group substituted with a halogen atom, a cycloalkyl group, a lower alkenyl group, a lower alkynyl group, an aralkyl group, an aryl group, or an aryl group substituted with one or more substituents selected from the group consisting of halogen atom, lower alkyl group, and lower alkoxy group; and R² represents a hydrogen atom or a lower alkyl group); Het B represents a group represented by any one of the following six formulas: 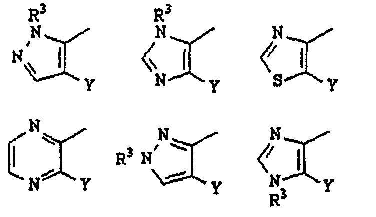 (wherein Y represents a hydrogen atom or a lower alkyl group; and R³ represents a hydrogen atom, a lower alkyl group, or a lower alkyl group substituted with a lower alkoxy group which may have a silyl group); and Het C represents a group represented by any one of the following six formulas: 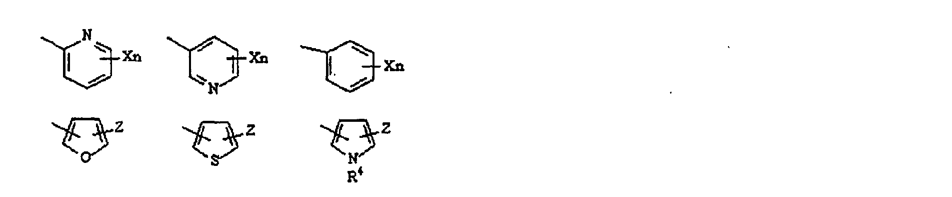 (wherein R⁴ represents a hydrogen atom or a lower alkyl group; X represents a hydrogen atom, a halogen atom, a lower alkyl group, a lower alkyl group substituted with a halogen atom, a lower alkoxy group, or a lower alkoxy group substituted with a halogen atom; Z represents a hydrogen atom, a halogen atom, or a lower alkyl group; and n is from 0 to 3).

5. An oxime derivative according to claim 2, wherein Het C is a group represented by any one of the ring structures of the following three formulas: 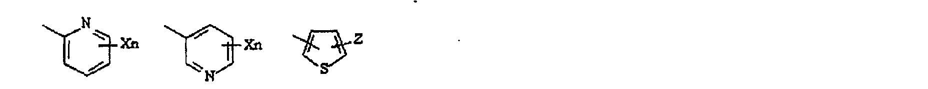 (wherein Z represents a hydrogen atom, a halogen atom, or a lower alkyl group; X represents a hydrogen atom, a halogen atom, a lower alkyl group, or a lower alkoxy group; and n is 0 or 1) in the general formula (1).

6. An oxime derivative according to claim 2, wherein Het A is a group represented by the following formula: 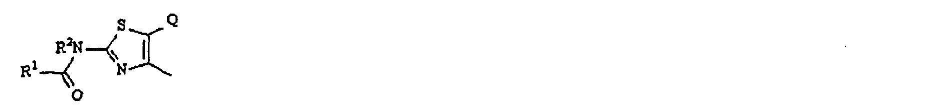 (wherein Q represents a hydrogen atom, a halogen atom, or a lower alkyl group; R¹ represents a lower alkyl group or a cycloalkyl group; and R² represents a hydrogen atom); and Het C is a group represented by the following formula: 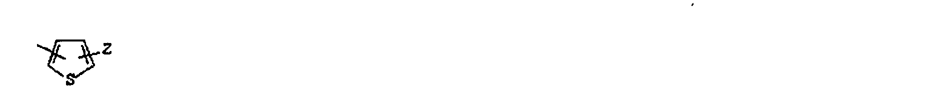 (wherein Z represents a hydrogen atom) in the general formula (1).

7. An oxime derivative according to claim 2, wherein Het A is a group represented by the following formula: 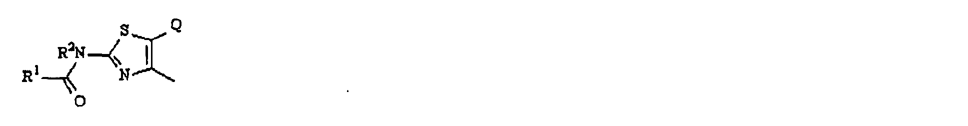 (wherein Q represents a hydrogen atom, a halogen atom, or a lower alkyl group; R¹ represents a lower alkyl group or a cycloalkyl group; and R² represents a hydrogen atom); and Het C is a group represented by the following formula: 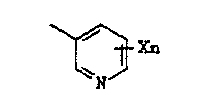 (wherein X represents a hydrogen atom or a halogen atom; and n is 0 or 1) in the general formula (1).

8. An oxime derivative according to claim 2, wherein Het A is a group represented by the following formula: 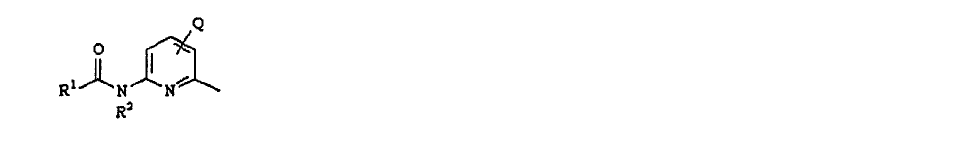 (wherein Q represents a hydrogen atom or a lower alkyl group; R¹ represents a lower alkyl group or a cycloalkyl group; and R² represents a hydrogen atom); and Het C is a group represented by any one of the following three formulas: 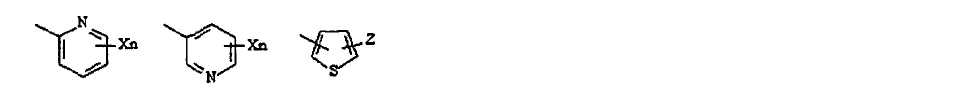 (wherein X represents a hydrogen atom or a halogen atom; z represents a hydrogen atom; and n is 0 or 1) in the general formula (1).

9. An oxime derivative according to claim 3, wherein Het A is a group represented by the following formula: 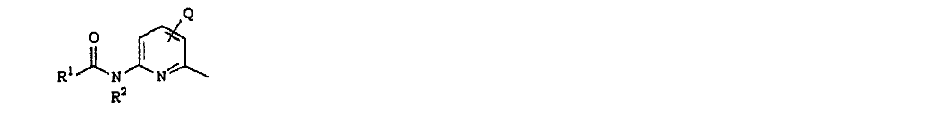 (wherein Q represents a hydrogen atom or a lower alkyl group; R¹ represents a lower alkyl group or a cycloalkyl group; and R² represents a hydrogen atom); and Het C is a group represented by the following formula: 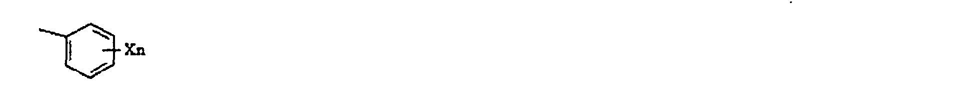 (wherein X represents a hydrogen atom, a halogen atom, a lower alkyl group, a lower alkyl group substituted with a halogen atom, or a cyano group; and n is from 0 to 3) in the general formula (1).

10. An oxime derivative according to claim 4, wherein Het C is a group represented by the following formula: 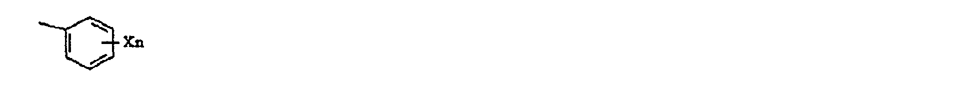 (wherein X represents a hydrogen atom, a halogen atom, a lower alkyl group, a lower alkyl group substituted with a halogen atom, a lower alkoxy group, or a lower alkoxy group substituted with a halogen atom; and n is from 0 to 3) in the general formula (1).

11. An oxime derivative according to claim 4, wherein Het A is a group represented by the following formula: 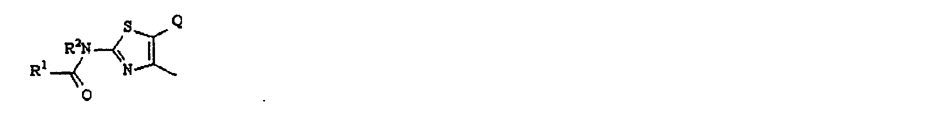 (wherein Q represents a hydrogen atom, a halogen atom, or a lower alkyl group; R¹ represents a lower alkyl group or a cycloalkyl group; and R² represents a hydrogen atom); Het B is a group represented by any one of the following three formulas: 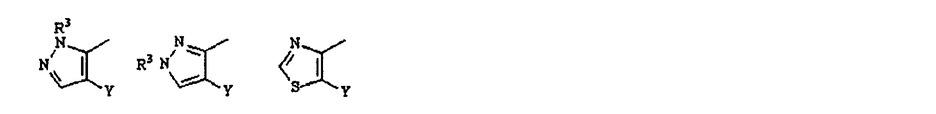 (wherein Y represents a hydrogen atom or a lower alkyl group; and R³ represents a hydrogen atom, a lower alkyl group, or a lower alkyl group substituted with a lower alkoxy group which may have a silyl group); and Het C is a group represented by the following formula: 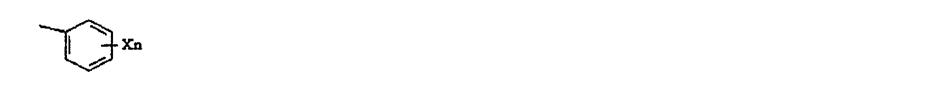 (wherein X represents a hydrogen atom, a halogen atom, a lower alkyl group, or a lower alkyl group substituted with a halogen atom; and n is 0 or 1) in the general formula (1).

12. A hydroxime derivative represented by the following general formula (2): 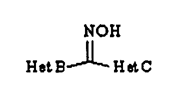 wherein Het B represents a group represented by any one of the following nine formulas: 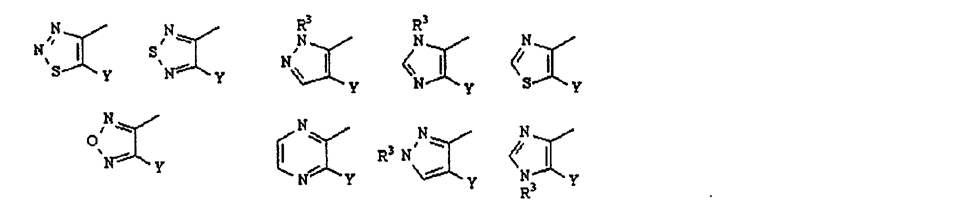 (wherein Y represents a hydrogen atom or a lower alkyl group; and R³ represents a hydrogen atom, a lower alkyl group, or a lower alkyl group substituted with a lower alkoxy group which may have a silyl group); and Het C represents a group represented by any one of the following nine formulas: 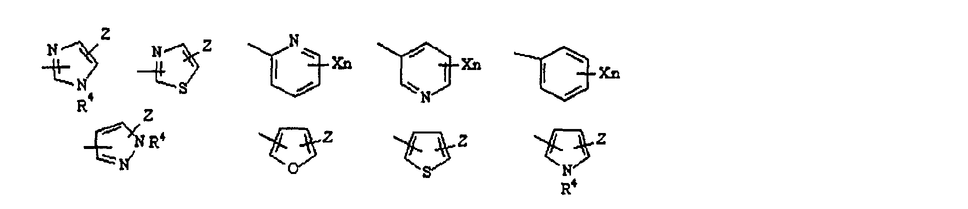 (wherein R⁴ represents a hydrogen atom, a lower alkyl group, or a lower alkyl group substituted with a lower alkoxy group which may have a silyl group; X represents a hydrogen atom, a halogen atom, a lower alkyl group, a lower alkyl group substituted with a halogen atom, a lower alkoxy group, or a lower alkoxy group substituted with a halogen atom; Z represents a hydrogen atom, a halogen atom, or a lower alkyl group; and n is from 0 to 3); with the exception of the case of the following compounds: 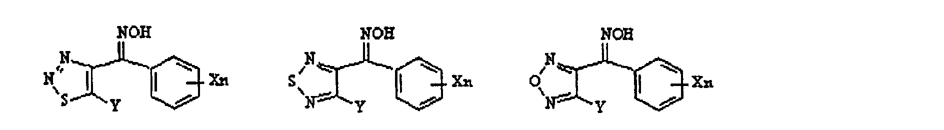 (wherein X represents a hydrogen atom, a halogen atom, a lower alkyl group, a lower alkyl group substituted with a halogen atom, a lower alkoxy group, a lower alkoxy group substituted with a halogen atom, or a cyano group; and n is 0 or 1), the following compound: 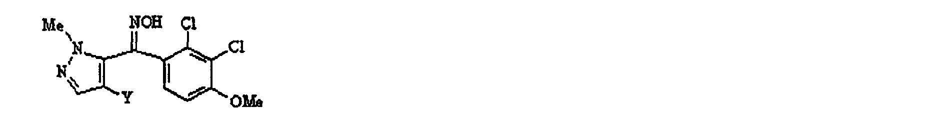 and the following compounds:

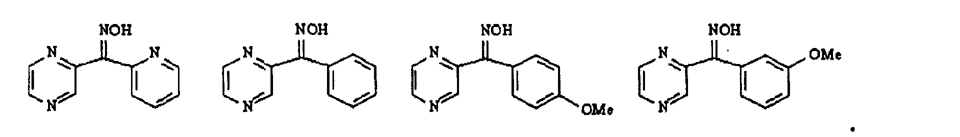

13. A hydroxime derivative according to claim 12, wherein Het B is a group represented by any one of the ring structures of the following three formulas: 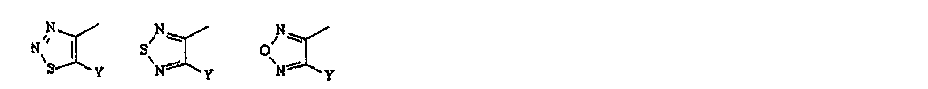 (wherein Y represents a hydrogen atom or a lower alkyl group); and Het C is a group represented by any one of the ring structures of the following eight formulas: 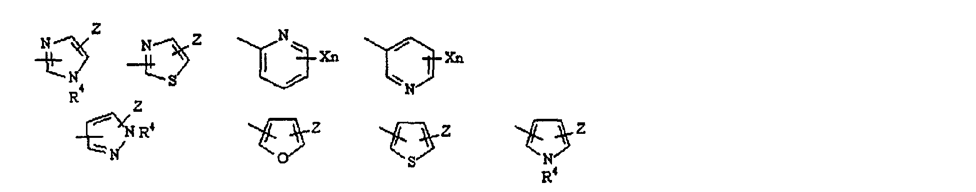 (wherein Z represents a hydrogen atom, a halogen atom, or a lower alkyl group; R⁴ represents a hydrogen atom, a lower alkyl group, or a lower alkyl group substituted with a lower alkoxy group which may have a silyl group; X represents a hydrogen atom, a halogen atom, a lower alkyl group, or a lower alkoxy group; and n is from 0 to 3) in the general formula (2).

14. A hydroxime derivative according to claim 12, wherein Het B is a group represented by any one of the following six formulas: 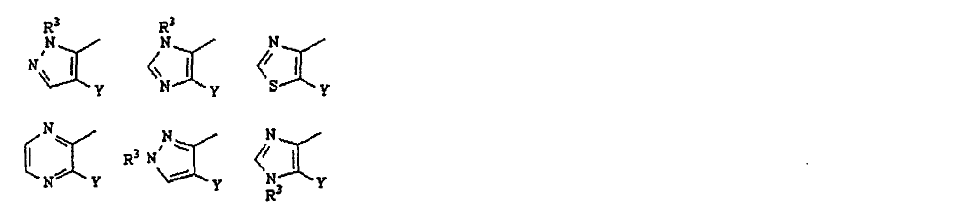 (wherein Y represents a hydrogen atom or a lower alkyl group; and R³ represents a hydrogen atom, a lower alkyl group, or a lower alkyl group substituted with a lower alkoxy group which may have a silyl group); and Het C is a group represented by any one of the following six formulas: 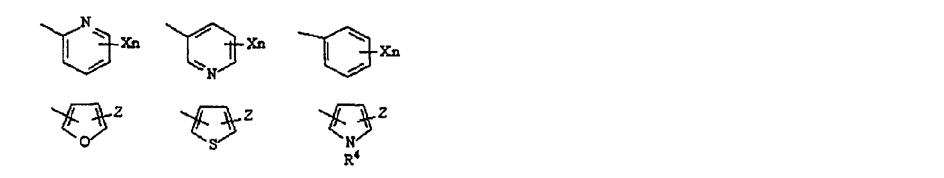 (wherein R⁴ represents a hydrogen atom or a lower alkyl group; X represents a hydrogen atom, a halogen atom, a lower alkyl group, a lower alkyl group substituted with a halogen atom, a lower alkoxy group, or a lower alkoxy group substituted with a halogen atom; Z represents a hydrogen atom, a halogen atom, or a lower alkyl group; and n is from 0 to 3) in the general formula (2).

15. A hydroxime derivative according to claim 13, wherein Het C is a group represented by any one of the ring structures of the following three formulas: 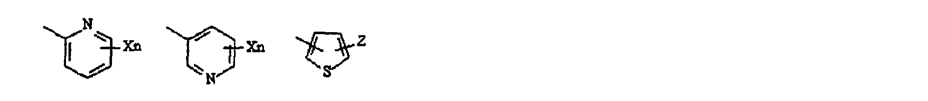 (wherein Z represents a hydrogen atom, a halogen atom, or a lower alkyl group; X represents a hydrogen atom, a halogen atom, a lower alkyl group, or a lower alkoxy group; and n is 0 or 1) in the general formula (2).

16. A hydroxime derivative according to claim 13, wherein Het C is a group represented by the following formula: 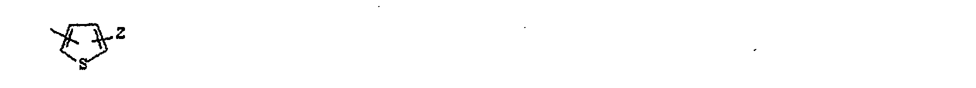 (wherein Z represents a hydrogen atom) in the general formula (2).

17. A hydroxime derivative according to claim 13, wherein Het C is a group represented by the following formula: 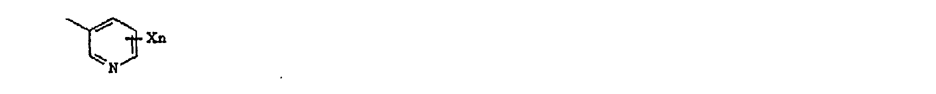 (wherein X represents a hydrogen atom or a halogen atom; and n is 0 or 1) in the general formula (2).

18. A hydroxime derivative according to claim 14, wherein Het C is a group represented by the following formula: 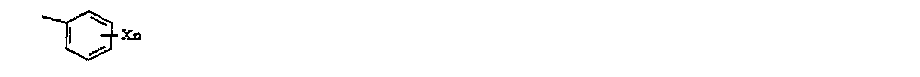 (wherein X represents a hydrogen atom, a halogen atom, a lower alkyl group, a lower alkyl group substituted with a halogen atom, a lower alkoxy group, or a lower alkoxy group substituted with a halogen atom; and n is from 0 to 3) in the general formula (2).

19. A hydroxime derivative according to claim 14, wherein Het B is a group represented by any one of the following three formulas: 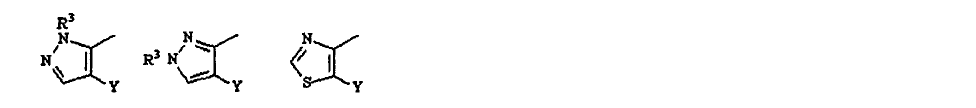 (wherein Y represents a hydrogen atom or a lower alkyl group; and R³ represents a hydrogen atom, a lower alkyl group, or a lower alkyl group substituted with a lower alkoxy group which may have a silyl group); and Het C is a group represented by the following formula: 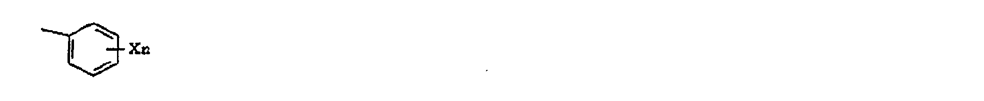 (wherein X represents a hydrogen atom, a halogen atom, a lower alkyl group, or a lower alkyl group substituted with a halogen; and n is 0 or 1) in the general formula (2).

20. A method of preparing a hydroxime derivative represented by the following general formula (2): 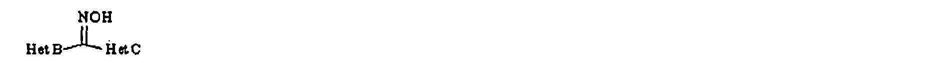 (wherein Het B and Het C are as defined in the general formula (3)), which comprises reacting a ketone compound represented by the following general formula (3): 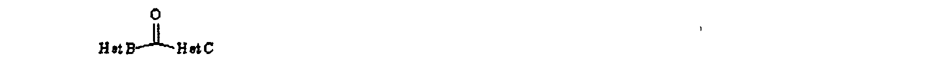 wherein Het B represents a group represented by any one of the following nine formulas: 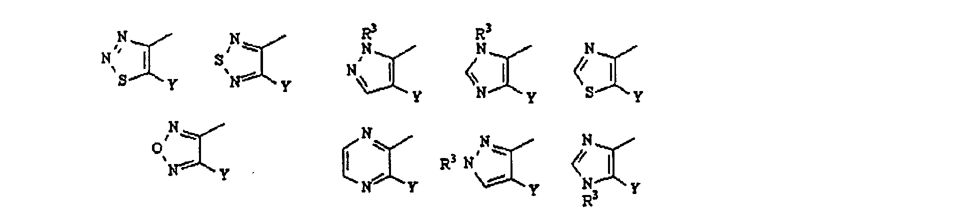 (wherein Y represents a hydrogen atom or a lower alkyl group; and R³ represents a hydrogen atom, a lower alkyl group, or a lower alkyl group substituted with a lower alkoxy group which may have a silyl group); and Het C represents a group represented by any one of the following nine formulas: 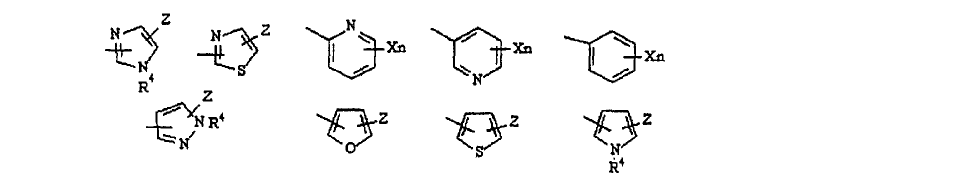 (wherein R⁴ represents a hydrogen atom, a lower alkyl group, or a lower alkyl group substituted with a lower alkoxy group which may have a silyl group; X represents a hydrogen atom, a halogen atom, a lower alkyl group, a lower alkyl group substituted with a halogen atom, a lower alkoxy group, or a lower alkoxy group substituted with a halogen atom; Z represents a hydrogen atom, a halogen atom, or a lower alkyl group; and n is from 0 to 3); with the exception of the case of the following compounds: 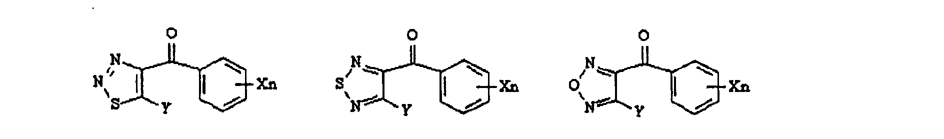 (wherein X represents a hydrogen atom, a halogen atom, a lower alkyl group, a lower alkyl group substituted with a halogen atom, a lower alkoxy group, a lower alkoxy group substituted with a halogen atom, or a cyano group; Y represents a hydrogen atom or a lower alkyl group; and n is 0 from 3), the following compound: 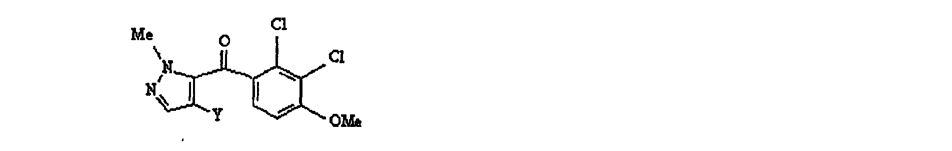 and the following compounds: 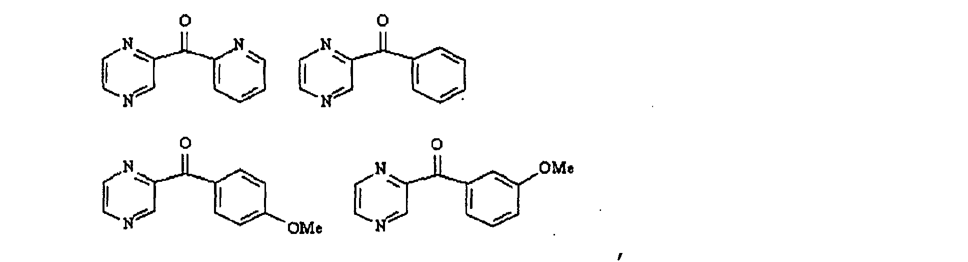 with an oximating agent.

21. A method of preparing a hydroxime derivative according to claim 20, wherein Het B is a group represented by any one of the ring structures of the following three formulas: 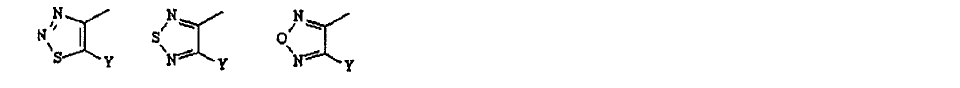 (wherein Y represents a hydrogen atom or a lower alkyl group); and Het C is a group represented by any one of the ring structures of the following eight formulas: 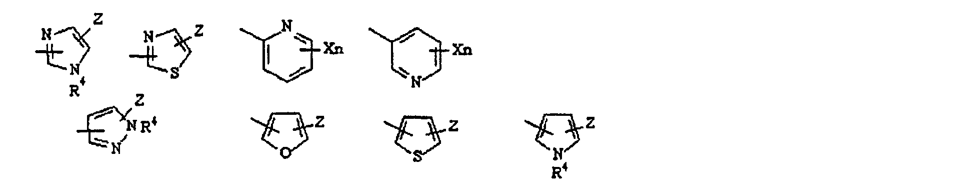 (wherein Z represents a hydrogen atom, a halogen atom, or a lower alkyl group; R⁴ represents a hydrogen atom, a lower alkyl group, or a lower alkyl group substituted with a lower alkoxy group which may have a silyl group; X represents a hydrogen atom, a halogen atom, a lower alkyl group, or a lower alkoxy group; and n is from 0 to 3) in the general formula (3).

22. A method of preparing a hydroxime derivative according to claim 20, wherein Het B is a group represented by any one of the following six formulas: 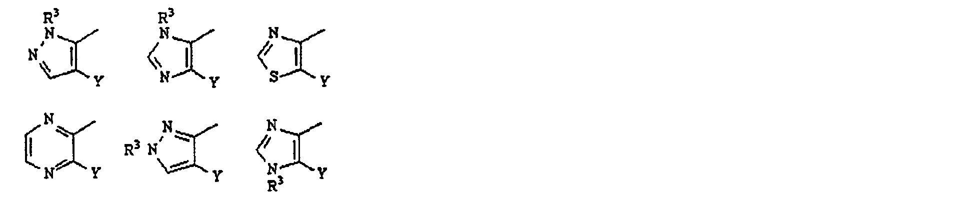 (wherein Y represents a hydrogen atom or a lower alkyl group; and R³ represents a hydrogen atom, a lower alkyl group, or a lower alkyl group substituted with a lower alkoxy group which may have a silyl group); and Het C is a group represented by any one the following six formulas: 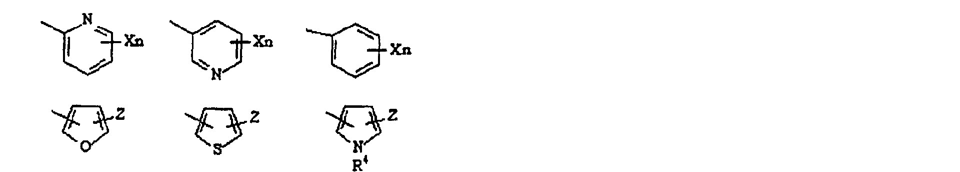 (wherein R⁴ represents a hydrogen atom or a lower alkyl group; X represents a hydrogen atom, a halogen atom, a lower alkyl group, a lower alkyl group substituted with a halogen atom, a lower alkoxy group, or a lower alkoxy group substituted with a halogen atom; Z represents a hydrogen atom, a halogen atom, or a lower alkyl group; and n is from 0 to 3) in the general formula (3).

23. A method of preparing a hydroxime derivative according to claim 21, wherein Het C is a group represented by any one of the ring structures of the following three formulas: 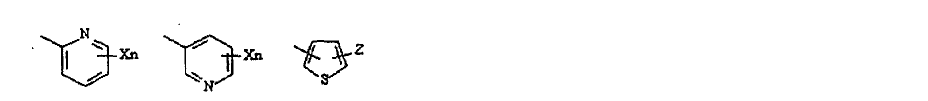 (wherein Z represents a hydrogen atom, a halogen atom, or a lower alkyl group; X represents a hydrogen atom, a halogen atom, a lower alkyl group, or a lower alkoxy group; and n is 0 or 1) in the general formula (3).

24. A method of preparing a hydroxime derivative according to claim 21, wherein Het C is a group represented by the following formula: 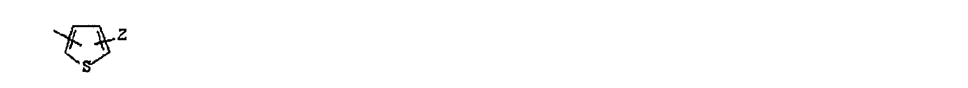 (wherein Z represents a hydrogen atom) in the general formula (3).

25. A method of preparing a hydroxime derivative according to claim 21, wherein Het C is a group represented by the following formula: 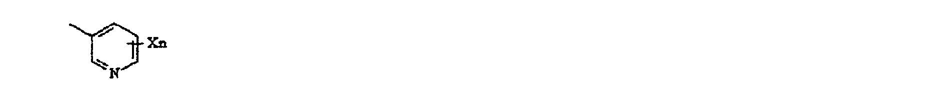 (wherein X represents a hydrogen atom or a halogen atom; and n is 0 or 1) in the general formula (3).

26. A method of preparing a hydroxime derivative according to claim 22, wherein Het C is a group represented by the following formula: 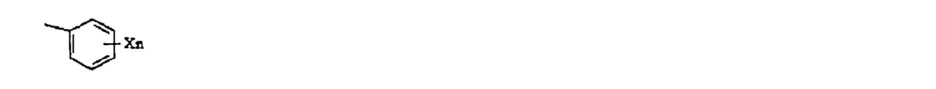 (wherein X represents a hydrogen atom, a halogen atom, a lower alkyl group, a lower alkyl group substituted with a halogen atom, a lower alkoxy group, or a lower alkoxy group substituted with a halogen atom; and n is from 0 to 3) in the general formula (3).

27. A method of preparing a hydroxime derivative according to claim 22, wherein Het B is a group represented by any one of the following three formulas: 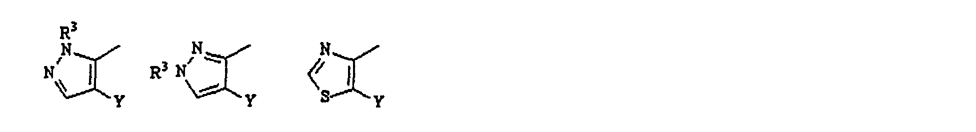 (wherein Y represents a hydrogen atom or a lower alkyl group; and R³ represents a hydrogen atom, a lower alkyl group, or a lower alkyl group substituted with a lower alkoxy group which may have a silyl group); and Het C is a group represented by the following formula: 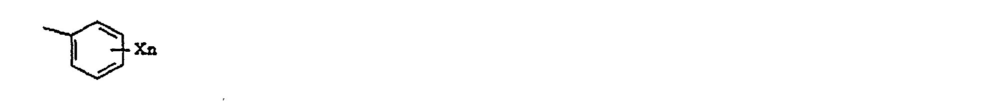 (wherein X represents a hydrogen atom, a halogen atom, a lower alkyl group, or a lower alkyl group substituted with a halogen atom; and n is 0 or 1) in the general formula (3).

28. A method of preparing an oxime derivative represented by the following general formula (1): 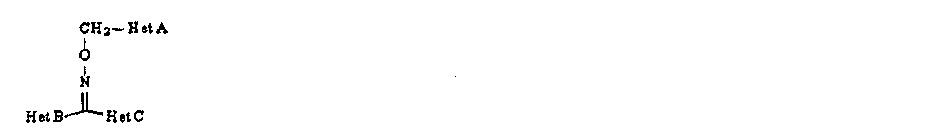 (wherein Het A is as defined in the general formula (4); and Het B and Het C are as defined in the general formula (2)), which comprises reacting a hydroxime compound represented by the following general formula (2): 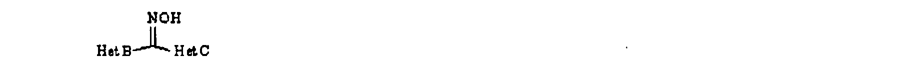 wherein Het B represents a group represented by any one of the following nine formulas: 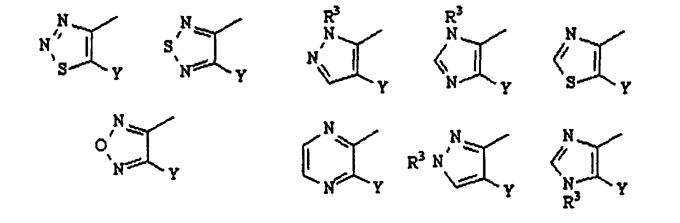 (wherein Y represents a hydrogen atom or a lower alkyl group; and R³ represents a hydrogen atom, a lower alkyl group, or a lower alkyl group substituted with a lower alkoxy group which may have a silyl group); and Het C represents a group represented by any one of the following nine formulas: 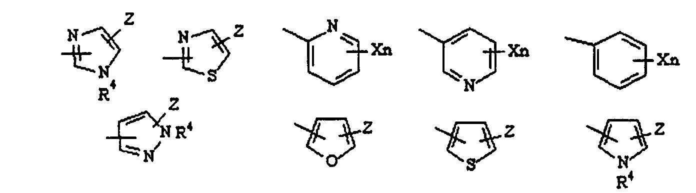 (wherein R⁴ represents a hydrogen atom, a lower alkyl group, or a lower alkyl group substituted with a lower alkoxy group which may have a silyl group; X represents a hydrogen atom, a halogen atom, a lower alkyl group, a lower alkyl group substituted with a halogen atom, a lower alkoxy group, a lower alkoxy group substituted with a halogen atom, or a cyano group; Z represents a hydrogen atom, a halogen atom, or a lower alkyl group; and n is from 0 to 3); with the exception of the case where Het A is a group represented by any one of the following two formulas: 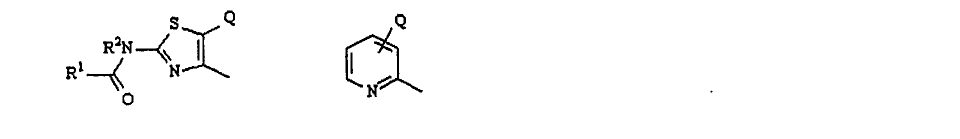 (wherein Q represents a hydrogen atom, a halogen atom, or a lower alkyl group; R¹ represents a hydrogen atom, a lower alkyl group, a lower alkyl group substituted with a halogen atom, a cycloalkyl group, a lower alkenyl group, a lower alkynyl group, an aralkyl group, an aryl group, or an aryl group substituted with one or more substituents selected from the group consisting of halogen atom, lower alkyl group, and lower alkoxy group; and R² represents a hydrogen atom or a lower alkyl group); Het B is a group represented by any one of the ring structures of the following three formulas: 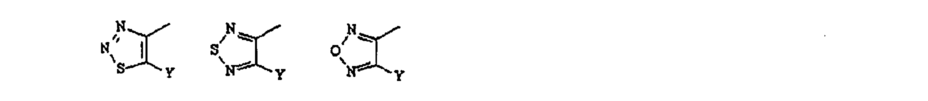 (wherein Y represents a hydrogen atom or a lower alkyl group); and Het C is a group represented by the following formula: 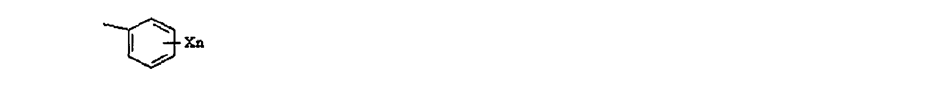 (wherein X represents a hydrogen atom, a halogen atom, a lower alkyl group substituted with a halogen atom, a lower alkoxy group, a lower alkoxy group substituted with a halogen atom, or a cyano group; and n is from 0 to 3),
with an alkylating agent represented by the following general formula (4):
Het A-CH₂L
wherein Het A represents a group represented by any one of the following three formulas: 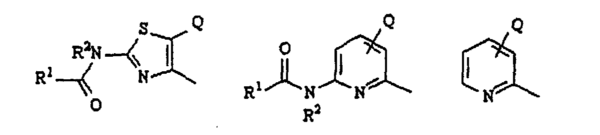 (wherein Q represents a hydrogen atom, a halogen atom, or a lower alkyl group; R¹ represents a hydrogen atom, a lower alkyl group, a lower alkyl group substituted with a halogen atom, a cycloalkyl group, a lower alkenyl group, a lower alkynyl group, an aralkyl group, an aryl group, or an aryl group substituted with one or more substituents selected from the group consisting of halogen atom, lower alkyl group, and lower alkoxy group; and R² represents a hydrogen atom or a lower alkyl group); and L represents a leaving group,
in the presence of a base.

29. A method of preparing an oxime derivative according to claim 28, wherein Het B is a group represented by any one of the ring structures of the following three formulas: 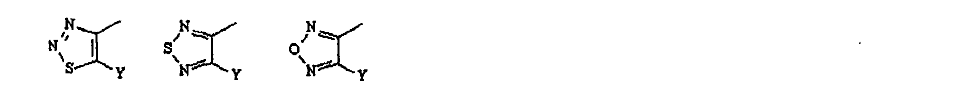 (wherein Y represents a hydrogen atom or a lower alkyl group); and Het C is a group represented by any one of the ring structures of the following eight formulas: 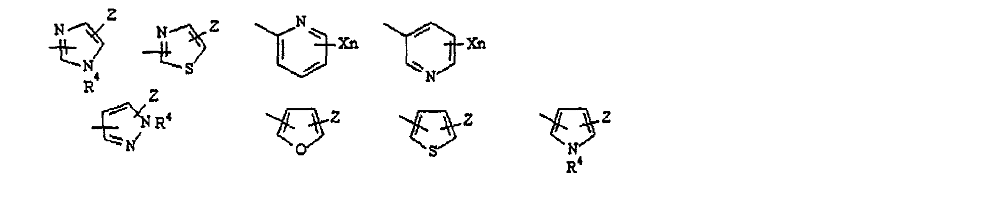 (wherein Z represents a hydrogen atom, a halogen atom, or a lower alkyl group; R⁴ represents a hydrogen atom, a lower alkyl group, or a lower alkyl group substituted with a lower alkoxy group which may have a silyl group; X represents a hydrogen atom, a halogen atom, a lower alkyl group, or a lower alkoxy group; and n is from 0 to 3) in the general formula (2).

30. A method of preparing an oxime derivative according to claim 28, wherein Het B is a group represented by any one of the ring structures of the following three formulas: 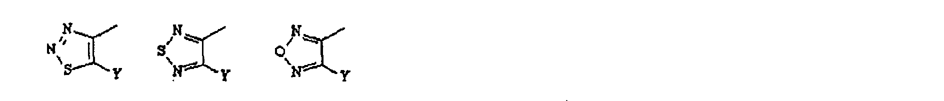 (wherein Y represents a hydrogen atom or a lower alkyl group); Het C is a group represented by any one of the ring structures of the following formula: 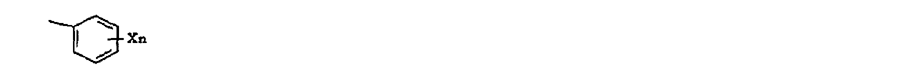 (wherein X represents a hydrogen atom, a halogen atom, a lower alkyl group, a lower alkyl group substituted with a halogen atom, a lower alkoxy group, a lower alkoxy group substituted with a halogen atom, or a cyano group; and n is from 0 to 3) in the general formula (2); and Het A is a group represented by the following formula: 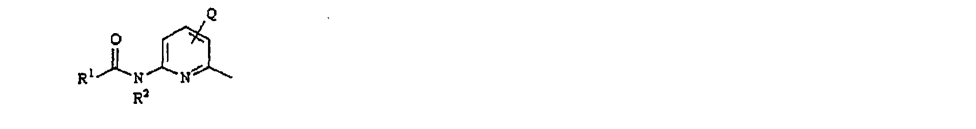 (wherein Q represents a hydrogen atom, a halogen atom, or a lower alkyl group; R¹ represents a hydrogen atom, a lower alkyl group, a lower alkyl group substituted with a halogen atom, a cycloalkyl group, a lower alkenyl group, a lower alkynyl group, an aralkyl group, an aryl group, or an aryl group substituted with one or more substituents selected from the group consisting of halogen atom, lower alkyl group, and lower alkoxy group; and R² represents a hydrogen atom or a lower alkyl group) in the general formula (4).

31. A method of preparing an oxime derivative according to claim 28, wherein Het B is a group represented by any one of the following six formulas: 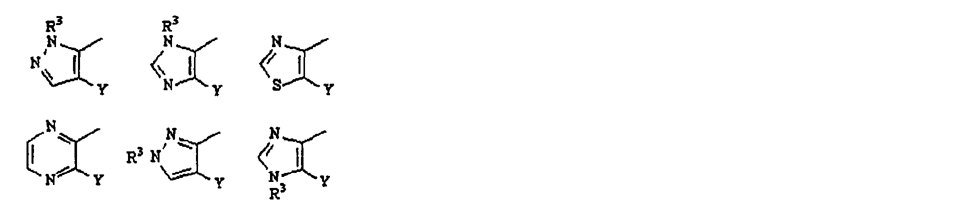 (wherein Y represents a hydrogen atom or a lower alkyl group; and R³ represents a hydrogen atom, a lower alkyl group, or a lower alkyl group substituted with a lower alkoxy group which may have a silyl group); and Het C is a group represented by any one of the following six formulas: 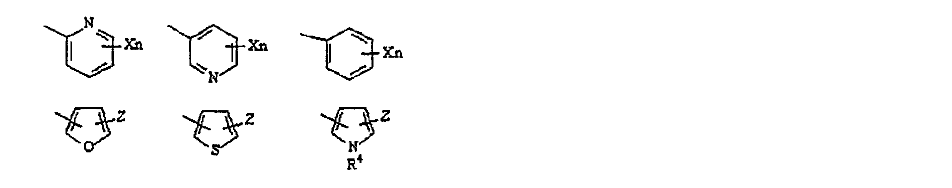 (wherein R⁴ represents a hydrogen atom or a lower alkyl group; X represents a hydrogen atom, a halogen atom, a lower alkyl group, a lower alkyl group substituted with a halogen atom, a lower alkoxy group, or a lower alkoxy group substituted with a halogen atom; Z represents a hydrogen atom, a halogen atom, or a lower alkyl group; and n is from 0 to 3) in the general formula (2).

32. A method of preparing an oxime derivative according to claim 29, wherein Het C is a group represented by any one of the ring structures of the following three formulas: 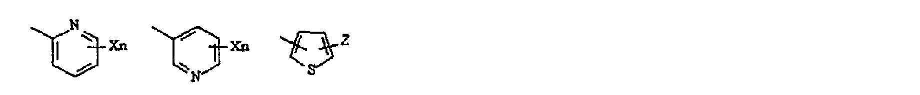 (wherein Z represents a hydrogen atom, a halogen atom, or a lower alkyl group; X represents a hydrogen atom, a halogen atom, a lower alkyl group, or a lower alkoxy group; and n is 0 or 1) in the general formula (2).

33. A method of preparing an oxime derivative according to claim 29, wherein Het C is a group represented by the following formula: 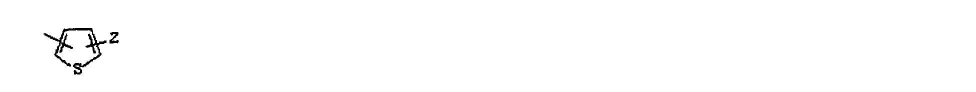 (wherein Z represents a hydrogen atom) in the general formula (2), and Het A is a group represented by the following formula: 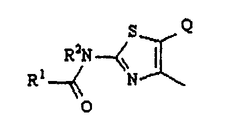 (wherein Q represents a hydrogen atom, a halogen atom, or a lower alkyl group; R¹ represents a lower alkyl group or a cycloalkyl group; and R² represents a hydrogen atom) in the general formula (4).

34. A method of preparing an oxime derivative according to claim 29, wherein Het C is a group represented by the following formula: 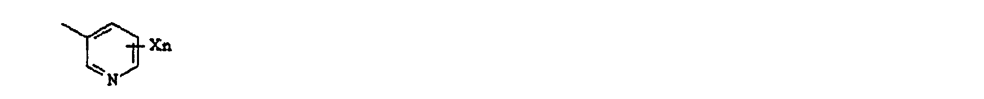 (wherein X represents a hydrogen atom or a halogen atom; and n is 0 or 1) in the general formula (2); and Het A is a group represented by the following formula: 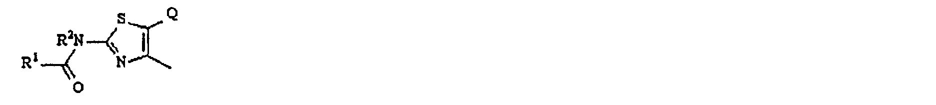 (wherein Q represents a hydrogen atom, a halogen atom, or a lower alkyl group; R¹ represents a lower alkyl group or a cycloalkyl group; and R² represents a hydrogen atom) in the general formula (4).

35. A method of preparing an oxime derivative according to claim 29, wherein Het C is a group represented by any one of the following three formulas: 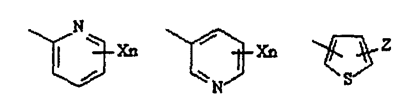 (wherein X represents a hydrogen atom or a halogen atom; Z represents a hydrogen atom; and n is 0 or 1) in the general formula (2); and Het A is a group represented by the following formula: 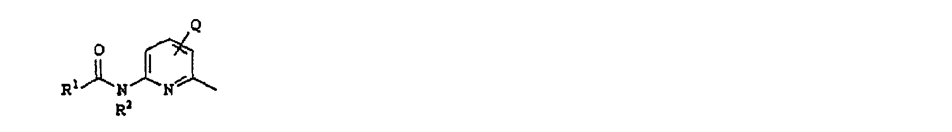 (wherein Q represents a hydrogen atom or a lower alkyl group; R¹ represents a lower alkyl group or a cycloalkyl group; and R² represents a hydrogen atom) in the general formula (4).

36. A method of preparing an oxime derivative according to claim 30, wherein Het C is a group represented by the following formula: 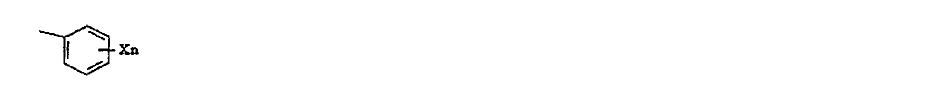 (wherein X represents a hydrogen atom, a halogen atom, a lower alkyl group, a lower alkyl group substituted with a halogen atom, or a cyano group; and n is from 0 to 3) in the general formula (2); and Het A is a group represented by the following formula: 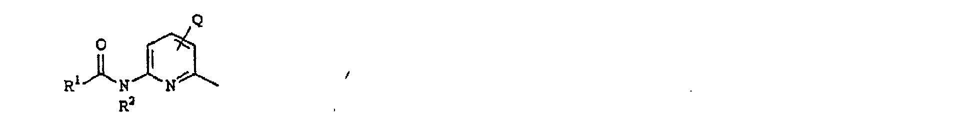 (wherein Q represents a hydrogen atom or a lower alkyl group; R¹ represents a lower alkyl group or a cycloalkyl group; and R² represents a hydrogen atom) in the general formula (4).

37. A method of preparing an oxime derivative according to claim 31, wherein Het C is a group represented by the following formula: 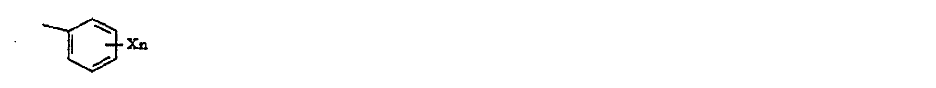 (wherein X represents a hydrogen atom, a halogen atom, a lower alkyl group, a lower alkyl group substituted with a halogen atom, a lower alkoxy group, or a lower alkoxy group substituted with a halogen atom; and n is from 0 to 3) in the general formula (2).

38. A method of preparing an oxime derivative according to claim 31, wherein Het B is a group represented by any one of the following three formulas: 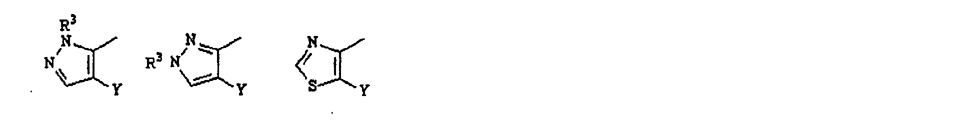 (wherein Y represents'a hydrogen atom or a lower alkyl group; and R³ represents a hydrogen atom, a lower alkyl group, or a lower alkyl group substituted with a lower alkoxy group which may have a silyl group); Het C is a group represented by the following formula: 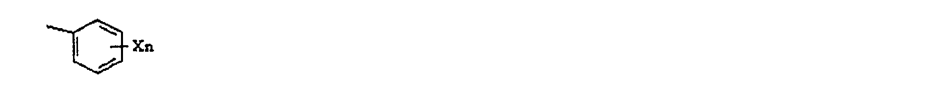 (wherein X represents a hydrogen atom, a halogen atom, a lower alkyl group, or a lower alkyl group substituted with a halogen atom; and n is 0 or 1) in the general formula (2); and Het A is a group represented by the following formula: 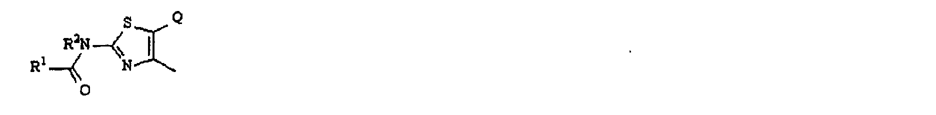 (wherein Q represents a hydrogen atom, a halogen atom, or a lower alkyl group; R¹ represents a lower alkyl group or a cycloalkyl group; and R² represents a hydrogen atom) in the general formula (4).

39. An agricultural chemical comprising an oxime derivative of any one of claims 1 to 11 as an active ingredient.

40. A plant disease controlling agent comprising the oxime derivative of any one of claims 1 to 11 as an active ingredient.

41. A plant disease controlling agent according to claim 40, which is effective against plant diseases caused by filamentous fungi.
